# EUROPEAN PATENT SPECIFICATION

(11) **EP 4 388 017 B1**
(45) Date of publication and mention of the grant of the patent: **07.10.2026**
(21) Application number: 22858012.2
(22) Date of filing: 22.08.2022
(51) Int. Cl.: A61K 40/31, A61K 40/11, A61K 40/42, A61K 40/46, C07K 19/00, A61P 31/12, A61P 35/00, A61P 37/02, C07K 5/10, C07K 14/705, C07K 16/08, C07K 16/10, C07K 16/30, C12N 5/10, C12N 5/0783, C12N 15/62, C12N 15/85, C07K 14/725, C07K 16/102, C07K 16/104

(54) **COMPOSITIONS AND METHODS FOR ANTI-VIRUS CHIMERIC ANTIGEN RECEPTOR**
ZUSAMMENSETZUNGEN UND VERFAHREN FÜR CHIMÄREN ANTIVIRUS-ANTIGENREZEPTOR
COMPOSITIONS ET PROCÉDÉS POUR RÉCEPTEUR ANTIGÉNIQUE CHIMÉRIQUE ANTI-VIRAL

(30) Priority: 20.08.2021 US 202163235647 P
(43) Date of publication of application: 26.06.2024
(60) Divisional application: 26158209.2 / 4 722 239
(73) Proprietor: Rudd, Christopher E., Montreal West, Québec H4X 1K3 (CA)
(72) Inventor: Rudd, Christopher E., Montreal West, Québec H4X 1K3 (CA)
(74) Representative: Mewburn Ellis LLP
(86) International application number: PCT/IB2022/057857
(87) International publication number: WO 2023/021494

(56) References cited:
- WO-A1-2014/031687
- WO-A1-2015/105522
- WO-A1-2015/164594
- WO-A1-2019/020088
- WO-A1-2019/144707
- WO-A1-2020/223445
- M. HUDECEK ET AL: "The Nonsignaling Extracellular Spacer Domain of Chimeric Antigen Receptors Is Decisive for In Vivo Antitumor Activity", CANCER IMMUNOLOGY RESEARCH, vol. 3, no. 2, 11 September 2014 (2014-09-11), US, pages 125 - 135, XP055177300, ISSN: 2326-6066, DOI: 10.1158/2326-6066.CIR-14-0127
- FINNEY HELENE M ET AL: "Activation of resting human primary T cells with chimeric receptors: costimulation from CD28, inducible costimulator, CD134, and CD137 in series with signals from the TCR zeta chain", THE JOURNAL OF IMMUNOLOGY, AMERICAN ASSOCIATION OF IMMUNOLOGISTS, vol. 172, no. 1, 1 January 2004 (2004-01-01), pages 104 - 113, XP002605937, ISSN: 0022-1767
- MALDINI ET AL.: "CAR T cells for infection, autoimmunity and allotransplantation", NATURE REVIEWS: IMMUNOLOGY, vol. 18, 25 July 2018 (2018-07-25), pages 605 - 616, XP037134951, [retrieved on 20220920], DOI: https://doi.org/10.1038/s41577-018-0042-2
- MA MINH, BADETI SAIADITYA, GENG KE, LIU DONGFANG: "Efficacy of Targeting SARS-CoV-2 by CAR-NK Cells", BIORXIV : THE PREPRINT SERVER FOR BIOLOGY, COLD SPRING HARBOR LABORATORY, 12 August 2020 (2020-08-12), XP055812652, Retrieved from the Internet <URL:https://www.biorxiv.org/content/10.1101/2020.08.11.247320v1.full.pdf> [retrieved on 20210610], DOI: 10.1101/2020.08.11.247320
- FU WENYAN, LEI CHANGHAI, MA ZETONG, QIAN KEWEN, LI TIAN, ZHAO JIAN, HU SHI: "CAR Macrophages for SARS-CoV-2 Immunotherapy", FRONTIERS IN IMMUNOLOGY, vol. 12, XP093038677, DOI: 10.3389/fimmu.2021.669103

## Description

### BACKGROUND

SARS-CoV-2 (severe acute respiratory syndrome coronavirus 2) is a positive-sense single-stranded RNA virus and the cause of Coronavirus Disease 2019 (COVID-19). The severity of COVID-19 ranges from asymptomatic to mild self-limiting disease to severe and acute respiratory distress syndrome, neurological symptoms and death (Amanat and Krammer, 2020; Tay et al., 2020; ter Meulen et al., 2006).SARS-CoV-2 has an incubation period of 4-7 days before symptom onset, followed in some cases by the progression to a severe disease (Peng et al., 2020). Current therapies include the use of vaccines, convalescent plasma (Bloch et al., 2020) and re-purposed anti-viral treatments (Sarzi-Puttini et al., 2020) such as umifenovir (Xu et al., 2020) and remdesivir/ribavirin (Jean et al., 2020). Other approaches include the use of anti-IL-7, anti-IL-6 receptor antibodies and corticosteroids to dampen the inflammatory immune response (Russell et al., 2020; Tharmarajah et al., 2021), and neutralizing monoclonal antibodies (mAbs) that bind to viral proteins that are critical for attachment and entry to mammalian cells (Altmann and Boyton, 2020).

SARS-CoV-2 contains glycosylated spike (S) protein facilitates viral attachment and cell entry and plays a critical role in the elicitation of the host immune response (Grifoni et al., 2020; Meckiff et al., 2020; Premkumar et al., 2020; Varchetta et al., 2020; Wu et al., 2020). It binds to the human angiotensin-converting enzyme 2 (ACE2) (Hoffmann et al., 2020b; Letko et al., 2020) which is the target of neutralizing antibodies (Cao et al., 2020; Shi et al., 2020). The S protein forms a trimer where each monomer has two subunits (S1 and S2) separated by a cleavage site that is recognized by host cell proteases (Hoffmann et al., 2020a). The S1 subunit is composed of the signal peptide (SP), N terminal domain (NTD), and receptor-binding domain (RBD), while the S2 subunit mediates membrane fusion. The RBD regions is comprised of a 5-stranded anti-parallel beta-sheet where between the β4-7 strands, is an extended insertion of short β5-6 strands. The extension carries the receptor-binding motif (RBM) that binds to the ACE2 receptor for entry into respiratory and digestive epithelial cells (Lan et al., 2020).The three RBDs on the S protein head show conformational variability. In a closed conformation, the three RBDs are flat with the RBM occluded, while in an open conformation, one or more RBDs lift to expose the RBM.

Severe COVID-19 patients show marked lymphopenia (Braun et al., 2020; Cizmecioglu et al., 2020), increased pro-inflammatory cytokines and chemokines (Song et al., 2020) and high neutrophil levels in peripheral blood (Song et al., 2020; Varchetta et al., 2020). Several studies have delineated aspects of cell immunity that determines disease outcomes (Braun et al., 2020; Campbell et al., 2020; Cheng et al., 2020; Weiskopf et al., 2020). Mild cases are associated with antibody response, CD4 and CD8 T cell responses, while severe cases involve the loss of T cells and reduced antibody responses (Rydyznski Moderbacher et al., 2020; Weiskopf et al., 2020). To date, the vaccines have been approved, although the long-term efficacy of antibodies induced by these vaccines is still under evaluation (Amanat and Krammer, 2020). Other approaches have included adoptive cell transfer of immune and stem cells (Shetty, 2020), serum from convalescent or recovered patients and the use of hexapeptides corresponding to the ACE2-interacting domain of SARS-CoV-2 (AIDS) to inhibit S1 subunit binding to the ACE2 receptor(Paidi et al., 2021).

However, a need remains in the art to treat an infection by SARS-CoV-2 or other viruses effectively and efficiently. This disclosure satisfies this need and provides related advantages as well.

WO2019020088A1 and WO2019144707A1 each disclose a CAR comprising a long IgG4 Fc CH2CH3 hinge.

### SUMMARY

The invention is defined as set out in the appended claims. In one aspect, provided is a polypeptide comprising, or consisting essentially of, or yet further consisting of: (a) an antibody or a fragment thereof that specifically binds a tumor antigen, (b) a hinge region, that comprises 120 or more amino acid residues, (c) one or more transmembrane domains, (d) one or more co-stimulatory domains, and (e) an intracellular signaling domain, wherein the hinge region comprises SEQ ID NO: 37. In some embodiments, the polypeptide comprises, or consists essentially of, or yet further consists of a chimeric antigen receptor (CAR).

The outlined novel combinations of antibody binding regions and hinge regions may be coupled to any variety of transmembrane regions or intracellular signaling domains. Non-limiting examples of cytoplasmic domains include any combination of the TCR-zeta or CD3-gamma, delta or epsilon or CD28, CTLA-4 or 4-1BB cytoplasmic signaling sequences. In one aspect, provided herein is a CAR comprising the antibody or antigen binding region thereof that binds a TAA, such as for example CD19, a hinge region from LAIR1 (144aa, SEQ ID NO: 37) and one or more co-stimulatory domains, a transmembrane domain and an intracellular signaling domain.

In a further aspect, provided is a polynucleotide encoding a polypeptide as disclosed herein, or a polynucleotide complementary thereto. In yet a further aspect, provided is a vector comprising, or consisting essentially of, or yet further consisting of a polynucleotide as disclosed herein. Additionally provided is a cell comprising one or more of: a polypeptide as disclosed herein, a polynucleotide as disclosed herein, or a vector as disclosed herein. In some embodiments, the cell is suitable for replicating a polynucleotide as disclosed herein, or a vector as disclosed herein, or both. Additionally or alternatively, the cell is suitable for expressing a polypeptide as disclosed herein. In further embodiments, the cell is a stem cell or an immune cell, optionally selected from a T cell, a B cell, a NK cell, a NKT cell, a dendritic cell, a myeloid cell, a monocyte, or a macrophage.

In one aspect, provided is a T cell comprising: (i) granzyme B, or a polynucleotide encoding the granzyme B, or both; (ii) perforin, or a polynucleotide encoding the perforin, or both; and (iii) one or more of: a polypeptide as disclosed herein, a polynucleotide as disclosed herein, or a vector as disclosed herein. In some embodiments, the T cell further comprises (iv) CD69, or a polynucleotide encoding the CD69, or both; or (v) IFN-y, or a polynucleotide encoding the IFN-y, or both; or both , or (vi) tumor necrosis factor-alpha (TNF-alpha), or a polynucleotide encoding the TNF-alpha, or both; or (vii) Fas-ligand (FasL), or a polynucleotide encoding the FasL, or both; or any combination of i-vii.

In a further aspect, provided is a histocompatible a T cell comprising one or more of: a polypeptide as disclosed herein, a polynucleotide as disclosed herein, or a vector as disclosed herein. In some embodiments, the T cell lacks a functional endogenous T cell receptor or a human leukocyte antigen (HLA) molecule or both.

Additionally provided is a cell population comprising, or consisting essentially of, or yet further consisting of a cell as disclosed herein. The population can be substantially homogeneous.

In one aspect, provided is a composition comprising, or consisting essentially of, or yet further consisting of a carrier and one or more of: a polypeptide as disclosed herein, a polynucleotide as disclosed herein, a vector as disclosed herein, a cell as disclosed herein, or a cell population as disclosed herein. In some embodiments, the carrier is a pharmaceutically acceptable carrier and optionally a preservative. Additionally or alternative, the composition further comprises a therapeutic agent or a prophylactic agent or both.

In another aspect, provided is an isolated complex comprising, or consisting essentially of, or yet further consisting of a cell as disclosed herein and the TAA.

In yet another aspect, provided is a method for producing a cell as disclosed herein. The method comprises, or consists essentially of, or yet further consists of introducing a polynucleotide as disclosed herein, a vector comprising a polynucleotide as disclosed herein, or both into a host cell. The cell can be a prokaryotic or eukaryotic cell. In some embodiments, the method further comprises culturing and expanding the cell to a population of cells or the cell introduced with the polynucleotide encoding the CAR or the vector comprising the polynucleotide encoding the CAR or both.

In another aspect, provided is a cell as disclosed herein for use in a method of treating a subject having or suspected of having cancer. The method comprises, or consists essentially of, or yet further consists of administering to the subject a cell as disclosed herein recognizing and binding to a TAA such as, e.g. CD19. The cell can be a stem cell or immune cell and can be autologous or allogeneic to the subject being treated. The cancer can be primary or metastatic. In some embodiments, the method further comprises treating the subject with a further anticancer therapy and/or immune enhancing combined therapy.

In a further aspect not encompassed by the claims, provided is a kit comprising, or consisting essentially of, or yet further consisting of instructions for use and one or more of: a polypeptide as disclosed herein, a polynucleotide as disclosed herein, a vector as disclosed herein, a cell as disclosed herein, a cell population as disclosed herein, or a composition as disclosed herein.

### BRIEF DESCRIPTION OF THE FIGURES

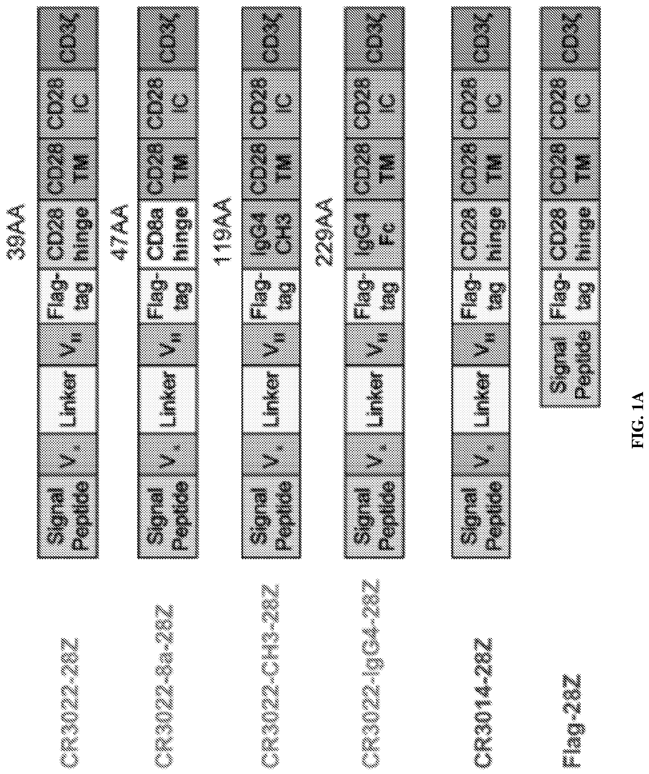

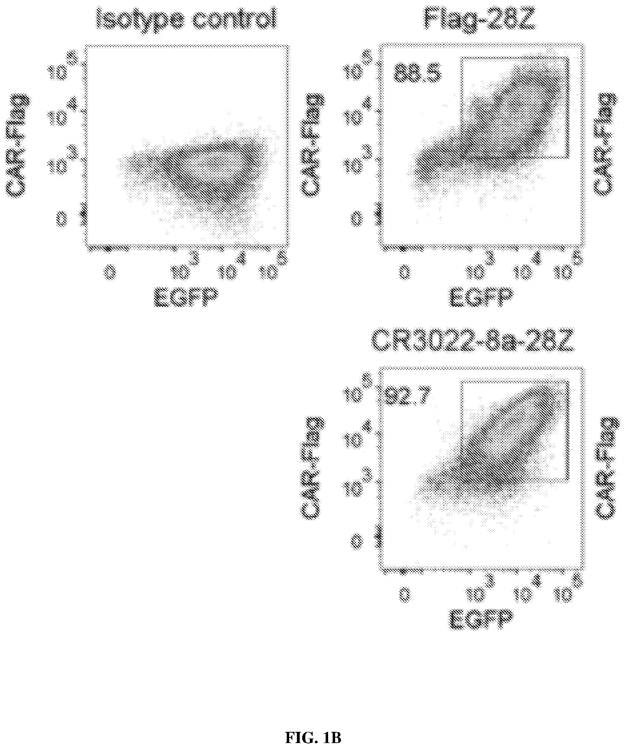

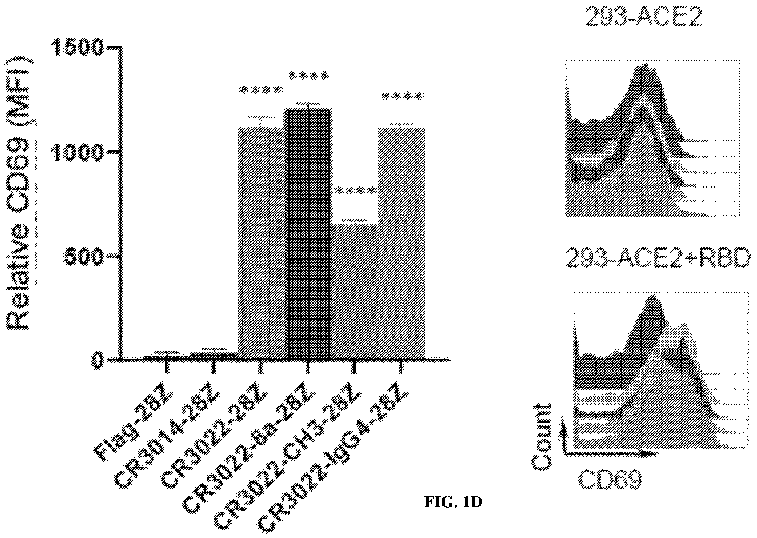

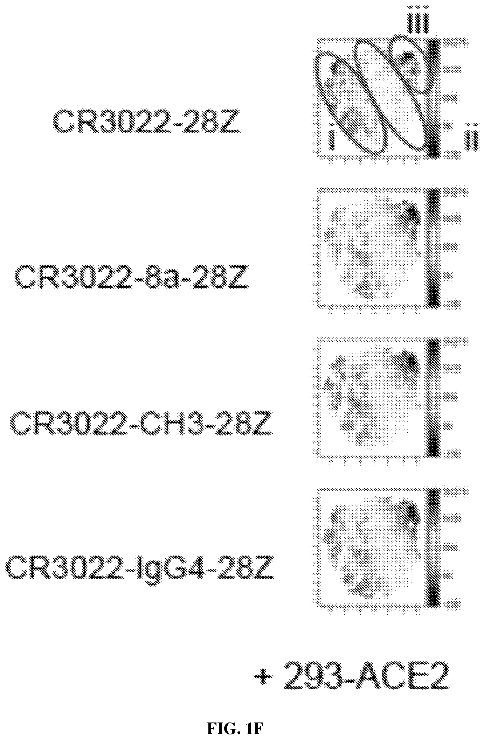

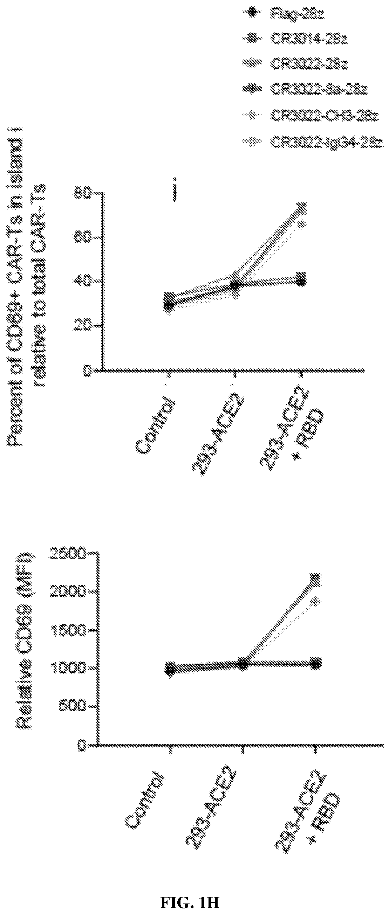

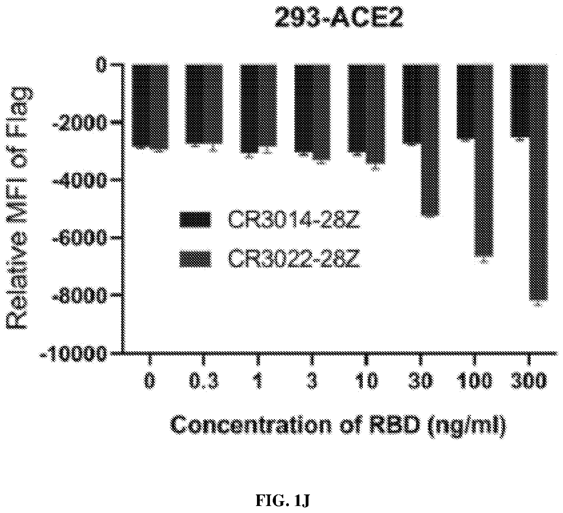

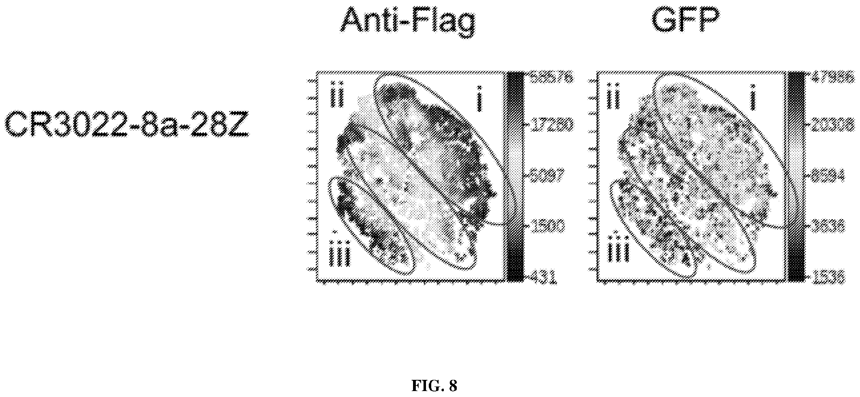

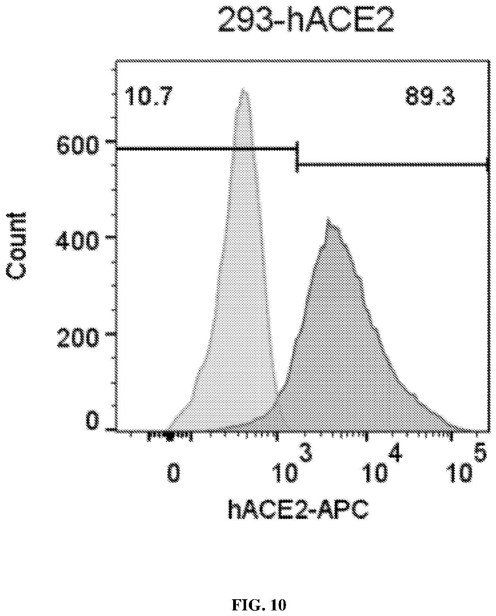

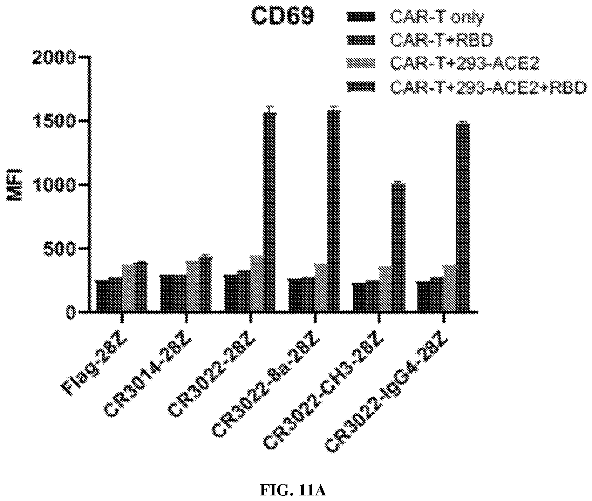

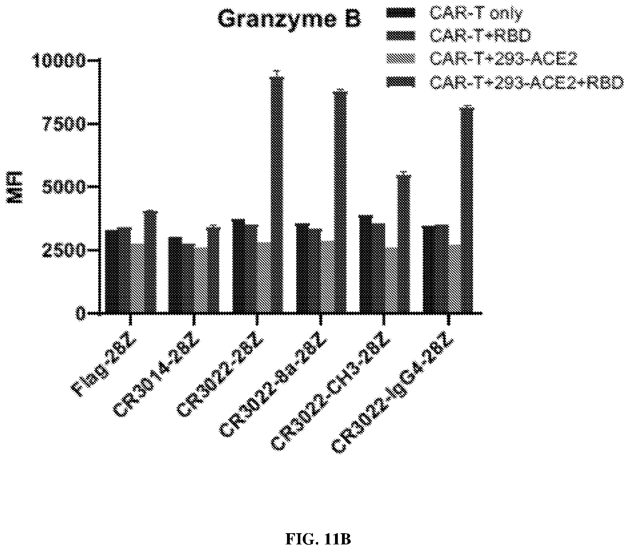

**FIGS. 1A - 1J****:** Design of anti-SARS-CoV-2 S1 CAR-Ts and effect on CD69 activation marker expression. **(****FIG. 1A****)** Design of SARS-CoV-2 S1 CAR-Ts. The CR3022scFv region was coupled to a Flag-Tag followed by different hinge regions and then the CD28 transmembrane (TM) domain and intracellular domain and a CD3zeta intracellular domain. The hinge regions included a39 amino acid CD28hinge (CR3022-28Z), a 47 aa CD8alpha chain (CR3022-8a-28Z), a 119aa CH3 region hinge (CR3022-CH3-28Z) or a 229aa IgG4 hinge (CR3022-IgG4-28Z). As a control, a CAR lacking a scFv region (Flag-28Z) and another with the scFv region of monoclonal antibody CR3014 (CR3014-28Z). **(****FIGS. 1B-1C****)** Flow cytometric profiles of the CAR construct expression in T-cells. CAR expression was detected by staining the Flag-tag and the EGFP was used for cell sorting (n=5). (also see **FIGS. 8-9E****). (****FIG. 1D****)** Expression of surface activation antigen CD69 on CAR-Ts. Left panel: Histogram showing that CD69 expression on different CAR-Ts (Flag-28Z, CR3014-28Z, CR3022-28Z, CR3022-8a-28Z, CR3022-CH3-28Z, and CR3022-IgG4-*28Z). Right profile:* FACS profile showing CD69 expression on the different CARs after incubation with 293-ACE2 or 293-ACE2-RBD cells for 20 hours. Data are shown as mean ± SD (N=3). **** represents *p*< 0.0001(compared with Flag-28Z by one-way ANOVA, n=5). (Also see **FIG. 10****). (FIG. E)** Increased CD69 expression on CR3022-28Z CAR-T in response to 293-ACE2 cells pre-incubated with different concentrations of the RBD peptide. CAR-T-cells were co-cultured for 20 hours with 293-ACE2 cells pre-incubated with various concentrations of the RBD peptide for 1h prior to co-culture. Data are shown as mean ± SD (N=3).* represents *p<* 0.05. *** represents *p<* 0.001. **** represents p<0.0001 (two-tailed Student's t-test, n=2). **(****FIGS. 1F** **and** **1H****) viSNE profiles of CAR and CD69 expression on CAR-Ts.** Left panel: Anti-Flag staining shows the surface expression of the Flag-tagged CARs in islands i, ii and iii after 20 hours of incubation with 293-ACE2 and293-ACE2-RBD cells, while anti-CD69 staining shows the expression of CD69 in CAR-Ts (CR3022-28Z, CR3022-8a-28Z, CR3022-CH3-28Z or CR3022-IgG4-28Z CAR-Ts)(n=3). Right panels: Histograms showing changes in the percent representation of CAR-Ts in island i relative to total CAR-Ts (upper right panel) and in the relative mean fluorescent intensity (MFI) values in island i (lower right panel) (also see **FIGS. 11A-11B). (FIG. 11****) Induction of S6 phosphorylation in response to 293-ACE2-RBD cells.** CR3022-8a-28Z CAR-T cells were incubated with 293-ACE2 or 293-ACE2-RBD cells for 20min prior to the detection of S6 phosphorylation by flow cytometry. In parallel, CR3022-8a-28Z CAR-T cells were incubated with PMA and ionomycin for the same period. Data are shown as mean ± SD (N=3). * represents *p*< 0.05. ** represents *p*< 0.01 (compared with CAR-T only group by one-way ANOVA, n=5). **(****FIG.1J****) CR3022-28Z downregulation in response to 293-ACE2 cells pre-incubated with different concentrations of the RBD peptide.** CAR-T-cells were co-cultured for 20 hours with 293-ACE2 cells pre-incubated with various concentrations of the RBD peptide for 1h prior to the co-culture. Data are shown as mean ± SD (N=3). * represents p< 0.05. **** represents p<0.0001 (two-tailed Student's t-test, n=2).

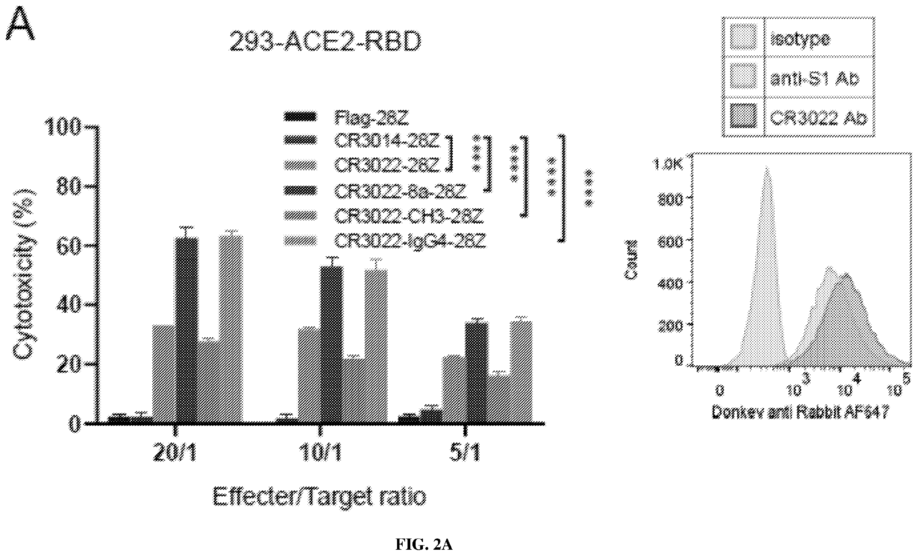

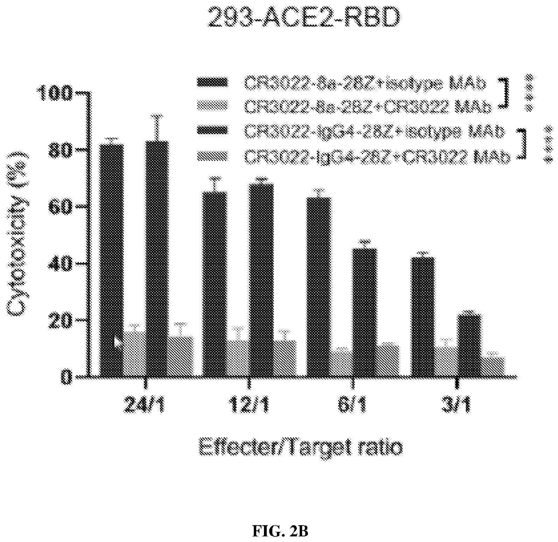

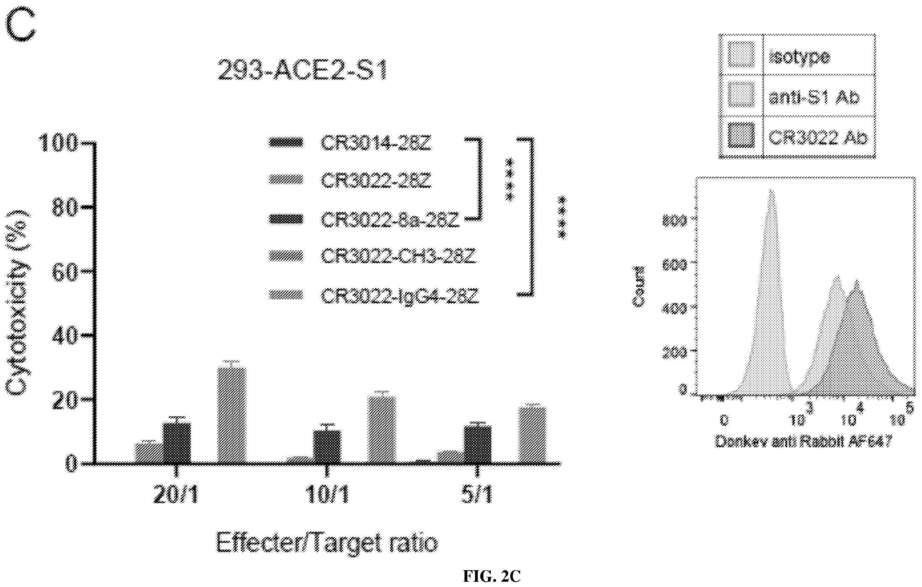

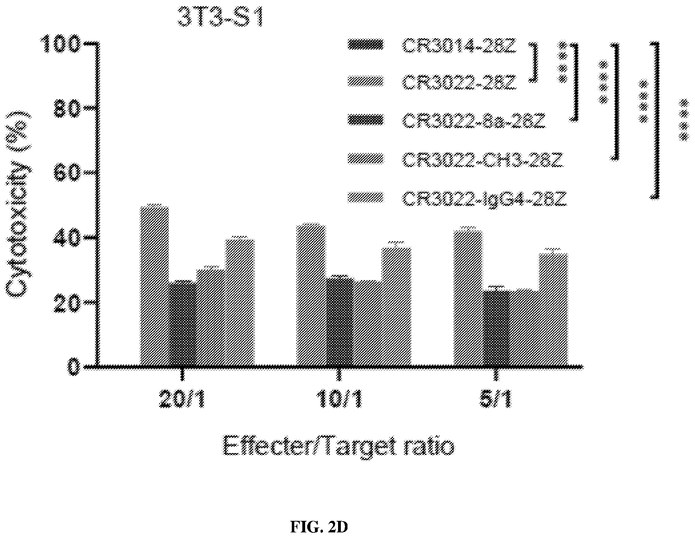

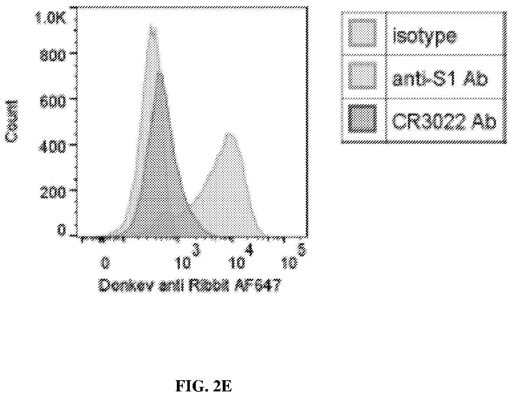

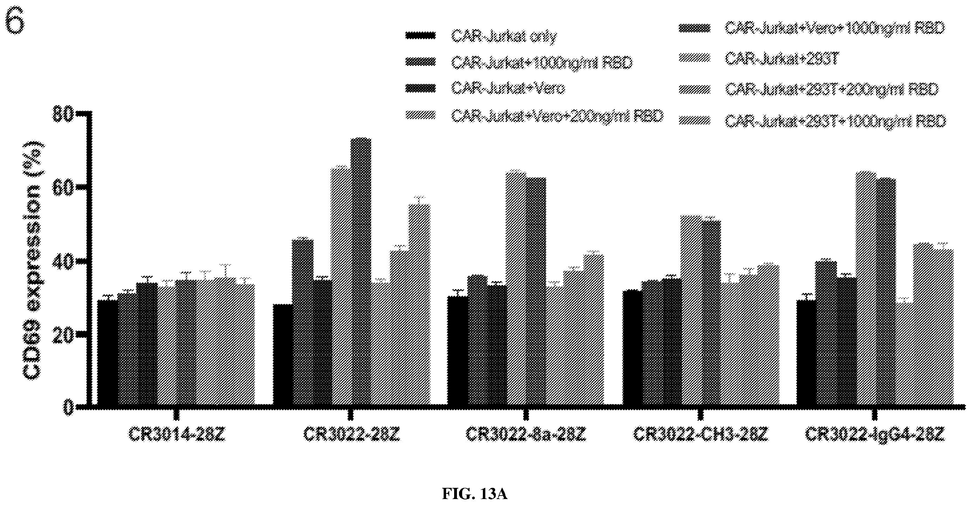

**FIGS. 2A - 2E****: Different CAR-Ts possess cytolytic activity that mediated the killing of RBD, S1 loaded and S1 expressing target cells (****FIG. 2A****).** Different CAR-Ts kill targets loaded with the RBD peptide. **Left panel:** 293-ACE2 cells were loaded with the RBD peptide followed by washing and incubation with CAR-Ts for 4-5 hours prior to a measure of killing. **Right panel:** The presence of RBD peptides on loaded 293-ACE2 cells was detected with anti-S1 and anti-RBD antibodies. Data are shown as mean ± SD (N=3). **** represents p < 0.0001 (compared with CR3014-28Z by two-way ANOVA, n=4). **(****FIG. 2B****)** CR3022 antibody blocks CR3022-8a-28Z and CR3022-IgG4-28Z kill RBD coated targets. 293-ACE2-RBD cells were incubated with CR3022, or the isotype control, at 37°C for 30min prior to the incubation with CAR-Ts. Data are shown as mean ± SD (N=3). **** represents p < 0.0001 (compared with isotype control groups by paired Student's t-test, two tailed, n=2). **(****FIG. 2C****)** Different CAR-Ts kill targets coated with S1 peptide. **Left panel:** 293-ACE2 cells were loaded with theS1 peptide followed by washing and incubation with CAR-Ts for 4-5 hours prior to a measure of killing. **Right panel:** The presence of S1 peptides on loaded 293-ACE2 cells was detected with anti-S1 and anti-RBD antibodies. Data are shown as mean ± SD (N=3). **** represents p < 0.0001 (compared with CR3014-28Z by two-way ANOVA, n=3). **(****FIGS. 2D** **and** **2E****)** Different CAR-Ts kill NIH/3T3 cells expressing the S1 peptide. **Lower panel:** The S1 over-expressing NIH/3T3 cell were sorted by FACS staining of S1.These cells showed high levels of anti-S1 staining, while the staining with the CR3022 antibody was lower. **Upper panel:** S1 expressing NIH/3T3 cells were co-cultured with CAR-Ts for 4-5 hours prior to a measure of killing. Data are shown as mean ± SD (N=3). **** represents p < 0.0001 (compared with CR3014-28Z by two-way ANOVA, n=3) (also see **FIGS. 13A-13B****).**

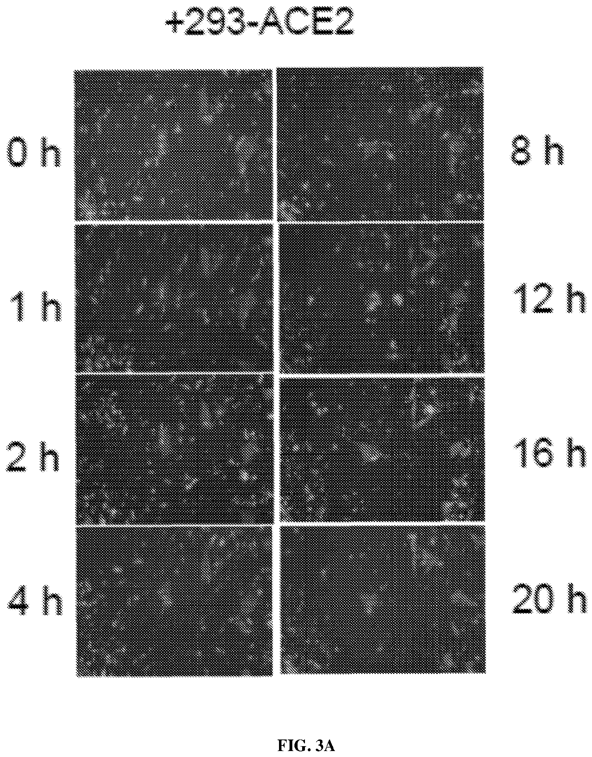

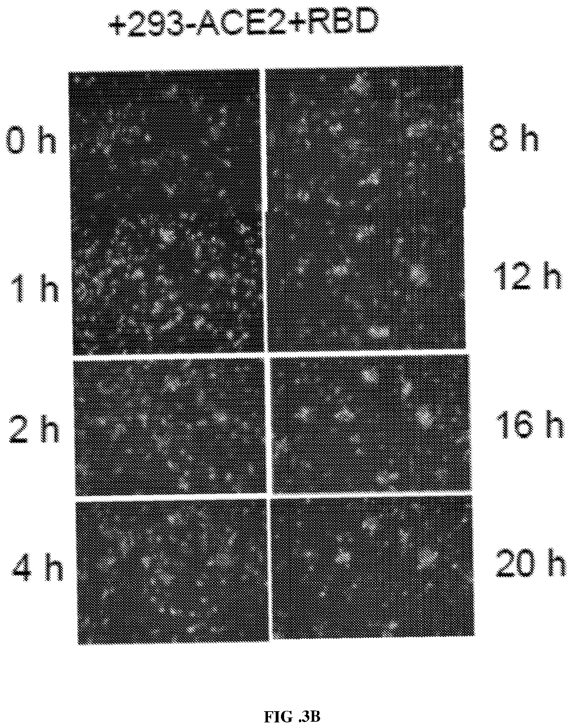

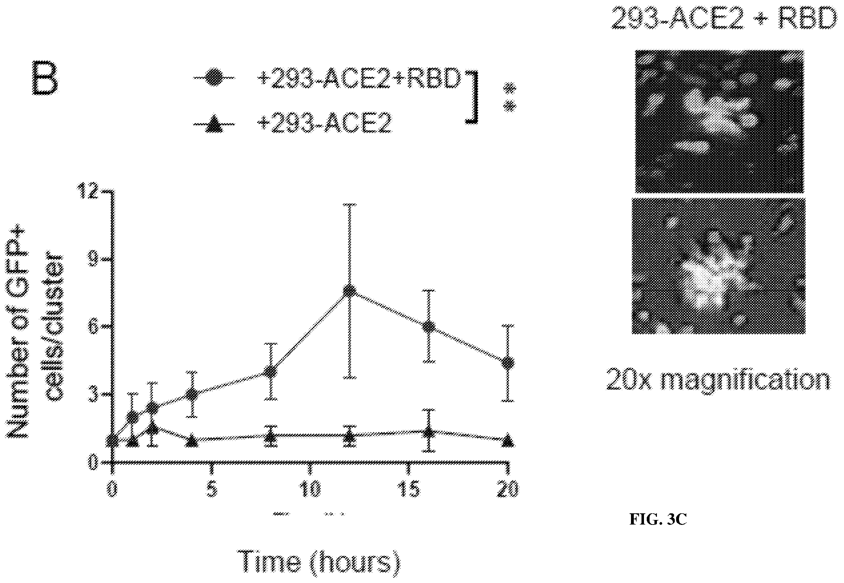

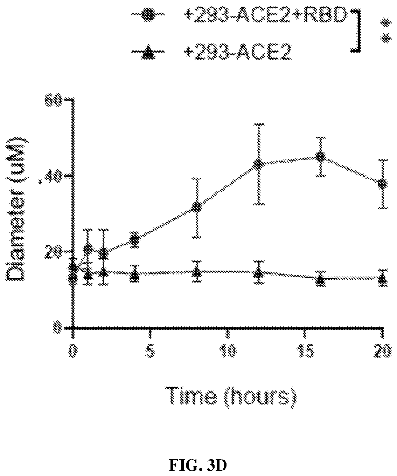

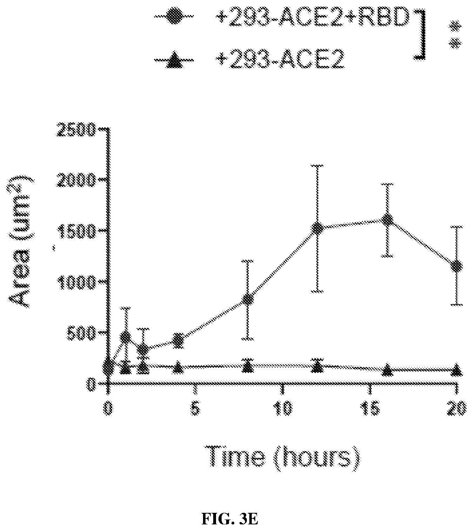

**FIGS. 3A - 3E****: Time lapse microscopy shows that CR3022-8a-28Z CAR-Ts form multi-cellular clusters in the killing of 293-ACE2-RBD target cells.**CR3022-8a-28Z CAR-Ts were co-cultured with 293-ACE2 cells or 293-ACE2-RBDcells and subjected to time-lapse microscopy for 20 hours. Red colored cells are 293-ACE2 cells or 293-ACE2-RBD transduced to express m-cherry (see Methods). **(****FIGS. 3A** **and** **3B****)** Images of CAR-Ts cells with 293-ACE2 cells (left panels) or 293-ACE2-RBD (right panels) over 20 hours. Examples of images of GFP+ CAR-Ts (green) in clusters with target cells (red-orange) (n=2). **(****FIG. 3C****)** Left panel: Histogram showing the numbers of GFP+ cells/cluster over 20 hours. Data are shown as mean ± SD (N=5). ** represents *p*< 0.01. *** represents *p*<0.001 (two-tailed Student's t-test, n=2). Right panel: Examples of 20× images of cell clusters between CR3022-8a-28Z CAR-Ts and 293-ACE2-RBD cells (n=2). **(****FIG. 3D****)** Histogram showing the diameter (um) of clusters over a time course of 20 hours. Data are shown as mean ± SD (N=5).* represents *p<* 0.05. ** represents *p<* 0.01. *** represents *p<* 0.001. **** represents *p*<0.0001 (two-tailed Student's t-test, n=2). **(****FIG. 3E****)** Histogram showing the area (um²) of clusters over a time course of 20 hours. Data are shown as mean ± SD (N=5). ** represents *p<* 0.01. *** represents *p<* 0.001. **** represents *p*<0.0001 (two-tailed Student's t-test, n=2).

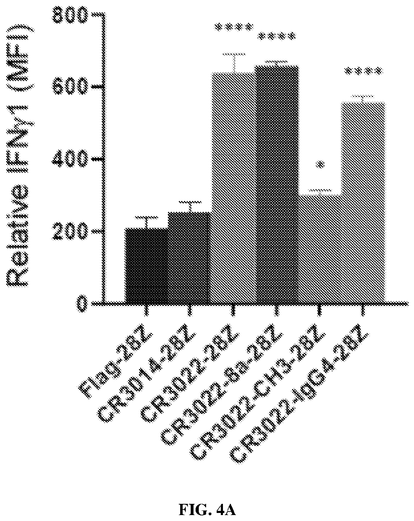

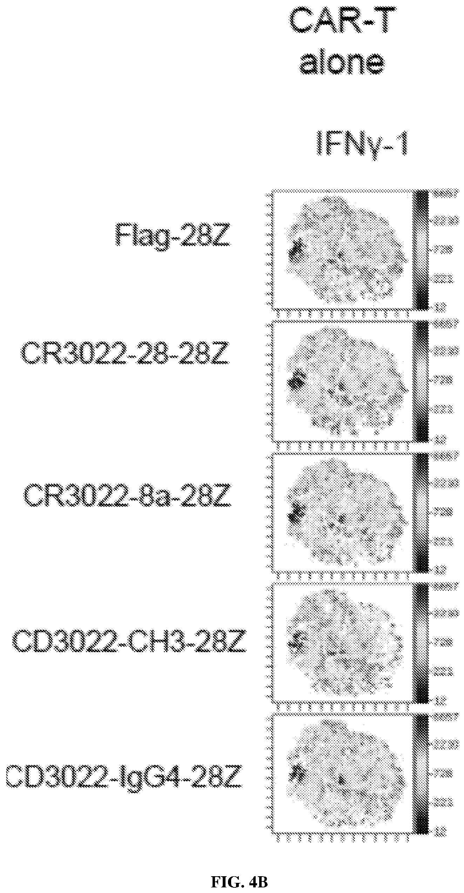

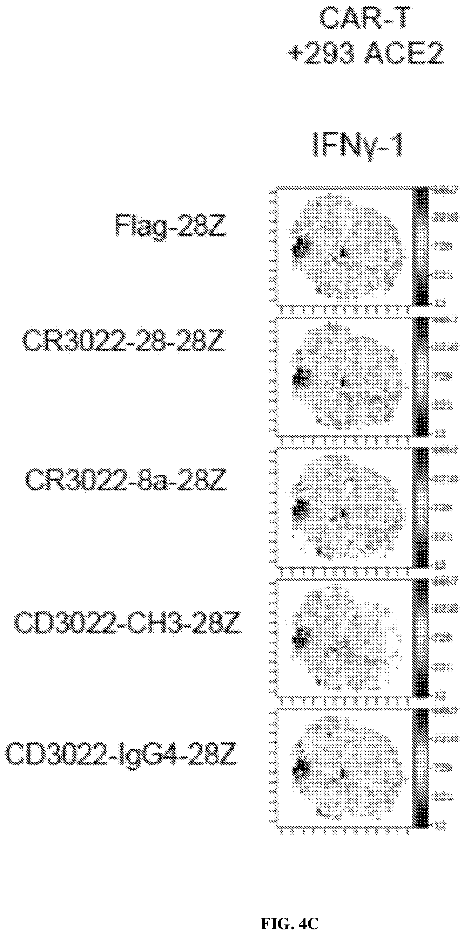

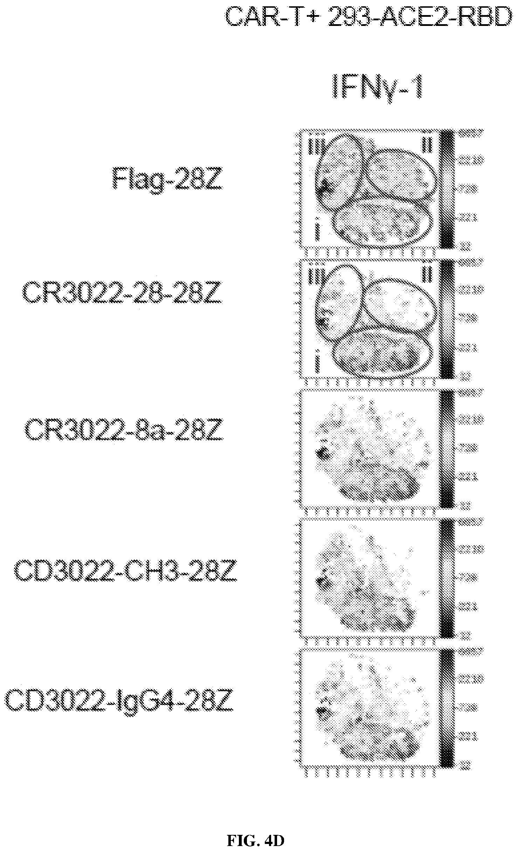

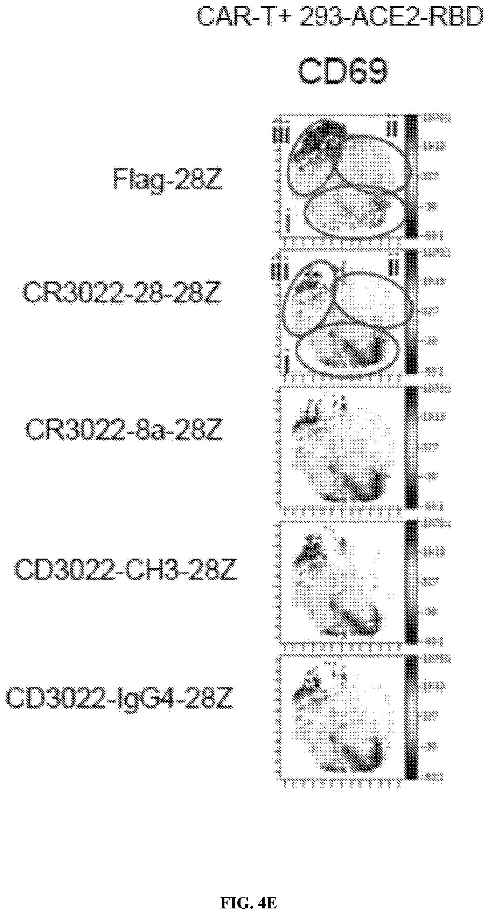

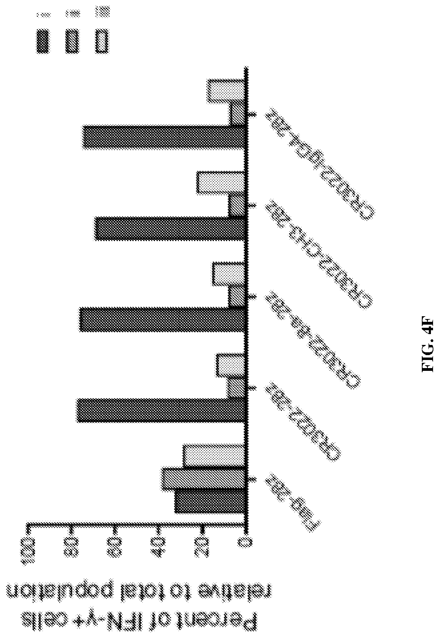

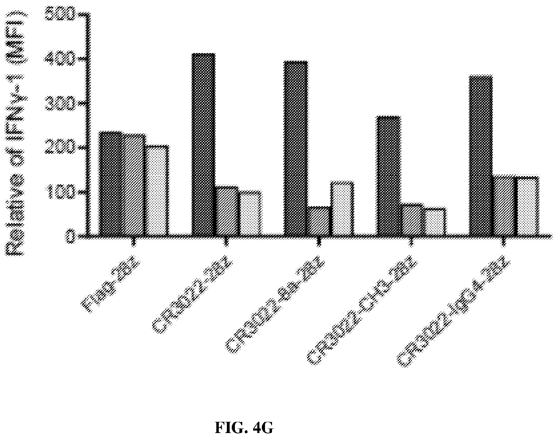

**FIGS. 4A** - **4G****: Expression of interferon y (IFN-y) in a CD69+ anti-SARS-CoV-2 CAR-Ts. (****FIG. 4A****)** Mean fluorescent intensity (MFI) values of IFN-γ expression on Flag-28Z, CR3014-28Z, CR3022-28Z, CR3022-8a-28Z, CR3022-CH3-28Z and CR3022-IgG4-28Z CAR-Ts in response to 293-ACE2-RBD cells after 20 hours of co-culture. Data are shown as mean ± SD (N=3).* represents *p<* 0.05. **** represents *p<* 0.0001 (compared with Flag-28Z by one-way ANOVA, n=3). **(****FIGS. 4B-4E****)** viSNE plots of the expression of IFN-γ on CAR-Ts following expansion after 3-week incubation in IL-2 supplemented media (CAR-T alone) (left panels) or following an additional 20 hours with 293-ACE2 cells (middle panel) or with 293-ACE2-RBD cells (right panel). ViSNE plots of CD69 expression on cells from the same donor (right panel) (n=3). **(****FIG. 4F****)** Histogram showing the changed distribution of cells expressing IFN-y in viSNE islands. Percentage of IFN-y cells relative to total population of CAR-Ts (n=3). **(****FIG. 4G****)** Histogram showing the changed expression of IFN-γ on individual cells in the viSNE islands. Relative MFI in islands i, ii and iii. (n=3).

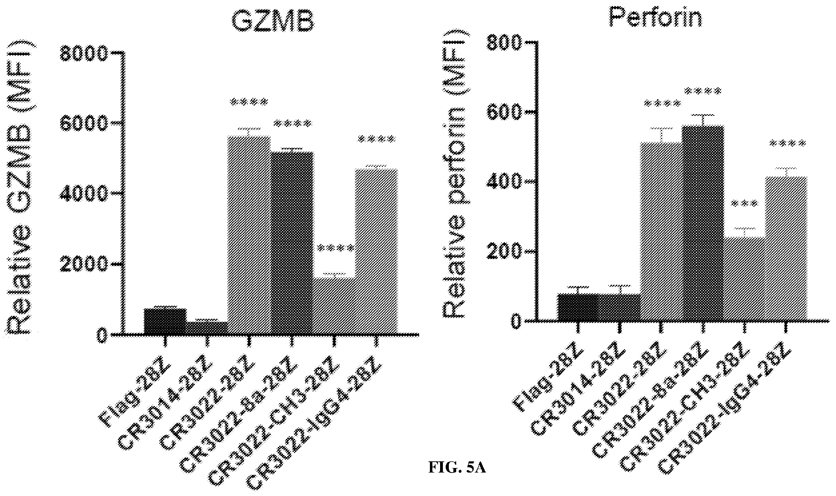

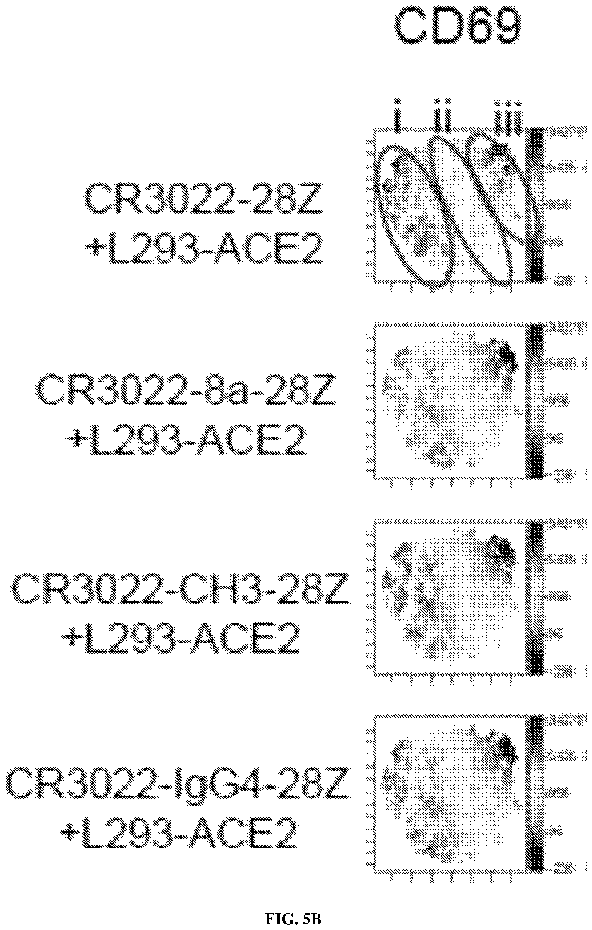

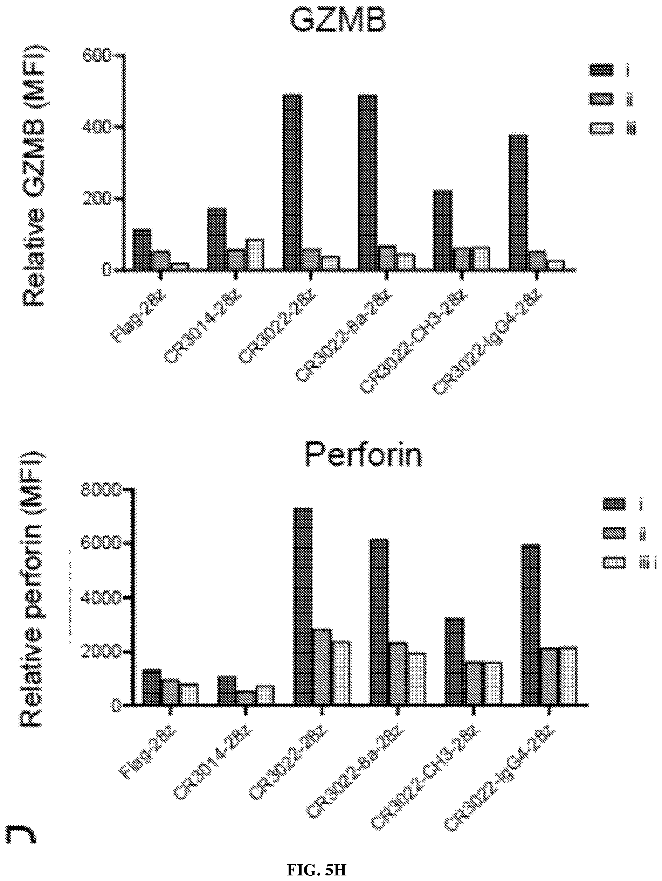

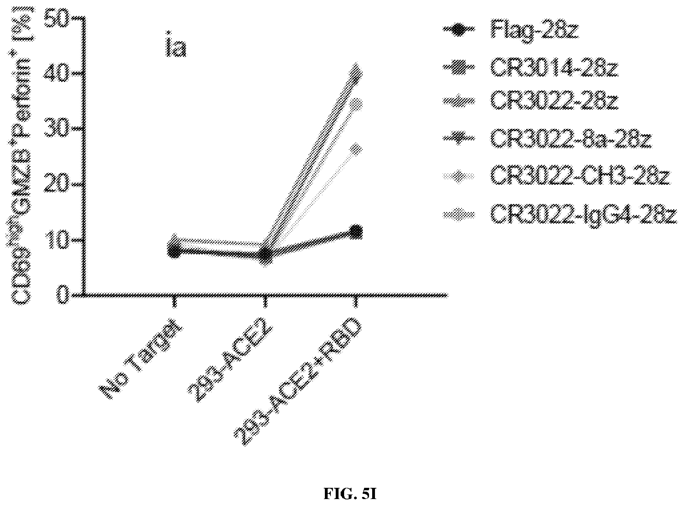

**FIGS. 5A - 5I****: Expression of Granzyme B (GZMB) and perforin on a subset of CD69+ anti-SARS-CoV-2 CAR-Ts. (****FIG. 5A****)** Mean fluorescent intensity (MFI) values of GZMB and perforin expression on Flag-28Z, CR3014-28Z, CR3022-28Z, CR3022-8a-28Z, CR3022-CH3-28Z and CR3022-IgG4-28Z CAR-Ts in response to 293-ACE2-RBD cells after 20 hours of co-culture. Data are shown as mean ± SD (N=3). *** represents *p*< 0.001*.* **** represents *p*< 0.0001(compared with Flag-28Z by one-way ANOVA, n=4). Upper panel: GZMB; lower panel: perforin. **(****FIGS. 5B-5G****)** viSNE plots for CD69, GZMB and perforin expression in SARS-CoV-2 CAR-Ts following incubation with 293-ACE2 (upper panels) or with 293-ACE2-RBD (lower panels) (n=4). **(****FIG. 5H****)**Histogram showing relative MFI values for GZMB (upper panel) and perforin (lower panel) in viSNE islands i, ii and iii (n=4). **(****FIG. 5I****)**Percent representation of CD69^{hi}GZMB+perforin+ subset in response to 293-ACE2 and 293-ACE2-RBD cells (n=4).

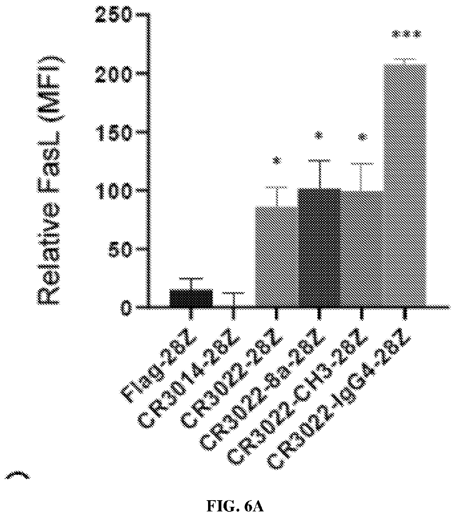

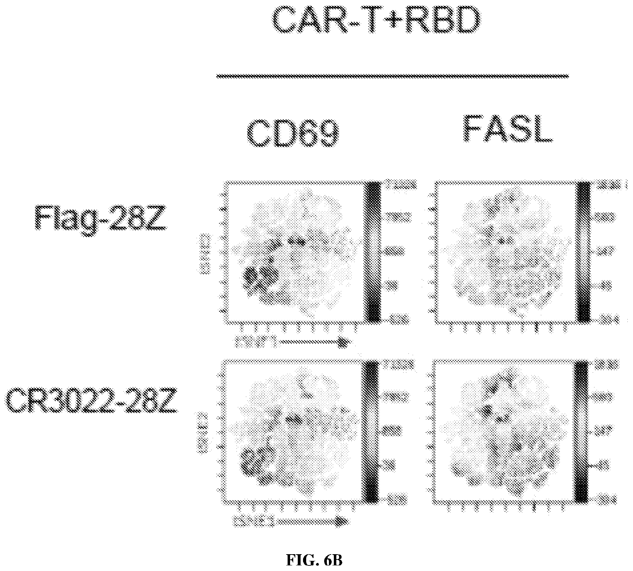

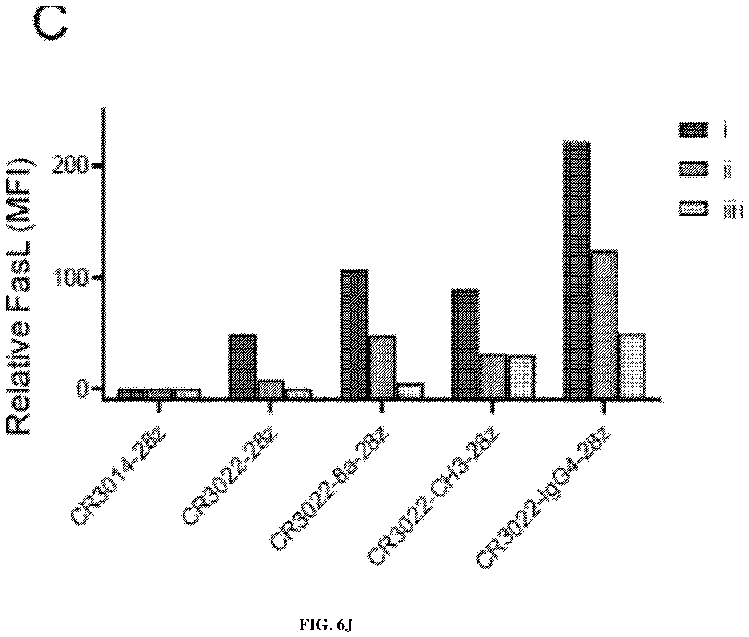

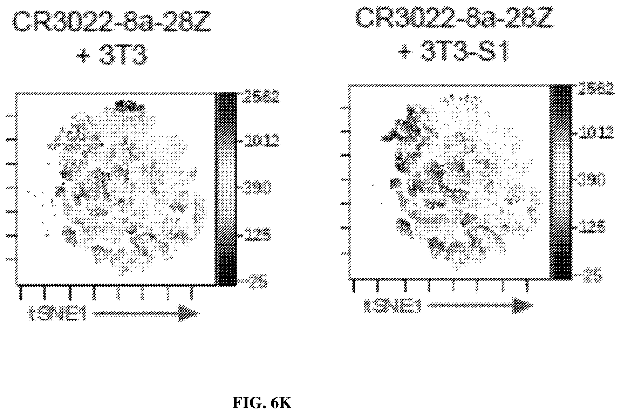

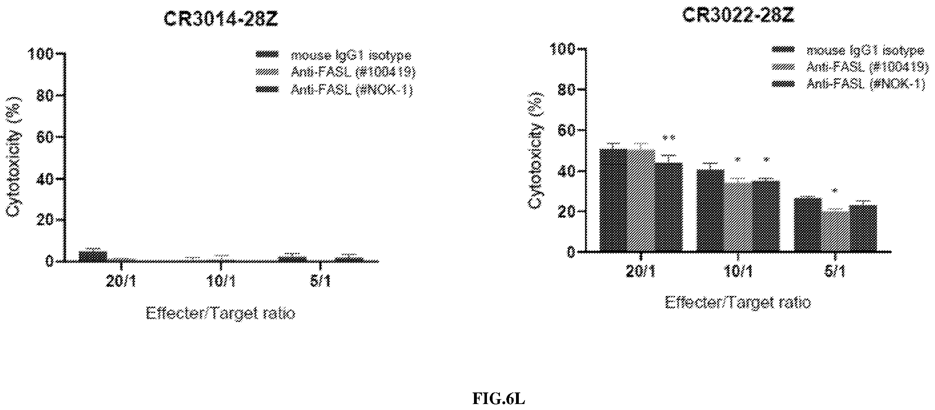

**FIGS. 6A - 6M****: FasL expression on anti-SARS-CoV-2 CAR-Ts. (****FIG. 6A****)** Relative mean fluorescent intensity (MFI) values for FasL expression on Flag-28Z, CR3014-28Z, CR3022-28Z, CR3022-8a-28Z, CR3022-CH3-28Z and CR3022-IgG4-28Z CAR-Ts in response to 293-ACE2-RBD cells after 24 hours of co-culture. Data are shown as mean ± SD (N=3).* represents *p<* 0.05. *** represents *p<* 0.001(compared with Flag-28Z by one-way ANOVA, n=3). **(****FIGS. 6B-6I****)** viSNE plots defining subsets of CD69 and FasL expression on CAR-Ts. Shown are the profiles of Flag-28Z, CR3022-28Z, CR3022-8a-28Z, CR3022-CH3-28Z and CR3022-IgG4-28Z CAR-Ts under conditions where cells were incubated for 24 hours with soluble RBD peptide in the absence of 293-ACE2 cells or with 293-ACE2 or 293-ACE2-RBD cells. **(****FIG. 6J****)** Histogram showing the increase in FasL expression on islands i, ii, iii on Flag-28Z, CR3014-28Z, CR3022-28Z, CR3022-8a-28Z, CR3022-CH3-28Z and CR3022-IgG4-28Z CAR-Ts after incubation with 293-ACE2-RBD cells (n=3). **(****FIG. 6K****)** viSNE profiles of FasL expression in CR3022-8a-28Z CAR-T following incubation without (left panel) or with NIH/3T3-S1 cells (right panel) (n=2). **(****FIGS. 6L-6M****)** Anti-FasL failed to interfere with the killing of anti-SARS-CoV-2 CAR-Ts. Two anti-FasL blocking antibodies were assessed for the ability to inhibit CR3022-28Z, CR3022-8a-28Z and CR3022-IgG4-28Z CAR-T killing of targets cells loaded with RBD peptide. Data are shown as mean ± SD (N=3). *represents p < 0.05. ** represents *p*< 0.01. (Compared with mouse IgG1 isotype group by paired Student's t-test, two tailed, n=2).

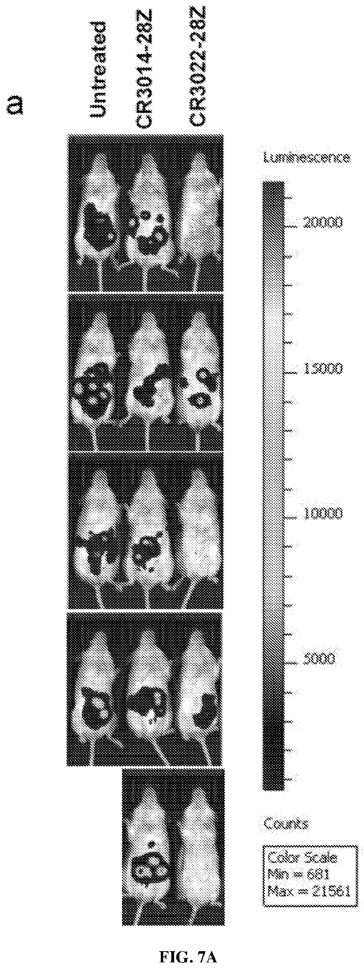

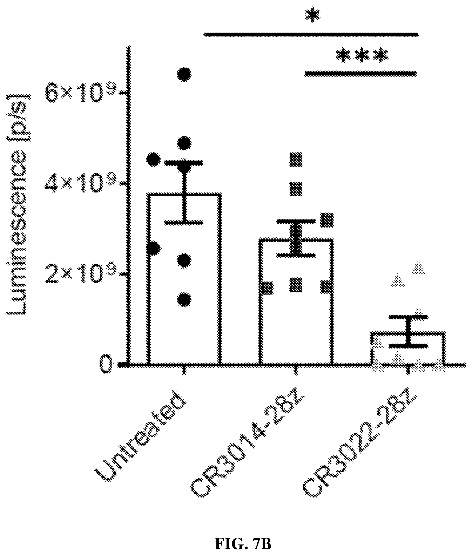

**FIGS. 7A - 7B****: The *in vivo* elimination of NIH/3T3 cells expressing SARS-CoV-2 S1 (****FIG. 7A****)** Images of NIH/3T3-S1 cells in mice. NIH/3T3-S1 cells express luciferase and the presence of luminescence after D-luciferin injection indicates the presence of NIH/3T3-S1 cells. A mixture of2×10⁶NIH/3T3-S1cells and4×10⁶ CR3014-28Z or CR3022-28Z CAR-T cells were injected into NOD-SCID IL2Rγ^{null}mice through the intraperitoneal route. 24h later, D-luciferin was injected and bioluminescence images were acquired. 4-5 mice are shown out of 8 mice per group in total. **(****FIG. 7B****)** Histogram showing the significant reduction of NIH/3T3-S1 cells *in* vivobyCR3022-8a-28Z but not by CR3014-28Z CAR T-cells. Data are shown as mean ± SD (n=8).* represents *p<* 0.05. *** represents *p<* 0.001(compared with untreated group by one-way ANOVA). This experiment was repeated twice.
**FIG 8****: ViSNE profiles of anti-Flag and GFP expression on CR3022-8a-28Z T-cells.** Histogram shows the expression intensity and cell numbers in islands i, ii and iii (following expansion and prior to incubation with 293-ACE2 cells). Anti-Flag shows the surface expression of the Flag-tagged CARs, while GFP shows the expression of EGFP by the pHIV bicistronic lentiviral construct containing IRESEGFP (n=3).

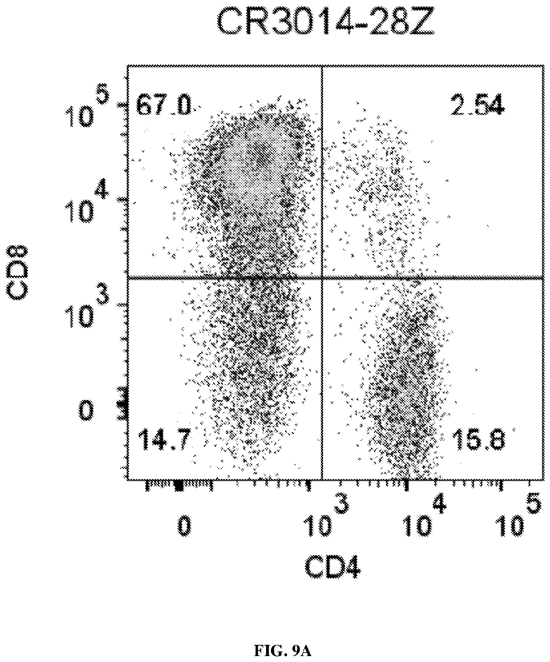

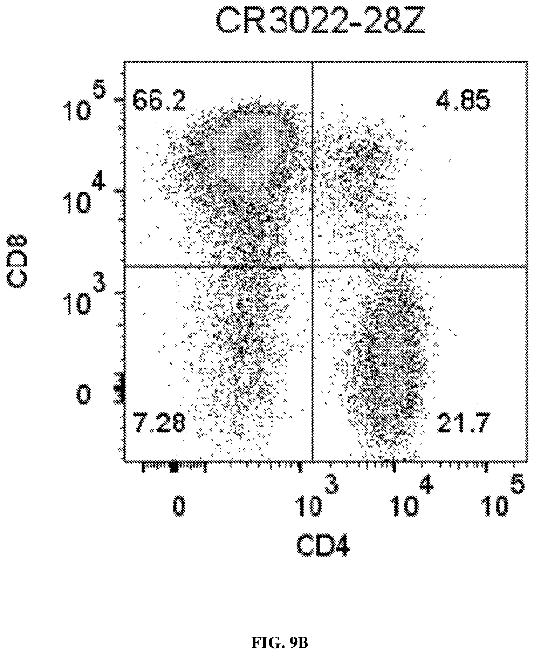

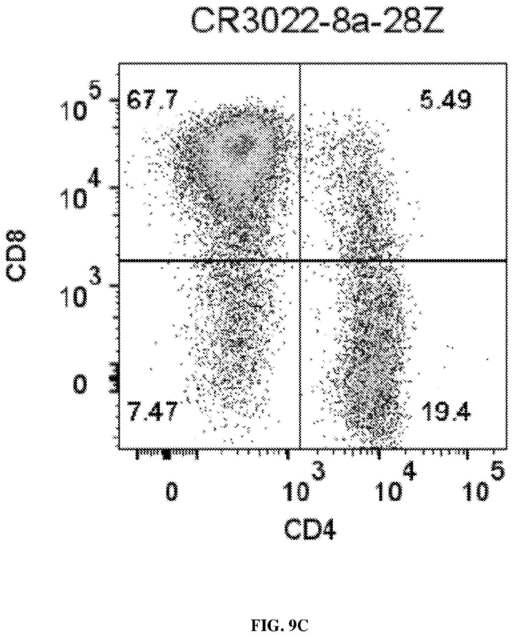

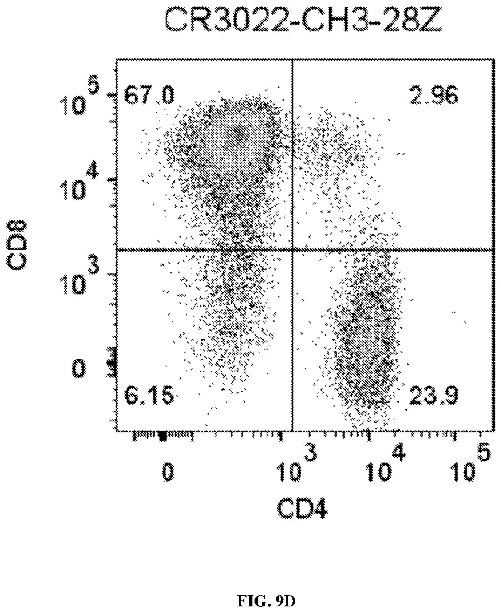

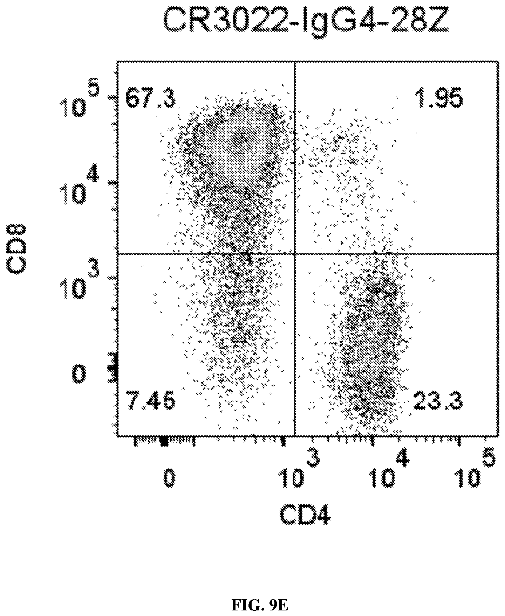

**FIGS. 9A-9E****: CD4 and CD8 CAR-Ts generated within the lentiviral transduced human peripheral.** Tcell population. Flow cytometric staining with anti-CD4 and anti-CD8 shows similar proportions of CD8+ and CD4+ cells amongst the **FIG. 9A****)** CR3022-28Z, **FIG. 9B****)** CR3022-28Z; **FIG. 9C****)** CR3022-8a-28Z, **FIG. 9D****)** CR3022-CH3-28Z or **FIG. 9E****)** CR3022-IgG4-28Z CAR-Ts.
**FIG. 10****: Flow cytometric profile of human ACE3 expression on 293 cells.** 293 cells were transfected to stably express the hACE2 receptor. Light blue: isotype control; pink: anti-h ACE3 receptor.
**FIGS. 11A-11B****: Incubation of different CAR-Ts with 293-ACE2-RBD cells induced the expression of CD69 and GZMB.** CAR-Ts were co-cultured for 20hrs either RBD peptide alone (negative control), 293-ACE2 cells (negative control) or 293-ACE2 cells that had been incubated with RBD peptide for 1 h prior (293-ACE2-RBD) prior to co-culturing with the CAR-Ts. CAR-T cells exposed to neither RBD or 293-ACE2 cells also served as a negative control. Cells where then purified, labelled with either anti-Flag or anti-CD69 tagged with different fluorochromes (i.e., PE, FITC) and analyzed by conventional flow cytometry. Cells gated for Flag expression were analyzed for CD69 expression **(****FIG. 11A****).** As in the previous panel, however, in this case, a separate collection of CAR expressing cells were permeabilized and stained for granzyme B (GZMB) **(****FIG. 11B****).**

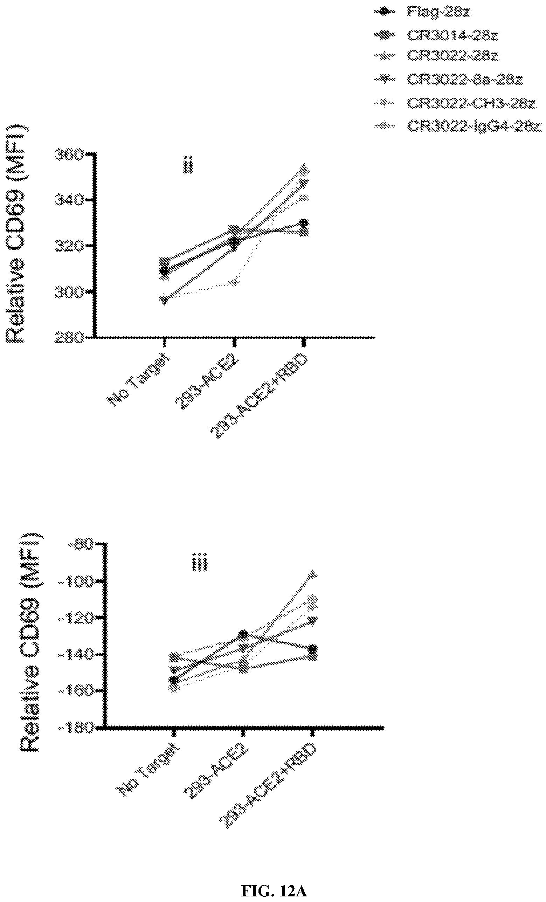

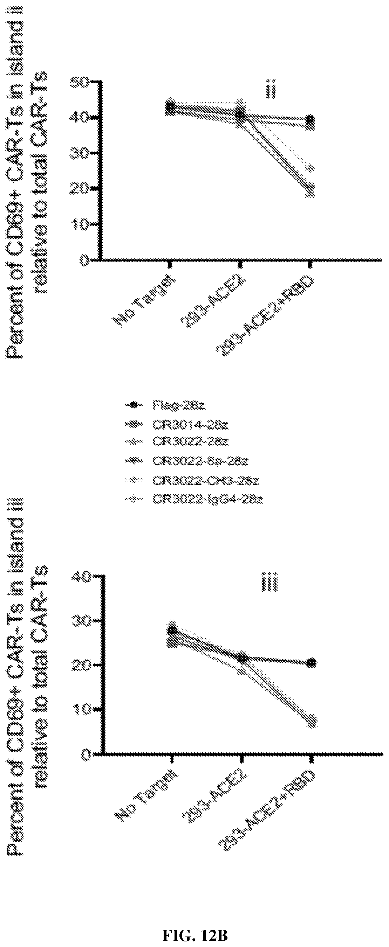

**FIGS. 12A-12B****: Changes in the presence or expression of CD69 in subsets of CAR-Ts following incubation with 293-ACE2 or 293-ACE2-RBD cells.** From **FIGS. 1F-****1G,** the viSNE is subdivided into Islands i ii and iii based on the surface expression of the activation marker CD69. **FIGS. 1F-1G****,** right panels shows the chances in expression for island i. **FIGS. 12A-12B** shows that change in the percent representation amongst CAR-Ts for islands ii and iii **(****FIG. 12A****);** MFI changes in expression **(****FIG. 12B****).**

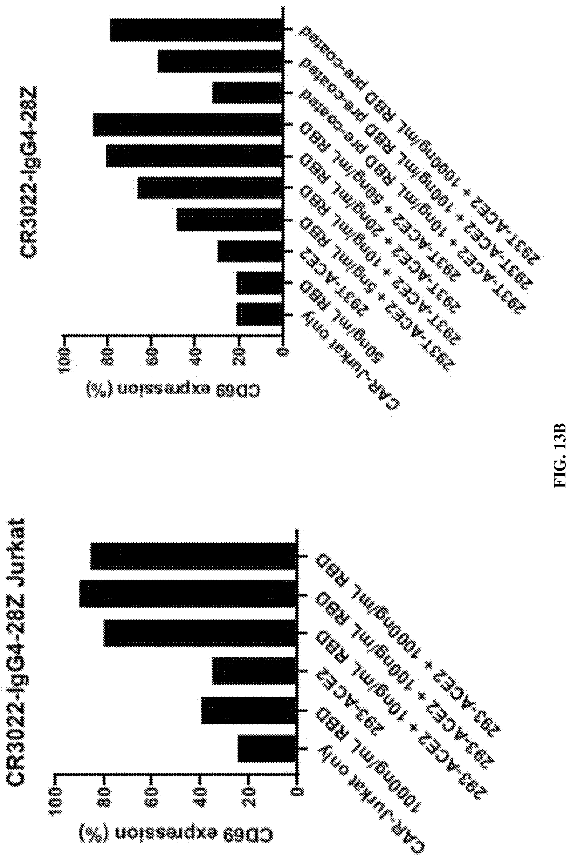

**FIGS. 13A-13B****: Response of transduced Jurkat T-cells expressing CARs.** **FIG. 13A****:** Induction of CD69 on Jurkat cells expressing different CARs in response to Vero or L293 cells loaded with RBD peptide. **FIG 13B****:** Expression of induced CD69 on CD8+ T-cells expressing CR3022-IgG4-28Z in response to 293-ACE2 cells that had been pre-incubated with different concentrations of the RBD peptide.

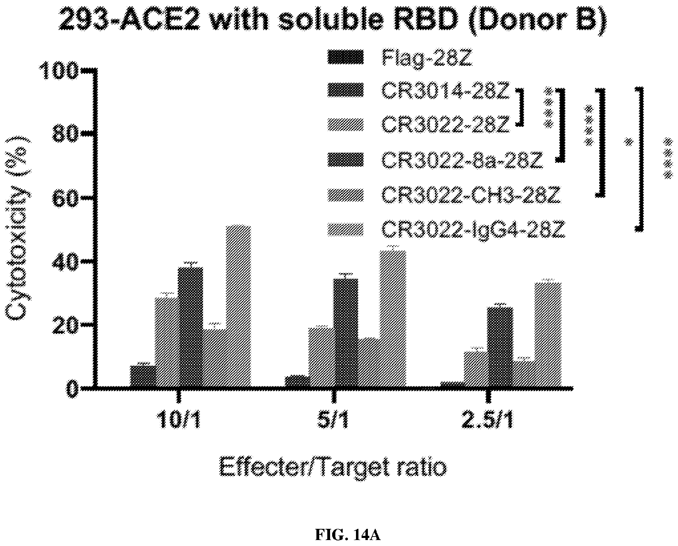

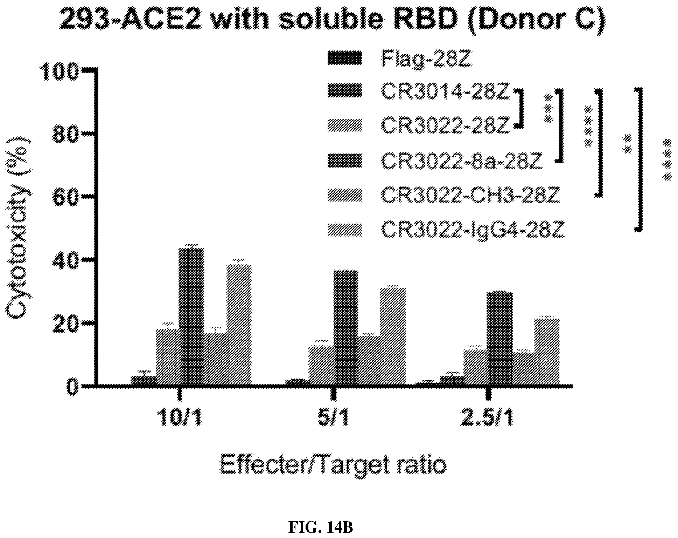

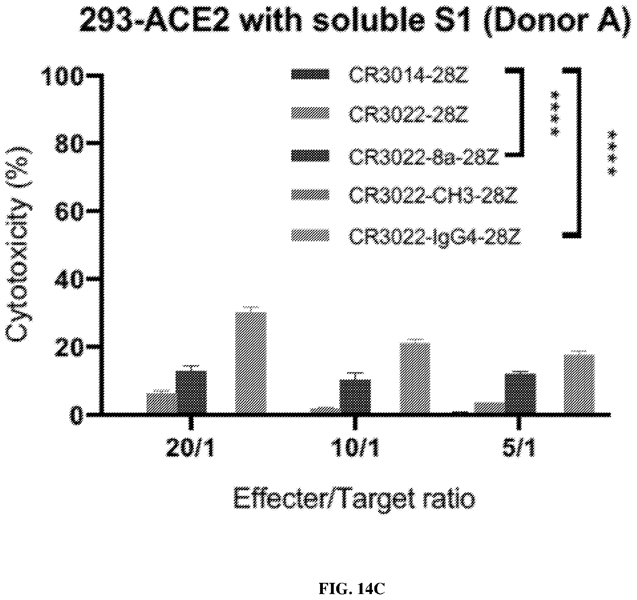

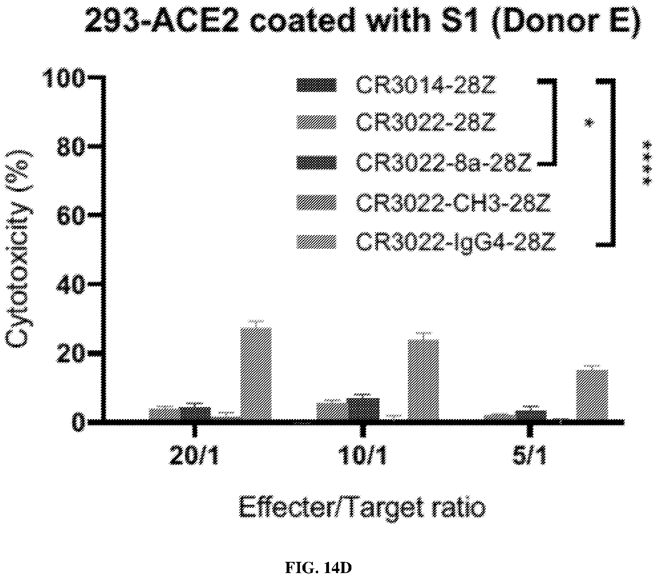

**FIGS. 14A-14D****: Examples of the cytolytic response of SARs CoV2 CAR-Ts with different donor to the RBD and S1 peptides.** The killing of 293-ACE2 target cells that had been pre-coated with either the RBD (**FIGS. 14A-14B****:** donors B and C) or S1 peptide **(****FIGS. 14C-14D****:** donors A and E).

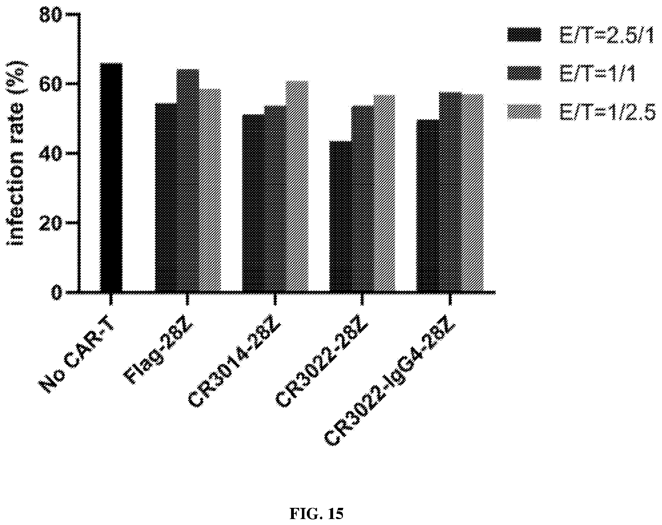

**FIG. 15****: CAR-Ts do not effectively block SARs-COV2 pseudotyped lentiviral particle entry into cells.** Recombinant pseudotyped lentiviral particles containing SARS-CoV-2 spike protein were used to mimic SARS-CoV-2 (2019nCoV) cell infection and cell entry. The SARS-CoV-2 pseudo-virus particles encode firefly luciferase and RFP in their lentiviral vector genome. The firefly luciferase and RFP gene will be strongly expressed after the SARS-CoV-2 pseudo-virus entry into ACE2-expressing cells. **FIG. 15** shows a trend in the blockade of pseudotyped lentiviral particle entry by both CR3014 and CR3022 at different ratios.

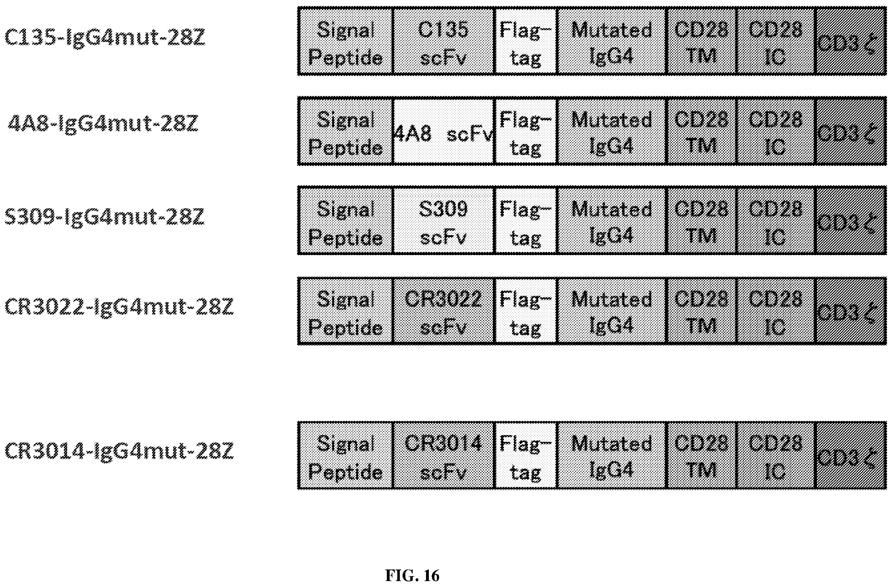

**FIG. 16****: Exemplary constructs.**

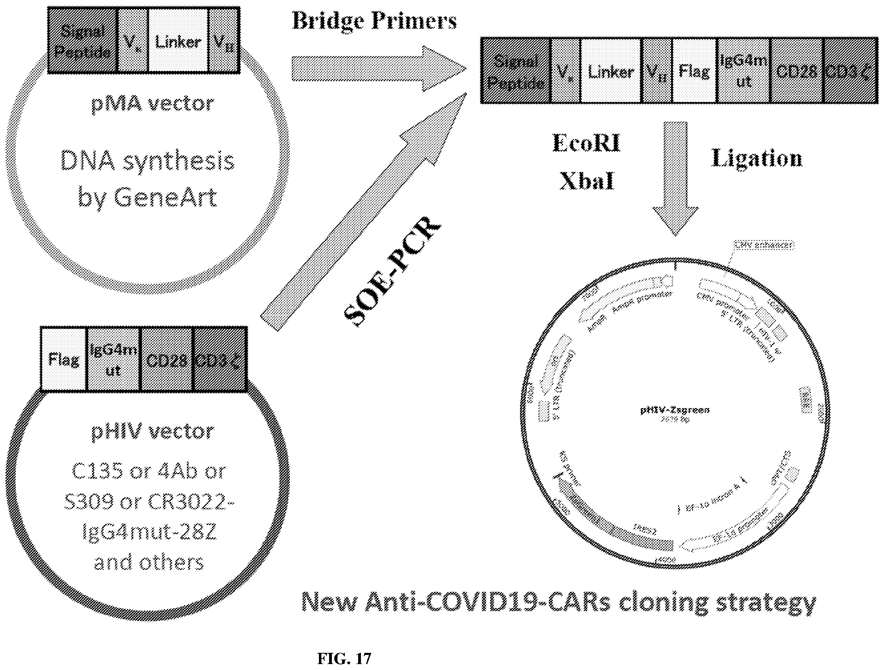

**FIG. 17****: General cloning strategy for constructs.**

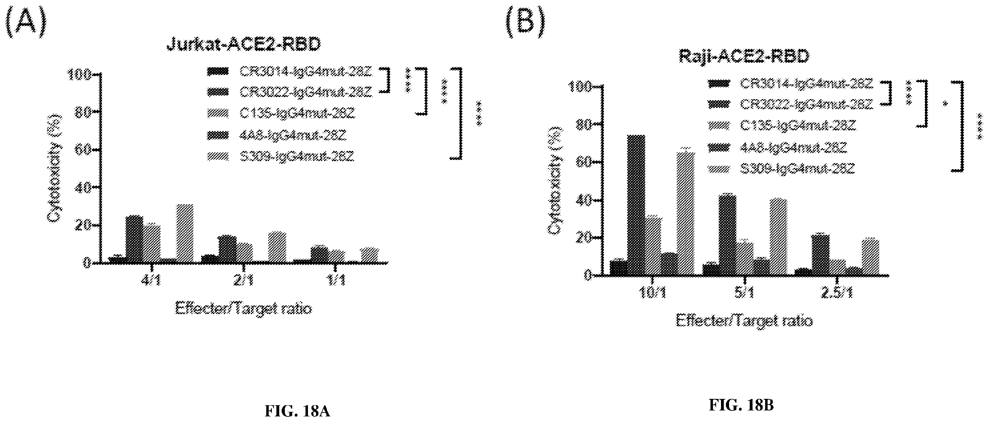

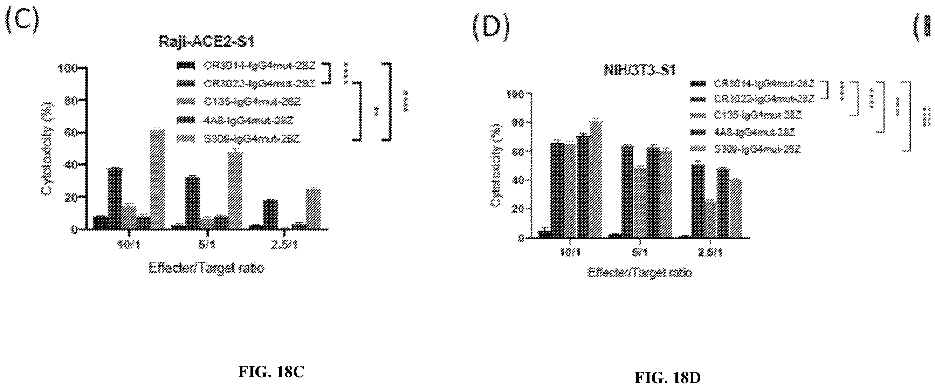

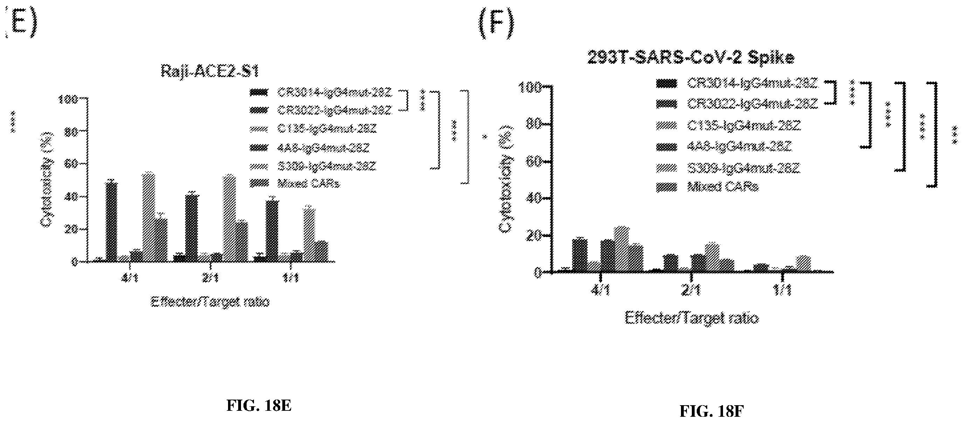

**FIGS. 18A-18F****: The CAR-Ts with different binding regions display differences in the killing of RBD, S1 and Spike expressing cells. (****FIG. 18A****) CAR-Ts with different binding regions killed Jurkat-ACE2 cells with the CoV2 RBD.** Jurkat-ACE2 cells were loaded with the CoV2 RBD peptide followed by washing and incubation with CAR-Ts for 4-5 hours prior to a measure of killing. Jurkat cells were transfected with ACE2 for stable expression. S309-IgGmut-28Z, C135 IgGmut-28 and CR3022-IgG4mut-28Z CAR-Ts effectively killed RBD pre-coated Jurkat-ACE2 cells. As expected, neither the CR3014-IgG4mut-28Z CAR-Ts (only recognizing CoV RBD) nor 4A8-IgGmut-28Z (only recognizing NTD sequences) killed the targets expressing CoV2 RBD peptide. This pattern was seen at each effecter-target ratio (4/1, 2/1, 1/1). **(****FIG. 18B****)** CAR-Ts with different binding regions killed Raji-ACE2 cells with the CoV2 RBD. Raji-ACE2 cells were loaded with the CoV2 RBD peptide followed by washing and incubation with CAR-Ts for 4-5 hours prior to a measure of killing. Raji cells were transfected with ACE2 for stable expression. S309-IgGmut-28Z and CR3022-IgG4mut-28Z were most effective in killing Raji-ACE2 cells were loaded with the CoV2 RBD peptide. As a negative control, CR3014-IgG4mut-28Z (only recognizing CoV RBD) CAR-Ts failed to kill targets. **(****FIG. 18C****)** CAR-Ts with different binding regions killed Raji-ACE2 cells with the CoV2 S1. Raji-ACE2 cells were loaded with the CoV2 RBD peptide followed by washing and incubation with CAR-Ts for 4-5 hours prior to a measure of killing. S309-IgGmut-28Z and CR3022-IgG4mut-28Z were most effective in killing Raji-ACE2 cells were loaded with the CoV2 S1 peptide. C135 IgGmut-28 and 4A8-IgGmut-28Z killed poorly. S309-IgGmut-28Z killed better than CR3022-IgG4mut-28Z against the Raji-ACE2-S1 target at ratios 10/1 and 5/1. As a negative control, CR3014-IgG4mut-28Z (only recognizing CoV RBD) CAR-Ts failed to kill targets. **(****FIG. 18D****)** CAR-Ts with different binding regions killed NIH/3T3 cells expressing CoV2 S1. NIH/3T3 cells, stably transfected with CoV2 S1 (a kind gift from Uri Saragovi, McGill University). They were incubated with CAR-Ts for 4-5 hours prior to a measure of killing. Each of the CAR-Ts were able to kill the target at different effector-target ratios. S309-IgG4mut-28Z killed better than the other CAR-Ts at a 10/1 ratio, while each of the CAR-Ts killed better than the C135 IgG4mut-28 at ratios 5/1 and 2.5/1. These data showed that each of the CAR-Ts kill with different levels of efficacy under different conditions of culture, a finding not predicted by simple antibody binding. As a control, CR3014-IgG4mut-28Z (only recognizing CoV RBD) CAR-T failed to kill target. **(****FIG. 18E****)** A mixture or cocktail of different CAR-Ts with different binding regions kill Raji-ACE2 cells expressing CoV2 S1. Raji-ACE2 cells were loaded with the CoV2 RBD peptide followed by washing and incubation with CAR-Ts for 4-5 hours prior to a measure of killing. As shown in panel C, S309-IgG4mut-28Z and CR3022-IgG4mut-28Z were most effective. A cocktail of each of the CAR-Ts nevertheless was also effective in killing targets at all effector-target ratios. As a control, CR3014-IgG4mut-28Z (only recognizing CoV RBD) CAR-T failed to kill target. **(****FIG. 18F****)** A mixture or cocktail of different CAR-Ts with different binding regions kill 293T-cells expressing ACE2 and CoV2 spike. 293T-ACE2 cells were transfected with the CoV2 spike protein. Cells were incubated with CAR-Ts for 4-5 hours prior to a measure of killing. S309-IgG4mut-28Z, 4A8-IgG4mut-28Z and CR3022-IgG4mut-28Z were most effective in killing. C135 IgG4mut-28 killed less well. A cocktail of each of the CAR-Ts nevertheless was also effective in killing targets at all effector-target ratios. The mixture killed to the same extent as the individual 4A8-IgG4mut-28Z and CR3022-IgG4mut-28Z CAR-Ts. They killed less well than the individual S309-IgG4mut-28Z and better than the C135 IgG4mut-28 CAR-T at ratios 4:1 and 2/1. The mixture killed better than the individual CAR-Ts at an effector to target ratio of 1/1. As a negative control, CR3014-IgG4mut-28Z (only recognizing CoV RBD) CAR-Ts failed to kill targets.

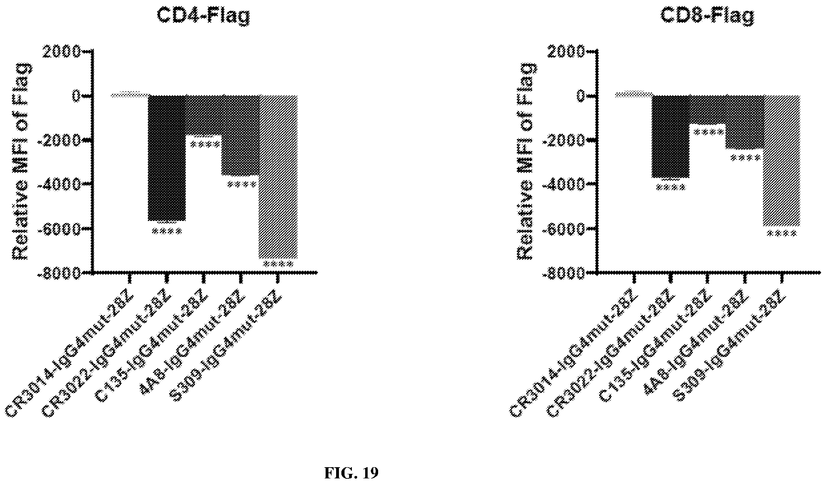

**FIG. 19****: Downregulation of CAR-T receptors on CD4 and CD8 T-cells following engagement with SARs CoV2 Spike proteins.** CAR-Ts were co-cultured for 4 h with 293T-spike cells. The presence of surface CAR was assessed using a PE tagged anti-Flag antibody to label cells for flow cytometry. Each of the S309-IgG4mut-28Z, 4A8-IgG4mut-28Z, CR3022-IgG4mut-28Z, C135 IgG4mut-28 and 4A8-IgG4mut-28Z was lost from the cell surface (i.e., shown as a change in the MFI). S309-IgG4mut-28Z and CR3022-IgG4mut-28Z showed the greatest degree of down-regulation. As a control, CR3014-IgG4mut-28Z showed no down-regulation. The effect was seen on both CD4 (left panel) and CD8+ (right panel) T-cells. Data are shown as mean ± SD of triplicate samples. ***** p* < 0.0001.

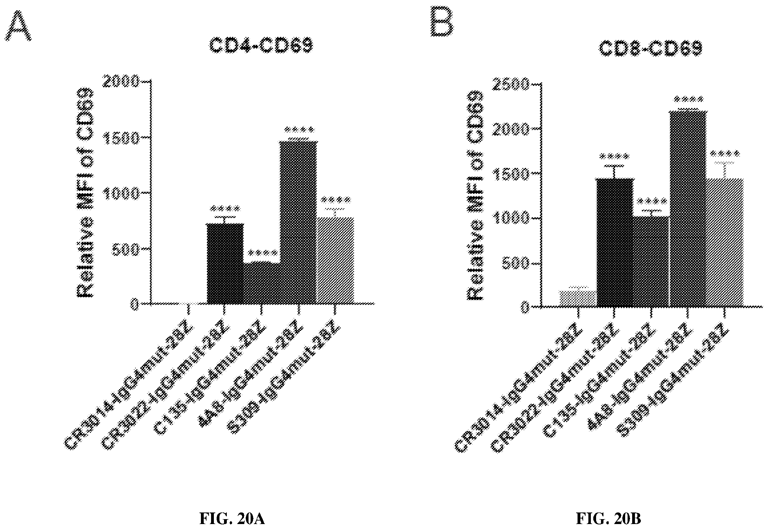

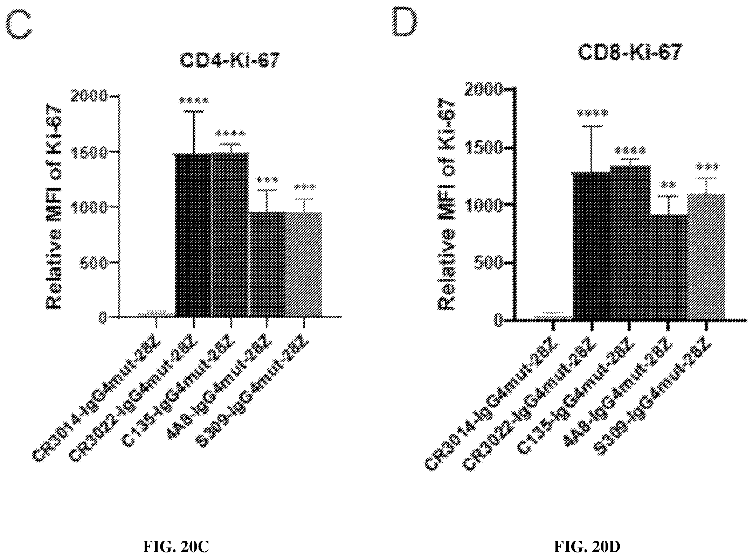

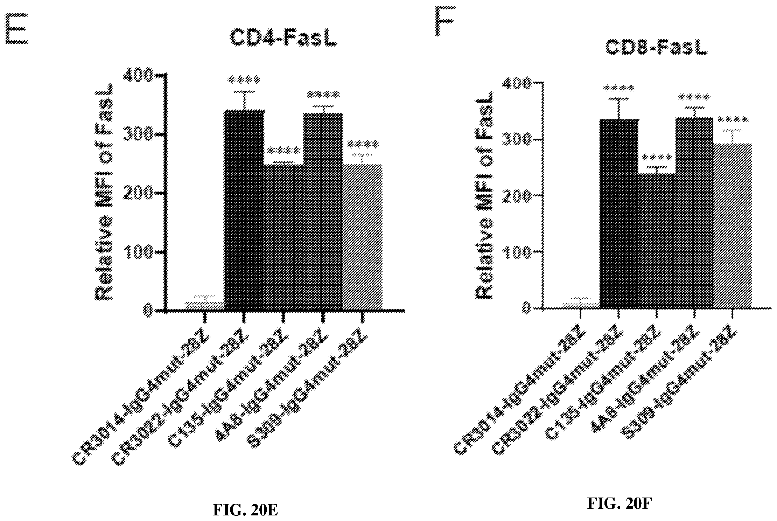

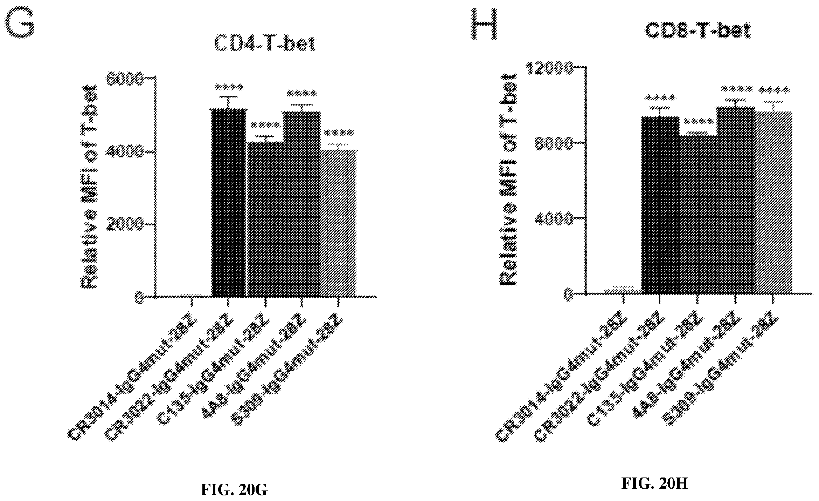

**FIGS. 20A-20H****: Histogram showing the expression of surface activation antigen CD69, cell cycle antigen Ki67, effector molecule FasL and transcription factor T-bet in CAR-Ts.** Histogram shows the expression in the different CAR-Ts after incubation 293T-Spike cells for 24 h. **(****FIG. 20A****)** CD69 on CD4+ T-cells; **(****FIG. 20B****)** CD69 on CD8+ T-cells; **(****FIG. 20C****)** Ki67 on CD4+ T-cells; **(****FIG. 20D****)** Ki67 on CD8+ T-cells; **(****FIG. 20E****)** FasL on CD4+ T-cells; **(****FIG. 20F****)** FasL on CD8+ T-cells; **(****FIG. 20G****)** T-bet in CD4+ T-cells and **(****FIG. 20H****)** T-bet in CD8+ T-cells. Each of the S309-IgG4mut-28Z, 4A8-IgG4mut-28Z, CR3022-IgG4mut-28Z, and C135-IgG4mut-28Z showed an increased in expression on CD4 and CD8 T-cells. 4A8-IgG4mut-28Z showed the greatest increase in CD69 expression on both CD4 **(****FIG. 20A****)** and CD8 **(****FIG. 20B****)** T-cells. CR3022-IgG4mut-28Z and 4A8-IgG4mut-28Z showed the greatest increase in FasL on CD4 **(****FIG. 20E****)** and CD8 **(****FIG. 20F****)** T-cells. CR3022-IgG4mut-28Z and 4A8-IgG4mut-28Z showed the greatest increase in FasL on CD4 **(****FIG. 20E****)** and CD8 **(****FIG. 20F****)** T-cells. CR3022-IgG4mut-28Z and 4A8-IgG4mut-28Z showed the greatest increase in T-bet expression in CD4 **(****FIG. 20G****)** T-cells. Data are shown as mean ± SD of triplicate samples. ***p* < 0.01, ****p* < 0.001, *****p <* 0.0001

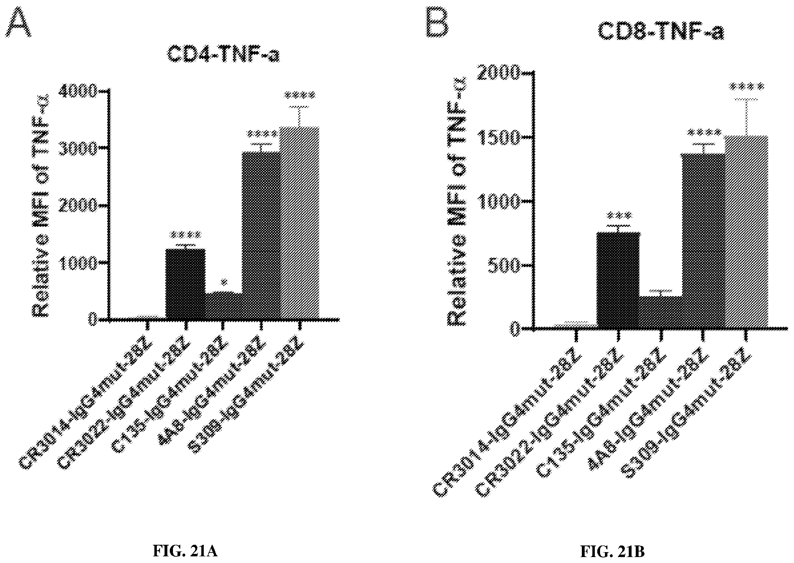

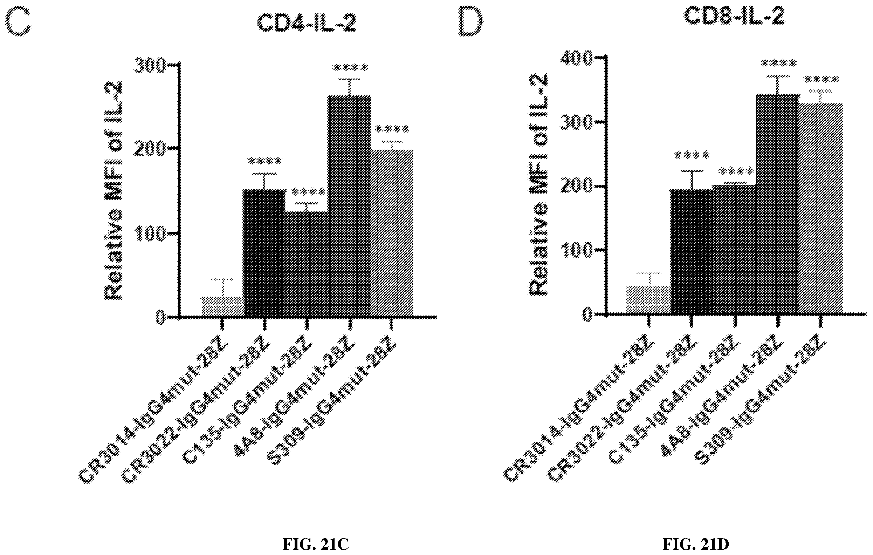

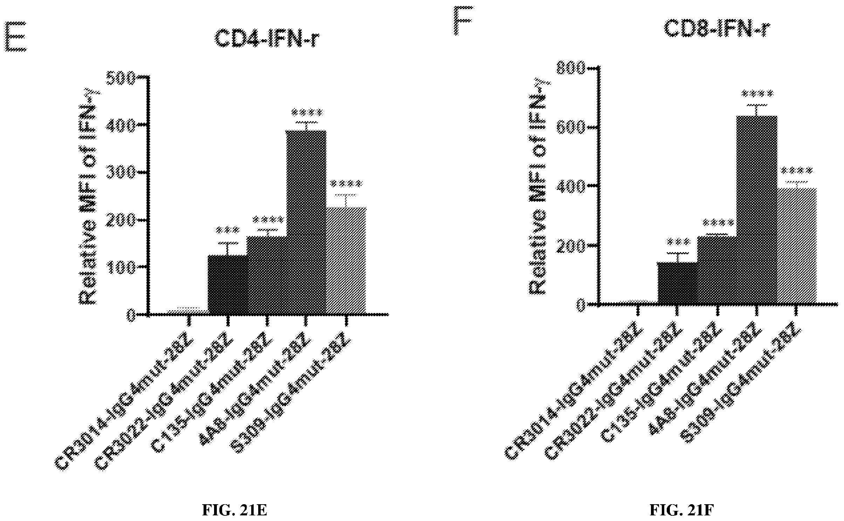

**FIGS. 21A-21F****: Histogram showing the expression of cytokines tumor necrosis factor TNF-a, interleukin 2 (IL-2) and interferon-gamma (IFN-gamma) in CAR-Ts.** Histogram showing expression in the different CAR-Ts after incubation 293T-Spike cells for 16 h. Each of the S309-IgG4mut-28Z, 4A8-IgG4mut-28Z, CR3022-IgG4mut-28Z, and C135-IgG4mut-28Z showed an increased in expression on CD4 and CD8 T-cells. **(****FIG. 21A****)** TNF-alpha on CD4+ T-cells; **(****FIG. 21B****)** TCF-alpha on CD8+ T-cells; **(****FIG. 21C****)** IL-2 on CD4+ T-cells; **(****FIG. 21D****)** IL-2 on CD8+ T-cells; **(****FIG. 21E****)** IFN-gamma on CD4+ T-cells; **(****FIG. 21F****)** IFN-gamma on CD8+ T-cells. 4A8-IgG4mut-28Z and S309-IgG4mut-28Z showed the greatest increase in TNF-alpha expression in CD4 **(****FIG. 21A****)** and CD8 **(****FIG. 21B****)** T-cells, IL-2 expression in CD4 **(****FIG. 21C****)** and CD8 **(****FIG. 21D****)** T-cells, IFN-gamma expression in CD4 **(****FIG. 21E****)** and CD8 **(****FIG. 21F****)** T-cells. Expectedly, 4A8-IgG4mut-28Z and S309-IgG4mut-28Z predominated in eliciting this cytokine response. Data are shown as mean ± SD of triplicate samples. **p <* 0.05, ****p <* 0.001, *****p <* 0.0001.

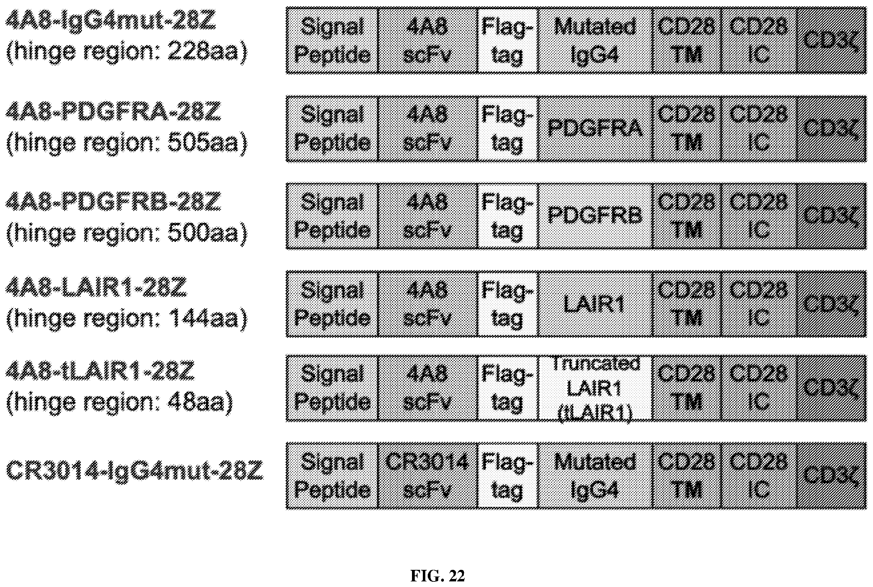

**FIG. 22****:** Diagram showing the design of 4A8 CAR-Ts with the IgG4, PDGFRA, PDGFRB, LAIR1 and tLAIR1 derived hinge regions. PDGFRA (505aa), PDGFRB (500aa), LAIR1 (144aa) and tLAIR1 (48aa) hinge regions.

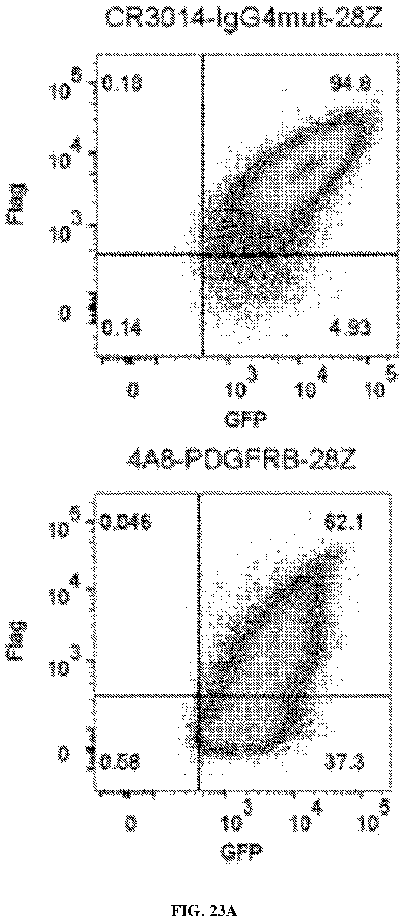

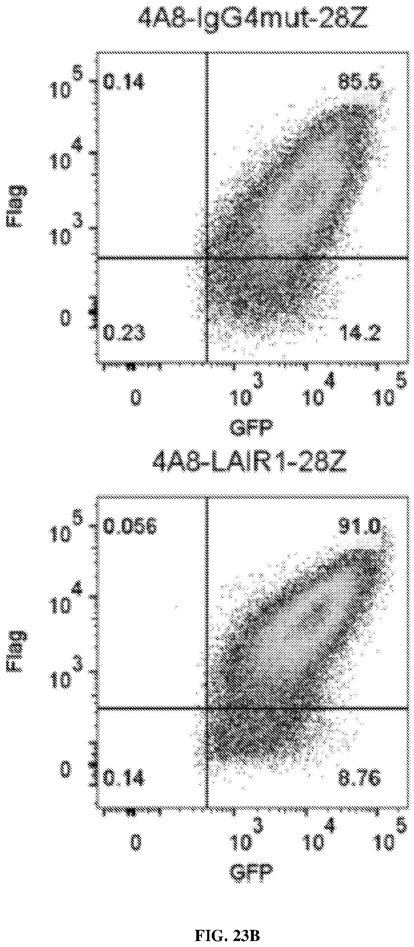

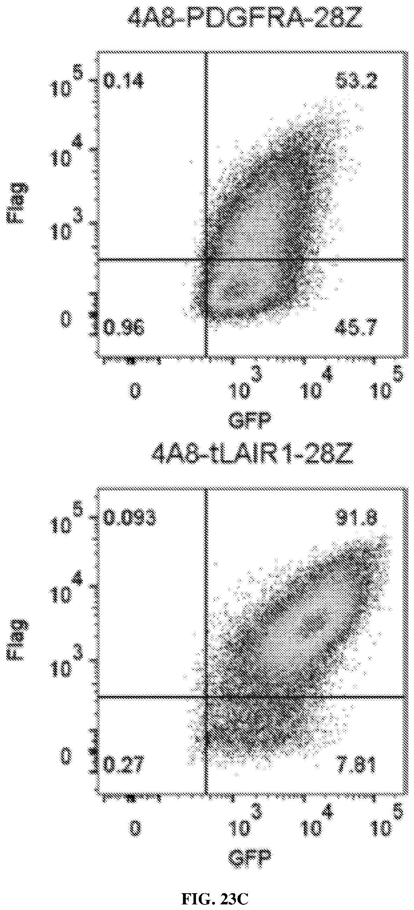

**FIGS. 23A-23C****: Surface expression of CAR-Ts with different hinge regions.** Human peripheral T-cells cells were transduced to express the individual CARs followed by culturing and expansion in vitro, cell sorting for CAR expression for flow cytometry and then a further expanded for several days in vitro prior to measure of expression by flow cytometry. CAR expression was detected by staining with PE tagged anti-Flag and the EGFP (n=2). The images show the profiles of individual CAR construct expression in T-cells. The data shows that the CR3014-IgG4mut-28Z, **(****FIG. 23A****,** upper panel); 4A8-PDGFA-28Z **(****FIG. 23A****,** lower panel); 4A8-IgG4mut-28Z **(****FIG. 23B****,** upper panel);, 4A8-LAIR1-28Z **(****FIG. 23B****,** lower panel), 4A8-PDGFA-28Z **(****FIG. 23C****,** upper panel) and 4A8-tLAIR1-28Z **(****FIG. 23C****,** lower panel) and were well expressed on the T-cells (i.e., >90% anti-Flag and GFP+). The data shows that the 4A8-PDGFA-28Z and 4A8-PDGFB-28Z were less well expressed, possibly due to the fact that the hinge regions and hence the CAR receptor was larger in size.

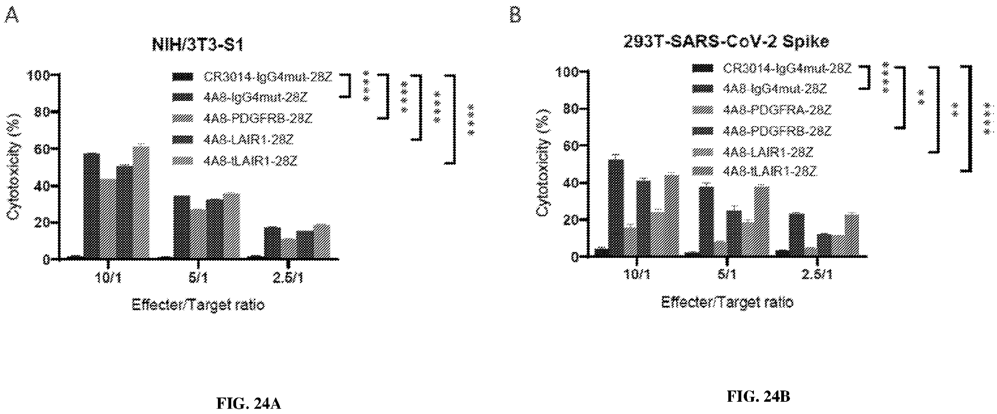

**FIGS. 24A-24B****: Different CAR-Ts kill target cells expressing S1 (****FIG. 24A****) or the entire Spike protein (****FIG. 24B****) transfected into NIH/3T3 (****FIG. 24A****) or 293T-cells (****FIG. 24A****).** Each of the 4A8-CAR-Ts were effective in killing targets expressing S1 **(****FIG. 24A****)** or the entire Spike **(****FIG. 24B****).** Unexpectedly, 4A8-tLAIR1 -28Z was the most efficient in killing the target expressing S1, significantly better than 4A8-IgG4mut-28Z and the other constructs at a ratio of 10/1 **(****FIG. 24A****).** 4A8-tLAIR1-28Z was also better in killing the target expressing S1 than 4A8-LAIR1-28Z and 4A8-PDGFB-28Z at all ratios (10/1, 5/1 and 2.5/1) **(****FIG. 24A****).** Unexpectedly, 4A8-PDGFB-28Z (despite lower expression levels), 4A8-IgG4mut-28Z and 4A8-tLAIR1-28Z were most effective in killing targets expressing the full-length Spike construct at a 10/1 effector/target ratio **(****FIG. 24B****).** 4A8-tLAIR1-28Z and 4A8-IgG4mut-28Z were the most effective at killing at all ratios **(****FIG. 24B****).**

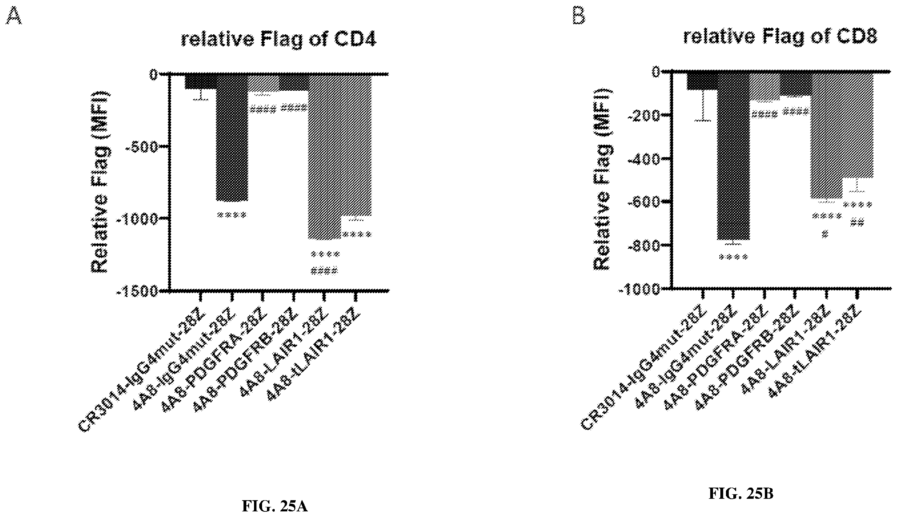

**FIGS. 25A-25B****: Downregulation of CAR-T receptors on CD4 and CD8 T-cells following engagement with SARs CoV2 Spike proteins.** CAR-Ts were co-cultured for 4 h with 293T-spike cells. The presence of surface CAR was assessed using a PE tagged anti-Flag antibody to label cells for flow cytometry. The 4A8-IgG4mut-28Z, 4A8-LAIR1-28Z and 4A8-tLAIR1-28Z were most effectively downregulated in CD4 **(****FIG. 25A****)** and CD8 **(****FIG. 25B****)** T-cells. It is possible that the enhanced killing by 4A8-PDGFB-28Z may be related to its longer retention time on the surface of T-cells. As a control, CR3014-IgG4mut-28Z showed no down-regulation. The effect was seen on both CD4 (left panel) and CD8+ (right panel) T-cells. Data are shown as mean ± SD of triplicate samples. **** *p* < 0.0001.

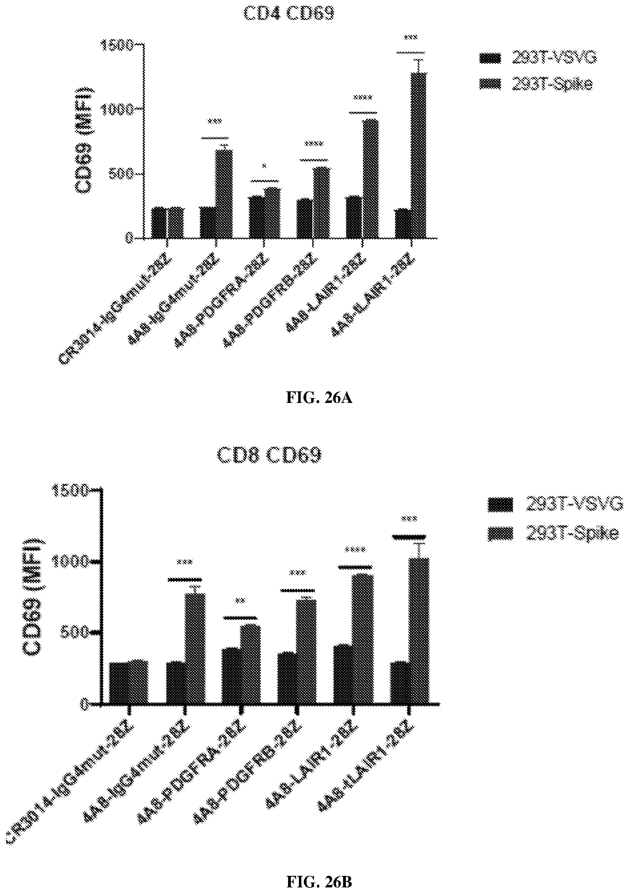

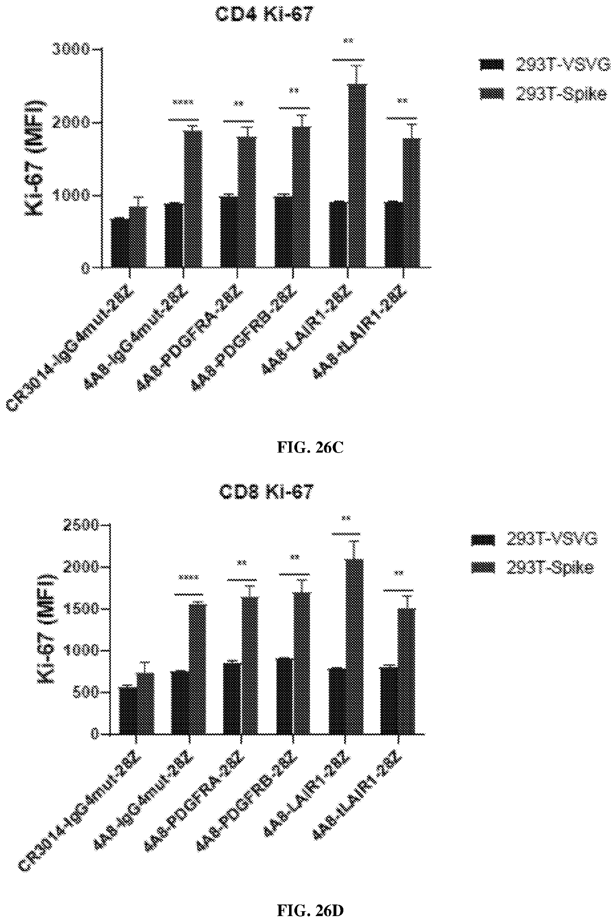

**FIGS. 26A-26D****: Histogram showing the induced expression of surface activation antigen CD69 and cell cycle antigen Ki67 on CD4 and CD8 expressing CAR-Ts.** Histogram shows the expression in the different CAR-Ts after incubation 293T-Spike or 293T-VSVG cells for 24 h. **(****FIG. 26A****)** CD69 on CD4+ T-cells; **(****FIG. 26B****)** CD69 on CD8+ T-cells; **(****FIG. 26C****)** Ki67 on CD4+ T-cells. 4A8-tLAIR1-28Z CAR-Ts showed the highest level of CD69 expression on CD4 **(****FIG. 26A****)** and CD8 **(****FIG. 26B****)** T-cells. LAIR1-28Z CAR-Ts showed the highest level of Ki67 expression on CD4 **(****FIG. 26C****)** and CD8 **(****FIG. 26D****)** T-cells. Nevertheless, each of the 4A8 CAR-Ts supported the induction of CD69 and Ki67 in comparing the response to 293T-Spike to 293T-VSVG control cells. As a control, CR3014-IgG4mut-28Z showed no induced expression in comparing the response to 293T-Spike to 293T-VSVG control cells. Data are shown as mean ± SD of triplicate samples. **p <* 0.05, ****p <* 0.001, *****p <* 0.0001.

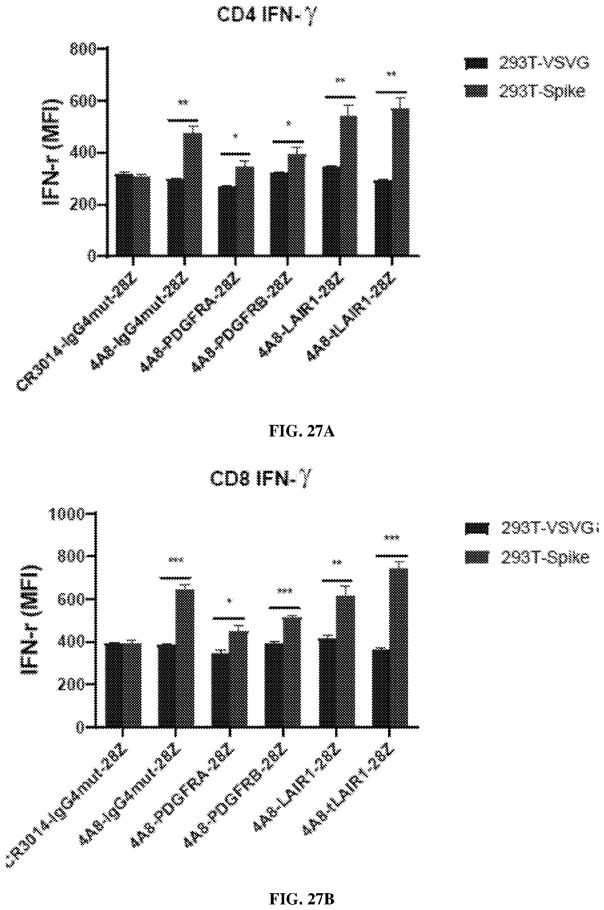

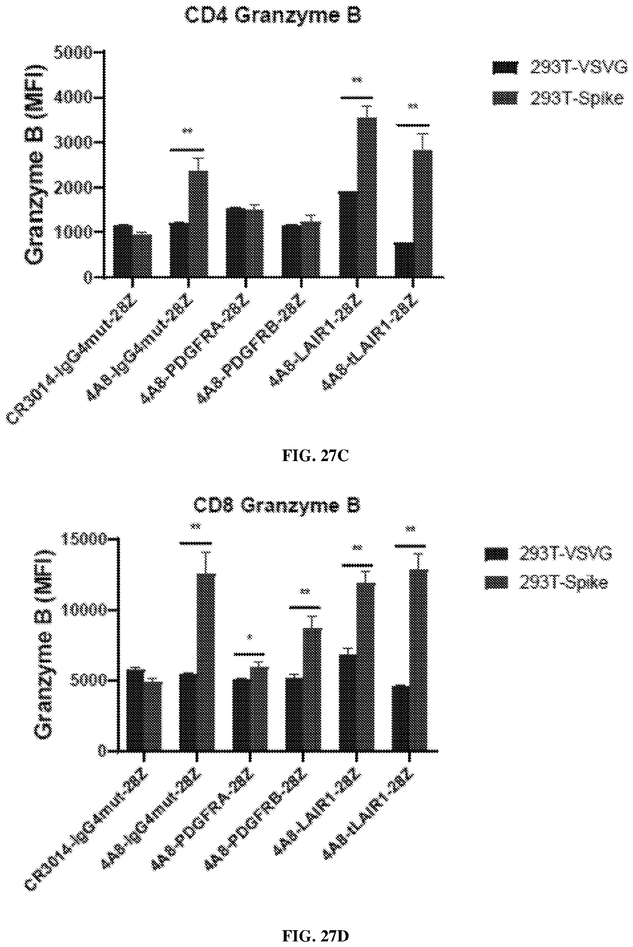

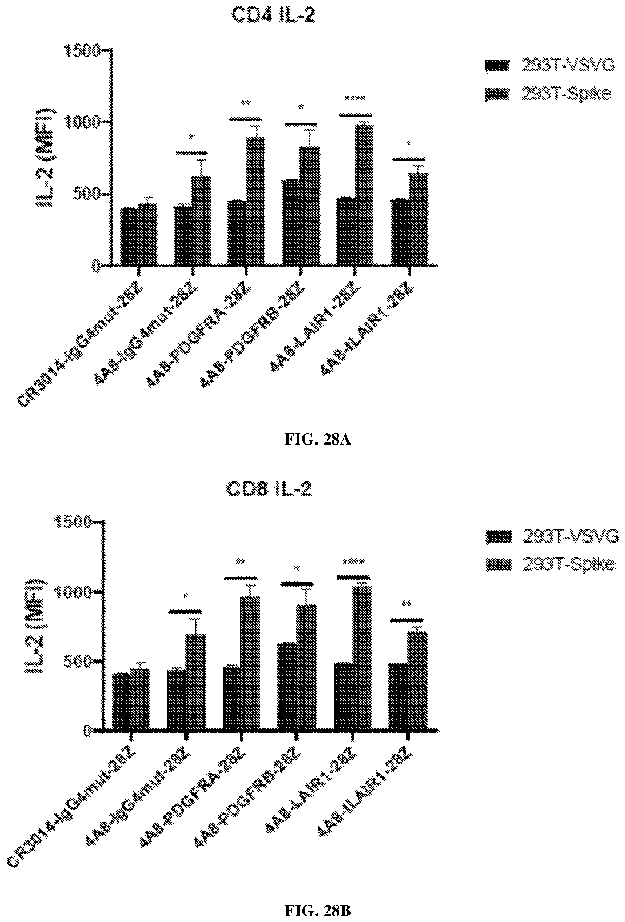

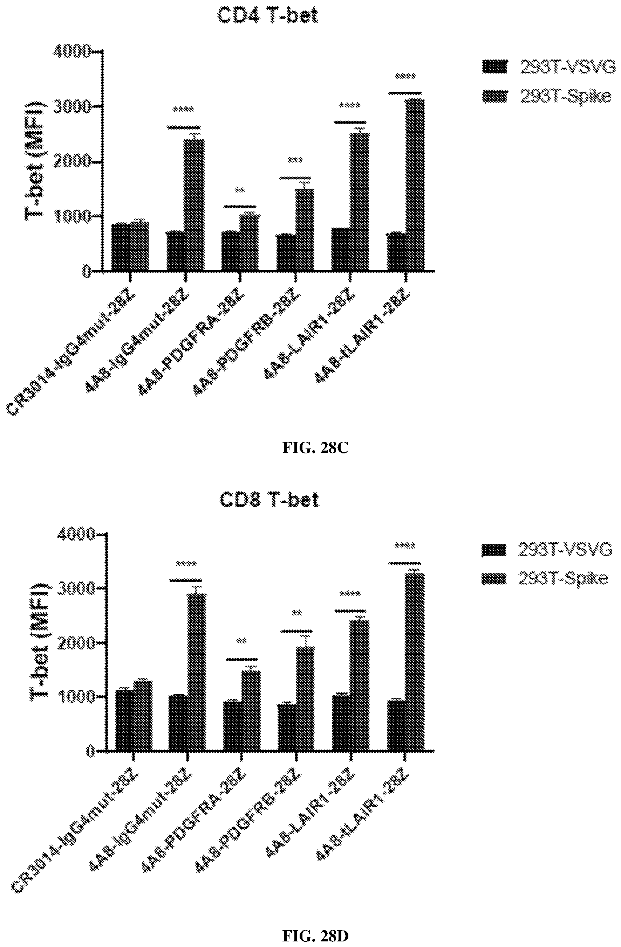

**FIGS. 27A-27D****: Histogram showing the induced expression of interferon-gamma (**FIGS. 27A**-B) and GZMB (**FIGS. 27C-D) in CD4+ (FIGS. A and C) and CD8 (**FIGS. 28** **B and D) CAR- T-cells.** Each of the CAR-Ts supported an increased in expression of both IFN-gamma and granzyme B (GZMB) on CD4 and CD8+ human T-cells in comparing the response to 293T-Spike to 293T-VSVG control cells. Unexpectedly, 4A8-IgG4mut-28Z, 4A8-LAIR1-28Z and 4A8-tLAIR1-28Z supported the induction of the highest levels of IFN-gamma expression. As a control, CR3014-IgG4mut-28Z showed no induced expression. As a control, CR3014-IgG4mut-28Z showed no induced expression in comparing the response to 293T-Spike to 293T-VSVG control cells. Data are shown as mean ± SD of triplicate samples. **p* < 0.05, ****p* < 0.001, *****p <* 0.0001.
**FIGS. 28A-28D****: Histogram showing the induction of interleukin 2 (IL-2) (****FIGS. 28A-28B****) and T-bet (****FIGS. 28C-28D****) in CD4+ (**FIGS. 28A **and C)** **and CD8 (**FIGS. 28Band D) CAR- T-cells. Each of the CAR-Ts supported an increased in expression of both IL-2 and T-bet on CD4 and CD8+ human T-cells in comparing the response to 293T-Spike to 293T-VSVG control cells. Unexpectedly, 4A8-PDGFA-28Z, 4A8-PDGFB-28Z and 4A8-LAIR1-28Z supported the induction of the highest levels of IL-2 expression. Unexpectedly, 4A8-IgG4mut-28Z, 4A8-LAIR1-28Z and 4A8-tLAIR1-28Z supported the induction of the highest levels of the expression of transcription factor T-bet (*Tbx21*) (C, D). As a control, CR3014-IgG4mut-28Z showed no induced expression in comparing the response to 293T-Spike to 293T-VSVG control cells. Data are shown as mean ± SD of triplicate samples. **p <* 0.05, ****p <* 0.001, *****p <* 0.0001.

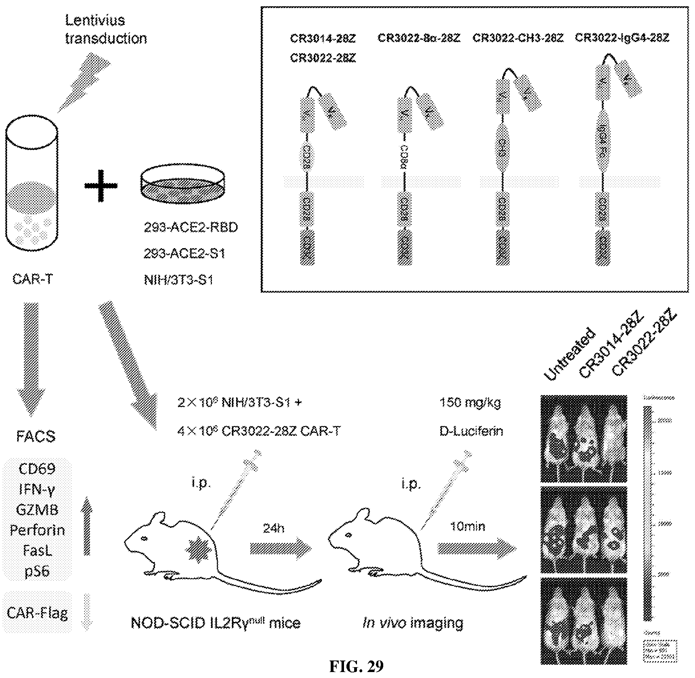

**FIG. 29****:** Exemplary therapeutic scheme for CAR-T treatment.

### DETAILED DESCRIPTION

### Definitions

As it would be understood, the section or subsection headings as used herein is for organizational purposes only and are not to be construed as limiting and/or separating the subject matter described.

Unless defined otherwise, all technical and scientific terms used herein have the same meanings as commonly understood by one of ordinary skill in the art to which this disclosure belongs.

The practice of the present disclosure will employ, unless otherwise indicated, conventional techniques of tissue culture, immunology, molecular biology, microbiology, cell biology and recombinant DNA, which are within the skill of t See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR 1: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Techique, 5th edition; Gait ed. (1984) Oligonucleotide Synthesis; U.S. Patent No. 4,683,195; Hames and Higgins eds. (1984) Nucleic Acid Hybridization; Anderson (1999) Nucleic Acid Hybridization; Hames and Higgins eds. (1984) Transcription and Translation; Immobilized Cells and Enzymes (IRL Press (1986)); Perbal (1984) A Practical Guide to Molecular Cloning; Miller and Calos eds. (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); Herzenberg et al. eds (1996) Weir's Handbook of Experimental Immunology; Manipulating the Mouse Embryo: A Laboratory Manual, 3rd edition (Cold Spring Harbor Laboratory Press (2002)); Sohail (ed.) (2004) Gene Silencing by RNA Interference: Technology and Application (CRC Press).

As used in the specification and claims, the singular form "a," "an" and "the" include plural references unless the context clearly dictates otherwise. For example, the term "a cell" includes a plurality of cells, including mixtures thereof.

As used herein, the term "comprising" is intended to mean that the compounds, compositions and methods include the recited elements, but not exclude others. "Consisting essentially of" when used to define compounds, compositions and methods, shall mean excluding other elements of any essential significance to the combination. Thus, a composition consisting essentially of the elements as defined herein would not exclude trace contaminants, e.g., from the isolation and purification method and pharmaceutically acceptable carriers, preservatives, and the like. "Consisting of" shall mean excluding more than trace elements of other ingredients or method steps. Embodiments defined by each of these transition terms are within the scope of this technology.

All numerical designations, e.g., pH, temperature, time, concentration, and molecular weight, including ranges, are approximations which are varied (+) or (-) by increments of 1.0 or 0.1, as appropriate or alternatively by a variation of +/- 15%, or alternatively 10% or alternatively 5% or alternatively 2%. It is to be understood, although not always explicitly stated, that all numerical designations are preceded by the term "about". It also is to be understood, although not always explicitly stated, that the reagents described herein are merely exemplary and that equivalents of such are known in the art.

As used herein, the term "about" is used to indicate that a value includes the standard deviation of error for the device or method being employed to determine the value. The term "about" when used before a numerical designation, e.g., temperature, time, amount, and concentration, including range, indicates approximations which may vary by (+) or (-) 15%, 10%, 5%, 3%, 2%, or 1 %.

As used herein, comparative terms as used herein, such as high, low, increase, decrease, reduce, or any grammatical variation thereof, can refer to certain variation from the reference. In some embodiments, such variation can refer to about 10%, or about 20%, or about 30%, or about 40%, or about 50%, or about 60%, or about 70%, or about 80%, or about 90%, or about 1 fold, or about 2 folds, or about 3 folds, or about 4 folds, or about 5 folds, or about 6 folds, or about 7 folds, or about 8 folds, or about 9 folds, or about 10 folds, or about 20 folds, or about 30 folds, or about 40 folds, or about 50 folds, or about 60 folds, or about 70 folds, or about 80 folds, or about 90 folds, or about 100 folds or more higher than the reference. In some embodiments, such variation can refer to about 1%, or about 2%, or about 3%, or about 4%, or about 5%, or about 6%, or about 7%, or about 8%, or about 0%, or about 10%, or about 20%, or about 30%, or about 40%, or about 50%, or about 60%, or about 70%, or about 75%, or about 80%, or about 85%, or about 90%, or about 95%, or about 96%, or about 97%, or about 98%, or about 99% of the reference.

As will be understood by one skilled in the art, for any and all purposes, all ranges disclosed herein also encompass any and all possible subranges and combinations of subranges thereof. Furthermore, as will be understood by one skilled in the art, a range includes each individual member.

"Optional" or "optionally" means that the subsequently described circumstance may or may not occur, so that the description includes instances where the circumstance occurs and instances where it does not.

As used herein, "and/or" refers to and encompasses any and all possible combinations of one or more of the associated listed items, as well as the lack of combinations when interpreted in the alternative ("or").

"Substantially" or "essentially" means nearly totally or completely, for instance, 95% or greater of some given quantity. In some embodiments, "substantially" or "essentially" means 95%, 96%, 97%, 98%, 99%, 99.5%, or 99.9%.

The terms or "acceptable," "effective," or "sufficient" when used to describe the selection of any components, ranges, dose forms, etc. disclosed herein intend that said component, range, dose form, etc. is suitable for the disclosed purpose.

The term "isolated" as used herein with respect to nucleic acids, such as DNA or RNA, refers to molecules separated from other DNAs or RNAs, respectively that are present in the natural source of the macromolecule. The term "isolated nucleic acid" is meant to include nucleic acid fragments which are not naturally occurring as fragments and would not be found in the natural state. The term "isolated" is also used herein to refer to polypeptides, proteins and/or host cells that are isolated from other cellular proteins and is meant to encompass both purified and recombinant polypeptides. In other embodiments, the term "isolated" means separated from constituents, cellular and otherwise, in which the cell, tissue, polynucleotide, peptide, polypeptide, protein, antibody or fragment(s) thereof, which are normally associated in nature. For example, an isolated cell is a cell that is separated form tissue or cells of dissimilar phenotype or genotype. As is apparent to those of skill in the art, a non-naturally occurring polynucleotide, peptide, polypeptide, protein, antibody or fragment(s) thereof, does not require "isolation" to distinguish it from its naturally occurring counterpart.

In some embodiments, the term "engineered" or "recombinant" refers to having at least one modification not normally found in a naturally occurring protein, polypeptide, polynucleotide, strain, wild-type strain or the parental host strain of the referenced species. In some embodiments, the term "engineered" or "recombinant" refers to being synthetized by human intervention. As used herein, the term "recombinant protein" refers to a polypeptide which is produced by recombinant DNA techniques, wherein generally, DNA encoding the polypeptide is inserted into a suitable expression vector which is in turn used to transform a host cell to produce the heterologous protein.

The terms "polynucleotide", "nucleic acid" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, DNA, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this disclosure that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

The expression "amplification of polynucleotides" includes methods such as PCR, ligation amplification (or ligase chain reaction, LCR) and amplification methods. These methods are known and widely practiced in the art. See, e.g., U.S. Pat. Nos. 4,683,195 and 4,683,202 and Innis et al., 1990 (for PCR); and Wu et al. (1989) Genomics 4:560-569 (for LCR). In general, the PCR procedure describes a method of gene amplification which is comprised of (i) sequence-specific hybridization of primers to specific genes within a DNA sample (or library), (ii) subsequent amplification involving multiple rounds of annealing, elongation, and denaturation using a DNA polymerase, and (iii) screening the PCR products for a band of the correct size. The primers used are oligonucleotides of sufficient length and appropriate sequence to provide initiation of polymerization, i.e. each primer is specifically designed to be complementary to each strand of the genomic locus to be amplified.

Reagents and hardware for conducting PCR are commercially available. Primers useful to amplify sequences from a particular gene region are preferably complementary to, and hybridize specifically to sequences in the target region or its flanking regions. Nucleic acid sequences generated by amplification may be sequenced directly. Alternatively, the amplified sequence(s) may be cloned prior to sequence analysis. A method for the direct cloning and sequence analysis of enzymatically amplified genomic segments is known in the art.

A "gene" refers to a polynucleotide containing at least one open reading frame (ORF) that is capable of encoding a particular polypeptide or protein after being transcribed and translated.

The term "express" refers to the production of a gene product, such as mRNA, peptides, polypeptides or proteins. As used herein, "expression" refers to the process by which polynucleotides are transcribed into mRNA or the process by which the transcribed mRNA is subsequently being translated into peptides, polypeptides, or proteins. If the polynucleotide is derived from genomic DNA, expression may include splicing of the mRNA in a eukaryotic cell.

A "gene product" or alternatively a "gene expression product" refers to the amino acid (e.g., peptide or polypeptide) generated when a gene is transcribed and translated. In some embodiments, the gene product may refer to an mRNA or other RNA, such as an interfering RNA, generated when a gene is transcribed.

The term "encode" as it is applied to polynucleotides refers to a polynucleotide which is said to "encode" a polypeptide if, in its native state or when manipulated by methods well known to those skilled in the art, it can be transcribed to produce the mRNA for the polypeptide or a fragment thereof, and optionally translated to produce the polypeptide or a fragment thereof. The antisense strand is the complement of such a nucleic acid, and the encoding sequence can be deduced therefrom. Further, as used herein an amino acid sequence coding sequence refers to a nucleotide sequence encoding the amino acid sequence.

As used herein, "complementary" sequences refer to two nucleotide sequences which, when aligned anti-parallel to each other, contain multiple individual nucleotide bases which pair with each other. Paring of nucleotide bases forms hydrogen bonds and thus stabilizes the double strand structure formed by the complementary sequences. It is not necessary for every nucleotide base in two sequences to pair with each other for sequences to be considered "complementary". Sequences may be considered complementary, for example, if at least 30%, 40%, 50%, 55%, 60%, 65%, 70%, 75%, 80%, 85%, 90%, 95%, 98%, 99%, or 100% of the nucleotide bases in two sequences pair with each other. In some embodiments, the term complementary refers to 100% of the nucleotide bases in two sequences pair with each other. In addition, sequences may still be considered "complementary" when the total lengths of the two sequences are significantly different from each other. For example, a primer of 15 nucleotides may be considered "complementary" to a longer polynucleotide containing hundreds of nucleotides if multiple individual nucleotide bases of the primer pair with nucleotide bases in the longer polynucleotide when the primer is aligned anti-parallel to a particular region of the longer polynucleotide. Nucleotide bases paring is known in the field, such as in DNA, the purine adenine (A) pairs with the pyrimidine thymine (T) and the pyrimidine cytosine (C) always pairs with the purine guanine (G); while in RNA, adenine (A) pairs with uracil (U) and guanine (G) pairs with cytosine (C). Further, the nucleotide bases aligned anti-parallel to each other in two complementary sequences, but not a pair, are referred to herein as a mismatch.

"Under transcriptional control", which is also used herein as "directing expression of", is a term well understood in the art and indicates that transcription of a polynucleotide sequence, usually a DNA sequence, depends on its being operatively linked to an element which contributes to the initiation of, or promotes, transcription. "Operatively linked" intends the polynucleotides are arranged in a manner that allows them to function in a cell.

The term "a regulatory sequence", "an expression control element" or "promoter" as used herein, intends a polynucleotide that is operatively linked to a target polynucleotide to be transcribed or replicated, and facilitates the expression or replication of the target polynucleotide. A promoter is an example of an expression control element or a regulatory sequence. Promoters can be located 5' or upstream of a gene or other polynucleotide, that provides a control point for regulated gene transcription. Polymerase II and III are examples of promoters. In some embodiments, a regulatory sequence is bidirectional, i.e., acting as a regulatory sequence for the coding sequences on both sides of the regulatory sequence. Such bidirectional regulatory sequence may comprises, or consists essentially of, or consists of a bidirectional promoter (see for example Trinklein ND, et al. An abundance of bidirectional promoters in the human genome. Genome Res. 2004 Jan; 14(1):62-6).

The term "promoter" as used herein refers to any sequence that regulates the expression of a coding sequence, such as a gene. Promoters may be constitutive, inducible, repressible, or tissue-specific, for example. A "promoter" is a control sequence that is a region of a polynucleotide sequence at which initiation and rate of transcription are controlled. It may contain genetic elements at which regulatory proteins and molecules may bind such as RNA polymerase and other transcription factors. Non-limiting examples of promoters include the EF1alpha promoter and the CMV promoter. The EF1alpha sequence is known in the art (see, e.g., addgene.org/11154/sequences/; ncbi.nlm.nih.gov/nuccore/J04617, each last accessed on March 13, 2019, and Zheng and Baum (2014) Int'l. J. Med. Sci. 11(5):404-408). The CMV promoter sequence is known in the art (see, e.g., snapgene.com/resources/plasmid-files/?set=basic_cloning_vectors&plasmid=CMV_promoter, last accessed on March 13, 2019 and Zheng and Baum (2014), supra.).

An enhancer is a regulatory element that increases the expression of a target sequence. A "promoter/enhancer" is a polynucleotide that contains sequences capable of providing both promoter and enhancer functions. For example, the long terminal repeats of retroviruses contain both promoter and enhancer functions. The enhancer/promoter may be "endogenous" or "exogenous" or "heterologous." An "endogenous" enhancer/promoter is one which is naturally linked with a given gene in the genome. An "exogenous" or "heterologous" enhancer/promoter is one which is placed in juxtaposition to a gene by means of genetic manipulation (i.e., molecular biological techniques) such that transcription of that gene is directed by the linked enhancer/promoter.

As used herein, the term "enhancer", as used herein, denotes sequence elements that augment, improve or ameliorate transcription of a nucleic acid sequence irrespective of its location and orientation in relation to the nucleic acid sequence to be expressed. An enhancer may enhance transcription from a single promoter or simultaneously from more than one promoter. As long as this functionality of improving transcription is retained or substantially retained (e.g., at least 70%, at least 80%, at least 90% or at least 95% of wild-type activity, that is, activity of a full-length sequence), any truncated, mutated or otherwise modified variants of a wild-type enhancer sequence are also within the above definition.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Hybridization reactions can be performed under conditions of different "stringency". In general, a low stringency hybridization reaction is carried out at about 40 °C in 10 x SSC or a solution of equivalent ionic strength/temperature. A moderate stringency hybridization is typically performed at about 50 °C in 6 x SSC, and a high stringency hybridization reaction is generally performed at about 60 °C in 1 x SSC. Hybridization reactions can also be performed under "physiological conditions" which is well known to one of skill in the art. A non-limiting example of a physiological condition is the temperature, ionic strength, pH and concentration of Mg2+ normally found in a cell.

When hybridization occurs in an antiparallel configuration between two single-stranded polynucleotides, the reaction is called "annealing" and those polynucleotides are described as "complementary." A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to form hydrogen bonding with each other, according to generally accepted base-pairing rules.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present disclosure.

A polynucleotide or polynucleotide region (or a polypeptide or polypeptide region) has a certain percentage (for example, 70%, 75%, 80%, 85%, 90%, 95%, 98% or 99%) of "sequence identity" to another sequence means that, when aligned, that percentage of bases (or amino acids) are the same in comparing the two sequences. This alignment and the percent homology or sequence identity can be determined using software programs known in the art, for example, those described in Ausubel et al. eds. (2007) Current Protocols in Molecular Biology. Preferably, default parameters are used for alignment. One alignment program is BLAST, using default parameters. In particular, programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: www.ncbi.nlm.nih.gov/cgi-bin/BLAST.

In some embodiments, the polynucleotide as disclosed herein is a RNA. In some embodiments, the polynucleotide as disclosed herein is a DNA. In some embodiments, the polynucleotide as disclosed herein is a hybrid of DNA and RNA.

In some embodiments, an equivalent to a reference nucleic acid, polynucleotide or oligonucleotide encodes the same sequence encoded by the reference. In some embodiments, an equivalent to a reference nucleic acid, polynucleotide or oligonucleotide hybridizes to the reference, a complement reference, a reverse reference, or a reverse-complement reference, optionally under conditions of high stringency.

Additionally or alternatively, an equivalent nucleic acid, polynucleotide or oligonucleotide is one having at least 70% sequence identity, or at least 75% sequence identity, or at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence, or alternatively at least 99 % sequence identity to the reference nucleic acid, polynucleotide, or oligonucleotide, or alternatively an equivalent nucleic acid hybridizes under conditions of high stringency to a reference polynucleotide or its complement. In one aspect, the equivalent must encode the same protein or a functional equivalent of the protein that optionally can be identified through one or more assays described herein. In addition or alternatively, the equivalent of a polynucleotide would encode a protein or polypeptide of the same or similar function as the reference or parent polynucleotide.

The term "transduce" or "transduction" or "introduce" as it is applied to the production of cells, such as chimeric antigen receptor cells, refers to the process whereby a foreign nucleotide sequence is introduced into a cell. In some embodiments, this transduction is done via a vector, viral or non-viral.

The term "suicide gene" refers to any gene that when activated, will induce apoptotic death of the cell in which the gene was activated. This pathway is most frequently induced through the p53 protein. Targeting suicide genes to cancerous cells is an attractive possibility for treatment.

The term "spike protein" (S protein) refers to a structural protein critical for the infectivity of SARS-CoV2 (COVID-19). Many copies of this protein exist on the outside of a SARS-CoV2 virion and bind to the host cell receptor angio-tension converting enzyme 2 (ACE2). In nature, this protein is glycosylated, but sometimes will lack glycosylation in a laboratory setting.

A "plasmid" is an extra-chromosomal DNA molecule separate from the chromosomal DNA which is capable of replicating independently of the chromosomal DNA. In many cases, it is circular and double-stranded. Plasmids provide a mechanism for horizontal gene transfer within a population of microbes and typically provide a selective advantage under a given environmental state. Plasmids may carry genes that provide resistance to naturally occurring antibiotics in a competitive environmental niche, or alternatively the proteins produced may act as toxins under similar circumstances.

"Plasmids" used in genetic engineering are called "plasmid vectors". Many plasmids are commercially available for such uses. The gene to be replicated is inserted into copies of a plasmid containing genes that make cells resistant to particular antibiotics and a multiple cloning site (MCS, or polylinker), which is a short region containing several commonly used restriction sites allowing the easy insertion of DNA fragments at this location. Another major use of plasmids is to make large amounts of proteins. In this case, researchers grow bacteria containing a plasmid harboring the gene of interest. Just as the bacterium produces proteins to confer its antibiotic resistance, it can also be induced to produce large amounts of proteins from the inserted gene. This is a cheap and easy way of mass-producing a gene or the protein it then codes for.

A "yeast artificial chromosome" or "YAC" refers to a vector used to clone large DNA fragments (larger than 100 kb and up to 3000 kb). It is an artificially constructed chromosome and contains the telomeric, centromeric, and replication origin sequences needed for replication and preservation in yeast cells. Built using an initial circular plasmid, they are linearized by using restriction enzymes, and then DNA ligase can add a sequence or gene of interest within the linear molecule by the use of cohesive ends. Yeast expression vectors, such as YACs, YIps (yeast integrating plasmid), and YEps (yeast episomal plasmid), are extremely useful as one can get eukaryotic protein products with posttranslational modifications as yeasts are themselves eukaryotic cells, however YACs have been found to be more unstable than BACs, producing chimeric effects.

A "viral vector" is defined as a recombinantly produced virus or viral particle that comprises a polynucleotide to be delivered into a host cell, either in vivo, ex vivo or in vitro. Examples of viral vectors include retroviral vectors, adenovirus vectors, adeno-associated virus vectors, alphavirus vectors and the like. Infectious tobacco mosaic virus (TMV)-based vectors can be used to manufacturer proteins and have been reported to express Griffithsin in tobacco leaves (O'Keefe et al. (2009) Proc. Nat. Acad. Sci. USA 106(15):6099-6104). Alphavirus vectors, such as Semliki Forest virus-based vectors and Sindbis virus-based vectors, have also been developed for use in gene therapy and immunotherapy. See, Schlesinger & Dubensky (1999) Curr. Opin. Biotechnol. 5:434-439 and Ying et al. (1999) Nat. Med. 5(7):823-827. In aspects where gene transfer is mediated by a retroviral vector, a vector construct refers to the polynucleotide comprising the retroviral genome or part thereof, and a therapeutic gene.

As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus.

In aspects where gene transfer is mediated by a DNA viral vector, such as an adenovirus (Ad) or adeno-associated virus (AAV), a vector construct refers to the polynucleotide comprising the viral genome or part thereof, and a transgene. Adenoviruses (Ads) are a relatively well characterized, homogenous group of viruses, including over 50 serotypes. See, e.g., PCT International Application Publication No. WO 95/27071. Ads do not require integration into the host cell genome. Recombinant Ad derived vectors, particularly those that reduce the potential for recombination and generation of wild-type virus, have also been constructed. See, PCT International Application Publication Nos. WO 95/00655 and WO 95/11984, Wild-type AAV has high infectivity and specificity integrating into the host cell's genome. See, Hermonat & Muzyczka (1984) Proc. Natl. Acad. Sci. USA 81:6466-6470 and Lebkowski et al. (1988) Mol. Cell. Biol. 8:3988-3996.

Vectors that contain both a promoter and a cloning site into which a polynucleotide can be operatively linked are well known in the art. Such vectors are capable of transcribing RNA in vitro or in vivo, and are commercially available from sources such as Stratagene (La Jolla, Calif.) and Promega Biotech (Madison, Wis.). In order to optimize expression and/or in vitro transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include DNA/liposome complexes, micelles and targeted viral protein-DNA complexes. Liposomes that also comprise a targeting antibody or fragment thereof can be used in the methods disclosed herein. In addition to the delivery of polynucleotides to a cell or cell population, direct introduction of the proteins described herein to the cell or cell population can be done by the non-limiting technique of protein transfection, alternatively culturing conditions that can enhance the expression and/or promote the activity of the proteins disclosed herein are other non-limiting techniques.

The term "protein", "peptide" and "polypeptide" are used interchangeably and in their broadest sense to refer to a compound of two or more subunit amino acids, amino acid analogs or peptidomimetics. The subunits (which are also referred to as residues) may be linked by peptide bonds. In another embodiment, the subunit may be linked by other bonds, e.g., ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics.

As used herein, the term "antibody" collectively refers to immunoglobulins or immunoglobulin-like molecules including by way of example and without limitation, IgA, IgD, IgE, IgG and IgM, combinations thereof, and similar molecules produced during an immune response in any vertebrate, for example, in mammals such as humans, goats, rabbits and mice, as well as non-mammalian species, such as shark immunoglobulins. Unless specifically noted otherwise, the term "antibody" includes intact immunoglobulins and "antibody fragments" or "antigen binding fragments" that specifically bind to a molecule of interest (or a group of highly similar molecules of interest) to the substantial exclusion of binding to other molecules (for example, antibodies and antibody fragments that have a binding constant for the molecule of interest that is at least 10³ M⁻¹ greater, at least 10⁴ M⁻¹ greater or at least 10⁵ M⁻¹ greater than a binding constant for other molecules in a biological sample). The term "antibody" also includes genetically engineered forms such as chimeric antibodies (for example, murine or humanized non-primate antibodies), heteroconjugate antibodies (such as, bispecific antibodies). See also, Pierce Catalog and Handbook, 1994-1995 (Pierce Chemical Co., Rockford, Ill.); Owen et al., Kuby Immunology, 7th Ed., W.H. Freeman & Co., 2013; Murphy, Janeway's Immunobiology, 8th Ed., Garland Science, 2014; Male et al., Immunology (Roitt), 8th Ed., Saunders, 2012; Parham, The Immune System, 4th Ed., Garland Science, 2014. In some embodiments, the term "antibody" refers to a single-chain variable fragment (scFv, or ScFV). In some embodiments, the term "antibody" refers to more than one single-chain variable fragments (scFv, or ScFV) linked with each other, optionally via a peptide linker or another suitable component as disclosed herein. In some embodiments, an antibody is a monoclonal antibody. In some embodiments, an antibody is a monospecific antibody or a multispecific antibody, such as a bispecific antibody or a trispecific antibody. The species of the antibody can be a human or non-human, e.g., mammalian.

As used herein, the term "monoclonal antibody" refers to an antibody produced by a single clone of B-lymphocytes or by a cell into which the light and heavy chain genes of a single antibody have been transfected. Monoclonal antibodies are produced by methods known to those of skill in the art, for instance by making hybrid antibody-forming cells from a fusion of myeloma cells with immune spleen cells. Monoclonal antibodies include humanized monoclonal antibodies.

In terms of antibody structure, an immunoglobulin has heavy (H) chains and light (L) chains interconnected by disulfide bonds. There are two types of light chain, lambda (λ) and kappa (κ). There are five main heavy chain classes (or isotypes) which determine the functional activity of an antibody molecule: IgM, IgD, IgG, IgA and IgE. Each heavy and light chain contains a constant region and a variable region, (the regions are also known as "domains"). In combination, the heavy and the light chain variable regions specifically bind the antigen. Light and heavy chain variable regions contain a "framework" region interrupted by three hypervariable regions, also called "complementarity-determining regions" or "CDRs". The extent of the framework region and CDRs have been defined (see, Kabat et al., Sequences of Proteins of Immunological Interest, US Department of Health and Human Services, 1991). The Kabat database is now maintained online. The sequences of the framework regions of different light or heavy chains are relatively conserved within a species. The framework region of an antibody, that is the combined framework regions of the constituent light and heavy chains, largely adopts a β-sheet conformation and the CDRs form loops which connect, and in some cases form part of, the β-sheet structure. Thus, framework regions act to form a scaffold that provides for positioning the CDRs in correct orientation by inter-chain, non-covalent interactions.

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located (heavy chain regions labeled CDRH and light chain regions labeled CDRL). Thus, a CDRH3 is the CDR3 from the variable domain of the heavy chain of the antibody in which it is found, whereas a CDRL1 is the CDR1 from the variable domain of the light chain of the antibody in which it is found. For example, an anti-BCMA antibody will have a specific VH region and the VL region sequence unique to the BCMA relevant antigen, and thus specific CDR sequences. Antibodies with different specificities (i.e., different combining sites for different antigens) have different CDRs. Although it is the CDRs that vary from antibody to antibody, only a limited number of amino acid positions within the CDRs are directly involved in antigen binding. These positions within the CDRs are called specificity determining residues (SDRs).

As used herein, a single-chain variable fragment (scFv or ScFV), also referred to herein as a fragment of an antibody, and is a fusion protein of the variable regions of the heavy (V_{H}) and light chains (V_{L}) of immunoglobulins, optionally connected with a short linker peptide of about 10 to about 25 amino acids. The linker is usually rich in glycine for flexibility, as well as serine or threonine for solubility, and can either connect the N-terminus of the V_{H} with the C-terminus of the V_{L}, or vice versa. This protein retains the specificity of the original immunoglobulin, despite removal of the constant regions and the introduction of the linker.

The polypeptide or an equivalent thereof, can be followed by an additional 50 amino acids, or alternatively about 40 amino acids, or alternatively about 30 amino acids, or alternatively about 20 amino acids, or alternatively about 10 amino acids, or alternatively about 5 amino acids, or alternatively about 4, or 3, or 2 or 1 amino acids at the carboxy-terminus (C-terminus). Additionally or alternatively, the polypeptide or an equivalent thereof can further comprises an additional 50 amino acids, or alternatively about 40 amino acids, or alternatively about 30 amino acids, or alternatively about 20 amino acids, or alternatively about 10 amino acids, or alternatively about 5 amino acids, or alternatively about 4, or 3, or 2 or 1 amino acids at the amine-terminus (N-terminus).

An equivalent of a reference polypeptide comprises, consists essentially of, or alternatively consists of an polypeptide having at least 80% amino acid identity to the reference polypeptide, such as the CAR as disclosed herein, or a polypeptide that is encoded by a polynucleotide that hybridizes under conditions of high stringency to the complement of a polynucleotide encoding the reference polypeptide, such as a CAR as disclosed herein, wherein conditions of high stringency comprises incubation temperatures of about 55°C to about 68°C; buffer concentrations of about 1x SSC to about 0.1x SSC; formamide concentrations of about 55% to about 75%; and wash solutions of about 1x SSC, 0.1x SSC, or deionized water.

Alternative embodiments include one or more of the CDRs (e.g., CDR1, CDR2, CDR3) from the LC variable region substituted with appropriate CDRs from other antibody CDRs, or an equivalent of each thereof. Accordingly, and as an example, the CDR1 and CDR2 from the LC variable region can be combined with the CDR3 of another antibody's LC variable region, and in some aspects, can include an additional 50 amino acids, or alternatively about 40 amino acids, or alternatively about 30 amino acids, or alternatively about 20 amino acids, or alternatively about 10 amino acids, or alternatively about 5 amino acids, or alternatively about 4, or 3, or 2 or 1 amino acids at the carboxy-terminus.

In some embodiments, the term "equivalent" or "biological equivalent" of an antibody means the ability of the antibody to selectively bind its epitope protein or a fragment thereof as measured by ELISA or other suitable methods is substantively maintained, for example, at a level of at least 50%, or at least 55%, or at least 60%, or at least 65%, or at least 70%, or at least 75%, or at least 80%, or at least 85%, or at least 90%, or at least 95%, or at least 99%, or more. Biologically equivalent antibodies include, but are not limited to, those antibodies, peptides, antibody fragments, antibody variant, antibody derivative and antibody mimetics that bind to the same epitope as the reference antibody. Additionally or alternatively, the equivalent and the reference antibody shares the same set of CDRs but other amino acids are modified.

It is to be inferred without explicit recitation and unless otherwise intended, that when the present disclosure relates to a polypeptide, protein, polynucleotide or antibody, an equivalent or a biologically equivalent of such is intended within the scope of this disclosure. As used herein, the term "biological equivalent thereof" is intended to be synonymous with "equivalent thereof" when referring to a reference protein, antibody, polypeptide or nucleic acid, intends those having minimal homology while still maintaining desired structure or functionality. Unless specifically recited herein, it is contemplated that any polynucleotide, polypeptide or protein mentioned herein also includes equivalents thereof. For example, an equivalent intends at least about 70% homology or identity, or at least 80 % homology or identity, or at least about 85 % homology or identity, or alternatively at least about 90 % homology or identity, or alternatively at least about 95 % homology or identity, or alternatively 98 % homology or identity and exhibits substantially equivalent biological activity to the reference protein, polypeptide or nucleic acid. Alternatively, when referring to polynucleotides, an equivalent thereof is a polynucleotide that hybridizes under stringent conditions to the reference polynucleotide or its complement.

As used herein, the term "specific binding" or "binding" means the contact between an antibody and an antigen with a binding affinity of at least 10⁻⁶ M. In certain embodiments, antibodies bind with affinities of at least about 10⁻⁷ M, and preferably at least about 10⁻⁸ M, at least about 10⁻⁹ M, at least about 10⁻¹⁰ M, at least about 10⁻¹¹ M, or at least about 10⁻¹² M.

As used herein, the term "antigen" refers to a compound, composition, or substance that may be specifically bound by the products of specific humoral or cellular immunity, such as an antibody molecule or T-cell receptor. Antigens can be any type of molecule including, for example, haptens, simple intermediary metabolites, sugars (e.g., oligosaccharides), lipids, and hormones as well as macromolecules such as complex carbohydrates (e.g., polysaccharides), phospholipids, and proteins. Common categories of antigens include, but are not limited to, viral antigens, bacterial antigens, fungal antigens, protozoa and other parasitic antigens, tumor antigens, antigens involved in autoimmune disease, allergy and graft rejection, toxins, and other miscellaneous antigens.

As used herein, a "cancer" is a disease state characterized by the presence in a subject of cells demonstrating abnormal uncontrolled replication and in some aspects, the term may be used interchangeably with the term "tumor." The term "cancer or tumor antigen" refers to an antigen known to be associated and expressed on the surface with a cancer cell or tumor cell or tissue, and the term "cancer or tumor targeting antibody" refers to an antibody that targets such an antigen. In some embodiments, the term "cancer" as used herein refers to multiple myeloma (MM). In some embodiments, the term "cancer" as used herein refers to acute myeloid leukemia (AML). Additionally or alternatively, the cancer as used herein expresses CD19. In some embodiment, the cancer is a relapsed cancer. In some embodiments, the cancer is a refractory cancer.

A "solid tumor" is an abnormal mass of tissue that usually does not contain cysts or liquid areas. Solid tumors can be benign or malignant, metastatic or non-metastatic. Different types of solid tumors are named for the type of cells that form them. Examples of solid tumors include sarcomas, carcinomas, and lymphomas.

CD19 is a molecule that functions as co-receptor for the B-cell antigen receptor complex (BCR) on B-lymphocytes. It decreases the threshold for activation of downstream signaling pathways and for triggering B-cell responses to antigens, and is required for normal B cell differentiation and proliferation in response to antigen challenges. See, for example, de Rie et al., Cell Immunol. 1989 Feb;118(2):368-81; and Carter and Fearon. Science. 1992 Apr 3;256(5053):105-7. The majority of B cell malignancies, such as Non-Hodgkin's Lymphoma (NHL), acute lymphoblastic leukemia (ALL), and chronic lymphocytic leukemia (CLL), express normal to high levels of CD19. In some embodiments, the CD19 is a human CD19. Non-limiting exemplary sequences of this protein or the underlying gene can be found under Gene Cards ID: GC16P033267, HGNC: 1633, NCBI Entrez Gene: 930, Ensembl: ENSG00000177455, OMIM^{®}: 107265, or UniProtKB/Swiss-Prot: P15391.

In some embodiments, CD19 refers to CD19 isoform 1 or CD19 isoform 2 or both. In further embodiments, CD19 isoform 1 comprises, or consists essentially of, or yet further consists of:
MPPPRLLFFLLFL TPMEVRPEEPL VVKVEEGDNA VLQCLKGTSDGPTQQL TWSRESP LKPFLKLSLGLPGLGIHMRPLAIWLFIFNVSQQMGGFYLCQPGPPSEKAWQPGWTVN VEGSGELFRWNVSDLGGLGCGLKNRSSEGPSSPSGKLMSPKLYVWAKDRPEIWEGE PPCLPPRDSLNQSLSQDLTMAPGSTLWLSCGVPPDSVSRGPLSWTHVHPKGPKSLLSL ELKDDRPARDMWVMETGLLLPRATAQDAGKYYCHRGNLTMSFHLEITARPVLWH WLLRTGGWKVSAVTLAYLIFCLCSLVGILHLQRALVLRRKRKRMTDPTRRFFKVTPP PGSGPQNQYGNVLSLPTPTSGLGRAQRWAAGLGGTAPSYGNPSSDVQADGALGSRS PPGVGPEEEEGEGYEEPDSEEDSEFYENDSNLGQDQLSQDGSGYENPEDEPLGPEDE DSFSNAESYENEDEELTQPVARTMDFLSPHGSAWDPSREATSLGSQSYEDMRGILYA APQLRSIRGQPGPNHEEDADSYENMDNPDGPDPAWGGGGRMGTWSTR (SEQ ID NO: 112), or aa 16 to aa 556 of this polypeptide SEQ ID NO: 112, or aa 20 to aa 556 of SEQ ID NO: 112. In further embodiments, CD19 isoform 2 comprises, or consists essentially of, or yet further consists of

MPPPRLLFFLLFLTPMEVRPEEPLVVKVEEGDNAVLQCLKGTSDGPTQQLTWSRESP
LKPFLKLSLGLPGLGIHMRPLAIWLFIFNVSQQMGGFYLCQPGPPSEKAWQPGWTVN
VEGSGELFRWNVSDLGGLGCGLKNRSSEGPSSPSGKLMSPKLYVWAKDRPEIWEGE
PPCLPPRDSLNQSLSQDLTMAPGSTLWLSCGVPPDSVSRGPLSWTHVHPKGPKSLLSL
ELKDDRPARDMWVMETGLLLPRATAQDAGKYYCHRGNLTMSFHLEITARPVLWH
WLLRTGGWKVSAVTLAYLIFCLCSLVGILHLQRALVLRRKRKRMTDPTRRFFKVTPP
PGSGPQNQYGNVLSLPTPTSGLGRAQRWAAGLGGTAPSYGNPSSDVQADGALGSRS
PPGVGPEEEEGEGYEEPDSEEDSEFYENDSNLGQDQLSQDGSGYENPEDEPLGPEDE
DSFSNAESYENEDEELTQPVARTMDFLSPHGSAWDPSREATSLAGSQSYEDMRGILY
AAPQLRSIRGQPGPNHEEDADSYENMDNPDGPDPAWGGGGRMGTWSTR (SEQ ID
NO: 113).

The term "chimeric antigen receptor" (CAR), as used herein, refers to a fused protein comprising an extracellular domain capable of binding to an antigen, a transmembrane domain derived from a polypeptide different from a polypeptide from which the extracellular domain is derived, and at least one intracellular domain. The "chimeric antigen receptor (CAR)" is sometimes called a "chimeric receptor", a "T-body", or a "chimeric immune receptor (CIR)." The "extracellular domain capable of binding to an antigen" means any oligopeptide or polypeptide that can bind to a certain antigen. The "intracellular domain" or "intracellular signaling domain" means any oligopeptide or polypeptide known to function as a domain that transmits a signal to cause activation or inhibition of a biological process in a cell, such as an immune cell. In certain embodiments, the intracellular domain may comprise, alternatively consist essentially of, or yet further consist of one or more costimulatory signaling domains in addition to the primary signaling domain. The "transmembrane domain" means any oligopeptide or polypeptide known to span the cell membrane and that can function to link the extracellular and signaling domains.

As used herein, "immune cells" includes, e.g., white blood cells (leukocytes, such as granulocytes (neutrophils, eosinophils, and basophils), monocytes, and lymphocytes (T cells, B cells, natural killer (NK) cells and NKT cells)) which may be derived from hematopoietic stem cells (HSC) produced in the bone marrow, lymphocytes (T cells, B cells, natural killer (NK) cells, and NKT cells) and myeloid-derived cells (neutrophil, eosinophil, basophil, monocyte, macrophage, dendritic cells). In some embodiments, the immune cell is derived from one or more of the following: progenitor cells, embryonic stem cells, embryonic stem cell derived cells, embryonic germ cells, embryonic germ cell derived cells, stem cells, stem cell derived cells, pluripotent stem cells, induced pluripotent stem cells (iPSc), haematopoietic stem cells (HSCs), or immortalized cells. In some embodiments, the HSC are derived from umbilical cord blood of a subject, peripheral blood of a subject, or bone marrow of a subject.

The term "culturing" refers to the in vitro or ex vivo propagation of cells or organisms on or in media of various kinds. It is understood that the descendants of a cell grown in culture may not be completely identical (i.e., morphologically, genetically, or phenotypically) to the parent cell.

As used herein, the term "NK cell," also known as natural killer cell, refers to a type of lymphocyte that originates in the bone marrow and play a critical role in the innate immune system. NK cells provide rapid immune responses against viral-infected cells, tumor cells or other stressed cell, even in the absence of antibodies and major histocompatibility complex on the cell surfaces. NK cells may either be isolated or obtained from a commercially available source. Non-limiting examples of commercial NK cell lines include lines NK-92 (ATCC^{®} CRL-2407^{™}), NK-92MI (ATCC^{®} CRL-2408^{™}). Further examples include but are not limited to NK lines HANK1, KHYG-1, NKL, NK-YS, NOI-90, and YT. Non-limiting exemplary sources for such commercially available cell lines include the American Type Culture Collection, or ATCC (www.atcc.org/) and the German Collection of Microorganisms and Cell Cultures (www.dsmz.de/).

As used herein, the term "T cell," refers to a type of lymphocyte that matures in the thymus. T cells play an important role in cell-mediated immunity and are distinguished from other lymphocytes, such as B cells, by the presence of a T-cell receptor on the cell surface. T-cells may either be isolated or obtained from a commercially available source. "T cell" includes all types of immune cells expressing CD3 including T-helper cells (CD4+ cells), cytotoxic T-cells (CD8+ cells), natural killer T-cells, T-regulatory cells (Treg) and gamma-delta T cells. A "cytotoxic cell" includes CD8+ T cells, natural-killer (NK) cells, and neutrophils, which cells are capable of mediating cytotoxicity responses. Non-limiting examples of commercially available T-cell lines include lines BCL2 (AAA) Jurkat (ATCC^{®} CRL-2902^{™}) BCL2 (S70A) Jurkat (ATCC^{®} CRL-2900^{™}), BCL2 (S87A) Jurkat (ATCC^{®} CRL-2901^{™}), BCL2 Jurkat (ATCC^{®} CRL-2899^{™}), Neo Jurkat (ATCC^{®} CRL-2898^{™}), TALL-104 cytotoxic human T cell line (ATCC # CRL-11386). Further examples include but are not limited to mature T-cell lines, e.g., such as Deglis, EBT-8, HPB-MLp-W, HUT 78, HUT 102, Karpas 384, Ki 225, My-La, Se-Ax, SKW-3, SMZ-1 and T34; and immature T-cell lines, e.g., ALL-SIL, Be13, CCRF-CEM, CML-T1, DND-41, DU.528, EU-9, HD-Mar, HPB-ALL, H-SB2, HT-1, JK-T1, Jurkat, Karpas 45, KE-37, KOPT-K1, K-T1, L-KAW, Loucy, MAT, MOLT-1, MOLT 3, MOLT-4, MOLT 13, MOLT-16, MT-1, MT-ALL, P12/Ichikawa, Peer, PER0117, PER-255, PF-382, PFI-285, RPMI-8402, ST-4, SUP-T1 to T14, TALL-1, TALL-101, TALL-103/2, TALL-104, TALL-105, TALL-106, TALL-107, TALL-197, TK-6, TLBR-1, -2, -3, and -4, CCRF-HSB-2 (CCL-120.1), J.RT3-T3.5 (ATCC TIB-153), J45.01 (ATCC CRL-1990), J.CaM1.6 (ATCC CRL-2063), RS4;11 (ATCC CRL-1873), CCRF-CEM (ATCC CRM-CCL-119); and cutaneous T-cell lymphoma lines, e.g., HuT78 (ATCC CRM-TIB-161), MJ[G11] (ATCC CRL-8294), HuT102 (ATCC TIB-162). Null leukemia cell lines, including but not limited to REH, NALL-1, KM-3, L92-221, are another commercially available source of immune cells, as well as cell lines derived from other leukemias and lymphomas, such as K562 erythroleukemia, THP-1 monocytic leukemia, U937 lymphoma, HEL erythroleukemia, HL60 leukemia, HMC-1 leukemia, KG-1 leukemia, U266 myeloma. Non-limiting exemplary sources for such commercially available cell lines include the American Type Culture Collection, or ATCC (www.atcc.org/) and the German Collection of Microorganisms and Cell Cultures (www.dsmz.de/).

As used herein, the term "animal" refers to living multi-cellular vertebrate organisms, a category that includes, for example, mammals and birds. The term "mammal" includes both human and non-human mammals.

The term "subject," "host," "individual," and "patient" are as used interchangeably herein to refer to animals, typically mammalian animals. Any suitable mammal can be treated by a method described herein. Non-limiting examples of mammals include humans, non-human primates (e.g., apes, gibbons, chimpanzees, orangutans, monkeys, macaques, and the like), domestic animals (e.g., dogs and cats), farm animals (e.g., horses, cows, goats, sheep, pigs) and experimental animals (e.g., mouse, rat, rabbit, guinea pig). In some embodiments, a mammal is a human. A mammal can be any age or at any stage of development (e.g., an adult, teen, child, infant, or a mammal in utero). A mammal can be male or female. In some embodiments, a subject is a human. In some embodiments, a subject has or is diagnosed of having or is suspected of having a disease.

In some embodiments, the cell as disclosed herein is a eukaryotic cell or a prokaryotic cell.

"Host cell" refers not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The host cell can be a prokaryotic or a eukaryotic cell.

"Eukaryotic cells" comprise all of the life kingdoms except monera. They can be easily distinguished through a membrane-bound nucleus. Animals, plants, fungi, and protists are eukaryotes or organisms whose cells are organized into complex structures by internal membranes and a cytoskeleton. The most characteristic membrane-bound structure is the nucleus. Unless specifically recited, the term "host" includes a eukaryotic host, including, for example, yeast, higher plant, insect and mammalian cells. Non-limiting examples of eukaryotic cells or hosts include simian, canine, bovine, porcine, murine, rat, avian, reptilian and human.

"Prokaryotic cells" that usually lack a nucleus or any other membrane-bound organelles and are divided into two domains, bacteria and archaea. Additionally, instead of having chromosomal DNA, these cells' genetic information is in a circular loop called a plasmid. Bacterial cells are very small, roughly the size of an animal mitochondrion (about 1-2µm in diameter and 10 µm long). Prokaryotic cells feature three major shapes: rod shaped, spherical, and spiral. Instead of going through elaborate replication processes like eukaryotes, bacterial cells divide by binary fission. Examples include but are not limited to bacillus bacteria, E. coli bacterium, and Salmonella bacterium.

The term "detectable marker" refers to any bio-compatible peptide, small molecule, lipid or nucleotide that is detectable with any method know in the art. Examples of such markers include, but are not limited to flag tags, His tags, Myc tags, and HA tags. Since such markers are made of peptides, they are known as "signal peptides."

A "composition" is intended to mean a combination of active agent and another compound or composition, inert (for example, a detectable agent or label) or active, such as an adjuvant , diluent, binder, stabilizer, buffers, salts, lipophilic solvents, preservative, adjuvant or the like and include pharmaceutically acceptable carriers.

Carriers also include pharmaceutical excipients and additives proteins, peptides, amino acids, lipids, and carbohydrates (e.g., sugars, including monosaccharides, di-, tri, tetra-oligosaccharides, and oligosaccharides; derivatized sugars such as alditols, aldonic acids, esterified sugars and the like; and polysaccharides or sugar polymers), which can be present singly or in combination, comprising alone or in combination 1-99.99% by weight or volume. Exemplary protein excipients include serum albumin such as human serum albumin (HSA), recombinant human albumin (rHA), gelatin, casein, and the like. Representative amino acid components, which can also function in a buffering capacity, include alanine, arginine, glycine, arginine, betaine, histidine, glutamic acid, aspartic acid, cysteine, lysine, leucine, isoleucine, valine, methionine, phenylalanine, aspartame, and the like. Carbohydrate excipients are also intended within the scope of this technology, examples of which include but are not limited to monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol.

A composition as disclosed herein can be a pharmaceutical composition. A "pharmaceutical composition" is intended to include the combination of an active agent with a carrier, inert or active, making the composition suitable for diagnostic or therapeutic use in vitro, in vivo or ex vivo.

"Pharmaceutically acceptable carriers" refers to any diluents, excipients, or carriers that may be used in the compositions disclosed herein. Pharmaceutically acceptable carriers include ion exchangers, alumina, aluminum stearate, lecithin, serum proteins, such as human serum albumin, buffer substances, such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Suitable pharmaceutical carriers are described in Remington's Pharmaceutical Sciences, Mack Publishing Company, a standard reference text in this field. They may be selected with respect to the intended form of administration, that is, oral tablets, capsules, elixirs, syrups and the like, and consistent with conventional pharmaceutical practices.

The compositions used in accordance with the disclosure can be packaged in dosage unit form for ease of administration and uniformity of dosage. The term "unit dose" or "dosage" refers to physically discrete units suitable for use in a subject, each unit containing a predetermined quantity of the composition calculated to produce the desired responses in association with its administration, i.e., the appropriate route and regimen. The quantity to be administered, both according to number of treatments and unit dose, depends on the result and/or protection desired. Precise amounts of the composition also depend on the judgment of the practitioner and are peculiar to each individual. Factors affecting dose include physical and clinical state of the subject, route of administration, intended goal of treatment (alleviation of symptoms versus cure), and potency, stability, and toxicity of the particular composition. Upon formulation, solutions are administered in a manner compatible with the dosage formulation and in such amount as is therapeutically or prophylactically effective. The formulations are easily administered in a variety of dosage forms, such as the type of injectable solutions described herein.

A combination as used herein intends that the individual active ingredients of the compositions are separately formulated for use in combination, and can be separately packaged with or without specific dosages. The active ingredients of the combination can be administered concurrently or sequentially.

An "effective amount" is an amount sufficient to effect beneficial or desired results. An effective amount can be administered in one or more administrations, applications or dosages. Such delivery is dependent on a number of variables including the time period for which the individual dosage unit is to be used, the bioavailability of the therapeutic agent, the route of administration, etc. It is understood, however, that specific dose levels of the therapeutic agents disclosed herein for any particular subject depends upon a variety of factors including the activity of the specific agent employed, bioavailability of the agent, the route of administration, the age of the animal and its body weight, general health, sex, the diet of the animal, the time of administration, the rate of excretion, the drug combination, and the severity of the particular disorder being treated and form of administration. In general, one will desire to administer an amount of the agent that is effective to achieve a serum level commensurate with the concentrations found to be effective in vivo. These considerations, as well as effective formulations and administration procedures are well known in the art and are described in standard textbooks.

"Therapeutically effective amount" of an agent refers to an amount of the agent that is an amount sufficient to obtain a pharmacological response; or alternatively, is an amount of the agent that, when administered to a patient with a specified disorder or disease, is sufficient to have the intended effect, e.g., treatment, alleviation, amelioration, palliation or elimination of one or more manifestations of the specified disorder or disease in the patient. A therapeutic effect does not necessarily occur by administration of one dose, and may occur only after administration of a series of doses. Thus, a therapeutically effective amount may be administered in one or more administrations.

In one embodiment not encompassed by the claims, the term "disease" or "disorder" as used herein refers to a virus infection, a status of being diagnosed with such infection, a status of being suspect of having such infection, a status of having being exposed to a virus, or a status of at high risk of being exposed to a virus. In one embodiment, the virus is a coronavirus. In one embodiment, the coronavirus is a respiratory virus. In a further embodiment, the disease is Coronavirus disease 2019 (COVID-19) caused by SARS-CoV-2. In yet a further embodiment, the disease is Severe acute respiratory syndrome (SARS) caused by SARS-CoV-1.

Coronaviruses constitute the subfamily Orthocoronavirinae, in the family Coronaviridae, order Nidovirales, and realm Riboviria. They are enveloped viruses with a positive-sense single-stranded RNA genome and a nucleocapsid of helical symmetry. The genome size of coronaviruses ranges from approximately 26 to 32 kilobases, one of the largest among RNA viruses. They have characteristic club-shaped spikes that project from their surface, which in electron micrographs create an image reminiscent of the solar corona, from which their name derives.

Coronaviridae express canonical polymerase genes, as well as structural genes, including S, E, M, and N, but also express a highly divergent set of accessory genes whose open reading frames are interspersed among the structural genes within the 3' one-third of the viral genome. The accessory genes are thought to contain "luxury" functions that are often not required for in-vitro virus replication. The severe acute respiratory syndrome coronavirus (SARS-CoV) expresses eight such accessory genes (ORF3a, -3b, -6, -7a, -7b, -8a, -8b, and - 9b), the most of any known coronavirus. Of these, 7a, 3a and 8 represent the 3rd, 4th and 5th most abundant transcripts behind N and S transcripts.

In some embodiments not encompassed by the claims, the coronavirus as used herein refers to a severe acute respiratory syndrome (SARS) associated coronavirus (SARS-CoV). In some embodiments, the coronavirus is either or both of SARS-CoV-1 and SARS-CoV-2. In some embodiments, the coronavirus comprises a virus selected from the group consisting of an Alphacoronavirus; a Colacovirus such as Bat coronavirus CDPHE15; a Decacovirus such as Bat coronavirus HKU10 or Rhinolophus ferrumequinum alphacoronavirus HuB-2013; a Duvinacovirus such as Human coronavirus 229E; a Luchacovirus such as Lucheng Rn rat coronavirus; a Minacovirus such as a Ferret coronavirus or Mink coronavirus 1; a Minunacovirus such as Miniopterus bat coronavirus 1 or Miniopterus bat coronavirus HKU8; a Myotacovirus such as Myotis ricketti alphacoronavirus Sax-2011; a nyctacovirus such as Nyctalus velutinus alphacoronavirus SC-2013; a Pedacovirus such as Porcine epidemic diarrhea virus or Scotophilus bat coronavirus 512; a Rhinacovirus such as Rhinolophus bat coronavirus HKU2; a Setracovirus such as Human coronavirus NL63 or NL63-related bat coronavirus strain BtKYNL63-9b; a Tegacovirus such as Alphacoronavirus 1; a Betacoronavirus; a Embecovirus such as Betacoronavirus 1, Human coronavirus OC43, China Rattus coronavirus HKU24, Human coronavirus HKU1 or Murine coronavirus; a Hibecovirus such as Bat Hp-betacoronavirus Zhejiang2013; a Merbecovirus such as Hedgehog coronavirus 1, Middle East respiratory syndrome-related coronavirus (MERS-CoV), Pipistrellus bat coronavirus HKU5 or Tylonycteris bat coronavirus HKU4; a Nobecovirus such as Rousettus bat coronavirus GCCDC1 or Rousettus bat coronavirus HKU9, a Sarbecovirus such as a Severe acute respiratory syndrome-related coronavirus, Severe acute respiratory syndrome coronavirus (SARS-CoV) or Severe acute respiratory syndrome coronavirus 2 (SARS-CoV-2, COVID-19); a Deltacoronavirus; an Andecovirus such as Wigeon coronavirus HKU20; a Buldecovirus such as Bulbul coronavirus HKU11, Porcine coronavirus HKU15, Munia coronavirus HKU13 or White-eye coronavirus HKU16; a Herdecovirus such as Night heron coronavirus HKU19; a Moordecovirus such as Common moorhen coronavirus HKU21; a Gammacoronavirus; a Cegacovirus such as Beluga whale coronavirus SW1; and an Igacovirus such as Avian coronavirus.

Symptoms of a coronavirus infection include, but are not limited to, mild symptoms, such as fatigues, tingling, tingling or numbness in the hands and feet, dizziness, confusion, brain fog, body ache, chills, loss of appetite, nausea, vomiting, abdominal pain or discomfort, loss of smell, inability to taste, muscle weakness, photophobia, adenopathy, headaches, cough, dry cough, shortness of breath, sore throat, lower extremity weakness/numbness, diarrhea, low blood O2, sneezing, runny nose or post-nasal drip; severe symptoms, such as ventilatory use, high fever, severe cough, delirium, seizures, stroke, systematic inflammation, cytokine storm; and other symptoms, such as fever, swollen adenoids, pneumonia, bronchitis, and Dyspnea.

As used herein, "treating" or "treatment" of a disease in a subject refers to (1) preventing the symptoms or disease from occurring in a subject that is predisposed or does not yet display symptoms of the disease; (2) inhibiting the disease or arresting its development; or (3) ameliorating or causing regression of the disease or the symptoms of the disease. As understood in the art, "treatment" is an approach for obtaining beneficial or desired results, including clinical results. For the purposes of the present technology, beneficial or desired results can include one or more, but are not limited to, alleviation or amelioration of one or more symptoms, diminishment of extent of a condition (including a disease), stabilized (i.e., not worsening) state of a condition (including disease), delay or slowing of condition (including disease), progression, amelioration or palliation of the condition (including disease), states and remission (whether partial or total), whether detectable or undetectable. When the disease is cancer, the following clinical end points are non-limiting examples of treatment: reduction in tumor burden, slowing of tumor growth, longer overall survival, longer time to tumor progression, inhibition of metastasis or a reduction in metastasis of the tumor. In one aspect, treatment excludes prophylaxis.

"Administration" or "delivery" of a cell or vector or other agent and compositions containing same can be performed in one dose, continuously or intermittently throughout the course of treatment. Methods of determining the most effective means and dosage of administration are known to those of skill in the art and will vary with the composition used for therapy, the purpose of the therapy, the target cell being treated, and the subject being treated. Single or multiple administrations can be carried out with the dose level and pattern being selected by the treating physician or in the case of animals, by the treating veterinarian. Suitable dosage formulations and methods of administering the agents are known in the art. Route of administration can also be determined and method of determining the most effective route of administration are known to those of skill in the art and will vary with the composition used for treatment, the purpose of the treatment, the health condition or disease stage of the subject being treated, and target cell or tissue. Non-limiting examples of route of administration include oral administration, intraperitoneal, infusion, nasal administration, inhalation, injection, and topical application. In some embodiments, the administration is an intratumoral administration, or administration to a tumor microenvironment, or both. In some embodiments, the administration is an infusion (for example to peripheral blood of a subject) over a certain period of time, such as about 30 minutes, about 1 hour, about 2 hours, about 3 hours, about 4 hours, about 5 hours, about 6 hours, about 7 hours, about 8 hours, about 9 hours, about 10 hours, about 11 hours, about 12 hours, about 24 hours or longer.

The term administration shall include without limitation, administration by oral, parenteral (e.g., intramuscular, intraperitoneal, intravenous, intracerebroventricular (ICV), intrathecal, intracisternal injection or infusion, subcutaneous injection, or implant), by inhalation spray nasal, vaginal, rectal, sublingual, urethral (e.g., urethral suppository) or topical routes of administration (e.g., gel, ointment, cream, aerosol, etc.) and can be formulated, alone or together, in suitable dosage unit formulations containing conventional non-toxic pharmaceutically acceptable carriers, adjuvants, excipients, and vehicles appropriate for each route of administration. The disclosure is not limited by the route of administration, the formulation or dosing schedule.

"Administration" can be performed in one dose, continuously or intermittently throughout the course of treatment. Methods of determining the most effective means and dosage of administration are known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents are known in the art. Route of administration can also be determined and method of determining the most effective route of administration are known to those of skill in the art and will vary with the composition used for treatment, the purpose of the treatment, the health condition or disease stage of the subject being treated, and target cell or tissue. In some embodiments, 1x10⁴ to 1x10¹⁵ or ranges in between of cells as disclosed herein are administrated to a subject, such as 1x10⁷ to 1x10¹⁰. In some embodiments, administering or a grammatical variation thereof also refers to more than one doses with certain interval. In some embodiments, the interval is 1 day, 2 days, 3 days, 4 days, 5 days, 6 days, 1 week, 10 days, 2 weeks, 3 weeks, 1 month, 2 months, 3 months, 4 months, 5 months, 6 months, 1 year or longer. In some embodiments, one dose is repeated for once, twice, three times, four times, five times, six times, seven times, eight times, nine times, ten times or more. For example, cells as disclosed herein may be administered to a subject weekly and for up to four weeks.

As used herein, the term "sample" and "biological sample" and "agricultural sample" are used interchangeably, referring to sample material derived from a subject. Biological samples may include tissues, cells, protein or membrane extracts of cells, and biological fluids (e.g., ascites fluid or cerebrospinal fluid (CSF)) isolated from a subject, as well as tissues, cells and fluids present within a subject. Biological samples may include, but are not limited to, samples taken from breast tissue, renal tissue, the uterine cervix, the endometrium, the head or neck, the gallbladder, parotid tissue, the prostate, the brain, the pituitary gland, kidney tissue, muscle, the esophagus, the stomach, the small intestine, the colon, the liver, the spleen, the pancreas, thyroid tissue, heart tissue, lung tissue, the bladder, adipose tissue, lymph node tissue, the uterus, ovarian tissue, adrenal tissue, testis tissue, the tonsils, thymus, blood, hair, buccal, skin, serum, plasma, CSF, semen, prostate fluid, seminal fluid, urine, feces, sweat, saliva, sputum, mucus, bone marrow, lymph, and tears. Agricultural samples include soil, foliage or any plant tissue or surface or other sample suspected of harboring virus. In addition, the sample can include industrial samples, such as those isolated from surfaces and the environment.

In some embodiments, the sample may be an upper respiratory specimen, such as a nasopharyngeal (NP) specimen, an oropharyngeal (OP) specimen, a nasal mid-turbinate swab, an anterior nares (nasal swab) specimen, or nasopharyngeal wash/aspirate or nasal wash/aspirate (NW) specimen.

In some embodiments, the samples include fluid from a subject, including, without limitation, blood or a blood product (e.g., serum, plasma, or the like), umbilical cord blood, amniotic fluid, cerebrospinal fluid, spinal fluid, lavage fluid (e.g., bronchoalveolar, gastric, peritoneal, ductal, ear, arthroscopic), washings of female reproductive tract, urine, feces, sputum, saliva, nasal mucous, prostate fluid, lavage, semen, lymphatic fluid, bile, tears, sweat, breast milk, breast fluid, the like or combinations thereof. In some embodiments, a liquid biological sample is a blood plasma or serum sample. The term "blood" as used herein refers to a blood sample or preparation from a subject. The term encompasses whole blood, blood product or any fraction of blood, such as serum, plasma, buffy coat, or the like as conventionally defined. In some embodiments, the term "blood" refers to peripheral blood. Blood plasma refers to the fraction of whole blood resulting from centrifugation of blood treated with anticoagulants. Blood serum refers to the watery portion of fluid remaining after a blood sample has coagulated. Fluid samples often are collected in accordance with standard protocols hospitals or clinics generally follow. For blood, an appropriate amount of peripheral blood (e.g., between 3-40 milliliters) often is collected and can be stored according to standard procedures prior to or after preparation.

When used for the treatment of cancer, it is apparent to the skilled artisan that the CAR is selected to bind to and induce an immune response against a cell that specifically binds the antibody fragment or antigen binding domain of the CAR such as for example, CD19. In some embodiments, the cancer cell is a primary cancer cell or a metastatic cancer cell. In some embodiments, the cancer cell is from a carcinoma, an adenocarcinoma, gallbladder adenocarcinoma and transitional cell carcinoma a sarcoma, a myeloma, a leukemia, or a lymphoma. In some embodiments, the cancer is selected from a liver cancer, a colon cancer, a colorectal cancer, an ovarian cancer, a kidney cancer, a thyroid cancer, a pancreatic cancer, a prostate cancer, urinary bladder cancer, a cervical cancer, an esophageal cancer, or a gastric cancer.

In some embodiments, the administering step may be repeated for once, twice, three times, four times, five times, six times, seven times, eight times, nine times, ten times or more. In further embodiments, two administrations are about 1 day apart, about 2 days apart, about 3 days apart, about 4 days apart, about 5 days apart, about 6 days apart, about 1 week apart, about 10 days apart, about 2 weeks apart, about 3 weeks apart, about 4 weeks apart, about 1 month apart, about 2 months apart, about 3 months apart, about 4 months apart, about 5 months apart, about 6 months apart, about 7 months apart, about 8 months apart, about 9 months apart, about 10 months a part, about 11 months apart, about 1 year apart, about 1.5 years apart, about 2 years apart, about 3 years apart, about 5 years apart, about 10 years apart or longer.

Administration of the cells or compositions can be performed in one dose, continuously or intermittently throughout the course of treatment and an effective amount to achieve the desired therapeutic benefit is provided. Methods of determining the most effective means and dosage of administration are known to those of skill in the art and will vary with the composition used for therapy, the purpose of the therapy and the subject being treated. Single or multiple administrations can be carried out with the dose level and pattern being selected by the treating physician. Suitable dosage formulations and methods of administering the agents are known in the art. In a further aspect, the cells and composition of the disclosure can be administered in combination with other treatments.

The cells and populations of cell can be administered to the host or subject using methods known in the art and described, for example, in WO2012079000A1. This administration of the cells or compositions of the disclosure can be done to generate an animal model of the desired disease, disorder, or condition for experimental and screening assays.

An "anti-cancer therapy," as used herein, includes but is not limited to surgical resection, chemotherapy, cryotherapy, radiation therapy, immunotherapy and targeted therapy. Agents that act to reduce cellular proliferation are known in the art and widely used. Chemotherapy drugs that kill cancer cells only when they are dividing are termed cell-cycle specific. These drugs include agents that act in S-phase, including topoisomerase inhibitors and anti-metabolites.

Topoisomerase inhibitors are drugs that interfere with the action of topoisomerase enzymes (topoisomerase I and II). During the process of chemo treatments, topoisomerase enzymes control the manipulation of the structure of DNA necessary for replication and are thus cell cycle specific. Examples of topoisomerase I inhibitors include the camptothecan analogs listed above, irinotecan and topotecan. Examples of topoisomerase II inhibitors include amsacrine, etoposide, etoposide phosphate, and teniposide.

Antimetabolites are usually analogs of normal metabolic substrates, often interfering with processes involved in chromosomal replication. They attack cells at very specific phases in the cycle. Antimetabolites include folic acid antagonists, e.g., methotrexate; pyrimidine antagonist, e.g., 5-fluorouracil, foxuridine, cytarabine, capecitabine, and gemcitabine; purine antagonist, e.g., 6-mercaptopurine and 6-thioguanine; adenosine deaminase inhibitor, e.g., cladribine, fludarabine, nelarabine and pentostatin; and the like.

Plant alkaloids are derived from certain types of plants. The vinca alkaloids are made from the periwinkle plant (Catharanthus rosea). The taxanes are made from the bark of the Pacific Yew tree (taxus). The vinca alkaloids and taxanes are also known as antimicrotubule agents. The podophyllotoxins are derived from the May apple plant. Camptothecan analogs are derived from the Asian "Happy Tree" (Camptotheca acuminata). Podophyllotoxins and camptothecan analogs are also classified as topoisomerase inhibitors. The plant alkaloids are generally cell-cycle specific.

Examples of these agents include vinca alkaloids, e.g., vincristine, vinblastine and vinorelbine; taxanes, e.g., paclitaxel and docetaxel; podophyllotoxins, e.g., etoposide and tenisopide; and camptothecan analogs, e.g., irinotecan and topotecan.

In some embodiments where the cancer is an immune cell cancer, an anti-cancer therapy may comprises, or consists essentially of, or consists of a hematopoietic stem cell transplantation.

In some embodiments, a therapeutic agent, such as a cell as disclosed herein, may be combined in treating a cancer with another anti-cancer therapy or a therapy depleting an immune cell. For example, lymphodepletion chemotherapy is performed followed by administration of a cell as disclosed herein, such as four weekly infusions. In further embodiments, these steps may be repeated for once, twice, three or more times until a partial or complete effect is observed or a clinical end point is achieved.

Gemcibabine (Gemzar^{®}) is an antimetabolite used to treat carcinomas and has been used as a first-line treatment for pancreatic cancer, and in combination with cisplatin for advanced or metastatic bladder cancer and advanced or metastatic non-small cell lung cancer. It is used as a second-line treatment in combination with carboplatin for ovarian cancer and in combination with paclitaxel for breast cancer that is metastatic or cannot be surgically removed. It is commercially available from Lilly Medical.

Aldoxorubicin is a tumor-targeted doxorubicin conjugate in development by CytRx. It is the (6-maleimidocaproyl) hydrazone of doxorubicin. Essentially, this chemical name describes doxorubicin attached to an acid-sensitive linker (N-ε-maleimidocaproic acid hydrazide, or EMCH).

Cryotherapy includes, but is not limited to, therapies involving decreasing the temperature, for example, hypothermic therapy.

Radiation therapy includes, but is not limited to, exposure to radiation, e.g., ionizing radiation, UV radiation, as known in the art. Exemplary dosages include, but are not limited to, a dose of ionizing radiation at a range from at least about 2 Gy to not more than about 10 Gy or a dose of ultraviolet radiation at a range from at least about 5 J/m² to not more than about 50 J/m², usually about 10 J/m².

The phrase "first line" or "second line" or "third line" refers to the order of treatment received by a patient. First line therapy regimens are treatments given first, whereas second or third line therapy are given after the first line therapy or after the second line therapy, respectively. The National Cancer Institute defines first line therapy as "the first treatment for a disease or condition. In patients with cancer, primary treatment can be surgery, chemotherapy, radiation therapy, or a combination of these therapies. First line therapy is also referred to those skilled in the art as "primary therapy and primary treatment." See National Cancer Institute website at www.cancer.gov, last visited on May 1, 2008. Typically, a patient is given a subsequent chemotherapy regimen because the patient did not show a positive clinical or sub-clinical response to the first line therapy or the first line therapy has stopped.

### Modes for Carrying Out the Disclosure

### CAR Constructs

Since the long-term efficacy of antibodies induced by vaccines and the overall resistance to new variants of SARS-CoV-2 is unclear, it will be important to consider alternate approaches. It is also unclear how well immunocompromised or aged individuals respond to immunization, especially for long-term immunity and immune memory. In influenza infection, CD8 T cells cannot prevent infection but are needed to resolve infections (Allan et al., 1990). In mouse models, T cells can promote recovery from lethal flu inflections in the absence of B-cells and antibodies (Epstein et al., 1998; Graham and Braciale, 1997). In humans, the number of influenza-specific cytolytic T cells (CTLs) correlates with the rate of viral clearance (McMichael et al., 1986). The presentation of viral peptides by major histocompatibility class 1 antigens activates CD8+ T cells induces the development of effector functions against host cells presenting viral determinants. Effectors control viral expansion via the production of cytokines such as interferon-gamma (IFN-y), the expression of Fas-ligand and the release of granules containing perforin and granzymes such as granzyme B (GZMB)(Topham et al., 1997). In keeping with the importance of cellular mediated immunity in COVID-19, a reduction in CD8 cells is correlated with a worse prognosis (Urra et al., 2020), while SARS-CoV-2-specific T cell responses promote disease resolution and reduced severity of infection (Rydyznski Moderbacher et al., 2020). The fact that CD4 and CD8 T-cell responses are induced during infection indicates that SARS-CoV-2 peptides can be processed and presented to T-cells (Grifoni et al., 2020; Meckiff et al., 2020; Premkumar et al., 2020; Rydyznski Moderbacher et al., 2020; Weiskopf et al., 2020). Mass-spectrometry has detected SARS-CoV-2 virus-derived peptides from the epithelial cells, or gargle, of patients with infections (Ihling et al., 2020; Nikolaev et al., 2020), while another study reported SARS-CoV-2 S protein expressed on the surface of infected cells, resulting in syncytia formation with ACE2-expressing cells (Buchrieser et al., 2021).

While the development and use of chimeric antigen receptor (CAR) is an effective immunotherapy against hematologic cancers, the potential of CAR-T cells in preventing or treating severe cases of COVID-19 has not yet been exploited (Kawalekar et al., 2016; Posey et al., 2016). CARs combine an antigen recognition domain of a specific antibody with an intracellular domain of the CD3-ζ chain or FcγRI protein into a single chimeric protein (Gross et al., 1989; Irving and Weiss, 1991). The inclusion of intracellular motifs from the co-receptors CD28 and CD137 (4-1BB) extend the longevity of CAR-Ts (Jena et al., 2010; Maus and June, 2016). CAR-Ts against relapsed acute lymphoblastic leukemia (ALL) have been associated with durable and sustained remissions for up to 24 months (Maude et al., 2014). Severe cytokine-release syndrome in a minority of patients has been effectively treated with the anti-interleukin-6 receptor antibody, tocilizumab (Maude et al., 2014). Three CAR-T based drugs (Breyanzi, Yescarta and Kymriah) have been approved by FDA, while other trials are presently underway in many academic medical centers(Sadelain et al., 2009). In the case of SARS-CoV-2(Grifoni et al., 2020; Meckiff et al., 2020; Premkumar et al., 2020; Rydyznski Moderbacher et al., 2020; Weiskopf et al., 2020), the induction of T-cell responses with different clonotypes indicates that SARS-CoV-2 derived peptides are likely available for recognition by appropriately designed CAR-Ts. Surprisingly, although some CAR-Ts have been used to target hepatitis B virus envelope proteins in mice (Krebs et al., 2013), the potential of CAR-T cells in the treatment of SARS-CoV-2 infection has not been explored (Seif et al., 2019). In one aspect, Applicant provides herein the design and generation of different CAR-T cells that recognize and kill target cells loaded with RBD region of the SARS-CoV-2 spike protein. The CAR-T cells can additionally express effector molecules, granzyme B, perforin, IFN-y and FasL and subsequently be phenotypically segregated into subsets. Applicant also demonstrates herein that CAR-T cells against SARS-CoV-2 can be generated to elicit the *in vitro* and *in vivo* killing of RBD and S1 bearing target cells. Thus, also provided herein is an in vitro method of killing RBD and S1 bearing target cells by contacting the cells with a CAR expressing the appropriate viral antigen, which can be used to develop personalized therapies, new drugs and combination therapies.

In a further aspect, the antigen comprises, or consists essentially of, or yet further consists of the RBD region of the SARS-CoV-2 spike protein. This approach offers a new potential therapeutic approach for the treatment of COVID-19 for longer-term immunity. In one aspect, provided is a polypeptide comprising, or consisting essentially of, or yet further consisting of (a) an antibody or a fragment thereof that specifically binds a viral antigen, (b) a hinge region, (c) a transmembrane domain, (d) one or more co-stimulatory domains, and (e) an intracellular signaling domain. In some embodiments, the polypeptide comprises, or consists essentially of, or yet further consist of a chimeric antigen receptor (CAR). In further embodiments, the polypeptide is a CAR. The viral antigen is in one aspect, a SARS-CoV-2 spike protein or fragment thereof from any SARS-CoV-2 variant, e.g., a delta or omicron variant. Examples of such are known in the art and provided herein. In a further aspect, the antigen comprises, or consists essentially of, or yet further consists of the RBD region of the SARS-CoV-2 spike protein.

In a further aspect, provided is a polypeptide comprising, or consisting essentially of, or yet further consisting of a chimeric antigen receptor (CAR) comprising, or consisting essentially of, or yet further consisting of (a) an antibody or a fragment thereof that specifically binds a TAA, (b) a hinge region, (c) one or more transmembrane domains, (d) one or more co-stimulatory domains, and (e) one or more intracellular signaling domain.

The CARs can comprise further elements such as for example, additional anti-TAA binding domains or elements that secrete antibodies or bispecific antibodies.

Without wishing to be bound by the theory, in one aspect, Applicant has discovered that a hinge region of the CARs comprising 120 or more amino acid residues unexpectedly promotes the binding, recognition or reactivity between the CAR and the TAA, the virus or the viral antigen, FasL expression and cytotoxic killing of the viral antigen or TAA expressing cell by a CAR expressing immune cell as disclosed herein.

In some embodiments not encompassed by the claims, the hinge region of the CAR comprises, or consists essentially of, or yet further consists of an IgG4 hinge region, In further embodiments, the IgG4 hinge region comprises, or consists essentially of, or yet further consists of ESKYGPPCPSCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNKGLPSS IEKTISKAKGQPREPQVYTLPPSQEEMTKNQVSLTCLVKGFYPSDIAVEWESNGQPEN NYKTTPPVLDSDGSFFLYSRLTVDKSRWQEGNVFSCSVMHEALHNHYTQKSLSLSL GK (SEQ ID NO: 1) or an equivalent thereof. In further embodiments not encompassed by the claims, the equivalent comprises, or consists essentially of, or yet further consists of a mutated SEQ ID NO: 1 comprising one or more mutations, such as comprising one or more of: a proline (P) corresponding to (such as aligned to) the 10^{th} amino acid residue of SEQ ID NO: 1 which is serine (S) (S10P), a P corresponding to the 15^{th} amino acid residue of SEQ ID NO: 1 which is glutamic acid (E) (E15P), a valine corresponding to the 16^{th} amino acid residue of SEQ ID NO: 1 which is phenylalanine (F) (F16V), a deletion (Δ) corresponding to the 17^{th} amino acid residue of SEQ ID NO: 1 which is leucine (L) (L17Δ), an alanine (A) corresponding to the 18^{th} amino acid residue of SEQ ID NO: 1 which is glycine (G18A), or a glutamine (Q) corresponding to the 79^{th} amino acid residue of SEQ ID NO: 1 which is asparagine (N). See, for example, Hudecek et al. Cancer Immunol Res. 2015 Feb; 3(2):125-35. In yet further embodiments not encompassed by the claims, the equivalent to SEQ ID NO: 1 comprises, or consists essentially of, or yet further consists of: ESKYGPPCPPCPAPPVAGPSVFLFPPKPKDTLMISRTPEVTCVVVDVSQEDPEVQFNW YVDGVEVHNAKTKPREEQFQSTYRVVSVLTVLHQDWLNGKEYKCKVSNKGLPSSIE KTISKAKGQPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQKSLSLSLG K (SEQ ID NO: 23) or an equivalent thereof.

In some embodiments not encompassed by the claims, the hinge region comprises, or consists essentially of, or yet further consists of an Platelet Derived Growth Factor Receptor Alpha (PDGFRA) hinge region, In further embodiments not encompassed by the claims, the PDGFRA hinge region comprises, or consists essentially of, or yet further consists of: 
QLSLPSILPNENEKVVQLNSSFSLRCFGESEVSWQYPMSEEESSDVEIRNEENNSGLFV
TVLEVSSASAAHTGLYTCYYNHTQTEENELEGRHIYIYVPDPDVAFVPLGMTDYLVI
VEDDDSAIIPCRTTDPETPVTLHNSEGVVPASYDSRQGFNGTFTVGPYICEATVKGKK
FQTIPFNVYALKATSELDLEMEALKTVYKSGETIVVTCAVFNNEVVDLQWTYPGEV
KGKGITMLEEIKVPSIKLVYTLTVPEATVKDSGDYECAARQATREVKEMKKVTISVH
EKGFIEIKPTFSQLEAVNLHEVKHFVVEVRAYPPPRISWLKNNLTLIENLTEITTDVEKI
QEIRYRSKLKLIRAKEEDSGHYTIVAQNEDAVKSYTFELLTQVPSSILDLVDDHHGST
GGQTVRCTAEGTPLPDIEWMICKDIKKCNNETSWTILANNVSNIITEIHSRDRSTVEGR
VTFAKVEETIAVRCLAKNLLGAENRELKLVAPTLRSELTVA (SEQ ID NO: 35)
 or an equivalent thereof.

In some embodiments not encompassed by the claims, the hinge region comprises, or consists essentially of, or yet further consists of an Platelet Derived Growth Factor Receptor Beta (PDGFRB) hinge region, In further embodiments not encompassed by the claims, the PDGFRB hinge region comprises, or consists essentially of, or yet further consists of: 
LVVTPPGPELVLNVSSTFVLTCSGSAPVVWERMSQEPPQEMAKAQDGTFSSVLTLTN
LTGLDTGEYFCTHNDSRGLETDERKRLYIFVPDPTVGFLPNDAEELFIFLTEITEITIPC
RVTDPQLVVTLHEKKGDVALPVPYDHQRGFSGIFEDRSYICKTTIGDREVDSDAYYV
YRLQVSSINVSVNAVQTVVRQGENITLMCIVIGNEVVNFEWTYPRKESGRLVEPVTD
FLLDMPYHIRSILHIPSAELEDSGTYTCNVTESVNDHQDEKAINITVVESGYVRLLGEV
GTLQFAELHRSRTLQVVFEAYPPPTVLWFKDNRTLGDSSAGEIALSTRNVSETRYVSE
LTLVRVKVAEAGHYTMRAFHEDAEVQLSFQLQINVPVRVLELSESHPDSGEQTVRC
RGRGMPQPNIIWSACRDLKRCPRELPPTLLGNSSEEESQLETNVTYWEEEQEFEVVST
LRLQHVDRPLSVRCTLRNAVGQDTQEVIVVPHSLPFKV (SEQ ID NO: 36) or an
 equivalent thereof.

In the claimed invention, the hinge region comprises, or consists essentially of, or yet further consists of a Leukocyte Associated Immunoglobulin like Receptor 1 (LAIR1) hinge region. The LAIR1 hinge region comprises, or consists essentially of, or yet further consists of: QEEDLPRPSISAEPGTVIPLGSHVTFVCRGPVGVQTFRLERESRSTYNDTEDVSQASPS ESEARFRIDSVSEGNAGPYRCIYYKPPKWSEQSDYLELLVKETSGGPDSPDTEPGSSA GPTQRPSDNSHNEHAPASQGLKAEHLY (SEQ ID NO: 37). In other embodiments not encompassed by the claims, the hinge region comprises, or consists essentially of, or yet further consists of a truncated LAIR1 hinge (tLAIR1). In further embodiments not encompassed by the claims, the tLAIR1 hinge region comprises, or consists essentially of, or yet further consists of: LLVKETSGGPDSPDTEPGSSAGPTQRPSDNSHNEHAPASQGLKAEHLY (SEQ ID NO: 42), or an equivalent thereof.

In some embodiments not encompassed by the claims, the hinge region comprises, or consists essentially of, or yet further consists of a CD8α hinge region. In further embodiments not encompassed by the claims, the CD8α hinge region comprises, or consists essentially of, or yet further consists of: KPTTTPAPRPPTPAPTIASQPLSLRPEACRPAAGGAVHTRGLDFACD (SEQ ID NO: 40, aa 136 to aa 182 of the sequence set forth as NP_001759.3) or an equivalent thereof. In some embodiments not encompassed by the claims, the hinge region comprises, or consists essentially of, or yet further consists of an IgG4 CH3 hinge. In further embodiments not encompassed by the claims, the IgG4 CH3 hinge region comprises, or consists essentially of, or yet further consists of the amino acid sequence: ESKYGPPCPPCPGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNHYTQKSL SLSLGK (SEQ ID NO: 41) or an equivalent thereof.

In some embodiments not encompassed by the claims, the hinge region comprises, or consists essentially of, or yet further consists of a CD28 hinge region. In further embodiments not encompassed by the claims, the CD28 hinge region comprises, or consists essentially of, or yet further consists of: IEVMYPPPYLDNEKSNGTIIHVKGKHLCPSPLFPGPSKP (SEQ ID NO: 39), or an equivalent thereof.

In some embodiments not encompassed by the claims, the viral antigen comprises, or consists essentially of, or yet further consists of a Spike (S) protein or a fragment thereof of the severe acute respiratory syndrome coronavirus 2 (SARS-CoV-2). In further embodiments not encompassed by the claims, the S protein or fragment thereof comprises, or consists essentially of, or yet further consists of: NITNLCPFGEVFNATKFPSVYAWERKKISNCVADYSVLYNSTFFSTFKCYGVSATKL NDLCFSNVYADSFVVKGDDVRQIAPGQTGVIADYNYKLPDDFMGCVLAWNTRNID ATSTGNYNYKYRYLRHGKLRPFERDISNVPFSPDGKPCTPPALNCYWPLNDYGFYTT TGIGYQPYRVVVLSFELLNAPATVCGPKLSTDLI (SEQ ID NO: 2) or an equivalent thereof.

The antibody CR3022 serves as a scaffold for some embodiments not encompassed by the claims. In some embodiments, wherein the antibody or fragment thereof comprises, or consists essentially of, or yet further consists of: a heavy chain complementarity determining region CDR 1 (HCDR1) comprising, or consisting essentially of, or yet further consisting of TYWIG (SEQ ID NO: 3); a heavy chain CDR 2 (HCDR2) comprising, or consisting essentially of, or yet further consisting of IIYPGDSETRYSPSFQG (SEQ ID NO: 4); a heavy chain CDR 3 (HCDR3) comprising, or consisting essentially of, or yet further consisting of GSGISTPMDV (SEQ ID NO: 5); a light chain CDR 1 (LCDR1) comprising, or consisting essentially of, or yet further consisting of KSSQSVLYSSINKNYLA (SEQ ID NO: 6); a light chain CDR 2 (LCDR2) comprising, or consisting essentially of, or yet further consisting of WASTRES (SEQ ID NO: 7); and a light chain CDR 3 (LCDR3) comprising, or consisting essentially of, or yet further consisting of QQYYSTPYT (SEQ ID NO: 8). See, for example, US Patent No. US8106170B2.

In some embodiments not encompassed by the claims, the antibody or fragment thereof comprises, or consists essentially of, or yet further consists of: CDRs of a heavy chain variable domain (VH) comprising, or consisting essentially of, or yet further consisting of: EVQLVQSGTEVKKPGESLKISCKGSGYGFITYWIGWVRQMPGKGLEWMGIIYPGDSE TRYSPSFQGQVTISADKSINTAYLQWSSLKASDTAIYYCAGGSGISTPMDVWGQGTT VTVSS (SEQ ID NO: 9); and CDRs of a light chain variable domain (VL) comprising, or consisting essentially of, or yet further consisting of:

DIVMTQSPDSLAVSLGERATINCKSSQSVLYSSINKNYLAWYQQKPGQPPKLLIYWA
STRESGVPDRFSGSGSGTDFTLTISSLQAEDVAVYYCQQYYSTPYTFGQGTKVEIK
(SEQ ID NO: 10).

In some embodiments not encompassed by the claims, the antibody or fragment thereof comprises, or consists essentially of, or yet further consists of: a heavy chain variable domain (VH) comprising, or consisting essentially of, or yet further consisting of: EVQLVQSGTEVKKPGESLKISCKGSGYGFITYWIGWVRQMPGKGLEWMGIIYPGDSE TRYSPSFQGQVTISADKSINTAYLQWSSLKASDTAIYYCAGGSGISTPMDVWGQGTT VTVSS (SEQ ID NO: 9) or an equivalent thereof retaining CDRs of SEQ ID NO: 9; or a light chain variable domain (VL) comprising, or consisting essentially of, or yet further consisting of: DIVMTQSPDSLAVSLGERATINCKSSQSVLYSSINKNYLAWYQQKPGQPPKLLIYWA STRESGVPDRFSGSGSGTDFTLTISSLQAEDVAVYYCQQYYSTPYTFGQGTKVEIK (SEQ ID NO: 10) or an equivalent thereof retaining CDRs of SEQ ID NO: 10; or both the VH and the VL or an equivalent of each thereof. See, for example, US Patent No. US8106170B2.

The antibody CR3014 serves as a scaffold for some embodiments not encompassed by the claims. In some embodiments, wherein the antibody or fragment thereof comprises, or consists essentially of, or yet further consists of: a heavy chain complementarity determining region CDR 1 (HCDR1) comprising, or consisting essentially of, or yet further consisting of FSDHYMDW (SEQ ID NO: 102); a heavy chain CDR 2 (HCDR2) comprising, or consisting essentially of, or yet further consisting of VGRTRNKANSYTTEYAASVKGR (SEQ ID NO: 103); a heavy chain CDR 3 (HCDR3) comprising, or consisting essentially of, or yet further consisting of CARGISPFYFDYW (SEQ ID NO: 104); a light chain CDR 1 (LCDR1) comprising, or consisting essentially of, or yet further consisting of CRASQSISSYLNW (SEQ ID NO: 105); a light chain CDR 2 (LCDR2) comprising, or consisting essentially of, or yet further consisting of YAASSLQSG (SEQ ID NO: 106); and a light chain CDR 3 (LCDR3) comprising, or consisting essentially of, or yet further consisting of CQQSYSTPPTF (SEQ ID NO: 107).

In some embodiments not encompassed by the claims, the antibody or fragment thereof comprises, or consists essentially of, or yet further consists of: CDRs of a heavy chain variable domain (VH) comprising, or consisting essentially of, or yet further consisting of: EVQLVESGGGLVQPGGSLRLSCAASGFTFSDHYMDWVRQAPGKGLEWVGRTRNKA NSYTTEYAASVKGRFTISRDDSKNSLYLQMNSLKTEDTAVYYCARGISPFYFDYWG QGTLVTVSS (SEQ ID NO: 22); and CDRs of a light chain variable domain (VL) comprising, or consisting essentially of, or yet further consisting of:

DIQMTQSPSSLSASVGDRVTITCRASQSISSYLNWYQQKPGKAPKLLIYAASSLQSGV
PSRFSGSGSGTDFTLTISSLQPEDFATYYCQQSYSTPPTFGQGTKVEIK (SEQ ID NO:
38).

In some embodiments not encompassed by the claims, the antibody or fragment thereof comprises, or consists essentially of, or yet further consists of: a heavy chain variable domain (VH) comprising, or consisting essentially of, or yet further consisting of: EVQLVESGGGLVQPGGSLRLSCAASGFTFSDHYMDWVRQAPGKGLEWVGRTRNKA NSYTTEYAASVKGRFTISRDDSKNSLYLQMNSLKTEDTAVYYCARGISPFYFDYWG QGTLVTVSS (SEQ ID NO: 22) or an equivalent thereof retaining CDRs of SEQ ID NO: 22; or a light chain variable domain (VL) comprising, or consisting essentially of, or yet further consisting of DIQMTQSPSSLSASVGDRVTITCRASQSISSYLNWYQQKPGKAPKLLIYAASSLQSGV PSRFSGSGSGTDFTLTISSLQPEDFATYYCQQSYSTPPTFGQGTKVEIK (SEQ ID NO: 38) or an equivalent thereof retaining CDRs of SEQ ID NO: 38; or both the VH and the VL or an equivalent of each thereof.

In further embodiments not encompassed by the claims, the S protein or fragment thereof comprises, or consists essentially of, or yet further consists of receptor binding domain (RBD) of the SAR-CoV2 S protein, such as 
RVVPSGDVVRFPNITNLCPFGEVFNATKFPSVYAWERKKISNCVADYSVLYNSTFFST
FKCYGVSATKLNDLCFSNVYADSFVVKGDDVRQIAPGQTGVIADYNYKLPDDFMGC
VLAWNTRNIDATSTGNYNYKYRYLRHGKLRPFERDISNVPFSPDGKPCTPPALNCY
WPLNDYGFYTTTGIGYQPYRVVVLSFELLNAPATVCGPKLSTDLIKNQCVNF (SEQ
ID NO: 28),
 
MFVFLVLLPLVSSQCVNLTTRTQLPPAYTNSFTRGVYYPDKVFRSSVLHSTQDLFLPF
FSNVTWFHVISGTNGTKRFDNPVLPFNDGVYFASIEKSNIIRGWIFGTTLDSKTQSLLI
VNNATNVVIKVCEFQFCNDPFLDHKNNKSWMESEFRVYSSANNCTFEYVSQPFLMD
LEGKQGNFKNLREFVFKNIDGYFKIYSKHTPIIVREPEDLPQGFSALEPLVDLPIGINIT
RFQTLLALHRSYLTPGDSSSGWTAGAAAYYVGYLQPRTFLLKYNENGTITDAVDCA
LDPLSETKCTLKSFTVEKGIYQTSNFRVQPTESIVRFPNITNLCPFDEVFNATRFASVY
AWNRKRISNCVADYSVLYNLAPFFTFKCYGVSPTKLNDLCFTNVYADSFVIRGDEVR
QIAPGQTGNIADYNYKLPDDFTGCVIAWNSNKLDSKVSGNYNYLYRLFRKSNLKPFE
RDISTEIYQAGNKPCNGVAGFNCYFPLRSYSFRPTYGVGHQPYRVVVLSFELLHAPA
TVCGPKKSTNLVKNKCVNFNFNGLKGTGVLTESNKKFLPFQQFGRDIADTTDAVRD
PQTLEILDITPCSFGGVSVITPGTNTSNQVAVLYQGVNCTEVPVAIHADQLTPTWRVY
STGSNVFQTRAGCLIGAEYVNNSYECDIPIGAGICASYQTQTKSHRRARSVASQSIIAY
TMSLGAENSVAYSNNSIAIPTNFTISVTTEILPVSMTKTSVDCTMYICGDSTECSNLLL
QYGSFCTQLKRALTGIAVEQDKNTQEVFAQVKQIYKTPPIKYFGGFNFSQILPDPSKP
SKRSFIEDLLFNKVTLADAGFIKQYGDCLGDIAARDLICAQKFKGLTVLPPLLTDEMI
AQYTSALLAGTITSGWTFGAGAALQIPFAMQMAYRFNGIGVTQNVLYENQKLIANQ
FNSAIGKIQDSLSSTASALGKLQDVVNHNAQALNTLVKQLSSKFGAISSVLNDIFSRL
 
DKVEAEVQIDRLITGRLQSLQTYVTQQLIRAAEIRASANLAATKMSECVLGQSKRVD
FCGKGYHLMSFPQSAPHGVVFLHVTYVPAQEKNFTTAPAICHDGKAHFPREGVFVS
NGTHWFVTQRNFYEPQIITTDNTFVSGNCDVVIGIVNNTVYDPLQPELDSFKEELDKY
FKNHTSPDVDLGDISGINASVVNIQKEIDRLNEVAKNLNESLIDLQELGKYEQYIKWP
WYIWLGFIAGLIAIVMVTIMLCCMTSCCSCLKGCCSCGSCCKFDEDDSEPVLKGVKL
HYT. (SEQ ID NO: 79),
 or

MFVFLVLLPLVSSQCVNLTTRTQLPPAYTNSFTRGVYYPDKVFRSSVLHSTQDLFLPF
FSNVTWFHVISGTNGTKRFDNPVLPFNDGVYFASIEKSNIIRGWIFGTTLDSKTQSLLI
VNNATNVVIKVCEFQFCNDPFLDHKNNKSWMESEFRVYSSANNCTFEYVSQPFLMD
LEGKQGNFKNLREFVFKNIDGYFKIYSKHTPIIVREPEDLPQGFSALEPLVDLPIGINIT
RFQTLLALHRSYLTPGDSSSGWTAGAAAYYVGYLQPRTFLLKYNENGTITDAVDCA
LDPLSETKCTLKSFTVEKGIYQTSNFRVQPTESIVRFPNITNLCPFDEVFNATRFASVY
AWNRKRISNCVADYSVLYNLAPFFTFKCYGVSPTKLNDLCFTNVYADSFVIRGDEVR
QIAPGQTGNIADYNYKLPDDFTGCVIAWNSNKLDSKVSGNYNYRYRLFRKSNLKPFE
RDISTEIYQAGNKPCNGVAGVNCYFPLQSYSFRPTYGVGHQPYRVVVLSFELLHAPA
TVCGPKKSTNLVKNKCVNFNFNGLKGTGVLTESNKKFLPFQQFGRDIADTTDAVRD
PQTLEILDITPCSFGGVSVITPGTNTSNQVAVLYQGVNCTEVPVAIHADQLTPTWRVY
STGSNVFQTRAGCLIGAEYVNNSYECDIPIGAGICASYQTQTKSHRRARSVASQSIIAY
TMSLGAENSVAYSNNSIAIPTNFTISVTTEILPVSMTKTSVDCTMYICGDSTECSNLLL
QYGSFCTQLKRALTGIAVEQDKNTQEVFAQVKQIYKTPPIKYFGGFNFSQILPDPSKP
SKRSFIEDLLFNKVTLADAGFIKQYGDCLGDIAARDLICAQKFKGLTVLPPLLTDEMI
AQYTSALLAGTITSGWTFGAGAALQIPFAMQMAYRFNGIGVTQNVLYENQKLIANQ
FNSAIGKIQDSLSSTASALGKLQDVVNHNAQALNTLVKQLSSKFGAISSVLNDIFSRL
DKVEAEVQIDRLITGRLQSLQTYVTQQLIRAAEIRASANLAATKMSECVLGQSKRVD
FCGKGYHLMSFPQSAPHGVVFLHVTYVPAQEKNFTTAPAICHDGKAHFPREGVFVS
NGTHWFVTQRNFYEPQIITTDNTFVSGNCDVVIGIVNNTVYDPLQPELDSFKEELDKY
FKNHTSPDVDLGDISGINASVVNIQKEIDRLNEVAKNLNESLIDLQELGKYEQYIKWP
WYIWLGFIAGLIAIVMVTIMLCCMTSCCSCLKGCCSCGSCCKFDEDDSEPVLKGVKL
HYT (SEQ ID NO: 108).

In further embodiments not encompassed by the claims, the antibody or fragment thereof comprises, or consists essentially of, or yet further consists of an anti-SARS-CoV-2 RBD antibody or a fragment thereof, such as Clone #: C135 as disclosed in RCSB PDB ID: 7K8R and Barnes et al. Nature. 2020 Dec; 588(7839):682-687. In some embodiments, wherein the antibody or fragment thereof comprises, or consists essentially of, or yet further consists of: a heavy chain complementarity determining region a heavy chain CDR 3 (HCDR3) comprising, or consisting essentially of, or yet further consisting of ASSSGYLFHSDY (SEQ ID NO: 94); a light chain CDR 3 (LCDR3) comprising, or consisting essentially of, or yet further consisting of QQYNSYPWT (SEQ ID NO: 95).

In yet further embodiments not encompassed by the claims, the antibody or fragment thereof comprises, or consists essentially of, or yet further consists of CDRs of a VH comprising, or consisting essentially of, or yet further consisting of: QVQLVESGGGVVQPGRSLRLSCAASGFTFSSYAMHWVRQAPGKGLEWVAVIPFDG RNKYYADSVTGRFTISRDNSKNTLYLQMNSLRAEDTAVYYCASSSGYLFHSDYWGQ GTLVTVSS (SEQ ID NO: 31), and CDRs of a VL comprising, or consisting essentially of, or yet further consisting of:

DIQMTQSPSTLSASVGDRVTITCRASQSISNWLAWFQQKPGKAPKLLIYEASSLESGV
PSRFSGSGSGTEFTLTISSLQPDDFATYYCQQYNSYPWTFGQGTKVEIK (SEQ ID NO:
32).

In some embodiments not encompassed by the claims, the antibody or fragment thereof comprises, or consists essentially of, or yet further consists of a VH comprising, or consisting essentially of, or yet further consisting of: QVQLVESGGGVVQPGRSLRLSCAASGFTFSSYAMHWVRQAPGKGLEWVAVIPFDG RNKYYADSVTGRFTISRDNSKNTLYLQMNSLRAEDTAVYYCASSSGYLFHSDYWGQ GTLVTVSS (SEQ ID NO: 31) or an equivalent thereof, and a VL comprising, or consisting essentially of, or yet further consisting of: DIQMTQSPSTLSASVGDRVTITCRASQSISNWLAWFQQKPGKAPKLLIYEASSLESGV PSRFSGSGSGTEFTLTISSLQPDDFATYYCQQYNSYPWTFGQGTKVEIK (SEQ ID NO: 32) or an equivalent thereof. In some embodiments, the equivalent of a VH or a VL retains the CDRs of the VH or VL.

In some embodiments not encompassed by the claims, the SARS-CoV-2 antigen comprises, or consists essentially of, or yet further consists of a SARS-CoV-2 S1 protein or a fragment thereof. In some embodiments, the SARS-CoV-2 antigen comprises, or consists essentially of, or yet further consists of a SARS-CoV-2 S1 protein or a fragment thereof not comprising the RBD. In further embodiments, the antibody or fragment thereof comprises, or consists essentially of, or yet further consists of an anti-SARS-CoV-2 S1 antibody or a fragment thereof, such as Clone #: 4A8 as disclosed in Chi et al. Science. 2020 Aug 7; 369(6504):650-655. In some embodiments, wherein the antibody or fragment thereof comprises, or consists essentially of, or yet further consists of: a heavy chain complementarity determining region CDR 1 (HCDR1) comprising, or consisting essentially of, or yet further consisting of GYTLTELS (SEQ ID NO: 96); a heavy chain CDR 2 (HCDR2) comprising, or consisting essentially of, or yet further consisting of FDPEDGET (SEQ ID NO: 97); a heavy chain CDR 3 (HCDR3) comprising, or consisting essentially of, or yet further consisting of ATSTAVAGTPDLFDYYYGMDV (SEQ ID NO: 98); a light chain CDR 1 (LCDR1) comprising, or consisting essentially of, or yet further consisting of QSLVHSDGNTY (SEQ ID NO: 99); a light chain CDR 2 (LCDR2) comprising, or consisting essentially of, or yet further consisting of KIS (SEQ ID NO: 100); and a light chain CDR 3 (LCDR3) comprising, or consisting essentially of, or yet further consisting of TQATQFPYT (SEQ ID NO: 101).

In some embodiments not encompassed by the claims, the antibody or fragment thereof comprises, or consists essentially of, or yet further consists of: CDRs of a heavy chain variable domain (VH) comprising, or consisting essentially of, or yet further consisting of: EVQLVESGAEVKKPGASVKVSCKVSGYTLTELSMHWVRQAPGKGLEWMGGFDPED GETMYAQKFQGRVTMTEDTSTDTAYMELSSLRSEDTAVYYCATSTAVAGTPDLFDY YYGMDVWGQGTTVTVSS (SEQ ID NO: 33), and CDRs of a VL comprising, or consisting essentially of, or yet further consisting of:

EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIK (SEQ
ID NO: 34).

In some embodiments not encompassed by the claims, the antibody or fragment thereof comprises, or consists essentially of, or yet further consists of a VH comprising, or consisting essentially of, or yet further consisting of: EVQLVESGAEVKKPGASVKVSCKVSGYTLTELSMHWVRQAPGKGLEWMGGFDPED GETMYAQKFQGRVTMTEDTSTDTAYMELSSLRSEDTAVYYCATSTAVAGTPDLFDY YYGMDVWGQGTTVTVSS (SEQ ID NO: 33) or an equivalent thereof, and a VL comprising, or consisting essentially of, or yet further consisting of: EIVMTQSPLSSPVTLGQPASISCRSSQSLVHSDGNTYLSWLQQRPGQPPRLLIYKISNR FSGVPDRFSGSGAGTDFTLKISRVEAEDVGVYYCTQATQFPYTFGQGTKVDIK (SEQ ID NO: 34) or an equivalent thereof. In some embodiments, the equivalent of a VH or a VL retains the CDRs of the VH or VL.

In some embodiments not encompassed by the claims, the SARS-CoV-2 antigen comprises, or consists essentially of, or yet further consists of a nucleocapsid (N) of a SARS-CoV-2. In further embodiments, the antibody or fragment thereof comprises, or consists essentially of, or yet further consists of an anti-SARS-CoV-2 N antibody S309 or fragment thereof. See, for example, Kang et al. Nat Commun. 2021 May 11; 12(1):2697. In yet further embodiments, the antibody or fragment S309 thereof comprises, or consists essentially of, or yet further consists of CDRs of a VH comprising, or consisting essentially of, or yet further consisting of: QVQLVESGGGVVQPGRSLRLSCAASGFTFSSYIMHWVRQAPGKGLEWVAVISYDGS NEAYADSVKGRFTISRDNSKNTLYLQMSSLRAEDTGVYYCARETGDYSSSWYDSW GRGTLVTVSS (SEQ ID NO: 29) and CDRs of a VL comprising, or consisting essentially of, or yet further consisting of: QLVLTQSPSASASLGASVKLTCTLSSGHSNYAIAWHQQQPEKGPRYLMKVNSDGSH TKGDGIPDRFSGSSSGAERYLTISSLQSEDEADYYCQTWGTGIQVFGGGTKLTVL (SEQ ID NO: 30). In some embodiments, HCDR1 of SEQ ID NO: 29 comprises, or consists essentially of, or yet further consist of GFTFSSY (SEQ ID NO: 44). In some embodiments, HCDR2 of SEQ ID NO: 29 comprises, or consists essentially of, or yet further consist of SYDGSN (SEQ ID NO: 45). In some embodiments, HCDR3 of SEQ ID NO: 29 comprises, or consists essentially of, or yet further consist of ETGDYSSSWYDS (SEQ ID NO: 46). In some embodiments, LCDR1 of SEQ ID NO: 30 comprises, or consists essentially of, or yet further consist of TLSSGHSNYAIA (SEQ ID NO: 47). In some embodiments, LCDR2 of SEQ ID NO: 30 comprises, or consists essentially of, or yet further consist of VNSDGSHTKGD (SEQ ID NO: 48). In some embodiments, LCDR3 of SEQ ID NO: 30 comprises, or consists essentially of, or yet further consist of QTWGTGIQV (SEQ ID NO: 49). In yet further embodiments, the antibody or fragment thereof comprises, or consists essentially of, or yet further consists of a VH comprising, or consisting essentially of, or yet further consisting of: QVQLVESGGGVVQPGRSLRLSCAASGFTFSSYIMHWVRQAPGKGLEWVAVISYDGS NEAYADSVKGRFTISRDNSKNTLYLQMSSLRAEDTGVYYCARETGDYSSSWYDSW GRGTLVTVSS (SEQ ID NO: 29) or an equivalent thereof and a VL comprising, or consisting essentially of, or yet further consisting of: QLVLTQSPSASASLGASVKLTCTLSSGHSNYAIAWHQQQPEKGPRYLMKVNSDGSH TKGDGIPDRFSGSSSGAERYLTISSLQSEDEADYYCQTWGTGIQVFGGGTKLTVL (SEQ ID NO: 30) or an equivalent thereof. In some embodiments, the equivalent of a VH or a VL retains the CDRs of the VH or VL.

In some embodiments not encompassed by the claims, other SARS-CoV-2 antigen can be specifically recognized or bound to by the antibody or fragment thereof and/or the CAR as disclosed herein. In some embodiments, other viral antigens can be specifically recognized or bound to by the antibody or fragment thereof and/or the CAR as disclosed herein.

In further embodiments, the antibody or antibody fragment binds to an oncogenic antigen or an antigen associated with cancer, also known as a tumor associated antigen.

In some embodiments, a fragment of the antibody is an antigen binding fragment. In some embodiments, the fragment comprises, or consists essentially of, or yet further consists of a single chain variable fragment (scFv) of an antibody that binds a TAA.

As used herein, the terms tumor associated antigen (TAA), cancer antigen, tumor antigen, cancer relevant antigen, and tumor relevant antigen are used interchangeably herein, referring to antigenic substance of a cancer or tumor cells. In some embodiments, a TAA presents on some tumor or cancer cells and also on some normal cells, optionally at a lower level. In some embodiments, a TAA only presents on a tumor or cancer cell but not on a normal cell. In some embodiments, a TAA refers to a TAA recognized and bound by a CAR as disclosed herein. In some embodiments, a TAA refers to a TAA recognized and bound by an antibody as disclosed herein. In some embodiments, a TAA is selected from BCMA, GPRC5D, FLT3, CD19, mesothelin, human epidermal growth factor receptor 2 (HER2), prostate stem cell antigen (PSCA), carcinoembryonic antigen (CEA), CD33, GTPase-activating protein (GAP), ganglioside G2 (GD2), CD5, prostate specific membrane antigen (PSMA), receptor tyrosine kinase-like orphan receptor 1 (ROR1), CD123, CD70, CD38, mucin 1, (Muc1), ephrin type-A receptor 2 precursor (EphA2), epidermal growth factor receptor variant III (EGFRVIII), interleukin 13 receptor alpha 2 (IL13Ra2), CD133, glypican 3 (GPC3), epithelial cell adhesion molecule precursor (EpCam), fibroblast activation protein alpha (FAP), vascular endothelial growth factor receptor 2 (VEGFR2), cancer/testis (CT), guanylyl cyclase C (GUCY2C), tumor-associated glycoprotein-72 (TAG-72), thymidine kinase 1 (TK1), or hypoxanthine guanine phosphoribosyltransferase (HPRT1). In one specific embodiment, the TAA is CD19 and the anti-TAA antibody or fragment thereof binds to CD19.

In some embodiments, the fragment comprises, or consists essentially of, or yet further consists of a single-chain variable fragment (scFv) comprising, or consisting essentially of, or yet further consisting of the VH, the VL, and a peptide linker between the VH and the VL.

In some embodiments, the peptide linker comprises, or consists essentially of, or yet further consists of GGGGSGGGGSGGGGS (SEQ ID NO: 11) or an equivalent thereof.

In some embodiments, the transmembrane domain comprises, or consists essentially of, or yet further consists of a CD28 transmembrane domain comprising, or consisting essentially of, or yet further consisting of FWVLVVVGGVLACYSLLVTVAFIIFWV (SEQ ID NO: 12) or an equivalent thereof.

Non-limiting exemplary co-stimulatory domains are provided herein. In some embodiments, the co-stimulatory domain comprises, or consists essentially of, or yet further consists of a CD28 co-stimulatory domain comprising, or consisting essentially of, or yet further consisting of RSKRSRLLHSDYMNMTPRRPGPTRKHYQPYAPPRDFAAYRS (SEQ ID NO: 13) or an equivalent thereof. In further embodiments, the equivalent of this sequence comprises, or consists essentially of, or yet further consists of a mutated sequence comprising one or more mutations, such as a G corresponding to the 7^{th} amino acid residue of SEQ ID NO: 13 which is L, or a G corresponding to the 8^{th} amino acid residue of this sequence which is L, or both. See, for example, Wang et al., Hum Gene Ther. 2007 Aug; 18(8):712-25. In yet further embodiments, the equivalent of this sequence comprises, or consists essentially of, or yet further consists of RSKRSRGGHSDYMNMTPRRPGPTRKHYQPYAPPRDFAAYRS (SEQ ID NO: 24) or an equivalent thereof.

As used herein, the term "4-1BB costimulatory signaling region" refers to a specific protein fragment associated with this name and any other molecules that have analogous biological function that share at least 70%, or alternatively at least 80% amino acid sequence identity, preferably 90% sequence identity, more preferably at least 95% sequence identity with the 4-1BB costimulatory signaling region sequence as shown herein. Non-limiting example sequences of the 4-1BB costimulatory signaling region are provided in U.S. Publication 20130266551A1 (filed as U.S. App. No. 13/826,258), such as the exemplary sequence provided below:
4-1BB costimulatory signaling region: KRGRKKLLYIFKQPFMRPVQTTQEEDGCSCRFPEEEEGGCEL (SEQ ID NO: 109) or an equivalent thereof.

As used herein, the term "2B4 costimulatory signaling region" refers to a specific protein fragment associated with this name and any other molecules that have analogous biological function that share at least 70%, or alternatively at least 80% amino acid sequence identity, preferably 90% sequence identity, more preferably at least 95% sequence identity with the 2B4 costimulatory signaling region sequence shown herein. 2B4 costimulatory signaling region: WRRKRKEKQSETSPKEFLTIYEDVKDLKTRRNHEQEQTFPGGGSTIYSMIQSQSSAPT SQEPAYTLYSLIQPSRKSGSRKRNHSPSFNSTIYEVIGKSQPKAQNPARLSRKELENFD VYS (SEQ ID NO: 110) or an equivalent thereof.

As used herein, the term "ICOS costimulatory signaling region" refers to a specific protein fragment associated with this name and any other molecules that have analogous biological function that share at least 70%, or alternatively at least 80% amino acid sequence identity, preferably 90% sequence identity, more preferably at least 95% sequence identity with the ICOS costimulatory signaling region sequence as shown herein. Non-limiting example sequences of the ICOS costimulatory signaling region are provided in U.S. Publication 2015/0017141A1 the exemplary polynucleotide sequence provided below.

ICOS costimulatory signaling region coding sequence:

ACAAAAAAGA AGTATTCATC CAGTGTGCAC GACCCTAACG GTGAATACAT
GTTCATGAGA GCAGTGAACA CAGCCAAAAA ATCCAGACTC ACAGATGTGA
CCCTA (SEQ ID NO: 114)

As used herein, the term "OX40 costimulatory signaling region" refers to a specific protein fragment associated with this name and any other molecules that have analogous biological function that share at least 70%, or alternatively at least 80% amino acid sequence identity, or alternatively 90% sequence identity, or alternatively at least 95% sequence identity with the OX40 costimulatory signaling region sequence as shown herein. Non-limiting example sequences of the OX40 costimulatory signaling region are disclosed in U.S. Publication 2012/20148552A1, and include the exemplary sequence OX40 costimulatory signaling region coding sequence: AGGGACCAG AGGCTGCCCC CCGATGCCCA CAAGCCCCCT GGGGGAGGCA GTTTCCGGAC CCCCATCCAA GAGGAGCAGG CCGACGCCCA CTCCACCCTG GCCAAGATC (SEQ ID NO: 111), and equivalents thereof.

As used herein, the term "DAP10 costimulatory signaling region" or "DAP10 costimulatory region" refers to a specific protein fragment associated with this name or any other molecules that have analogous biological function that share at least about 70%, or alternatively at least about 80% amino acid sequence identity, or alternatively at least about 90% sequence identity, or alternatively at least about 95% sequence identity with the DAP10 costimulatory signaling region sequence as shown herein. Non-limiting example sequences of the DAP10 costimulatory signaling region are disclosed in U.S. Patent No. 9,587,020B2, and include the exemplary sequence: RPRRSPAQDGKVYINMPGRG (SEQ ID NO: 115), or equivalents thereof.

As used herein, the term "DAP12 costimulatory signaling region" or "DAP12 costimulatory region" refers to a specific protein fragment associated with this name or any other molecules that have analogous biological function that share at least about 70%, or alternatively at least about 80% amino acid sequence identity, or alternatively at least about 90% sequence identity, or alternatively at least about 95% sequence identity with the DAP12 costimulatory signaling region sequence as disclosed in U.S. Patent No. 9,587,020B2. Non-limiting example sequences of the DAP12 costimulatory signaling region are disclosed in U, and include the exemplary sequence: ESPYQELQGQRSDVYSDLNTQ (SEQ ID NO: 116), or equivalents thereof.

In some embodiments, the intracellular signaling domain comprises, or consists essentially of, or yet further consists of a CD3ζ intracellular signaling domain comprising, or consisting essentially of, or yet further consisting of RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDALHMQAL PPR (SEQ ID NO: 14) or an equivalent thereof.

In some embodiments, a polypeptide as disclosed herein further comprises a detectable marker. In further embodiments, the detectable marker comprises, or consists essentially of, or yet further consists of any one or more of: a flag tag comprising, or consisting essentially of, or yet further consisting of DYKDDDDK (SEQ ID NO: 15), a His tag comprising, or consisting essentially of, or yet further consisting of HHHHHH (SEQ ID NO: 16), a Myc tag comprising, or consisting essentially of, or yet further consisting of EQKLISEEDL (SEQ ID NO: 17), an HA tag comprising, or consisting essentially of, or yet further consisting of YPYDVPDYA (SEQ ID NO: 18), or an equivalent of each thereof.

In some embodiments, a polypeptide as disclosed herein further comprises a signal peptide at the N terminus of the CAR. In further embodiments, the signal peptide comprises, or consists essentially of, or yet further consists of any one or more of: a CD8α signal peptide comprising, or consisting essentially of, or yet further consisting of MALPVTALLLPLALLLHAARP (SEQ ID NO: 25), a CD4 signal peptide comprising, or consisting essentially of, or yet further consisting of MNRGVPFRHLLLVLQLALLPAATQG (SEQ ID NO: 26), a CD28 signal peptide comprising, or consisting essentially of, or yet further consisting of MLRLLLALNLFPSIQVTG (SEQ ID NO: 27), a GM-CSF signal peptide comprising, or consisting essentially of, or yet further consisting of MWLQSLLLLGTVACSIS (SEQ ID NO: 19) or an equivalent thereof.

In some embodiments, the equivalent of any one of SEQ ID NOS: 1-19, 22-37, 39-42, 45-49, 79, and 94-107 is at least 80%, or at least 85%, or at least 90%, or at least 95%, or at least 96%, or at least 97%, or at least 98%, or at least 99% identical to the one of SEQ ID NOS: 1-19, 22-37, 39-42, 45-49, 79, and 94-107, respectively.

Some embodiments not encompassed by the claims comprise a construct whose subcomponents are shown in FIG. 1A. Other embodiments not encompassed by the claims comprise a construct whose subcomponents are shown in FIG. 16. Still other embodiments not encompassed by the claims comprise a construct whose subcomponents are shown in FIG. 22.

In some embodiments, a polypeptide as disclosed herein further comprises a suicide gene product. In further embodiments, the suicide gene product comprises, or consists essentially of, or yet further consists of one or more of: thymidine kinase (TK) optionally herpes simplex virus thymidine kinase (HSV-TK), purine nucleoside phosphorylase (PNP), cytosine deaminase (CD), carboxypetidase G2, cytochrome P450, linamarase, beta-lactamase, nitroreductase (NTR), carboxypeptidase A, inducible caspase 9, or truncated epidermal growth factor receptor (EGFR).

In some embodiments, a polypeptide as disclosed herein further comprises a cleavable peptide between the suicide gene product and the CAR.

In some embodiments, a polypeptide as disclosed herein is recombinant or isolated.

In some embodiments not encompassed by the claims, a polypeptide as disclosed herein comprises, or consists essentially of, or yet further consists of MALPVTALLLPLALLLHAARPDIVMTQSPDSLAVSLGERATINCKSSQSVLYSSINKN YLAWYQQKPGQPPKLLIYWASTRESGVPDRFSGSGSGTDFTLTISSLQAEDVAVYYC QQYYSTPYTFGQGTKVEIKGGGGSGGGGSGGGGSEVQLVQSGTEVKKPGESLKISCK GSGYGFITYWIGWVRQMPGKGLEWMGIIYPGDSETRYSPSFQGQVTISADKSINTAY LQWSSLKASDTAIYYCAGGSGISTPMDVWGQGTTVTVSSDYKDDDDKESKYGPPCP PCPAPPVAGPSVFLFPPKPKDTLMISRTPEVTCVVVDVSQEDPEVQFNWYVDGVEVH NAKTKPREEQFQSTYRVVSVLTVLHQDWLNGKEYKCKVSNKGLPSSIEKTISKAKG QPREPQVYTLPPSQEEMTKNQVSLTCLVKGFYPSDIAVEWESNGQPENNYKTTPPVL DSDGSFFLYSRLTVDKSRWQEGNVFSCSVMHEALHNHYTQKSLSLSLGKFWVLVVV GGVLACYSLLVTVAFIIFWVRSKRSRGGHSDYMNMTPRRPGPTRKHYQPYAPPRDF AAYRSRVKFSRSADAPAYQQGQNQLYNELNLGRREEYDVLDKRRGRDPEMGGKPR RKNPQEGLYNELQKDKMAEAYSEIGMKGERRRGKGHDGLYQGLSTATKDTYDALH MQALPPR (SEQ ID NO: 20), the sequence SEQ ID NO: 80, the sequence SEQ ID NO: 81, the sequence SEQ ID NO: 82, the sequence SEQ ID NO: 83, the sequence SEQ ID NO. 85, the sequence SEQ ID NO. 86, the sequence SEQ ID NO. 87, the sequence SEQ ID NO: 88, the sequence SEQ ID NO: 89, the sequence SEQ ID NO: 90, the sequence SEQ ID NO: 91, the sequence SEQ ID NO: 92, the sequence SEQ ID NO: 93 or an equivalent thereof.

In a further aspect not encompassed by the claims, provided is a polynucleotide encoding a polypeptide as disclosed herein, or a polynucleotide complementary thereto. In some embodiments not encompassed by the claims, the polynucleotide comprises, or consists essentially of, or yet further consists of: 
ATGGCTCTGCCTGTGACAGCTCTGCTGCTGCCTCTGGCTCTGCTTCTGCATGCCGC
CAGACCTGACATCGTGATGACACAGAGCCCTGACAGCCTGGCCGTGTCTCTGGG
AGAAAGAGCCACCATCAACTGCAAGAGCAGCCAGAGCGTGCTGTACTCCAGCAT
CAACAAGAACTACCTGGCCTGGTATCAGCAGAAGCCCGGCCAGCCTCCTAAGCT
GCTGATCTACTGGGCCAGCACCAGAGAAAGCGGCGTGCCCGATAGATTTTCTGG
CAGCGGCTCTGGCACCGACTTCACCCTGACAATTAGCTCCCTGCAGGCCGAGGAT
GTGGCCGTGTACTACTGCCAGCAGTACTACAGCACCCCTTACACCTTTGGCCAGG
 
GCACCAAGGTGGAAATCAAAGGCGGCGGAGGATCTGGCGGAGGTGGAAGTGGC
GGAGGCGGATCTGAAGTTCAGCTGGTGCAGAGCGGCACCGAAGTGAAGAAGCCT
GGCGAGAGCCTGAAGATCTCCTGCAAAGGCTCCGGCTACGGCTTCATCACCTACT
GGATCGGCTGGGTCCGACAGATGCCTGGCAAAGGCCTTGAGTGGATGGGCATCA
TCTACCCCGGCGACAGCGAGACAAGATACAGCCCTAGCTTCCAGGGCCAAGTGA
CCATCAGCGCCGACAAGAGCATCAATACCGCCTACCTGCAGTGGTCCAGCCTGA
AGGCCTCTGACACCGCCATCTACTATTGTGCCGGCGGAAGCGGCATCAGCACCCC
AATGGATGTTTGGGGCCAGGGAACCACCGTGACCGTTTCTTCTGACTACAAGGAC
GACGACGACAAGGAATCTAAGTACGGCCCTCCTTGTCCTCCATGTCCTGCTCCAC
CTGTGGCCGGACCCTCCGTGTTCCTGTTTCCTCCAAAGCCTAAGGACACCCTGAT
GATCAGCAGAACCCCTGAAGTGACCTGCGTGGTGGTGGACGTTTCCCAAGAGGA
CCCTGAGGTGCAGTTCAATTGGTACGTGGACGGCGTGGAAGTGCACAACGCCAA
GACCAAGCCTAGAGAGGAACAGTTCCAGAGCACCTACAGAGTGGTGTCCGTGCT
GACCGTGCTGCACCAGGATTGGCTGAACGGCAAAGAGTACAAGTGCAAGGTGTC
CAACAAGGGCCTGCCTAGCAGCATCGAGAAAACCATCAGCAAGGCCAAGGGCC
AGCCAAGAGAACCCCAGGTGTACACACTGCCTCCAAGCCAAGAGGAAATGACCA
AGAACCAGGTGTCCCTGACCTGCCTGGTCAAGGGCTTCTACCCTTCCGATATCGC
CGTGGAATGGGAGAGCAATGGCCAGCCTGAGAACAACTACAAGACCACACCTCC
TGTGCTGGACAGCGACGGCTCATTCTTCCTGTACAGCAGACTGACCGTGGACAAG
AGCAGATGGCAAGAGGGCAACGTGTTCAGCTGCAGCGTGATGCACGAGGCCCTG
CACAACCACTACACCCAGAAGTCTCTGAGCCTGAGCCTGGGCAAGTTCTGGGTG
CTCGTTGTTGTTGGCGGCGTGCTGGCCTGTTACTCTCTGCTGGTTACCGTGGCCTT
CATCATCTTTTGGGTCCGAAGCAAGCGGAGCAGAGGCGGCCACAGCGACTACAT
GAACATGACCCCTAGACGGCCCGGACCAACCAGAAAGCACTACCAGCCTTACGC
TCCTCCTAGAGACTTCGCCGCCTACCGGTCCAGAGTGAAGTTCAGCAGATCCGCC
GATGCTCCCGCCTATCAGCAGGGACAGAACCAGCTGTACAACGAGCTGAACCTG
GGGAGAAGAGAAGAGTACGACGTGCTGGACAAGCGGAGAGGCAGAGATCCTGA
GATGGGCGGCAAGCCCAGACGGAAGAATCCTCAAGAGGGCCTGTATAATGAGCT
GCAGAAAGACAAGATGGCCGAGGCCTACAGCGAGATCGGAATGAAGGGCGAGC
GCAGAAGAGGCAAGGGACACGATGGACTGTACCAGGGCCTGAGCACCGCCACC
AAGGATACCTATGATGCCCTGCACATGCAGGCCCTGCCTCCAAGATAA (SEQ ID
NO: 21),
 AUGGCUCUGCCUGUGACAGCUCUGCUGCUGCCUCUGGCUCUGCUUCUGCAUGC CGCCAGACCUGACAUCGUGAUGACACAGAGCCCUGACAGCCUGGCCGUGUCUC UGGGAGAAAGAGCCACCAUCAACUGCAAGAGCAGCCAGAGCGUGCUGUACUCC AGCAUCAACAAGAACUACCUGGCCUGGUAUCAGCAGAAGCCCGGCCAGCCUCC UAAGCUGCUGAUCUACUGGGCCAGCACCAGAGAAAGCGGCGUGCCCGAUAGAU UUUCUGGCAGCGGCUCUGGCACCGACUUCACCCUGACAAUUAGCUCCCUGCAG GCCGAGGAUGUGGCCGUGUACUACUGCCAGCAGUACUACAGCACCCCUUACAC CUUUGGCCAGGGCACCAAGGUGGAAAUCAAAGGCGGCGGAGGAUCUGGCGGA GGUGGAAGUGGCGGAGGCGGAUCUGAAGUUCAGCUGGUGCAGAGCGGCACCG AAGUGAAGAAGCCUGGCGAGAGCCUGAAGAUCUCCUGCAAAGGCUCCGGCUAC GGCUUCAUCACCUACUGGAUCGGCUGGGUCCGACAGAUGCCUGGCAAAGGCCU UGAGUGGAUGGGCAUCAUCUACCCCGGCGACAGCGAGACAAGAUACAGCCCUA GCUUCCAGGGCCAAGUGACCAUCAGCGCCGACAAGAGCAUCAAUACCGCCUAC CUGCAGUGGUCCAGCCUGAAGGCCUCUGACACCGCCAUCUACUAUUGUGCCGG CGGAAGCGGCAUCAGCACCCCAAUGGAUGUUUGGGGCCAGGGAACCACCGUGA CCGUUUCUUCUGACUACAAGGACGACGACGACAAGGAAUCUAAGUACGGCCCU CCUUGUCCUCCAUGUCCUGCUCCACCUGUGGCCGGACCCUCCGUGUUCCUGUU UCCUCCAAAGCCUAAGGACACCCUGAUGAUCAGCAGAACCCCUGAAGUGACCU GCGUGGUGGUGGACGUUUCCCAAGAGGACCCUGAGGUGCAGUUCAAUUGGUA CGUGGACGGCGUGGAAGUGCACAACGCCAAGACCAAGCCUAGAGAGGAACAGU UCCAGAGCACCUACAGAGUGGUGUCCGUGCUGACCGUGCUGCACCAGGAUUGG CUGAACGGCAAAGAGUACAAGUGCAAGGUGUCCAACAAGGGCCUGCCUAGCAG CAUCGAGAAAACCAUCAGCAAGGCCAAGGGCCAGCCAAGAGAACCCCAGGUGU ACACACUGCCUCCAAGCCAAGAGGAAAUGACCAAGAACCAGGUGUCCCUGACC UGCCUGGUCAAGGGCUUCUACCCUUCCGAUAUCGCCGUGGAAUGGGAGAGCAA UGGCCAGCCUGAGAACAACUACAAGACCACACCUCCUGUGCUGGACAGCGACG GCUCAUUCUUCCUGUACAGCAGACUGACCGUGGACAAGAGCAGAUGGCAAGAG GGCAACGUGUUCAGCUGCAGCGUGAUGCACGAGGCCCUGCACAACCACUACAC CCAGAAGUCUCUGAGCCUGAGCCUGGGCAAGUUCUGGGUGCUCGUUGUUGUU GGCGGCGUGCUGGCCUGUUACUCUCUGCUGGUUACCGUGGCCUUCAUCAUCUU UUGGGUCCGAAGCAAGCGGAGCAGAGGCGGCCACAGCGACUACAUGAACAUGA CCCCUAGACGGCCCGGACCAACCAGAAAGCACUACCAGCCUUACGCUCCUCCU AGAGACUUCGCCGCCUACCGGUCCAGAGUGAAGUUCAGCAGAUCCGCCGAUGC UCCCGCCUAUCAGCAGGGACAGAACCAGCUGUACAACGAGCUGAACCUGGGGA GAAGAGAAGAGUACGACGUGCUGGACAAGCGGAGAGGCAGAGAUCCUGAGAU GGGCGGCAAGCCCAGACGGAAGAAUCCUCAAGAGGGCCUGUAUAAUGAGCUGC AGAAAGACAAGAUGGCCGAGGCCUACAGCGAGAUCGGAAUGAAGGGCGAGCG CAGAAGAGGCAAGGGACACGAUGGACUGUACCAGGGCCUGAGCACCGCCACCA AGGAUACCUAUGAUGCCCUGCACAUGCAGGCCCUGCCUCCAAGAUAA (SEQ ID NO: 50), the sequence SEQ ID NO: 51, the sequence SEQ ID NO: 52, the sequence SEQ ID NO: 53, the sequence SEQ ID NO: 54, the sequence SEQ ID NO: 55, the sequence SEQ ID NO: 56, the sequence SEQ ID NO: 57, the sequence SEQ ID NO: 58, the sequence SEQ ID NO: 59, the sequence SEQ ID NO: 60, the sequence SEQ ID NO: 61, the sequence SEQ ID NO: 62, the sequence SEQ ID NO: 63, the sequence SEQ ID NO: 64, the sequence SEQ ID NO: 65, the sequence SEQ ID NO: 66, the sequence SEQ ID NO: 67, the sequence SEQ ID NO: 68, the sequence SEQ ID NO: 69, the sequence SEQ ID NO: 70, the sequence SEQ ID NO: 71, the sequence SEQ ID NO: 72, the sequence SEQ ID NO: 73, the sequence SEQ ID NO: 74, the sequence SEQ ID NO: 75, the sequence SEQ ID NO: 76, the sequence SEQ ID NO: 77, the sequence SEQ ID NO: 78, or an equivalent thereof encoding the amino acid sequence as set forth in SEQ ID NO: 20 or 80-93.

In particular embodiments, the CAR polypeptide comprises, or consists essentially of or yet further consists of a antibody or antigen binding domain that binds to a TAA, a hinge region as described herein, a CD28 transmembrane domain, a CD28 costimulatory domain and a CD3ζ intracellular signaling domain. The hinge region is LAIR1 (144aa). The CAR polypeptide can further comprise one or more linker polypeptide and/or a signal peptide.

In a further aspect, the CARs comprise a detectable or purification label.

### Polynucleotides, Vectors and Cells

Also provided is a polynucleotide encoding the CAR as disclosed herein, as well as the complement of such polynucleotide. The polynucleotide can be DNA, RNA or hybrid DNA/RNA.

In yet a further aspect, provided is a vector comprising, or consisting essentially of, or yet further consisting of a polynucleotide encoding the CAR as disclosed herein.

In some embodiments, the vector further comprises a first regulatory sequence operatively linked to the polynucleotide and directing the expression of the polynucleotide. In further embodiments, the first regulatory sequences comprise one or more of the following: a promoter, an intron, an enhancer, or a polyadenylation signal. In some embodiments, the vector further comprises a second regulatory sequence operatively linked to the polynucleotide and directing the replication of the polynucleotide.

In some embodiments, the vector is a non-viral vector. In further embodiments, the non-viral vector is a plasmid or lipid nanoparticle.

In some embodiments, the vector is a viral vector. In further embodiments, the viral vector is selected from the group of a retroviral vector, a lentiviral vector, an adenoviral vector, an adeno-associated viral vector, or a Herpes viral vector. In yet further embodiments not encompassed by the claims, the lentiviral vector comprises, or consists essentially of, or yet further consists of a viral genome comprising, or consisting essentially of, or yet further consisting of a polynucleotide encoding the amino acid sequence as set forth in SEQ ID NO: 20, the sequence SEQ ID NO: 80, the sequence SEQ ID NO: 81, the sequence SEQ ID NO: 82, the sequence SEQ ID NO: 83, the sequence SEQ ID NO. 85, the sequence SEQ ID NO. 86, the sequence SEQ ID NO. 87, the sequence SEQ ID NO: 88, the sequence SEQ ID NO: 89, the sequence SEQ ID NO: 90, the sequence SEQ ID NO: 91, the sequence SEQ ID NO: 92, the sequence SEQ ID NO: 93 or an equivalent thereof. In one aspect, provided is a cell comprising one or more of: a polypeptide as disclosed herein, a polynucleotide as disclosed herein, or a vector as disclosed herein.

In some embodiments, the cell is a prokaryotic cell. For example, the cell is an *E. coli* cell suitable for replicating a polynucleotide as disclosed herein or a vector as disclosed herein or both.

In some embodiments, the cell is a eukaryotic cell. In further embodiments, the eukaryotic cell is selected from an animal cell, a mammalian cell, a bovine cell, a feline cell, a canine cell, a murine cell, an equine cell, or a human cell. Additionally or alternatively, the eukaryotic cell is a stem cell or an immune cell. In further embodiments, the immune cell is selected from a T cell, a B cell, a NK cell, a NKT cell, a dendritic cell, a myeloid cell, a monocyte, or a macrophage.

In one aspect, provided is an immune cell comprising one or more of: a polypeptide as disclosed herein, a polynucleotide as disclosed herein, or a vector as disclosed herein. In some embodiments, the immune cell is selected from a T cell, a B cell, a NK cell, a NKT cell, a dendritic cell, a myeloid cell, a monocyte, or a macrophage. In some embodiments, the immune cell expresses a CAR on the cell surface.

In a further aspect, provided herein is a T cell, stem cell or immune cell that comprises the CAR polypeptide that comprises, or consists essentially of or yet further consists of a antibody or antigen binding domain that binds to a TAA, a hinge regions as described herein, a CD28 transmembrane domain, a CD28 costimulatory domain and a CD3ζ intracellular signaling domain. The hinge region is LAIR1 (144aa). The CAR polypeptide can further comprise one or more linker polypeptide, and/or a signal peptide.

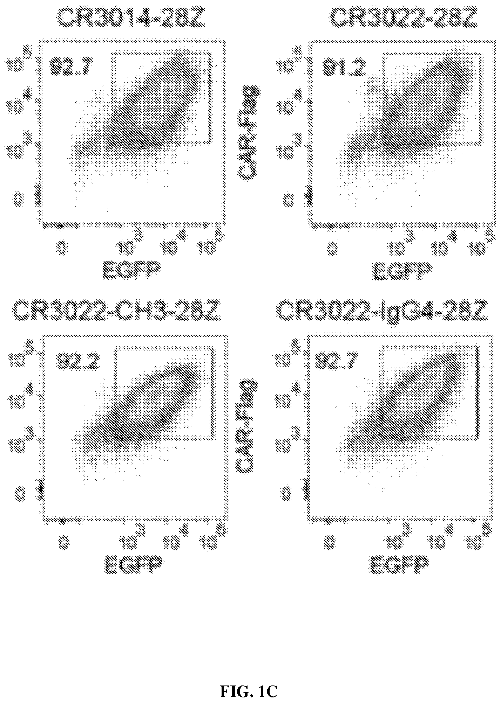

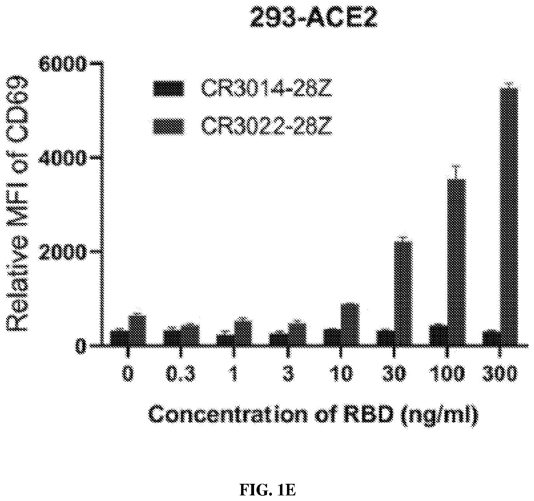

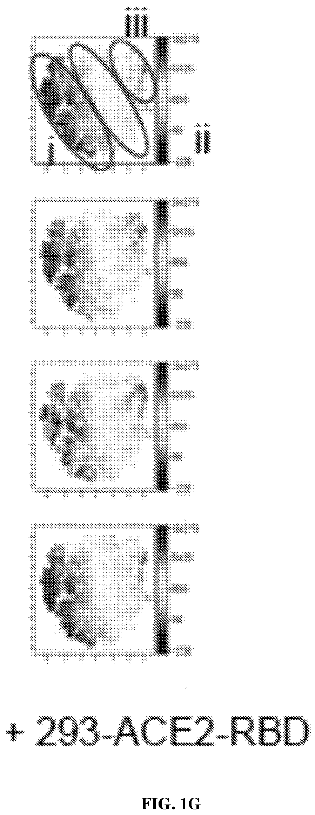

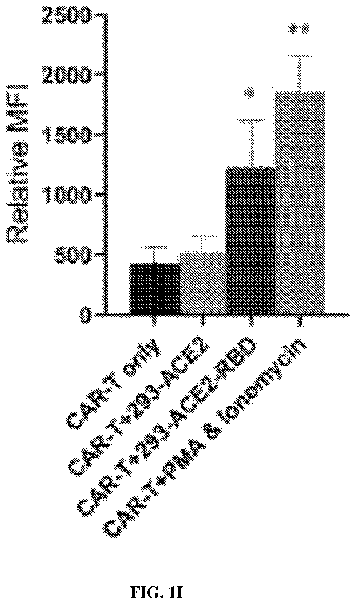

In another aspect, provided is a T cell, stem cell, or immune cell comprising: (i) granzyme B, or a polynucleotide encoding the granzyme B, or both; (ii) perforin, or a polynucleotide encoding the perforin, or both; and (iii) one or more of: a CAR polypeptide as disclosed herein, a polynucleotide as disclosed herein, or a vector as disclosed herein. In some embodiments, the T cell, stem cell, or immune cell further comprises (iv) CD69, or a polynucleotide encoding the CD69, or both; or (v) IFN-γ, or a polynucleotide encoding the IFN-γ, or both; or both (iv) and (v). In one aspect, the CAR polypeptide comprises, or consists essentially of or yet further consists of a antibody or antigen binding domain that binds to a TAA, a hinge regions as described herein, a CD28 transmembrane domain, a CD28 costimulatory domain and a CD3ζ intracellular signaling domain. The hinge region is LAIR1 (144aa). The CAR polypeptide can further comprise one or more linker polypeptide, optionally located as shown in FIG. 1. and/or a signal peptide.

In yet another aspect, provided is a histocompatible T cell, stem cell, or immune cell comprising one or more of a CAR polypeptide as disclosed herein, a polynucleotide as disclosed herein, or a vector as disclosed herein. In some embodiments, the histocompatible T cell, stem cell, or immune cell lacks a functional endogenous T cell receptor or a human leukocyte antigen (HLA) molecule or both. In one aspect, the CAR polypeptide comprises, or consists essentially of or yet further consists of an antibody or antigen binding domain that binds to a TAA, a hinge regions as described herein, a CD28 transmembrane domain, a CD28 costimulatory domain and a CD3ζ intracellular signaling domain. The hinge region is LAIR1 (144aa). The CAR polypeptide can further comprise one or more linker polypeptide, and/or a signal peptide.

In one aspect, provided is a cell population comprising, or consisting essentially of, or yet further consisting of a cell as disclosed herein. The cell population can be substantially homogeneous, e.g. at least 70%, or at least 80%, or at least 90%, or at least 95% of the cells of the population comprise the same CAR. Alternatively, provided herein is a cell population comprises cells wherein the CARs of the population are different from each other.

In another aspect, provided is a composition comprising, or consisting essentially of, or yet further consisting of a carrier and one or more of: a polypeptide as disclosed herein, a polynucleotide as disclosed herein, a vector as disclosed herein, a cell as disclosed herein, or a cell population as disclosed herein. In some embodiments, the carrier is a pharmaceutically acceptable carrier.

In further embodiments, a composition or combination of a CAR of this disclosure and a further agent, such as a therapeutic agent or a prophylactic agent or both, optionally selected from one or more of:
an anti-viral agent, optionally remdesivir, lopinavir, ritonavir, ivermectin, tamiflu, or favipiravir;
an anti-inflammatory agent optionally dexamethasone, tocilizumab, kevzara, colcrys, hydroxychloroquine, chloroquine, or a kinase inhibitor;
a covalescent plasma from a subject recovered from the viral infection;
an antibody binding to the virus, optionally bamlanivimab, etesevimab, casirivimab, or imdevimab;
an anti-cancer agent,
an antibiotic agent, optionally azithromycin; or
a vaccine.

In yet another aspect, provided is an isolated complex comprising, or consisting essentially of, or yet further consisting of a cell as disclosed herein and the viral antigen or a virus comprising the viral antigen.

In one aspect, provided is a method for producing a cell as disclosed herein. The method comprises, or consists essentially of, or yet further consists of introducing a polynucleotide as disclosed herein, a vector as disclosed herein, or both to a cell. In some embodiments, the method further comprises isolating the cell introduced with the polynucleotide or the vector or both.

### Therapeutic Methods

All references to methods of treatment by therapy or surgery in the following description are provided for information only and are to be interpreted as references to compounds and compositions of the present invention for use in those methods.

In another aspect not encompassed by the claims, provided is a method of one or more of: (a) treating a subject having or suspect of having an infection of a virus, (b) conferring anti-virus passive immunity to a subject in need thereof, (c) conferring or inducing an immune response to the virus in a subject in need thereof, or (d) neutralizing the virus in a subject in need thereof. The method comprises, or consists essentially of, or yet further consists of administering to the subject a cell as disclosed herein recognizing and binding to a viral antigen of the virus. In one aspect, T cell, stem cell or immune cell comprises a CAR polypeptide comprises, or consists essentially of or yet further consists of an antibody or antigen binding domain that binds to an S protein, a hinge regions as described herein, a CD28 transmembrane domain, a CD28 costimulatory domain and a CD3ζ intracellular signaling domain. Non-limiting examples of hinge regions are selected from one or more of PDGFRA (505aa), PDGFRB (500aa), LAIR1 (144aa) and tLAIR1 (48aa). The CAR polypeptide can further comprise one or more linker polypeptide, optionally located as shown in FIG. 1. and/or a signal peptide. The cell can be autologous or allogeneic to the subject being treated. The subject can be an animal or a human patient.

Also provided but not encompassed by the claims is an in vitro method comprising contacting a CAR as disclosed herein with a cell comprising the virus and/or expressing the viral antigen.

In some embodiments not encompassed by the claims, the virus is SARS-CoV-2.

In some embodiments, the cell was isolated from the subject and introduced with a polynucleotide as disclosed herein, a vector as disclosed herein, or both.

In some embodiments, the method further comprises treating the subject with a combined therapy. In further embodiments, the combination therapy comprises, or consists essentially of, or yet further consists of one or more of: an anti-viral agent, optionally remdesivir, lopinavir, ritonavir, ivermectin, tamiflu, or favipiravir; an anti-inflammatory agent optionally dexamethasone, tocilizumab, kevzara, colcrys, hydroxychloroquine, chloroquine, or a kinase inhibitor; a covalescent plasma from a subject recovered from the viral infection; an antibody binding to the virus, optionally bamlanivimab, etesevimab, casirivimab, or imdevimab; an antibiotic agent, optionally azithromycin; or a vaccine such as a vaccine against SARs CoV2 that would include but not exclusive to mRNA-1273 (Moderna) (US); BNT162b2 (Pfizer-BioNtech) (US); ASd26.CoV2.S (Janssen/Johnson and Johnson) (US); ChAd0x1 (Astrazeneca)(UK); NVXX-CoV2373 (Novavax) (US); CVnCoV (CureVac.GlaxoSmithKline) (Germany); Gam-COVID-Vac (Sputnik V)) (Gamaleya National Centre for Epidemiology and Microbiology) (Russia); CoronaVac (Sinovac Biotech) (China) and BBIBP-CorV (Sinopharm 1,2) (China).

In another aspect, provided is a method of one or more of treating a subject having or suspect of having cancer, in a subject in need thereof. The method comprises, or consists essentially of, or yet further consists of administering to the subject a cell as disclosed herein recognizing and binding to a TAA such as, e.g. CD19.

In one aspect, T cell, stem cell or immune cell comprises a CAR polypeptide comprises, or consists essentially of or yet further consists of an antibody or antigen binding domain that binds to a TAA, a hinge regions as described herein, a CD28 transmembrane domain, a CD28 costimulatory domain and a CD3ζ intracellular signaling domain. The hinge region is LAIR1 (144aa). The CAR polypeptide can further comprise one or more linker polypeptide, optionally located as shown in FIG. 1. and/or a signal peptide. The cell can be autologous or allogeneic to the subject being treated. The subject can be an animal or a human patient.

Also provided is an in vitro method comprising contacting a CAR as disclosed herein with a cell expressing the TAA.

In a further aspect, the method further comprises a therapy practiced prior to, concurrently or after the administration of the cells as described herein. Non-limiting examples include for example, cytoreductive therapy or the administration of a second cancer agent, e.g., gemcibabine or aldoxorubicin.

In some embodiments, the cytoreductive therapy comprises, or consists essentially of, or yet further consists of one or more of the following: a chemotherapy, a cryotherapy, a hyperthermia, a targeted therapy, or a radiation therapy.

In some embodiments, the administration is applied to the subject as a first line therapy, or a second line therapy, or third line therapy, or a fourth line therapy.

In some embodiments, the second anti-cancer therapy comprises administering an antibody that recognizes and binds to a suicide gene product, after the administration of the cells, thereby eliminating the suicide-gene-product-expressing cells. In further embodiments, the antibody is selected from one or more of the following: rituximab, ocrelizumab, ofatumumab, binutuzumab, ibritumomab, or iodine i 131 tositumomab. In yet further embodiments, the administration of the antibody is about 4 weeks, or about 1.5 months, or about 2 months, or about 3 months, or about 4 months, or about 5 months, or about 6 months, or about 7 months, or about 8 months, or about 9 months, or about 10 months, or about 11 months, or about 12 months, or about 1.5 years after the administration of the cells.

In some embodiments, the cell (such as the isolated or engineered cell) is autologous or allogeneic to the subject in need. In some embodiments, the cell (such as the isolated or engineered cell) is allogenic to the subject in need.

In some embodiments of any aspect as disclosed herein, the subject is a mammal, a canine, a feline, an equine, a murine, or a human patient.

In one aspect of the above methods, the methods further comprise, or consist essentially of, or yet further consist of administering the subject a second anti-cancer agent prior to, concurrent with or subsequent to the CAR therapy. They made be delivered in the same or different mode of contacting as determined by the treating physician or veterinarian.

In some aspects of these methods, after administration of the agent or the second agent, the subject experiences one or more endpoints selected from tumor response, reduction in tumor size, reduction in tumor burden, increase in overall survival, increase in progression free survival, inhibiting metastasis, improvement of quality of life, minimization of toxicity, and avoidance of side-effects.

In some embodiments, the combined therapy comprises a second agent which is selected from the group of: 5-fluorouracil, pemetrexed, raltitrexed, nolatrexed, plevitrexed, GS7904L, capecitabine, methotrexate, pralatrexate, CT-900, NUC-3373, FOLFOX, FOLFOX4, FOLFIRI, MOF, deflexifol, or a combination of 5-FU with one or more selected from radiation, methyl-CCNU, leucovorin, oxaliplatin (such as cisplatin), irinotecan, mitomycin, cytarabine, and levamisole.

In some embodiments, the second agent comprises an inhibitor of folate-mediated one-carbon metabolism. In another aspect, the second agent comprises anthracycline or other topoisomerase II inhibitor comprises daunorubicin, doxorubicin, epirubicin, idarubicin, valrubicin, mitoxantrone, etoposide and teniposide.

In some embodiments, the second agent comprises one or more selected from monoclonal antibodies, optionally selected from a monospecific antibody, a bispecific antibody, multispecific antibody, a bispecific immune cell engager, or an antibody-drug conjugate. Non-limiting examples of monoclonal antibodies are selected from rituximab, blinatumomab, alemtuzumab, ibritumomab tiuxetan, bevacizumab, bevacizumab-awwb, cetuximab, panitumumab, ofatumumab, denosumab, pertuzumab, obinutuzumab, elotuzumab, ramucirumab, dinutuximab, daratumumab, trastuzumab, trastuzumab-dkst, nivolumab, pembrolizumab, cemiplimab, spartalizumab, camrelizumab, sintilimab, tislelizumab, toripalimab, AMF 514 (MEDI0680), balstilimab, avelumab, durvalumab, atezolizumab, ipilimumab, tremelimumab, zalifrelimab, and AGEN1181. Non-limiting examples of antibody-drug conjugates are selected from moxetumomab pasudotox-tdfk, brentuximab vedotin, trastuzumab emtansine, inotuzumab ozogamicin, gemtuzumab ozogamicin, tagraxofusp-erzs, polatuzumab vedotin-piiq, enforfumab vedotin-ejfv, trastuzumab deruxtecan, and sacituzumab govitecan-hziy.

In some embodiments, the second agent comprises a second CAR therapy selected from a CAR NK therapy, a CAR-T therapy, a CAR cytotoxic T therapy, or a CAR gamma-delta T therapy. In some embodiments, the second CAR therapy is a CAR T-cell therapy selected from tisagenlecleucel and axicabtagene ciloleucel.

In some embodiments, the second agent comprises an immune regulator. In some embodiments, the immune regulator is selected from an interleukin, an aldesleukin, interferon alfa-2a/2b, pexidartinib, erythropoietin, granulocyte-macrophage colony-stimulating factor (GM-CSF), granulocyte colony-stimulating factor (G-CSF), thalidomide, lenalidomide, pomalidomide, or imiquimod.

In some embodiments, the second agent comprises a cancer vaccine. In some embodiments, the cancer vaccine is selected from CG live (THERACYS^{®}) and sipuleucel-T (PROVENGE^{®}).

In some embodiments, the second agent comprises an oncolytic virus therapy. In some embodiments, the oncolytic virus therapy is selected from oncorine (H101) or talimogene laherparepvec (IMLYGIC^{®}).

In some embodiments, the second agent comprises a checkpoint inhibitor. In some embodiments, the checkpoint inhibitor is selected from GS4224, AMP-224, CA-327, CA-170, BMS-1001, BMS-1166, peptide-57, M7824, MGD013, CX-072, UNP-12, NP-12, or a combination of two or more thereof.

Additional checkpoint inhibitors comprises one or more selected from an anti-PD-1 agent, an anti-PD-L1 agent, an anti-CTLA-4 agent, an anti-LAG-3 agent, an anti-TIM-3 agent, an anti-TIGIT agent, an anti-VISTA agent, an anti-B7-H3 agent, an anti-BTLA agent, an anti-ICOS agent, an anti-GITR agent, an anti-4-1BB agent, an anti-OX40 agent, an anti-CD27 agent, an anti-CD28 agent, an anti-CD40 agent, and an anti-Siglec-15 agent. In a further aspect, the checkpoint inhibitor comprises an anti-PD1 agent or an anti-PD-L1 agent. In one aspect, the anti-PD1 agent comprises an anti-PD1 antibody or an antigen binding fragment thereof. In a further aspect, the anti-PD1 antibody comprises nivolumab, pembrolizumab, cemiplimab, spartalizumab, camrelizumab, sintilimab, tislelizumab, toripalimab, AMF 514, or a combination of two or more thereof. In another aspect, the anti-PD-L1 agent comprises an anti-PD-L1 antibody or an antigen binding fragment thereof. In a further aspect, the anti-PD-L1 antibody comprises avelumab, durvalumab, atezolizumab, envafolimab, or a combination of two or more thereof.

In some embodiments, the checkpoint inhibitor comprises an anti- Cytotoxic T Lymphocyte Antigen 4 (CTLA-4; CD152) agent. In another aspect, the anti-CTLA-4 agent comprises an anti-CTLA-4 antibody or an antigen binding fragment thereof. In a yet further aspect, the anti-CTLA-4 antibody comprises ipilimumab, tremelimumab, zalifrelimab, or AGEN1181, or a combination thereof or other checkpoint inhibitors against Lymphocyte-activation Gene 3 (LAG-3; CD223); T Cell Immunoglobulin and Mucin-3 (TIM-3; HAVcr-2); T Cell Immunoreceptor with Immunoglobulin and ITIM domains (TIGIT); CD137 (4-1BB; TNFRSF9); Glucocorticoid-induced TNFR-related protein (GITR; CD357; TNFRSF18); OX40 (CD134; TNFRSF4); HVEM (CD270; TNFRSF14); CD40-CD40L; CD27, CD48, CD244; CD200; B7-H3; B7-H4 and Bf-H5 (VISTA) or B7H6 and others or Ig-based fusion proteins encompassing these checkpoint blockade receptors or in combination with cytokines such as IL-2 or multimeric versions of checkpoint blockade receptors, TNF ligands or cytokine fusion proteins or, CD40, Inducible T Cell Co-Stimulator (ICOS; CD278).

In some embodiments, the subject is selected for the administration if the antibody or fragment as disclosed herein binds to a component of a biological sample isolated from the subject.

### Kits

As set forth herein, the present disclosure provides methods for producing and administering CAR cells. In one particular aspect, the present disclosure provides kits for performing these methods as well as instructions for carrying out the methods of the present disclosure such as collecting cells or tissues, performing the screen/transduction/etc., analyzing the results, or any combination thereof

Additionally provided is a kit comprising, or consisting essentially of, or yet further consisting of instructions for use and one or more of: a polypeptide as disclosed herein; a polynucleotide as disclosed herein; a vector as disclosed herein; a cell as disclosed herein; a cell population as disclosed herein; a composition as disclosed herein; an anti-viral agent, optionally remdesivir, lopinavir, ritonavir, ivermectin, tamiflu, or favipiravir; an anti-inflammatory agent optionally dexamethasone, tocilizumab, kevzara, colcrys, hydroxychloroquine, chloroquine, or a kinase inhibitor; a covalescent plasma from a subject recovered from the viral infection; an antibody binding to the virus, optionally bamlanivimab, etesevimab, casirivimab, or imdevimab; an antibiotic agent, optionally azithromycin; an anti-cancer agent, or a vaccine.

In one aspect, the kit comprises, or alternatively consists essentially of, or yet further consists of, any one or more of: a polypeptide as disclosed herein, a CAR as disclosed herein, a polynucleotide as disclosed herein, a vector as disclosed herein, a vector system as disclosed herein, a cell as disclosed herein, such as isolated allogenic cells, preferably T cells or NK cells, a cell population as disclosed herein, a composition as disclosed herein, an isolated complex as disclosed herein, or an optional instruction for use in a method as disclosed herein, for example, on the procuring of autologous cells from a patient. Such a kit may also comprise, or alternatively consist essentially of, or yet further comprise media and other reagents appropriate for the transduction, selection, activation, expansion or any combination thereof of CAR expressing cells, such as those disclosed herein.

In one aspect the kit comprises, or alternatively consists essentially of, or yet further consists of, an isolated CAR expressing cell or population thereof. In some embodiments, the cells of this kit may require activation or expansion or both prior to administration to a subject in need thereof. In further embodiments, the kit may further comprise, or consist essentially of, media and reagents, such as those covered in the disclosure above, to activate or expand or both activate and expand the isolated CAR expressing cell. In some embodiments, the cell is to be used for a CAR therapy. In further embodiments, the kit comprises instructions on the administration of the isolated cell to a patient in need of a CAR therapy.

The kits of this disclosure can also comprise, e.g., a buffering agent, a preservative or a protein-stabilizing agent. The kits can further comprise components necessary for detecting the detectable-label, e.g., an enzyme or a substrate. The kits can also contain a control sample or a series of control samples, which can be assayed and compared to the test sample. Each component of a kit can be enclosed within an individual container and all of the various containers can be within a single package, along with instructions for interpreting the results of the assays performed using the kit. The kits of the present disclosure may contain a written product on or in the kit container. The written product describes how to use the reagents contained in the kit.

As amenable, these suggested kit components may be packaged in a manner customary for use by those of skill in the art. For example, these suggested kit components may be provided in solution or as a liquid dispersion or the like.

### Experimental Methods

### Plasmids

The lentiviral vector pHIV-EGFP was a kind gift from Bryan Welm& Zena Werb (Addgene # 18121; http://n2t.net/addgene:18121; RRID: Addgene_18121) (Welm et al., 2008). The packaging plasmids pMD2.G (Addgene # 12259; http://n2t.net/addgene:12259; RRID: Addgene_12259) and psPAX2 (Addgene # 12260; http://n2t.net/addgene:12260; RRID: Addgene_12260) were kind gifts from Didier Trono. The lentiviral vector pCDH-EF1a-eFFly-mCherry was a kind gift from Irmela Jeremias (Addgene # 104833; http://n2t.net/addgene:104833; RRID: Addgene_104833) (Ebinger et al., 2016). The plasmid pGBW-m4137382, encoding the SARS-CoV-2 surface glycoprotein (Spike) for VSV pseudotyping, was a kind gift from Ginkgo Bioworks (Addgene # 149539; http://n2t.net/addgene: 149539; RRID: Addgene_149539).

### Cell lines

Cell lines included human embryonic kidney 293 and 293T cells, Vero (naturally express monkey ACE2) cells, NIH/3T3 fibroblast cells and the human T lymphoblastic leukemia Jurkat cells (American Type Culture Collection (ATCC). 293, 293Tand Vero cells were cultured in DMEM supplemented with 10% heat-inactivated FBS (Gibco), 2 mM L-Glutamine (Corning), 10 mM HEPES (Corning), 100 U/ml Penicillin and Streptomycin (Sigma).By contrast, Jurkat cells were cultured in RPMI-1640 medium supplemented with 10% heat-inactivated FBS, 2 mM L-Glutamine, 10 mM HEPES, 100 U/ml Penicillin and Streptomycin. Transduced 293 cells stably over-expressing human ACE2 and transduced NIH/3T3 cells stably expressing S1 domain of SARS-CoV-2 spike glycoprotein were cultured in RPMI-1640 medium supplemented with 10% heat-inactivated FBS, 2 mM L-glutamine, 10 mM HEPES and drug selection by 50 µg/ml hygromycin B. The 293-hACE2 cells and NIH/3T3-S1 cells were also transduced with firefly luciferase and mCherry by using the lentiviral vectorpCDH-EF1a-eFFly-mCherry (Addgene), followed by sorting based on mCherry expression. All cell lines were confirmed for gene transduction before experiments.

### Mouse study

Experimental procedures were approved by the Animal Care Committee of the CR-HMR (#2021-2509). The NOD-SCID IL2Rγ^{null} mice were purchased from The Jackson Laboratory (Cat# 005557) and bred in the animal facility of the Research Center of Maisonneuve Rosemont Hospital, Montreal (#2017-1346, 2017-JA-001). Both female and male mice, the age of which range from 9 to 15 weeks old, were included and randomized to treatment groups based on sex, age and weight.

### Human subjects

Human blood samples from healthy donors were provided by HÉMA-QUÉBEC with donors' written informed consent and experiments using human PBMC were approved by the Research Ethics Committee of CIUSSS de l'Est-de-l'Île-de-Montréal (protocol # 2019-1184).

### Method Details

### CAR constructs

The sequence of anti-SARS &SARS-CoV-2 spike glycoprotein antibody CR3022 and anti-SARS spike glycoprotein antibody CR3014 were obtained from a patent. To construct CAR expression vector, the VH and Vκ of CR3022 were fused with a (Gly4Ser) 3 linker. The resulting single chain variable fragment (scFv) was linked to a Flag-tag (DYKDDDDK), a spacer, the CD28 trans-membrane and intracellular domain (68AA, 153-220, NP_006130.1) and the CD3ζ intracellular domain (112AA, 52-163, NP_000725.1) in tandem. The spacer is either a truncated CD28 hinge (39AA, 114-152, NP_006130.1), a CD8a hinge (47AA, 136-182, NP_001759.3), the CH3 domain of human IgG4 (119AA, UniProt ID P01861) or a mutated heavy chain of human IgG4 (CH2-CH3, 229AA, 99-327, UniProt ID P01861) as described elsewhere (Hudecek et al., 2015). The whole CAR construct was codon-optimized and synthesized by GeneArt (Thermo Scientific) and sub-cloned into pHIV-EGFP vector between EcoRI and XbaI cloning site.

### Preparation, titration and infection of pseudo-typed SARS-CoV-2 virus

293T cells were pre-plated in 6 well plates in 2 ml complete DMEM per well 24h prior to transfection. After being cultured overnight, 3 µg pGBW-m4137382 was mixed with 8 µl X-tremeGENE HP DNA transfection reagent and incubated for 30 min at room temperature before being added to the plate. After 24 h of transfection, the supernatant was discarded and the transfected cells were infected with pseudotyped ΔG-DsRed rVSV at multiplicity of infection (MOI) of 5 at 37°C. After 1 h of infection, 1 ml fresh complete RPMI-1640 medium was added to the plate. The next day, the virus supernatant was collected and the titre was determined by serial dilution.

For infection assay, 293-ACE2 cells were pre-labeled with Tag-it Violet^{™} Tracking Dye (Biolegend) according to the manufacturer's instruction. Then, 2×10⁵ per tube of labeled 293-ACE2 cells were co-cultured with 5×10⁵ per tube of effector cells at 37°C in the presence of pseudo-typed SARS-CoV-2 virus (MOI = 0.1) in a final volume of 500 µl complete RPMI-1640 medium. After 20 h of incubation, cells were washed and stained for viability, Flag and CD69 and analyzed by FACS. All the experiments were carried out in the laboratory of biosafety level 2.

### Preparation of lentivirus and generation of CAR-Jurkat cells and CAR-T cells

293T cells were co-transfected with CAR-encoding vectors, pMD2.G and psPAX2 by using X-tremeGENE HP DNA transfection reagent (Roche) according to the manufacturer's instruction. After overnight transfection, the supernatant was replaced with fresh RPMI-1640 complete medium. The supernatant containing lentivirus was harvested at 48 h and 72 h post-transfection and stored at -80°C.

To generate CAR-T cells, 3×10⁶ PBMC were resuspended in 1ml X-VIVO15 serum-free medium (Lonza) with soluble 1 µg/ml anti-hCD28 antibody (Clone # 9.3) and seeded into a 24 well plate pre-coated with 2µg/ml anti-hCD3 antibody (Clone # OKT-3). After 72 h of stimulation, cells were washed 2 times, and the medium was exchanged with 2 ml virus supernatant containing 10µg/ml protamine sulfate and spinoculated at 1200g for 60 min at 32°C. After 1 h of incubation at 37°C, the supernatant was replaced with 2 ml fresh X-VIVO15 serum-free medium and cultured for 4 days before FACS sorting based on EGFP expression. The sorted CAR-T cells were expanded in X-VIVO15 medium with 1000 IU/ml recombinant human IL-2 (Stemcell) and sub-cultured every 2~3 days with fresh media.

To generate CAR-Jurkat, 2×10⁵Jurkat cells were resuspended with 1 ml virus supernatant containing 10 µg/ml protamine sulfate and spinoculated at 1200 g for 60 min at 32°C. After 1 h of incubation at 37°C, supernatant was replaced with 2 ml fresh RPMI-1640 complete medium and the transduced cells were sub-cultured for 3 passages before sorting with cell sorter (SONY SH800) based on EGFP expression.

### Flow cytometry

For flow cytometric surface staining (anti-Flag, CD69 and FasL), CAR-Ts and other cells were incubated with fluorochrome-conjugated antibodies at 4°C for 30min followed by washing and fixation with 4 percent PFA followed by additional washing and analysis by either FACS Celesta (BD) or Fortessa X-20 (BD) as previously described (Raab et al., 2010). In certain instances, indirect two step staining was conducted with an unconjugated primary antibody for 30min on ice (anti-S1, anti-RBD) followed by washing and incubation with a conjugated secondary antibody for another 30 min at 4°Cand analyzed by FACS. For intracellular staining (IFN-γ, GZMB, perforin, pS6), cells were permeabilized by using the intracellular staining kit (Invitrogen), according to the manufacturer's protocol. Data analysis was performed by using FlowJo V10.0 (Tree Star) or t-Distributed Stochastic Neighbor Embedding (t-SNE) and Cytobank analysis (Alfei et al., 2019; McLane et al., 2019).

### In vitro functional assays

Three types of *in vitro* functional assays were conducted. In one case, freshly expanded CAR-Ts (10⁶/cell/ml) were incubated with 293-ACE2-RBD or 293-ACE2 cells (10⁶cells CAR-Ts and 5.0 x 10⁵/ml 293-ACE2 cells) for 20min prior to permeabilization kit (Invitrogen) and staining with phospho-S6 (1: 1000 dilution). As a control, phorbol ester (PMA) (Sigma) and ionomycin (Sigma) was also used to stimulate the CAR-T for 20min. In another instance, the same proportions of cells were incubated for 18-20hrs followed by cell permeabilization and staining with anti-GZMB, anti-perforin and anti-IFN-g, or for 24h and stained for CD69 and FasL. In the third *in vitro* assay, Applicant measured the cytolytic killing of target cells. 293-ACE2 target cells were either left untreated or were coated with the RBD-His (#SPD-C52H3, Acro Biosystems) or S1-His peptides (#S1N-C82E8, Acro Biosystems). For coating the 293-ACE2 cells, 1 µg/ml RBD or S1 protein was added to 1×10⁶ /ml cells at 37°C for 1hr followed by a gentle wash in media before used in experiments with CAR-Ts. In other experiments, NIH/3T3 cells expressing S1 was also used as described above. The effector cells were isolated from the same donor for each individual experiment and harvested after 2~3 weeks of cellular expansion in culture *in vitro* with IL-2 supplement media.

For the cytotoxicity assay, 1×10⁴ per well of target cells were co-cultured with different effector cells at the ratio of 1/20, 1/10 and 1/5 in U-bottom 96-well plates (Corning) in a final volume of 100 µl RPMI-1640 medium (5% FBS, phenol red free) per well. All samples were set in triplicate. After 4 h of incubation at 37°C, 50 µl supernatant of each well was collected to measure the LDH release by using CytoTox 96^{®} Non-Radioactive Cytotoxicity Assay kit (Promega) according to the manufacturer's protocol. The cytotoxicity was calculated as follows: Cytotoxicity (%) = (Experimental Release - Effector Spontaneous Release - Target Spontaneous Release) / (Target Maximum Release- Target Spontaneous Release) × 100.For statistics, *: <0.05; **: <0.01; ***: <0.001; ****: <0.0001

In certain instances, antibodies to the RBD epitope or FasL were used to block the response. For blocking the RBD epitope, 1 µg/ml RBD protein was added to 1×10⁶ /ml 293-ACE2 cells and incubated at 37°C for 1 h. After that, the cells were washed twice, resuspended with 400 µl RPMI-1640 medium (5% FBS, phenol red free) and split into two EP tubes equally. 1 µg/ml anti-RBD antibody (clone # CR3022, Abcam) or 1 µg/ml rabbit IgG isotype control antibody (Clone # 60024B, R&D) was added to each tube (5×10⁵ 293-ACE2-RBD in 200 µl medium), respectively, and incubated at 37°C for 30 min. Then cells were directly diluted with RPMI-1640 medium to a final volume of 2 ml/tube without wash, and co-cultured with different CAR-T cells at the ratio of 1/24, 1/12, 1/6 and 1/3 in a U-bottom 96-well plate (Corning).

Similarly, for the blockade of FasL, 2 µg/ml anti-hFasL antibody (NOK-1 or 100419) was added to 1×10⁶ /ml CAR-T cells, incubated at 37°C for 1 h and co-cultured with target cells without wash.

### Time-lapse imaging

5×10⁴ 293-ACE2-mCherry cells were resuspended with 500 µl RPMI-1640 medium (5% FBS, phenol red free) and pre-plated in a cover-glass bottom dish (SPL life sciences) at 37°C. After being cultured overnight, 1 µg/ml RBD protein was added to cells and incubated at 37°C for 1 h. Then 2×10⁶ CR3022-8a-28Z-EGFPCAR-T cells were resuspended with 1.5 ml RPMI-1640 medium (5% FBS, phenol red free) and added to the dish for co-culture in an inverted fluorescence microscope with climate control (Zeiss Observer Z1). Images were captured from the beginning of the co-culture with a 2 min interval for 24h. Videos were exported and analyzed by using ZEN Lite software (Zeiss).

### In vivo cytolytic assay

Mice were injected i.p. with either 2×10⁶NIH/3T3-S1-Luc cells (NT group) or a combination of 2×10⁶NIH/3T3-S1-Luc cells and CAR-T cells in the ratio of 1/2 premixed in 300 µl of RPMI-1640 medium (CR3014-28z and CR3022-28z groups). At 24 h post-implantation, mice were injected i.p. with 150 mg/kg RediJect D-Luciferin (PerkinElmer; #770505).Mice were anaesthetized with 2% isoflurane and transferred to the IVIS Lumina III (PerkinElmer) for *in vivo* imaging at 10 min post-injection. Mice from three different groups were imaged simultaneously with 20 sec of exposure. Bioluminescence images were overlapped with bright field images of mice and displayed in the same intensity range. Images were acquired and analyzed by using Aura Imaging Software (Spectral Instruments Imaging). The total radiance (Photon/s and counts) was measured and calculated for each region of interest (ROI).

### Quantification and Statistical Analysis

The n represents individual donors from a random pool of donors provided by the blood service Hema-Quebec (Quebec). The response of CAR-Ts was compared from one donor to another. This measure applied to all figures in the paper. Although CAR-T cell numbers were expanded in vitro from a given individual donor, all cells were used at a single time from the individual donor for cytolytic killing assays and flow cytometry. No repeat experiments from individual donors was done due to the lack of repeat access to individual donors. Statistical analysis was performed by using GraphPad Prism^{®} software v8.0 (GraphPad). Two-tailed Student's t-test was used to compare two normally distributed independent groups with continuous endpoints. The one-way ANOVA was applied when more than two groups were compared and the two-way ANOVA was applied when there are two independent variables. Dunnett's post hoc test was used for multiple comparisons. All experiments were repeated at least two times, and all experimental data in figures and texts are shown as the mean ± standard deviation. *p* values less than 0.05 was considered statistically significant.

### Results

### Anti-SARS-CoV-2 CAR Design

A neutralizing antibody from a convalescent SARS patient termed CR3022 binds to the RBD region of both SARS-CoV-1 and SARS-CoV-2 spike protein (Tian et al., 2020). Based on its sequence, the CR3022single chain variable fragment (scFv) was cloned into the bicistronic lentiviral vector (pHIV) followed by a Flag-tag sequence (DYKDDDDK), a hinge region, the CD28 transmembrane (TM) and intracellular domain and a CD3zeta intracellular domain. CARs comprised of different hinge regions were generated that included either a 39 amino acid CD28hinge (CR3022-28Z) (residues 114-152), a 47 aa CD8alpha chain (residues 136-182) (CR3022-8a-28Z), a 119aa CH3 region hinge (residues 221-327) (CR3022-CH3-28Z) or a 229aa IgG4 region hinge (residues 99-327) (CR3022-IgG4-28Z) (FIG. 1A). As a control, Applicant generated a CAR lacking an scFv region (Flag-28Z) and another with the scFv region of another monoclonal antibody termed CR3014 that reacts only with the RBD region of SARS-CoV-1 (CR3014-28Z). The n represents the number of single experiments, each from a different, individual donor. This measure applied to all figures in the paper. Each pHIV bicistronic lentiviral construct contained IRES-EGFP allowing for sorting and tracking the CAR-T cells.

To generate CAR-T cells, peripheral blood mononuclear cells (PBMCs) from a given donor were stimulated *in vitro* with anti-CD3 and anti-CD28 antibodies for 72 hours followed by spinoculation with lentiviral supernatant, further culturing for 4 days in X-VIVO15 serum-free medium followed by fluorescence-activated cell sorting (FACS) based on EGFP expression. Sorted CAR-Ts cells were then expanded in the same media supplemented with interleukin 2 (IL-2) for 2-3 weeks before their use in assays. Lentiviral driven expression of the CR3022-28Z, CR3022-8a-28Z, CR3022-CH3-28Z or CR3022-IgG4-28Z constructs was confirmed by EGFP expression (FIGS. 1B and 1C). The CR3022-CH3-28Z construct was routinely observed to be expressed at slightly lower levels than the other constructs. CAR expression on the surface of cells was confirmed by anti-Flag staining (FIGS. 1B, 1C. and 8). As a representative example, using CR3022-8a-28Z transduced cells, anti-Flag-APC and EGFP showed a similar heterogeneous viSNE pattern for CAR receptor expression. It could roughly be divided into populations of cells with high (island i), intermediate (island ii) and low-nil expression (island iii). The islands showed some overlap but defined a general trend of heterogeneity within the CAR-T population. Lastly, flow cytometric staining with anti-CD4 and anti-CD8 showed similar proportions of CD8+ and CD4+ cells amongst the CR3022-28Z, CR3022-8a-28Z, CR3022-CH3-28Z or CR3022-IgG4-28Z expressing CAR-Ts (FIGS. 9A-9E). These data showed that Applicant were able to successfully transduce and maintain CAR-Ts.

### The binding to the S1 RBD peptide activates SARS-CoV-2 CAR-Ts

Applicant next assessed whether the RBD viral sequence presented on the surface of cells could activate the various CAR-Ts (FIG. 1D). For this, 293-ACE2 cells were incubated with RBD peptide for 1 h (293-ACE2-RBD) prior to co-culturing with the CAR-Ts for 20h. The expression of hACE2 on the 293-ACE2 cells was confirmed by flow cytometry (FIG. 10). Applicant initially examined changes in the expression of CD69 on CAR-Ts, a well-established activation marker on surface of T-cells (Cibrian and Sanchez-Madrid, 2017).Indeed, the co-culturing with 293-ACE2-RBD cells induced a marked increase in CD69 expression on the CR3022-28Z, CR3022-8a-28Z, CR3022-CH3-28Z or CR3022-IgG4-28Z CAR-Ts(left panel). As a negative control, neither Flag-28Znor CR3014-28Z CAR-T cells showed an increase in CD69. Similarly, standard flow cytometry profiles of a representative donor showed that 293-ACE2-RBD cells induced an increase in CD69expressionon each of the CR3022 CAR-Ts (upper right panel), while no increase was seen with incubation with 293-ACE2 cells lacking RBD (lower right panel). Further, in a titration experiment, CD69 expression was seen to increase in response to increasing concentrations of the RBD peptide pre-incubated with the 293-ACE2 cells FIG. 1E). In this case, Applicant observed a titratable increase in the response to peptide as low as 10ng/ml. No response of the CR3014-28Z CAR-Ts was noted. The viability of cells in control and stimulation assays was similar. These data showed clearly that the presentation of the RBD peptide induced the expression of CD69on the various CR3022 CAR-Ts.

Applicant next assessed the heterogeneity of CD69 expression on CAR-Ts in more detail by viSNE and Cytobank analysis (Alfei et al., 2019; McLane et al., 2019) (FIGS. 1F - 1H). The CD69 population could be divided into three islands based on levels of expression (island i with highest level, island ii intermediate levels (yellow/turquoise) and island iii with low-nil levels of CD69 (dark blue)). Importantly, the CD69 expression patterns changed in response to 293-ACE2-RBD cells with an increase in the percentage of CAR-Ts in island i relative to the total CAR-T population (from 35-40% to 65-70%; upper right panel) and in an increase in the relative mean fluorescent intensity (MFI) (from 1000 to 1800-2000; lower right panel). No change in the expression of CD69 on Flag-28Z or CR3014-28Z was seen. By contrast, islands ii and iii showed much lower increase in expression and a decrease in their percent representation in the total CAR-T population (FIGS. 11A-11B, left and right panels, respectively).

Applicant also transduced Jurkat T-cells to express different CARs and monitored CD69 expression in response to RBD peptide presented by Vero cells (FIGS. 12A-12B). Vero cells were co-incubated with 200 or 1000ng/ml RBD peptide. The CR3022 Jurkat cells, but not the Jurkat CR3014 cells, showed an increase in CD69 expression in response to peptide in the presence of Vero cells.

In addition, as a nearly intracellular signaling event, Applicant assessed the phosphorylation of the ribosomal protein S6, a key signal transduction event that regulates cell division, protein translation and metabolism (Roux et al., 2007) (FIG. 11). As an example, CR3022-8a-28Z CAR-Ts were incubated with 293-ACE2 or 293-ACE2-RBD cells for 20min prior to the detection of S6 phosphorylation by flow cytometry. The incubation of CAR-Ts with 293-ACE2-RBD induced S6 phosphorylation (ie. pS6) in the T cells, while no change was observed in response to incubation with the control, 293-ACE2 cells. As a positive control, the incubation of cells with PMA and ionomycin also increased S6 phosphorylation. These data indicate CAR-T engagement generated a key early intracellular signaling event needed for cell proliferation.

Lastly, the recognition of the RBD peptide led to the downregulation of the CARs from the surface of cells (FIG. 1J). CAR downregulation has been previously reported (Li et al., 2020). The 20 h incubation period was sufficient to allow for both the induction of signaling and the subsequent downregulation of the receptors. In agreement, the CR3022-28Z CAR receptors were downregulated with increasing RBD peptide concentrations in a manner that inversely mirrored the induction of CD69 expression. As a control, no titratable response of the CR3014-28Z CAR-T was observed. These data confirmed that the CR3022-based CARs became engaged in a specific manner when presented with the RBD peptide. CAR down-regulation can serve as an indicator of receptor engagement and activation.

### Anti-SARS-CoV-2 CAR-Ts kill RBD/S1-coated targets in vitro

Applicant next assessed whether anti-SARS-CoV-2CAR-Ts were cytolytic in killing SARS-CoV-2 RBD/S1 loaded cells (FIG. 2). CAR-Ts were co-cultured with different ratios (20:1, 10:1, 5:1) of 293-ACE2-RBD/CAR-Ts for 5 hours for an assessment of killing. Each of the CR3022-based CAR-Ts was seen to kill the targets over various ratios (FIG. 2A, left panel). The presence of the RBD peptide on the 293-ACE2-RBD cells was confirmed by staining with anti-S1 and the CR3022 antibody (right panel). By contrast, as a negative control, T cells expressing Flag-28Z or CR3014-28Z failed to kill targets. Interestingly, CAR-Ts with different hinge regions showed differences in the efficiency of killing. The CD8a (CR3022-8a-28Z) and IgG4 (CR3022-IgG4-28Z) CAR-T expressing cells were the most effective killers followed by CR3022-28Z and CR3022-CH3-28Z.

Specificity was confirmed by CR3022 antibody blockade of killing (FIG. 2B). 293-ACE2-RBD cells were incubated with CR3022, or the isotype control, at 37°C for 30min prior to the incubation with CAR-Ts. As shown in a representative experiment, the CR3022 antibody abrogated the killing of targets by the CR3022-8a-28Z and CR3022-IgG4-28Z CAR-Ts by 70-80 percent at various effector to target ratios. By contrast, the isotype control antibody had no effect on killing.

Applicant also assessed the killing of 293-ACE2 target cells that had been pre-coated with the full length S1 peptide (FIG. 2C).The presence of the S1 peptide on the cells was confirmed by staining with anti-S1 and the CR3022 antibody (right **panel**).CR3022-28Z, CR3022-8a-28Z and CR3022-IgG4-28Z CAR-Ts killed S1 peptide loaded targets, albeit at lower levels (FIG. 2C, left panel). The CR3022-CH3-28Z CAR-T or the control CR3014-28Z CAR-T showed no killing. In this case, the CR3022-IgG4-28Z CAR-T was most effective. Overall, the CAR-Ts were less able to kill targets with only 10-30% for S1 peptide-coated cells compared to the 30-60% for RBD peptide-loaded target. This may be due to the mode of the specific RBD sequence presentation in the S1 protein as recognized by the CAR-Ts. Examples of the similar response of CAR-Ts from different donors to the RBD and S1 peptides are also shown in FIGS. 13A-13B.

Applicant next assessed SARS-CoV-2 CAR-T killing of NIH/3T3 cells that had been transduced to express the S1 protein (FIGS. 2D and 2E). FACS profiles showed high levels of anti-S1 staining but a lower level of staining with the CR3022 antibody against the RBD peptide (lower panel). The reason for this difference is not clear but is likely related to the accessibility of the CR3022 epitope. The RBD is only accessible when at least two RBDs are open simultaneously (Yuan et al., 2020) and the frequency of the opening RBD is usually less than 30% (Wrobel et al., 2020). Despite this, the various CR3022CAR-Ts were able to effectively kill targets (upper panel). Each of the CR3022-28Z, CR3022-8a-28Z, CR3022-CH3-28Z and CR3022-IgG4-28Z CAR-Ts killed 20-50 percent of target cells, dependent on the CAR-T to target ratio. In this assay, the CR3022-28Z CAR-T and the CR3022-IgG4-28Z CAR-T cells were the most effective.

Overall, these data showed that the CAR-Ts could be generated that recognize and kill targets coated with the RBD and S1 peptides or the S1 expressing NIH/3T3 cells. The CAR-Ts with different sized extracellular domains also varied in their killing efficacy of different target cells expressing the S1 protein. These CAR-T variants will enable the eventual testing efficacy against S1 variants.

### Anti-SARS-CoV-2 CAR-Ts form multi-cellular clusters with targets

The interaction of the CAR-T cells with the target cells was next examined using time lapse live-cell microscopy (FIG. 3). Applicant analyzed the behavior of CR3022-8a-28Z CAR-Ts co-cultured with the 293-ACE2 and 293-ACE2-RBD cells over 20 hours. The EGFP+ CAR-T cells were observed to be green due to EGFP+ expression, while the 293 cells were seen as red-orange color due to the lentiviral transduction with pCDH-EF1a-eFFly-mCherry. CR3022-8a-28Z formed clusters with 293-ACE2-RBD clusters within 30-60 minutes and were present for 10-15 hours followed by a slight reduction in clusters (FIGS. 3A and 3B). No clusters were seen with 293-ACE2 negative control cells. On average, there were 3-5 CAR-Ts in each cluster, although this varied depending on the timing of the analysis (FIG. 3C, also high magnification examples from two separate experiments in right upper panel). No clusters were seen with the 293-ACE2 cells alone. Cluster diameter also increased from 60 min to 10-12 hours with an average of diameter of 30-40 um as assessed by ZEN Lite software (FIG. 3D). Similarly, the surface area of the clusters increased from 500 to 1500um² over the same time period (FIG. 3E). An example of tracking between CR3022-8a-28Z CAR-Ts co-cultured with the 293-ACE2 pre-loaded with S1 peptide was also captured. Overall, these data confirmed that CR3022-8a-28Z CAR-Ts formed clusters with 293-ACE2-RBD targets dependent on the presence of the RBD/S1 peptides.

### IFN-γ is induced on the CD69 subset of SARS-CoV-2 CAR-Ts

Applicant next examined the inducible expression of immune effector molecules on the CAR-Ts. The cytokine interferon-γ (IFN-γ) promotes differentiation into Tₕ1 cells and the up-regulation of MHC antigens on dendritic cells(Schoenborn and Wilson, 2007). Indeed, Applicant observed an increase in IFN-γ expression in response to 293-ACE2-RBD cells on each of the CAR-Ts (FIG. 4A). The highest increase in expression was seen on CR3022-28Z, CR3022-8a-28Z and CR3022-IgG4-28ZCAR-Ts. In a separate experiment, viSNE analysis showed that IFN-γ was already widely expressed on the majority of CAR-Ts after their expansion in IL-2 supplemented media (FIGS. 4B-4E) or after the incubation with 293-ACE2 cells (middle panel). A small increase in expression was observed after incubation with 293-ACE2 cells alone. However, in response to 293-ACE2-RBD cells, the CAR-Ts caused a major change in the distribution of expressing cells and expression intensity of IFN-γ as seen by viSNE. There was a loss of cells in islands ii and iii concurrent with an increase in the density of cells in island i. Each of the CR3022 based CAR-Ts showed a similar trend. In keeping with this, there was an increase in the percentage of IFN-γ+ cells in island i relative to total CAR-T population (FIG. 4F) as well as an increase in the relative MFI for IFN-γ in island i (FIG. 4G). Lastly, viSNE analysis showed that the same island of cells with increased IFN-γ expression in island i also showed an increase in CD69 expression (FIGS. 4B-4E). Overall, these data indicated that the recognition of the RBD peptide by CAR-Ts caused a major increase in the expression of IFN-γ expression in the same subset of cells showing an increase in CD69 expression.

### Granzyme B/perforin is induced in a subset of CD69+ anti-SARS-CoV-2 CAR-Ts

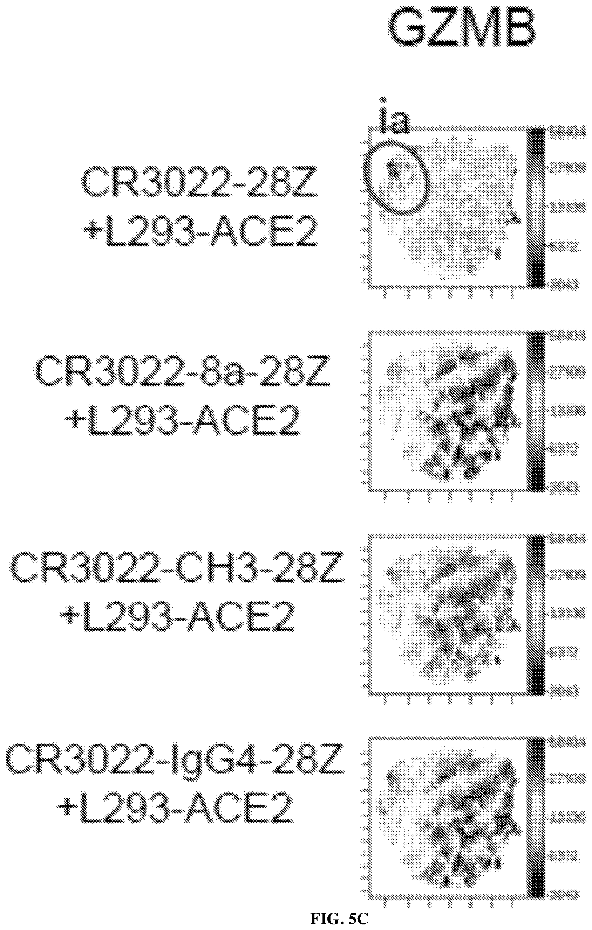

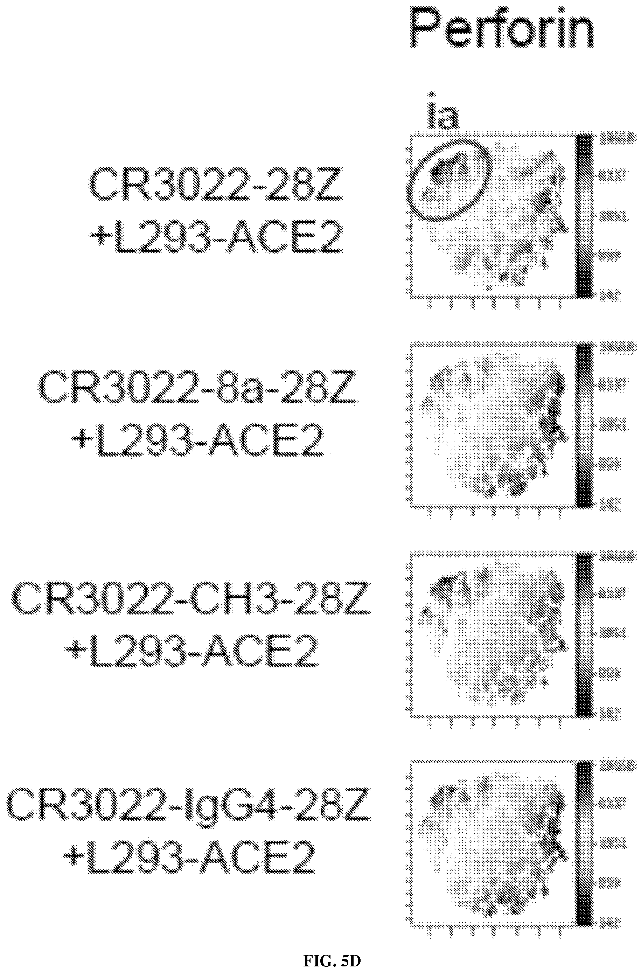

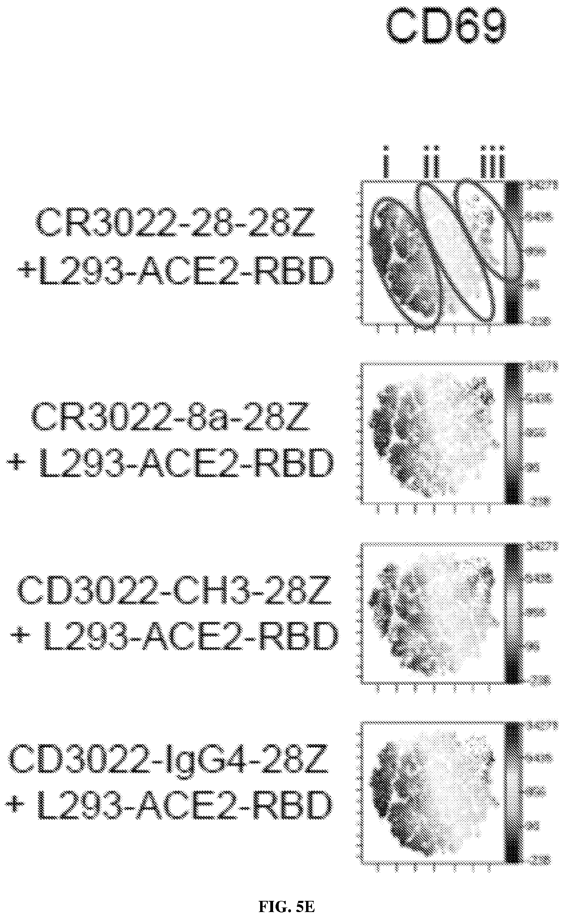

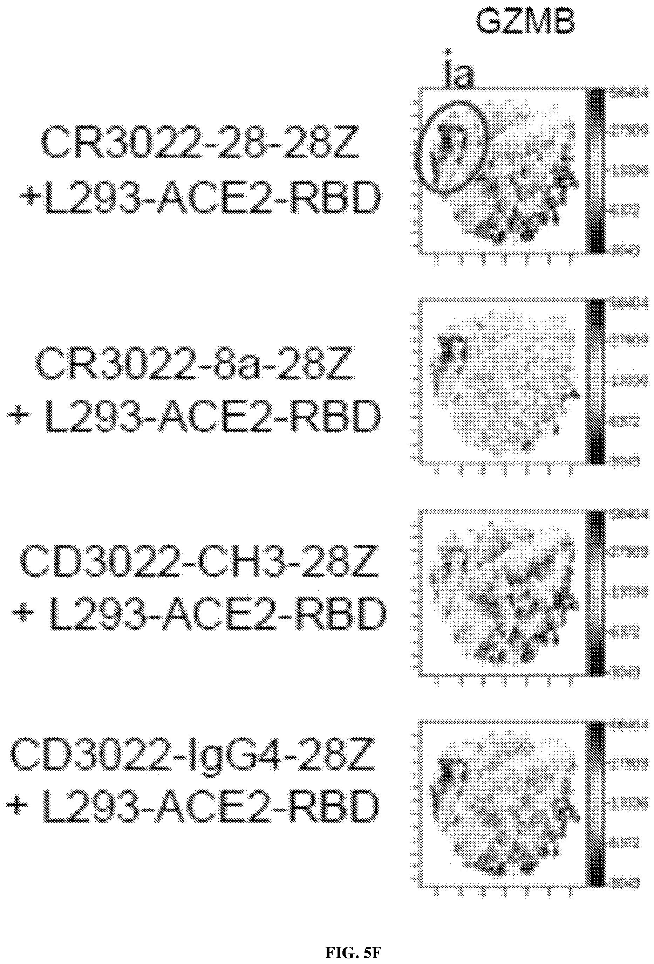

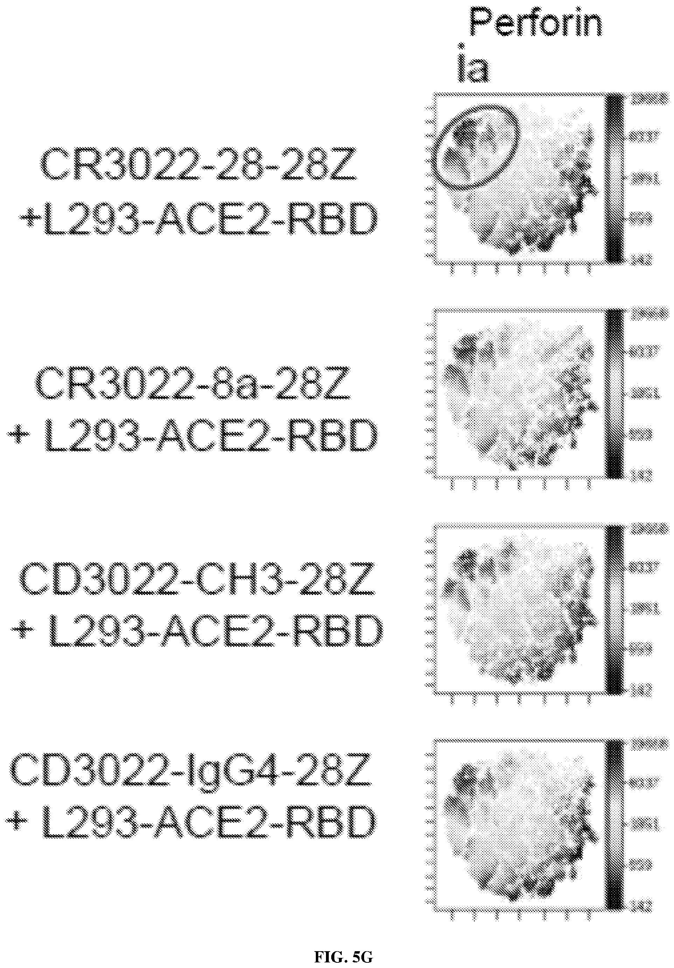

Given the ability of the various CAR-Ts to kill RBD or S1 bearing targets, Applicant next assessed the expression of the cytolytic effectors, granzyme B (GZMB) and perforin (FIG. 5). Both are key effector molecules that mediate the killing of targets(Kurschus and Jenne, 2010). Perforin is a pore-forming cytolytic protein that facilitates GZMB entry to kill target cells(Smyth et al., 1996).Indeed, each CR3022 based CAR-T showed an increase in the level of expression of GZMB (FIG. 5A) and perforin (FIG. 5A) when incubated with RBD loaded target cells. CR3022-28Z, CR3022-8a-28Z and CR3022-IgG4-28Z CAR-Ts showed the highest level of GZMB and perforin expression. No increase was observed upon incubation with 293-ACE2 cells lacking RBD, or in the negative controls, Flag-28Z and CR3014-28Z.

Unlike in the case of IFN-γ, GZMB and perforin were expressed in a highly localized subset of CD69+ CAR-Ts within island i (termed this subset as sub-island ia) (FIGS. 5B-5G). A basal level of GZMB and perforin expression was seen in CAR-T cells incubated with 293-ACE2 cells. Both molecules were expressed within the same subset of CAR-Ts. Incubation with 293-ACE2-RBD cells increased the expression of both proteins (lower panels). Each of the CAR-Ts showed a similar increase except for the CR3022-CH3-28Z which showed a more modest increase. An increase in the MFI for GZMB (FIG. **5H**) and for perforin (FIG. **5H**) was observed. No increase in GZMB in islands ii and iii was observed (upper panel), while a modest increase in perforin expression in the same islands was seen. Overall, there was a striking increase in the presence of CD69^{hi}GZMB⁺perforin⁺ CAR-Ts in sub-island ia (FIG. 5I). These data showed that the recognition of RBD peptide by CAR-Ts induced a detectable increase in the expression of GZMB and perforin within a subset of CD69 high expressing CAR-Ts.

### FasL (CD95L) is preferentially expressed on CAR-T cells with high CD69 expression

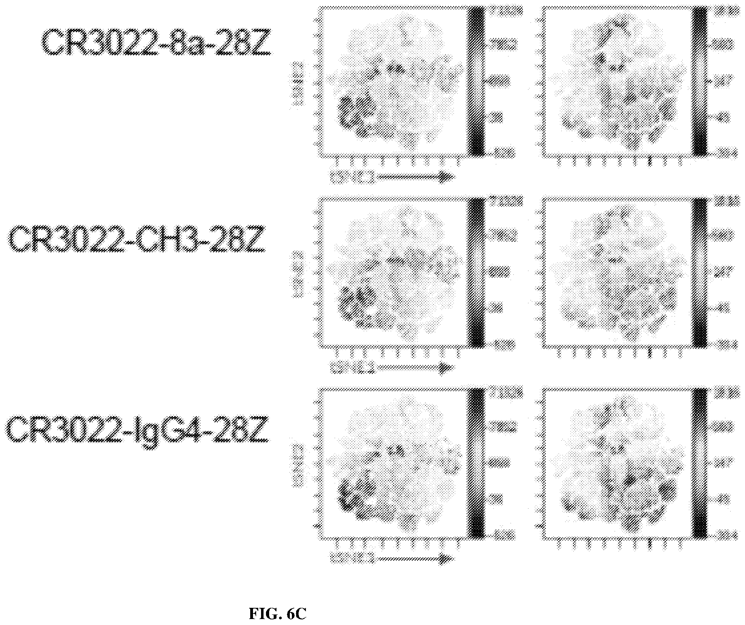

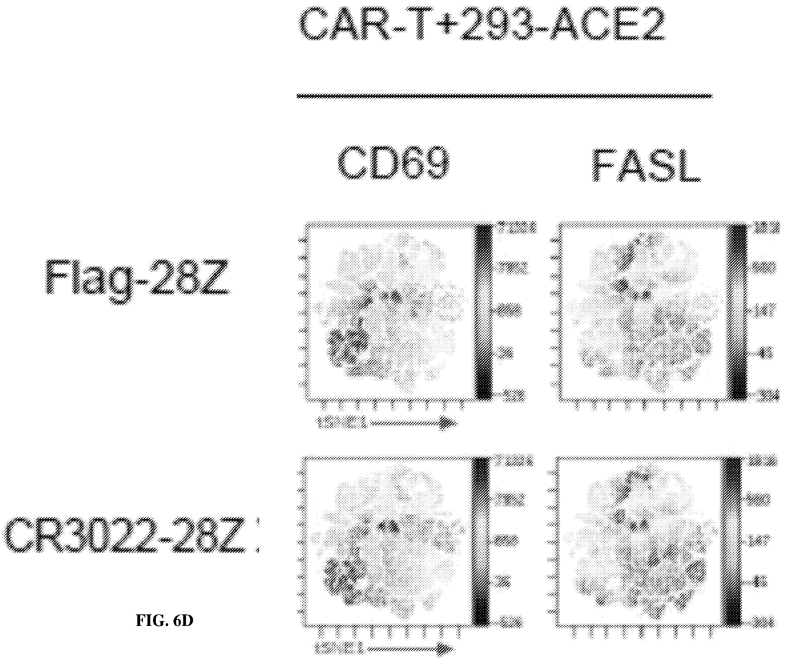

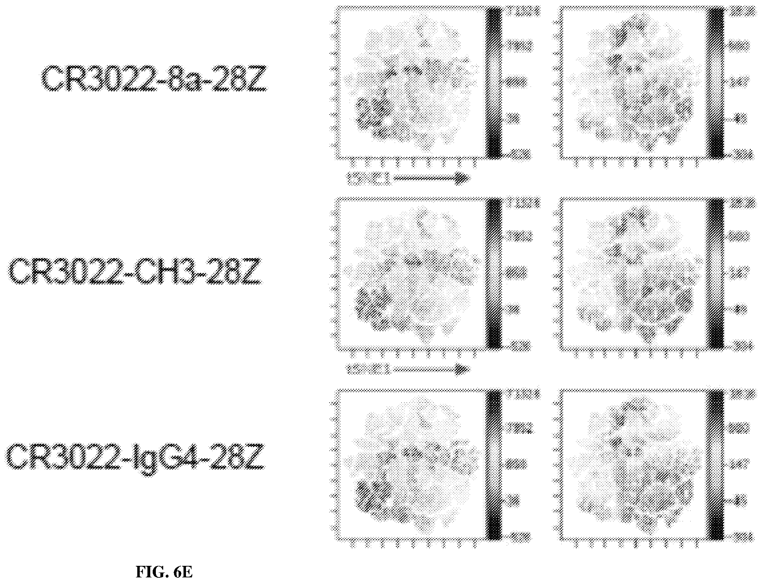

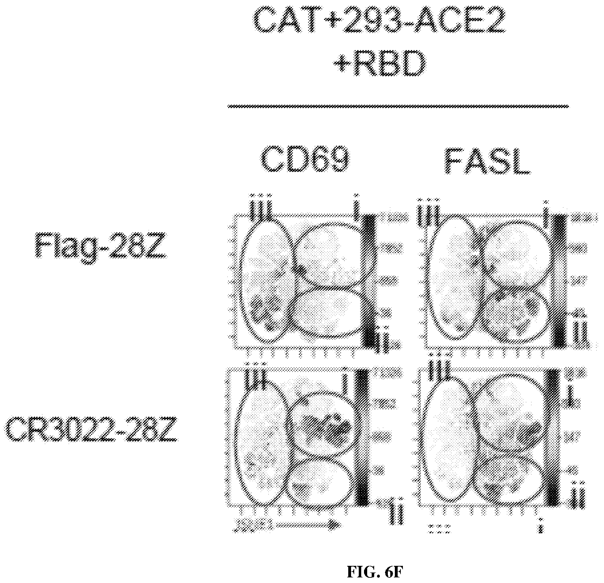

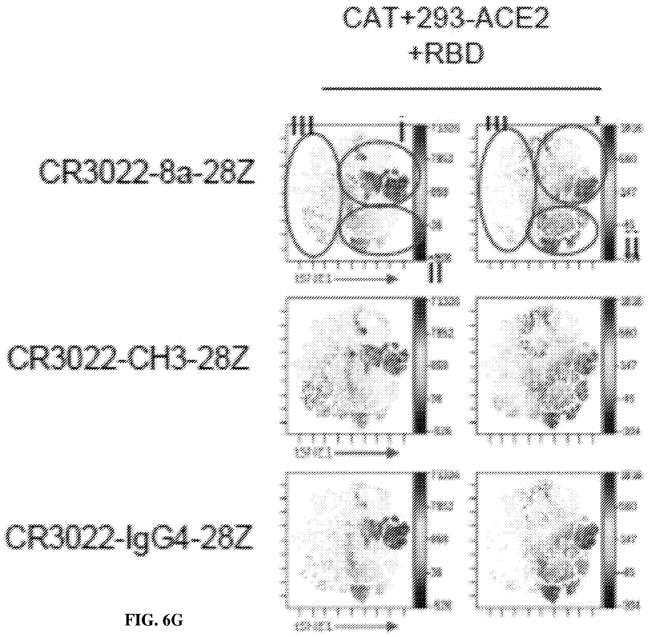

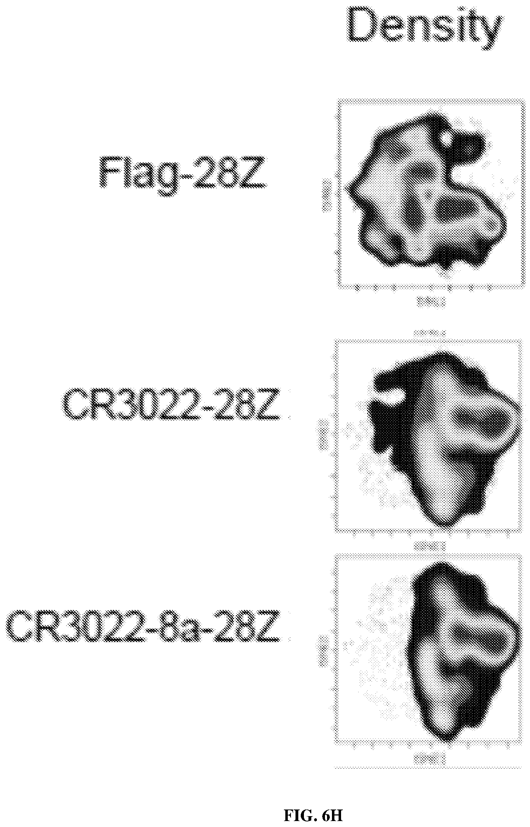

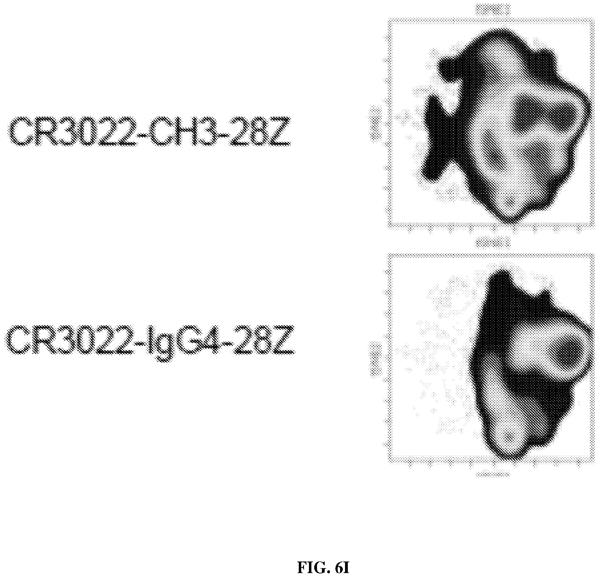

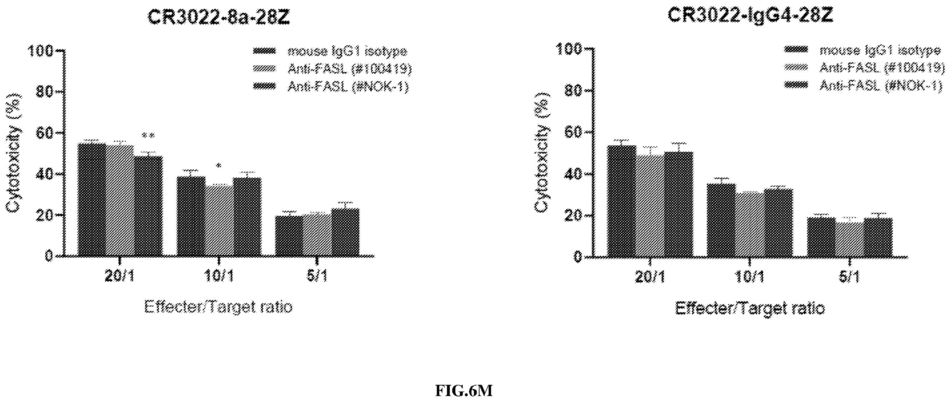

A second mechanism of cytolytic mediated killing involves Fas ligand (FasL) (FIG. 6). FasL is a TNF superfamily member that binds to the Fas receptor on target cells leading to their apoptosis and death(Russell and Ley, 2002).Each CR3022 CAR-T increased FasL expression upon incubation with 293-ACE2-RBD cells (FIG. 6A). In this case, the CR3022-IgG4-28Z CAR-T showed the greatest increase. viSNE analysis showed that FasL was broadly expressed on the majority of CAR-Ts that seen with the other markers (FIGS. 6B-6J). As in the case of CD69, three islands of cells could be defined based on receptor expression (islands i,ii,iii). Unlike for CD69, an increase in FasL was observed (albeit to a lesser extent) in both islands i and ii. The increase in the positive signal in island ii was accompanied by the loss of FasL negative cells (see dark blue), as most evident in the viSNE patterns for the CR3022-IgG4-28Z CAR-Ts. As negative controls, the incubation of CAR-Ts with soluble RBD peptide without 293-ACE2 cells, or the incubation of Flag-28Z cells with 293-ACE2 or 293-ACE2-RBD showed background staining (upper panels). This indicated that SARS-CoV-2 recognition led to an increase in FasL on a broader population of cells than observed for CD69 (FIGS. 6B-6K). As an alternate system, Applicant also assessed changes in FasL expression in response to NIH/3T3 cells that had been stably transduced to express the S1 peptide (FIG. 6K).In this case, CR3022-8a-28Z cells also showed a broad increase in FasL expression. These data showed that the recognition of the RBD peptide or the S1 protein on cells induced the expression of FasL, and that the pattern of induced expression was broader than seen with the expression of CD69, IFN-γ or GZMB/perforin.

Given this, Applicant next assessed the importance of FasL expression to the killing of RBD-coated cells by adding anti-FasL blocking antibodies to the killing assay (FIGS. 6M-6N). CR3022-28Z, CR3022-8a-28Z and CR3022-IgG4-28Z CAR-T cells were incubated with 293-ACE2-RBD cells in the presence or absence of anti-FasL blocking mAbs, #100419 or #NOK-1, for 5 hours prior to and during the killing assay. NOK-1 has been previously reported to block cell mediated killing (Kayagaki et al., 1995). A minor reduction in killing was observed in some ratios of effector to target cells for CR3022-28Z, and CR3022-8a-28Z CAR-Ts; however, the patterns were not consistent. A minor but statistically significant effect was observed on the killing by CR3022-28Z (FIG-6M, right panel) and CD3022-8a-28Z (FIG-6N, left panel). A trend was seen with the CD3022-IgG4-28Z CAR-T cells (FIG-6N, right panel). From these data, Applicant observed that the FasL pathway plays a minor but significant role in the killing of RBD-coated ACE2-expressing cells in our systems. The participation of some FasL mediated killing might vary with different target cells, in particular, depending on the level or nature of Fas or FasL expression in the target cells expressing components of the SARs-CoV2 virus. The statistical dependency of certain CAR-Ts such as CR3022-28Z and CD3022-8a-28Z on the Fas-FasL pathway would not have been predicted by any previous data.

### Anti-SARS-CoV-2 CAR-Ts kill S1-expressing targets in vivo

Lastly, it was important to assess whether CAR-Ts could elicit the killing of targets *in vivo* in mice (FIG. 7). For this, Applicant injected a mixture of 2×10⁶NIH/3T3-S1 cells and 4×10⁶ CR3014-28Z/ CR3022-28Z CAR-T cells to NOD-SCID IL2Rγ^{null} mice via the intraperitoneal route. The NIH/3T3-S1 cells, in addition to expressing S1 from SARS-CoV-2, express luciferase, and the presence of NIH/3T3-S1 cells can be assessed by injecting D-luciferin. For analysis, bioluminescence images were acquired at 24 h after injection of the cells by administering D-luciferin intraperitoneally (i.p.). Applicant found that the adoptive transfer of CR3022-28Z reduced the presence of target cells in mice (FIG. 7A). While injection with NIH/3T3-S1 cells alone resulted in nearly 4 x 10⁹ Luminescence (p/s), injection of NIH/3T3-S1 cells with CR3022-28Z CAR-T cells reduced the luminescence measurement to <1x 10⁹,and CR3014-28Z CAR-T cells had no effect on the presence of the target (FIG. 7B). In comparison to the untreated samples, the CR3022-28Z reduced the presence of NIH/3T3-S1 cells (i.e. luminescence) by 80%. However, relative to the CR3014-28Z, the luminescence was reduced by 60%. There was the loss of signal in 5/8 mice (shown for 3/5 mice). Overall, these data confirm that CAR-T cells also are capable of killing S1 expressing cells *in vivo* in mice.

Applicant next assessed whether the SARS-CoV-2 CAR-Ts could also block viral entry (FIGS. **14A-14D****).** Applicant expected that the efficiency of blockade would be low given the difficulty in having cells compete for smaller scale protein-protein interactions. Recombinant pseudo-typed VSV particles containing SARS-CoV-2 spike protein were used to mimic SARS-CoV-2 cell infection and cell entry. The SARS-CoV-2 pseudo-virus particles encode DsRed in the virus genome. DsRed was strongly expressed after the SARS-CoV-2 pseudo-virus entry into ACE2-expressing cells (Millet et al., 2019). From this, using SARS-CoV-2 as a control with an infection rate of 60%, Applicant observed that co-incubation with CAR-Ts expressing the various constructs reduced viral entry in response to different ratios of T-cells to target. As expected, the efficacy of blockade was low and observed for bothCR3014 and CR3022 based CAR-Ts (FIGS. **14A-14D****).** Both Flag28z and CR3014 reduced binding to the same degree as CAR-T's expressing specific receptors. Overall, the data showed that CAR-Ts are unlikely to sterically block viral entry into cells, but rather limit viral infection, as seen in the case of most viral infections by mediating CTL killing responses against infected cells expressing viral antigens (Moskophidis and Kioussis, 1998).

### Additional Anti-SARS-CoV-2 CAR designs

Various CARs were generated using the scFv regions of antibodies: (i) neutralizing antibody from a convalescent SARS patient termed CR3022 binds to the RBD region of both SARS-CoV-1 and SARS-CoV-2 spike protein (Tian et al., 2020), (ii) 4A8 (Chi et al., 2020), (iii) C135 (Robbiani et al., 2020)and (iv) S309 (Pinto et al., 2020). 4A8 exhibits high neutralization potency against both authentic and pseudotyped SARS-CoV-2 but does not bind the RBD, instead binds to the N-terminal domain (NTD) of the S protein (Chi et al., 2020). C135 is a potent neutralizing antibody against the RBD sequence on S1 (Barnes et al., 2020; Robbiani et al., 2020). S309 also neutralizes SARS-CoV-2 and SARS-CoV pseudoviruses as well as authentic SARS-CoV-2, by binding to RBD of the S glycoprotein (Pinto et al., 2020). The single-chain variable fragment (scFv) of each antibody was cloned into the bicistronic lentiviral vector (pHIV) followed by a Flag-tag sequence (DYKDDDDK), a hinge IgG4 mutant region, the CD28 transmembrane (TM), and intracellular domain and the CD3ζ intracellular domain. As a control, Applicant generated another CAR with the scFv region of another monoclonal antibody termed CR3014 that reacts only with the RBD region of SARS-CoV-1 (CR3014-28Z) (FIG. 16).

Each pHIV bicistronic lentiviral construct contained IRES-EGFP, allowing for sorting and tracking the CAR-Ts. To generate CAR-Ts, peripheral blood mononuclear cells (PBMCs) from a given donor were stimulated *in vitro* with anti-CD3 and anti-CD28 antibodies for 72 h followed by spinoculation with lentiviral supernatant, further culturing for 4 days in X-VIVO15 serum-free medium followed by fluorescence-activated cell sorting (FACS) based on EGFP expression. Sorted CAR-Ts were then expanded in the same media (FIG. 17).

Applicant assessed whether each of the different anti-SARS-CoV-2 CAR-Ts were cytolytic in killing SARS-CoV-2 RBD/S1-loaded cells (FIGS. 18A-18F). CAR-Ts were co-cultured with 293-ACE2-RBD and other target cells at different effector to target ratios (10:1, 5:1, 2.5:1) for 5h for the assessment of killing. Each of the CARs except 4A8 could kill RBD pre-coated Jurkat-ACE2 cells (A), RBD peptide pre-coated Raji-ACE2 cells (B) (FIGS. 18A-18F). The lack of killing by the 4A8 based CAR-T was not expected since it does not bind the RBD, and instead binds to the N-terminal domain (NTD) of the S protein (Chi et al., 2020). Instead, 4A8 did kill S1 expressing NIH/3T3 S1 expressing cells (FIG. 18D) and 293t-SARS-CoV2 spike expressing cells (FIG. 18F). For unknown reasons, it was less effective in killing Raj-ACE2 expressing S1 cells (FIG. 18E). Unexpectedly, CAR-Ts showed differences in killing that would not have been predicted from simple antibody binding to RBD sequences as described by others (Pinto et al., 2020; Robbiani et al., 2020; Tian et al., 2020). For example, the CR30220-IgG4mut-28Z and S309-IgG4mut- killed more effectively than C135-IgG4mut CAR-Ts when Jurkat-ACE2-RBD, Raji-ACE-S1 or 293T-SARs-CoV2 Spike cells were used as targets (A, C, E, and F). This would not have been predicted on the basis of simple antibody binding.

Applicant also showed that mixed CARs were less effective than the individual CARs against S1 peptide pre-coated Raji-ACE2 cells (FIG. 18E) or against spike protein transfected 293 T-cells (FIG. 18F). S309-IgGmut-28Z and CR3022-IgG4mut-28Z, while C135 IgGmut-28Z was less effective in killing RBD pre-coated Jurkat-ACE2 cells. The predominance of S309-IgGmut-28Z and CR3022-IgG4mut-28Z was most routinely seen against Raji-ACE2-RBD and Raji-ACE2-S1 targets. Although the mixed cocktail of CARs was less effective than S309-IgG4mut-28Z and CR3022-IgG4mut-28Z, it was more effective than C135- IgG4mut-28Z. The mixed cocktail of CARs was similarly effective with individual CR3022-IgG4mut-28Z, 4A8-IgGmut-28Z and S309-IgG4mut-28Z but more effective than the individual C135- IgG4mut-28Z against 293T-SARS-CoV2 Spike protein (FIG. 18F).

Applicant next looked at the downregulation or endocytosis of the CAR-Ts which Applicant showed is an indicator of the engagement and activation of CARs receptor (Guo et al., 2021) (FIG. 19). In this context, the cytoplasmic tail, the regulator of endocytosis is the same for each of the CARs. CAR receptors on CD4 and CD8 cells are downregulated in response to 293T cells transfected with the SARS-CoV-2 spike protein. CAR-Ts were co-cultured for 4 h with 293T-spike cells. In keeping with enhanced ability to kill targets, the CR3022-IgG4mut-28Z and the S309-IgG4mut-28Z but more effective than the C135-IgG4mut-28Z in downregulating on CD4 and CD8+ T-cells (left and right panels). The 4A8-IgGmut-28Z showed intermediate effects on CAR down-regulation.

Applicant next assessed the expression of activation antigens on T-cells (FIGS. 20A-20H). Both CD4 and CD8+ T-cells showed increases in the expression of CD69 (FIGS. 5A-5B), the cell cycle indicator Ki67 (FIGS. 5C-5D), the effector mediator FasL (FIGS. 5E-5F) and the transcription factor Tbet (FIGS. 5G-5H). Although no effect on the negative control CAR-T was seen (CR3014), minor differences in expression were seen between the different CARs. 4A8-IgGmut-28Z was most effective in inducing CD69 expression on CD4 and CD8 T-cells.

In terms of cytokines, more variation was observed (FIGS. 21A-21F). 4A8-IgGmut-28Z and S309-IgG4mut-28Z were most effective in inducing the secretion of tumor necrosis factor alpha (FIGS. 21A-21B), interleukin 2 (FIGS. 21C-21D) and IFN-gamma (FIGS. 21E-21F). These effects could not be predicted by simple antibody binding or neutralization efficacies. Further, while 4A8-IgGmut-28Z showed intermediate effects on CAR down-regulation, it was statistically, the most potent CAR in the induction of interferon gamma production (FIGS. 21E-21F).

### Hinge regions

Applicant next examined the effect of different hinge regions on the reactivity of CAR-Ts against SARs CoV2 (FIG. 22). Various CARs were generated and compared by using the scFv regions of the 4A8 antibody that exhibits high neutralization potency against both authentic and pseudotyped SARS-CoV-2 but binds to the N-terminal domain (NTD) of the S protein (Chi et al., 2020). Using this as the backbone, Applicant generated versions using different sized hinge regions that included: (i) from the provisional application, Applicant used the PDGFRA (hinge region: 505aa); PDGFRB (hinge region: 500aa) and LAIR1 (hinge region: 144aa) sequences. Applicant also included a smaller sequence of the LAIR1 sequence that Applicant called 4A8-tLAIR1-28Z (hinge region: 48aa). This is seen in different killing assays as well as in the induction of T-cell activation, transcription factors, cytokines and effector molecules used to kill targets.

Each of the CAR-Ts was seen to be expressed at higher levels than the 4A8-PDGFRA-28Z, possible due to the larger size of the 4A8-PDGFRA-28Z CAR (FIGS. 23A-23C). Anti-Flag was used in flow cytometry to detect the CAR expression.

Differences were noted in their ability to kill NIH/3T3-S1 (FIGS. 24A-24B) or 293T-SARS-CoV2-Spike (i.e., full length with S1 and S2) targets (FIG. 24B). Each showed similar killing of NIH-3T3-S1 relative to the CD3014-IgG4mut-28Z control (FIG. 24A). By contrast, against the full-length Spike protein, major differences were seen. CD3014-IgG4mut-28Z, 4A8-PDGFRB-28Z, 4A8-tLAIR1-28Z were generally at all ratios examined (i.e., ratios 10/1 and 5/1 and 2.5/1). 4A8-IgG4mut-28Z, 4A8-tLAIR1-28Z predominated at an effecter to target ratio of 2.5/1, while 4A8-PDGFRB-28Z was also effective at similar levels to 4A8-IgG4mut-28Z at ratios 10/1 and to a lesser extent at 5/1. Despite this, it is important to state that 4A8-PDGFRB-28Z and 4A8-LAIR1-28Z also showed significant killing relative to the CR3014-IgG4mut-28Z CAR-T.

The dominance of CD3014-IgG4mut-28Z, 4A8-tLAIR1-28Z was also seen in the downregulation of CARs (FIGS. 25A-25B). However, were Applicant also observed potent 4A8-LAIR1-28Z downregulation on CD4 and CD8+ T-cells (FIGS. 25A-25B). This effect was significantly different from the CD3014-IgG4mut-28Z control.

In terms of induction of activation antigens and other markers, Applicant also compared the reactivity of different CAR-Ts after incubation with 293T-VSVG or 293T-Spike transduced cells for 24 h (FIGS. 26A-26D). While 293T-VSVG had no effect in inducing expression, 293-T-Spike effectively induced CD69 expression, in particular, on 4A8- LAIR1-28Z and 4A8-tLAIR1-28Z cells. This effect or 4A8-tLAIR1-28Z was significantly different from the CD3014-IgG4mut-28Z control on CD4+ T-cells (FIGS. 26A-26D). Similarly the on 4A8- LAIR1-28Z was most potent in inducing Ki67, although each of the new CAR-Ts induced significant expression (FIGS. 26A-26D).

In terms of cytokine production, 4A8- LAIR1-28Z and 4A8-tLAIR1-28Z also induced high levels of interferon-gamma (FIGS. 27A-27D) on CD4 and CD8+ T-cells as well as the effector granzyme B (GZMB) on CD4 and CD8+ T-cells (FIGS. 27A-27D). 4A8- LAIR1-28Z and the PDGFRA and PDGFRB elicited the highest effect on IL-2 production on CD4 (FIGS. 28A-28D) and CD8 T-cells (FIGS. 28A-28D); In terms of transcription factor T-bet expression, 4A8- LAIR1-28Z and 4A8-tLAIR1-28Z also induced high levels together with CD3014-IgG4mut-28Z. T-bet is the key transcription factor responsible for the generation of Th1 CD4+ T-cells and cytolytic CD8+ T-cells (Glimcher, 2007).

### Discussion

Given the ongoing threat of SARS-CoV-2 and the uncertainties related to the long-term efficacy of vaccines against new variants of the virus, new therapeutic approaches may be needed. The development of CAR therapy has been proven effective in the treatment of various types of cancers (Kawalekar et al., 2016; Posey et al., 2016). In this study, Applicant show that it is possible to generate CAR-T cells capable of responding to the SARS-CoV-2 and to elicit the *in vitro* and *in vivo* killing of cells loaded with RBD/S1 peptides or which express the S1 protein. Our findings outline the potential use of anti-SARS-CoV-2 CAR-Ts as a therapeutic option against COVID-19.

CR3022 based CAR-Ts were stimulated by the recognition of the RBD peptide as shown by the increased expression of CD69 and the phosphorylation of ribosomal S6, a key regulator of metabolism, translation, and cell division (Roux et al., 2007). CD69 expression was induced in a peptide concentration dependent manner, with responses to cells that had been loaded with a peptide-concentrations as low as 10ng/ml. This denotes a remarkable sensitivity to the peptide. CD69 expression also promotes the retention of T-cells in the lymphoid organs (Cibrian and Sanchez-Madrid, 2017) and serves as a marker for resident memory T cells (TRMs) (Osborn et al., 2019; Ziegler et al., 1994). In this way, the increased CD69 expression on CAR-Ts may alter their residency for enhanced responses against SARS-CoV-2 infected cells.

Exposure to 293-ACE2-RBD cells also potently downregulated CARs from the surface of CAR-Ts. This property has been previously reported for CD19-CARs (Li et al., 2020) and is another measure of receptor-mediated engagement (Monjas et al., 2004; San Jose et al., 2000; Schneider et al., 1999). In this manner, the level of CAR downregulation inversely mirrored the effect of different peptide concentrations on CD69 expression. While confirming that CAR-Ts were responsive to the RBD peptide, the loss of the surface CAR might ultimately limit the longevity of responses against targets as seen in tumor models (Li et al., 2020).

Importantly, SARS-CoV-2 CAR-Ts were cytolytic in the specific killing of target cells loaded with either the RBD peptide, S1 peptide or which express the S1 protein. Specificity was seen by the fact that CR3014 based CAR-Ts failed to kill SARS Cov-2 expressing target cells. In keeping with killing, time lapse microscopy showed the formation of multi-cellular CAR-T clusters around single target cells. Further, CAR-Ts with different sized hinge regions varied in the efficacy of killing where the IgG4 hinge CAR-T gave the most consistent results in the killing of targets. However, these differences appeared to vary depending on the mode of RBD or S1 presentation. For example, the IgG4 hinge CAR-Ts predominated in response to RBD peptide loaded on 293-ACE cells, while CD28 and IgG4 hinge CAR-Ts were most effective in response to S1 peptide presented on the surface of 3T3 cells. The different hinge regions may therefore affect the ability of CARs to bind and respond to peptides depending on their mode of presentation. By contrast, Applicant did not observe blockade of viral entry into cells based on the use of recombinant pseudo-typed VSV particles containing SARS-CoV-2 spike protein. These were used to mimic SARS-CoV-2 cell infection and cell entry. CAR-Ts are therefore unlikely to sterically block viral entry into cells, but rather limit viral infection in a classic fashion involving CTL-like killing of infected cells expressing viral antigens (Moskophidis and Kioussis, 1998; Rouse and Sehrawat, 2010).

The extent of SARS-CoV-2 viral peptide antigen expression during infection remains to be established. Various labs have shown that COVID-19 is accompanied by a robust induction of CD4 and CD8 responses against a variety of SARS-CoV-2 peptide antigens (Grifoni et al., 2020; Meckiff et al., 2020; Premkumar et al., 2020; Rydyznski Moderbacher et al., 2020; Weiskopf et al., 2020). Given that CD4 and CD8 T-cells are generated by the presentation of peptide antigens, these findings clearly show that peptides derived from various regions of SARS-CoV-2 are presented during infection. The same peptides could be targeted by CAR-Ts. Further, mass-spectrometry has detected SARS-CoV-2 virus-derived peptides in patients (Nikolaev et al., 2020),while SARS-CoV-2 infected cells undergo syncytia formation of ACE2-expressing cells, which can be facilitated by antigen recognition (Buchrieser et al., 2021).

In terms of mechanism, there are two well-established modes of killing by cytolytic T-cells, one involving Fas-FasL and another perforin/granzymes (Williams and Bevan, 2007). The loss of perforin and Fas/Fas ligand can cause severe autoimmune and inflammation in mice (Peng et al., 1998; Spielman et al., 1998). In our model, the expression of FasL and GZMB/perforin increased in response to RBD presenting cells. However, the blockade of FasL with antibodies had only a weak effect, but with certain CAR-Ts such as CD3022-28Z and CR3022-8a-28Z, a statistically significant effect on the killing of targets by the anti-SARS-CoV-2 CAR-Ts was observed. The observation points to the GZMB/perforin pathway as a major mechanism by which our anti-SARS-CoV-2 CAR-Ts kill targets with the participation of some Fas-FasL killing.

In this context, the effectors GZMB and perforin were expressed within a relatively small subset of CD69 high expressing anti-SARS-CoV-2 CAR-Ts. Perforin allows for GZMB into the target cell for cell death, although GZMB may also enter via other pathways (Smyth et al., 1996). While GZMB and perforin were expressed in the same subset, not all cells expressing high levels of CD69 expressed GZMB and perforin. This indicates that factors other than the mere activation of cells influenced whether these effectors were expressed.

By contrast, FasL and IFN-γ were more widely expressed on populations of CAR-Ts. viSNE analysis showed that FasL expression occurred in both CD69 high and intermediate populations, while the increase in IFN-γ tended to more restricted to the CD69 high population. These differences underscore the heterogeneity in the responses of individual SARS-CoV-2 CAR-Ts. IFN-γ has both anti-viral and immunoregulatory properties (Schroder et al., 2004) and can alter the transcription of multiple genes including major histocompatibility antigens on antigen-presenting cells (Schroder et al., 2004). Higher levels of type I interferons have also been associated with a lower risk of SARS-CoV-2 infection and amelioration of disease severity(Sui et al., 2021). IFN-γ also primes alveolar macrophages against secondary bacterial infections which may have relevance to COVID-19 associated pulmonary disease (Yao et al., 2018). It could also assist by inducing STAT3 activation which can activate GZMB expression for enhanced CTL effector function (Lu et al., 2019). The secretion of IFN-γ by CAR-T cells would, therefore, be expected to promote these beneficial events during the progression of SARS-CoV-2 infection.

CAR-Ts could be most effective in patients who are immunocompromised or who respond weakly to the virus or vaccines (Grifoni et al., 2020; Meckiff et al., 2020; Premkumar et al., 2020; Rydyznski Moderbacher et al., 2020; Weiskopf et al., 2020). Further, they may provide longer term memory responses in patients that fail to develop sufficient memory against vaccines. CAR-Ts against relapsed ALL cancers have shown sustained remissions for 24 months (Maude et al., 2014). The therapeutic approach could involve the retroviral transduction of T-cells extracted from patient blood and who are reinfused with CAR-Ts as shown for CD19-CAR-T approaches against cancer (Pfeiffer et al., 2018). Alternatively, the transfection or transduction of stem cells with our CAR-Ts would be used to reconstitute the immune system of patients with COV-2 reactive T-cells. In addition, the full or partial infusion of patients with their T-cells transduced to express the CARs or the expression CAR-Ts by other means such as in bone marrow transplantation or the injection of formations of our CAR-Ts as a vaccine to infect immune cells, each could be used in conjunction with the present and future vaccines encoding or expressing the component of SARs Cov2 such as the Spike protein, NTD sequences or the full virus. The vaccine would be recognized by the CAR-Ts leading to their stimulation which in turn, would provide help for antibody production against SARs CoV2 and/or promote the development of T-cell response to the vaccines. CAR-Ts would undergo cell expansion, as we demonstrated with Ki67 staining, and also develop into cytolytic T-cells (CTLs) which could kill SARs CoV2 infected cells. CAR-Ts might also contribute the development of other endogenous cells such as myeloid cells, dendritic T-cells which present antigen to other T-cells of the immune system. In this manner, our different hinge regions exhibited unexpected difference in their killing ability. For example, 4A8-tLAIRE-28Z killed as effectively or cases in certain cases, more effectively than 4A8-IgG4mut-28Z against 293T cells expressing the SARs-CoV2 spike proteins. Similarly, the 4A8-PDGFRB-28Z killed as effectively as 4A8-tLAIRE-28Z and 4A8-IgG4mut-28Z CAR-Ts despite its lower level of surface expression. The observation that 4A8-PDGFRB-28Z was poorly endocytosed following engagement might contribute to it efficacy given that the CAR would be expected to spend more time on the cell surface to mediate CTL function. Again, these properties could not have been predicted a priori. In this context, 4A8-LAIRE-28Z and 4A8-tLAIRE-28Z cells produced higher levels of interferon-gamma on CD4+ T-cells which promote more effective (DC) function by promoting the expression of MHC antigens and co-receptors needed to co-ligate and co-stimulate T-cells. Each of the CR3022 CAR-Ts with CD28alpha and IgG4 supported the induction of interferon-gamma (FIG. 4G). Unexpectedly, 4A8-LAIRE-28Z and 4A8-tLAIRE-28Z also induced the highest levels of GZMB expression in both CD4 and CD8 T-cells, a, effect that could not have been predicted beforehand based simply on the domain structure of their IG-like domains. The use of our small bridging sequence in CD3022-28Z was also effective in supporting the induction of the same level of GZMB and perforin, correlated with its ability to support the killing of 3T3-S 1 cells. However, surprisingly, it did not support the killing of 293-ACE2-S1 cells which were most effectively killed by the CR30220IgG4-28Z CAR with a bone fide Ig-like domain hinge region. These findings were unexpected. In a similar sense, it was also unexpected that the CR3022-IgG4-28Z CAR was most effective in supporting an increase FasL expression (FIG. 6A). We found that generally, the FasL pathway provided a minor but statistically significant contribution to the killing of targets (in addition to CTL-GZMB mediated killing) but its relative contribution would be expected to increase with higher levels of FasL expression. Lastly, 4A8-tLAIRE-28Z induced high levels of the transcription factor T-bet which could not have been predicted. T-bet is known to promote CD4+ Th1 subset development that provides help in the development of CD8+ CTLs. Further, they may provide longer term memory responses in patients that fail to develop sufficient memory against vaccines. CAR-Ts against relapsed ALL cancers have shown sustained remissions for 24 months (Maude et al., 2014). Surprisingly, we also found that the non-mutated form of the IgG4 hinge domain was effective in supporting the development of CAR-Ts against the RBD sequences as the mutated version. The non-mutated version promotes ADCC which is abrogated by mutation. This differs from CAR-Ts against tumor associated antigens where the non-mutated version is less effective than the mutated version. In fact, that non-mutated IgG4 domain in the CD3022-IgG4-28Z CAR provided the highest level of the S1 protein as presented by 293-ACE2 cells (FIG. 2C). It was also as effective as the CR3022-28Z CAR-T in killing 3T3 cells expressing S1 (FIG. 2D). Further, it would also unexpected that a small bridging sequence CR3022-28Z would be so effective in supporting the killing of targets. Lastly, although others have reported generation of other CARs in NK cells or macrophages against SARs Cov2 which mediate some in vitro killing (Ma et al, 2021; Fu et al 2021), our findings are the first to show to define unique CAR-Ts with other ligand binding regions against different regions of the Spike (i.e. RBD and NTD) with unique functions and, importantly, the first to show the efficacy of our CAR-Ts such as CR3022-28Z the in vivo killing of cells expressing S1in mice **(**FIGS. 7A-B). This represents a major advance since in vitro killing assays do not reliability indicate whether the same cells will be effective in a living organism. This demonstrates that our CAR-Ts can function in vivo, and as such, will have applications in immunotherapy for the treatment of COVID-19. Lastly, we also showed that our mutated IgG4 hinge region was effective in supporting CARs with different scFV regions such as C135, 4A8, S309 and CD3022. Unexpectedly, S309-IgG4mut-28Z and CD30220IgG4mut-28Z were generally the most effective in killing of RBD and S1 expressing target cells (**FIGS. 18A, 18B****,** **18C****,** **18E**). By contrast, 4A8-IgG4mut and S309-IgG4mut-CD28Z were the most supportive of the induction of tumor necrosis factor-alpha and interleukin 2 expression on CD4 and CD8 CAR-Ts **(****FIGS. 21A, 21B****,** **21C, 21D****),** while 4A8-IgG4mut-CD28Z was most effective in inducing interferon-gamma expression **(****FIGS.** 21E-F). These data indicate that CAR-Ts with different hinge regions can differentially induce different downstream events involved in killing and the modulation of the immune response that cannot be predicted a priori based on expression or structure.

### Equivalents

Unless otherwise defined, all technical and scientific terms used herein have the same meaning as commonly understood by one of ordinary skill in the art to which this technology belongs.

### References

1. Alfei, F., Kanev, K., Hofmann, M., Wu, M., Ghoneim, H.E., Roelli, P., Utzschneider, D.T., von Hoesslin, M., Cullen, J.G., Fan, Y., et al. (2019). TOX reinforces the phenotype and longevity of exhausted T cells in chronic viral infection. Nature 571, 265-269.
2. Allan, W., Tabi, Z., Cleary, A., and Doherty, P.C. (1990). Cellular events in the lymph node and lung of mice with influenza. Consequences of depleting CD4+ T cells. J Immunol 144, 3980-3986.
3. Altmann, D.M., and Boyton, R.J. (2020). SARS-CoV-2 T cell immunity: Specificity, function, durability, and role in protection. Sci Immunol 5, eabd6160.
4. Amanat, F., and Krammer, F. (2020). SARS-CoV-2 Vaccines: Status Report. Immunity 52, 583-589.
5. Barnes, C.O., C.A. Jette, M.E. Abernathy, K.A. Dam, S.R. Esswein, H.B. Gristick, A.G. Malyutin, N.G. Sharaf, K.E. Huey-Tubman, Y.E. Lee, D.F. Robbiani, M.C. Nussenzweig, A.P. West, Jr., and P.J. Bjorkman. 2020. SARS-CoV-2 neutralizing antibody structures inform therapeutic strategies. Nature 588:682-687.
6. Bloch, E.M., Shoham, S., Casadevall, A., Sachais, B.S., Shaz, B., Winters, J.L., van Buskirk, C., Grossman, B.J., Joyner, M., Henderson, J.P., et al. (2020). Deployment of convalescent plasma for the prevention and treatment of COVID-19. J Clin Invest 130, 2757-2765.
7. Braun, J., Loyal, L., Frentsch, M., Wendisch, D., Georg, P., Kurth, F., Hippenstiel, S., Dingeldey, M., Kruse, B., Fauchere, F., et al. (2020). SARS-CoV-2-reactive T cells in healthy donors and patients with COVID-19. Nature 587, 270-274.
8. Buchrieser, J., Dufloo, J., Hubert, M., Monel, B., Planas, D., Rajah, M.M., Planchais, C., Porrot, F., Guivel-Benhassine, F., Van der Werf, S., et al. (2021). Syncytia formation by SARS-CoV-2-infected cells. EMBO J 40, e107405.
9. Campbell, K.M., Steiner, G., Wells, D.K., Ribas, A., and Kalbasi, A. (2020). Prediction of SARS-CoV-2 epitopes across 9360 HLA class I alleles. bioRxiv, https://doi.org/10.1101/2020.1103.1130.016931.
10. Cao, Y., Su, B., Guo, X., Sun, W., Deng, Y., Bao, L., Zhu, Q., Zhang, X., Zheng, Y., Geng, C., et al. (2020). Potent Neutralizing Antibodies against SARS-CoV-2 Identified by High-Throughput Single-Cell Sequencing of Convalescent Patients' B Cells. Cell 182, 73-84 e16.
11. Cheng, M.H., Zhang, S., Porritt, R.A., Noval Rivas, M., Paschold, L., Willscher, E., Binder, M., Arditi, M., and Bahar, I. (2020). Superantigenic character of an insert unique to SARS-CoV-2 spike supported by skewed TCR repertoire in patients with hyperinflammation. Proc Natl Acad Sci U S A 117, 25254-25262.
12. Chi, X., R. Yan, J. Zhang, G. Zhang, Y. Zhang, M. Hao, Z. Zhang, P. Fan, Y. Dong, Y. Yang, Z. Chen, Y. Guo, J. Zhang, Y. Li, X. Song, Y. Chen, L. Xia, L. Fu, L. Hou, J. Xu, C. Yu, J. Li, Q. Zhou, and W. Chen. 2020. A neutralizing human antibody binds to the N-terminal domain of the Spike protein of SARS-CoV-2. Science 369:650-655.
13. Cibrian, D., and Sanchez-Madrid, F. (2017). CD69: from activation marker to metabolic gatekeeper. Eur J Immunol 47, 946-953.
14. Cizmecioglu, A., Cizmecioglu, H.A., Goktepe, M.H., Emsen, A., Korkmaz, C., Tasbent, F.E., Colkesen, F., and Artac, H. (2020). Apoptosis-Induced T Cell Lymphopenia Is Related to COVID-19 Severity. J Med Virol.
15. Ebinger, S., Ozdemir, E.Z., Ziegenhain, C., Tiedt, S., Castro Alves, C., Grunert, M., Dworzak, M., Lutz, C., Turati, V.A., Enver, T., et al. (2016). Characterization of Rare, Dormant, and Therapy-Resistant Cells in Acute Lymphoblastic Leukemia. Cancer Cell 30, 849-862.
16. Epstein, S.L., Lo, C.Y., Misplon, J.A., and Bennink, J.R. (1998). Mechanism of protective immunity against influenza virus infection in mice without antibodies. J Immunol 160, 322-327.
17. Fu, W., Lei, C., Ma, Z., Qian, K., Li, T., Zhao, J., and Hu, S. (2021). CAR Macrophages for SARS-CoV-2 Immunotherapy. Front Immunol 12, 669103.
18. Galdiero, M.R., Garlanda, C., Jaillon, S., Marone, G., and Mantovani, A. (2013). Tumor associated macrophages and neutrophils in tumor progression. J Cell Physiol 228, 1404-1412.
19. Glimcher, L.H. 2007. Trawling for treasure: tales of T-bet. Nat Immunol 8:448-450.
20. Graham, M.B., and Braciale, T.J. (1997). Resistance to and recovery from lethal influenza virus infection in B lymphocyte-deficient mice. J Exp Med 186, 2063-2068.
21. Grifoni, A., Weiskopf, D., Ramirez, S.I., Mateus, J., Dan, J.M., Moderbacher, C.R., Rawlings, S.A., Sutherland, A., Premkumar, L., Jadi, R.S., et al. (2020). Targets of T Cell Responses to SARS-CoV-2 Coronavirus in Humans with COVID-19 Disease and Unexposed Individuals. Cell 181, 1489-1501 e1415.
22. Gross, G., Gorochov, G., Waks, T., and Eshhar, Z. (1989). Generation of effector T cells expressing chimeric T cell receptor with antibody type-specificity. Transplant Proc 21, 127-130.
23. Guedan, S., Ruella, M., and June, C.H. (2019). Emerging Cellular Therapies for Cancer. Annu Rev Immunol 37, 145-171.
24. Guo, X., A. Kazanova, S. Thurmond, H.U. Saragovi, and C.E. Rudd. 2021. Effective chimeric antigen receptor T-cells (CAR-Ts) against SARS-CoV-2. iScience 103295.
25. Hoffmann, M., Kleine-Weber, H., and Pohlmann, S. (2020a). A Multibasic Cleavage Site in the Spike Protein of SARS-CoV-2 Is Essential for Infection of Human Lung Cells. Mol Cell 78, 779-784 e775.
26. Hoffmann, M., Kleine-Weber, H., Schroeder, S., Kruger, N., Herrler, T., Erichsen, S., Schiergens, T.S., Herrler, G., Wu, N.H., Nitsche, A., et al. (2020b). SARS-CoV-2 Cell Entry Depends on ACE2 and TMPRSS2 and Is Blocked by a Clinically Proven Protease Inhibitor. Cell 181, 271-280 e278.
27. Hudecek, M., Sommermeyer, D., Kosasih, P.L., Silva-Benedict, A., Liu, L., Rader, C., Jensen, M.C., and Riddell, S.R. (2015). The nonsignaling extracellular spacer domain of chimeric antigen receptors is decisive for in vivo antitumor activity. Cancer Immunol Res 3, 125-135.
28. Ihling, C., Tanzler, D., Hagemann, S., Kehlen, A., Huttelmaier, S., Arlt, C., and Sinz, A. (2020). Mass Spectrometric Identification of SARS-CoV-2 Proteins from Gargle Solution Samples of COVID-19 Patients. J Proteome Res 19, 4389-4392.
29. Irving, B.A., and Weiss, A. (1991). The cytoplasmic domain of the T cell receptor zeta chain is sufficient to couple to receptor-associated signal transduction pathways. Cell 64, 891-901.
30. Jean, S.S., Lee, P.I., and Hsueh, P.R. (2020). Treatment options for COVID-19: The reality and challenges. J Microbiol Immunol Infect 53, 436-443.
31. Jena, B., Dotti, G., and Cooper, L.J. (2010). Redirecting T-cell specificity by introducing a tumor-specific chimeric antigen receptor. Blood 116, 1035-1044.
32. Kawalekar, O.U., O'Connor, R.S., Fraietta, J.A., Guo, L., McGettigan, S.E., Posey, A.D., Jr., Patel, P.R., Guedan, S., Scholler, J., Keith, B., et al. (2016). Distinct Signaling of Coreceptors Regulates Specific Metabolism Pathways and Impacts Memory Development in CAR T Cells. Immunity 44, 380-390.
33. Kayagaki, N., Kawasaki, A., Ebata, T., Ohmoto, H., Ikeda, S., Inoue, S., Yoshino, K., Okumura, K., and Yagita, H. (1995). Metalloproteinase-mediated release of human Fas ligand. J Exp Med 182, 1777-1783.
34. Krebs, K., Bottinger, N., Huang, L.R., Chmielewski, M., Arzberger, S., Gasteiger, G., Jager, C., Schmitt, E., Bohne, F., Aichler, M., et al. (2013). T cells expressing a chimeric antigen receptor that binds hepatitis B virus envelope proteins control virus replication in mice. Gastroenterology 145, 456-465.
35. Kurschus, F.C., and Jenne, D.E. (2010). Delivery and therapeutic potential of human granzyme B. Immunol Rev 235, 159-171.
36. Lan, J., Ge, J., Yu, J., Shan, S., Zhou, H., Fan, S., Zhang, Q., Shi, X., Wang, Q., Zhang, L., and Wang, X. (2020). Structure of the SARS-CoV-2 spike receptor-binding domain bound to the ACE2 receptor. Nature 581, 215-220.
37. Letko, M., Marzi, A., and Munster, V. (2020). Functional assessment of cell entry and receptor usage for SARS-CoV-2 and other lineage B betacoronaviruses. Nat Microbiol 5, 562-569.
38. Li, W., Qiu, S., Chen, J., Jiang, S., Chen, W., Jiang, J., Wang, F., Si, W., Shu, Y., Wei, P., et al. (2020). Chimeric antigen receptor designed to prevent ubiquitination and downregulation showed durable antitumor efficacy. Immunity 53, 456-470 e456.
39. Lu, C., Klement, J.D., Ibrahim, M.L., Xiao, W., Redd, P.S., Nayak-Kapoor, A., Zhou, G., and Liu, K. (2019). Type I interferon suppresses tumor growth through activating the STAT3-granzyme B pathway in tumor-infiltrating cytotoxic T lymphocytes. J Immunother Cancer 7, 157.
40. Ma, M.T., Badeti, S., Chen, C.H., Kim, J., Choudhary, A., Honnen, B., Reichman, C., Calianese, D., Pinter, A., Jiang, Q., et al. (2021). CAR-NK Cells Effectively Target SARS-CoV-2-Spike-Expressing Cell Lines In Vitro. Front Immunol 12, 652223.
41. Maude, S.L., Frey, N., Shaw, P.A., Aplenc, R., Barrett, D.M., Bunin, N.J., Chew, A., Gonzalez, V.E., Zheng, Z., Lacey, S.F., et al. (2014). Chimeric antigen receptor T cells for sustained remissions in leukemia. N Engl J Med 371, 1507-1517.
42. Maus, M.V., and June, C.H. (2016). Making Better Chimeric Antigen Receptors for Adoptive T-cell Therapy. Clin Cancer Res 22, 1875-1884.
43. McLane, L.M., Abdel-Hakeem, M.S., and Wherry, E.J. (2019). CD8 T cell exhaustion during chronic viral infection and cancer. Annu Rev Immunol.
44. McMichael, A.J., Michie, C.A., Gotch, F.M., Smith, G.L., and Moss, B. (1986). Recognition of influenza A virus nucleoprotein by human cytotoxic T lymphocytes. J Gen Virol 67 ( Pt 4), 719-726.
45. Meckiff, B.J., Ramirez-Suastegui, C., Fajardo, V., Chee, S.J., Kusnadi, A., Simon, H., Eschweiler, S., Grifoni, A., Pelosi, E., Weiskopf, D., et al. (2020). Imbalance of Regulatory and Cytotoxic SARS-CoV-2-Reactive CD4(+) T Cells in COVID-19. Cell 183, 1340-1353 e1316.
46. Millet, J.K., Tang, T., Nathan, L., Jaimes, J.A., Hsu, H.L., Daniel, S., and Whittaker, G.R. (2019). Production of Pseudotyped Particles to Study Highly Pathogenic Coronaviruses in a Biosafety Level 2 Setting. J Vis Exp.
47. Monjas, A., Alcover, A., and Alarcon, B. (2004). Engaged and bystander T cell receptors are down-modulated by different endocytotic pathways. J Biol Chem 279, 55376-55384.
48. Moskophidis, D., and Kioussis, D. (1998). Contribution of virus-specific CD8+ cytotoxic T cells to virus clearance or pathologic manifestations of influenza virus infection in a T cell receptor transgenic mouse model. J Exp Med 188, 223-232.
49. Nikolaev, E.N., Indeykina, M.I., Brzhozovskiy, A.G., Bugrova, A.E., Kononikhin, A.S., Starodubtseva, N.L., Petrotchenko, E.V., Kovalev, G.I., Borchers, C.H., and Sukhikh, G.T. (2020). Mass-Spectrometric Detection of SARS-CoV-2 Virus in Scrapings of the Epithelium of the Nasopharynx of Infected Patients via Nucleocapsid N Protein. J Proteome Res 19, 4393-4397.
50. Osborn, J.F., Hobbs, S.J., Mooster, J.L., Khan, T.N., Kilgore, A.M., Harbour, J.C., and Nolz, J.C. (2019). Central memory CD8+ T cells become CD69+ tissue-residents during viral skin infection independent of CD62L-mediated lymph node surveillance. PLoS Pathog 15, e1007633.
51. Paidi, R.K., Jana, M., Mishra, R.K., Dutta, D., Raha, S., and Pahan, K. (2021). ACE-2-interacting Domain of SARS-CoV-2 (AIDS) Peptide Suppresses Inflammation to Reduce Fever and Protect Lungs and Heart in Mice: Implications for COVID-19 Therapy. J Neuroimmune Pharmacol.
52. Peng, S.L., Moslehi, J., Robert, M.E., and Craft, J. (1998). Perforin protects against autoimmunity in lupus-prone mice. J Immunol 160, 652-660.
53. Peng, Y., Mentzer, A.J., Liu, G., Yao, X., Yin, Z., Dong, D., Dejnirattisai, W., Rostron, T., Supasa, P., Liu, C., et al. (2020). Broad and strong memory CD4(+) and CD8(+) T cells induced by SARS-CoV-2 in UK convalescent individuals following COVID-19. Nat Immunol 21, 1336-1345.
54. Pfeiffer, A., Thalheimer, F.B., Hartmann, S., Frank, A.M., Bender, R.R., Danisch, S., Costa, C., Wels, W.S., Modlich, U., Stripecke, R., et al. (2018). In vivo generation of human CD19-CAR T cells results in B-cell depletion and signs of cytokine release syndrome. EMBO Mol Med 10.
55. Pinto, D., Y.J. Park, M. Beltramello, A.C. Walls, M.A. Tortorici, S. Bianchi, S. Jaconi, K. Culap, F. Zatta, A. De Marco, A. Peter, B. Guarino, R. Spreafico, E. Cameroni, J.B. Case, R.E. Chen, C. Havenar-Daughton, G. Snell, A. Telenti, H.W. Virgin, A. Lanzavecchia, M.S. Diamond, K. Fink, D. Veesler, and D. Corti. 2020. Cross-neutralization of SARS-CoV-2 by a human monoclonal SARS-CoV antibody. Nature 583:290-295.
56. Posey, A.D., Jr., Schwab, R.D., Boesteanu, A.C., Steentoft, C., Mandel, U., Engels, B., Stone, J.D., Madsen, T.D., Schreiber, K., Haines, K.M., et al. (2016). Engineered CAR T Cells Targeting the Cancer-Associated Tn-Glycoform of the Membrane Mucin MUC1 Control Adenocarcinoma. Immunity 44, 1444-1454.
57. Premkumar, L., Segovia-Chumbez, B., Jadi, R., Martinez, D.R., Raut, R., Markmann, A., Cornaby, C., Bartelt, L., Weiss, S., Park, Y., et al. (2020). The receptor binding domain of the viral spike protein is an immunodominant and highly specific target of antibodies in SARS-CoV-2 patients. Sci Immunol 5.
58. Raab, M., Wang, H., Lu, Y., Smith, X., Wu, Z., Strebhardt, K., Ladbury, J.E., and Rudd, C.E. (2010). T cell receptor "inside-out" pathway via signaling module SKAP1-RapL regulates T cell motility and interactions in lymph nodes. Immunity 32, 541-556.
59. Robbiani, D.F., C. Gaebler, F. Muecksch, J.C.C. Lorenzi, Z. Wang, A. Cho, M. Agudelo, C.O. Barnes, A. Gazumyan, S. Finkin, T. Hagglof, T.Y. Oliveira, C. Viant, A. Hurley, H.H. Hoffmann, K.G. Millard, R.G. Kost, M. Cipolla, K. Gordon, F. Bianchini, S.T. Chen, V. Ramos, R. Patel, J. Dizon, I. Shimeliovich, P. Mendoza, H. Hartweger, L. Nogueira, M. Pack, J. Horowitz, F. Schmidt, Y. Weisblum, E. Michailidis, A.W. Ashbrook, E. Waltari, J.E. Pak, K.E. Huey-Tubman, N. Koranda, P.R. Hoffman, A.P. West, Jr., C.M. Rice, T. Hatziioannou, P.J. Bjorkman, P.D. Bieniasz, M. Caskey, and M.C. Nussenzweig. 2020. Convergent antibody responses to SARS-CoV-2 in convalescent individuals. Nature 584:437-442.
60. Rouse, B.T., and Sehrawat, S. (2010). Immunity and immunopathology to viruses: what decides the outcome? Nat Rev Immunol 10, 514-526.
61. Roux, P.P., Shahbazian, D., Vu, H., Holz, M.K., Cohen, M.S., Taunton, J., Sonenberg, N., and Blenis, J. (2007). RAS/ERK signaling promotes site-specific ribosomal protein S6 phosphorylation via RSK and stimulates cap-dependent translation. J Biol Chem 282, 14056-14064.
62. Russell, B., Moss, C., George, G., Santaolalla, A., Cope, A., Papa, S., and Van Hemelrijck, M. (2020). Associations between immune-suppressive and stimulating drugs and novel COVID-19-a systematic review of current evidence. Ecancermedicalscience 14, 1022.
63. Russell, J.H., and Ley, T.J. (2002). Lymphocyte-mediated cytotoxicity. Annu Rev Immunol 20, 323-370.
64. Rydyznski Moderbacher, C., Ramirez, S.I., Dan, J.M., Grifoni, A., Hastie, K.M., Weiskopf, D., Belanger, S., Abbott, R.K., Kim, C., Choi, J., et al. (2020). Antigen-Specific Adaptive Immunity to SARS-CoV-2 in Acute COVID-19 and Associations with Age and Disease Severity. Cell 183, 996-1012 e1019.
65. Sadelain, M., Brentjens, R., and Riviere, I. (2009). The promise and potential pitfalls of chimeric antigen receptors. Curr Opin Immunol 21, 215-223.
66. San Jose, E., Borroto, A., Niedergang, F., Alcover, A., and Alarcon, B. (2000). Triggering the TCR complex causes the downregulation of nonengaged receptors by a signal transduction-dependent mechanism. Immunity 12, 161-170.
67. Sarzi-Puttini, P., Giorgi, V., Sirotti, S., Marotto, D., Ardizzone, S., Rizzardini, G., Antinori, S., and Galli, M. (2020). COVID-19, cytokines and immunosuppression: what can we learn from severe acute respiratory syndrome? Clin Exp Rheumatol 38, 337-342.
68. Schneider, H., Martin, M., Agarraberes, F.A., Yin, L., Rapoport, I., Kirchhausen, T., and Rudd, C.E. (1999). Cytolytic T lymphocyte-associated antigen-4 and the TCR zeta/CD3 complex, but not CD28, interact with clathrin adaptor complexes AP-1 and AP-2. J Immunol 163, 1868-1879.
69. Schoenborn, J.R., and Wilson, C.B. (2007). Regulation of interferon-gamma during innate and adaptive immune responses. Adv Immunol 96, 41-101.
70. Schroder, K., Hertzog, P.J., Ravasi, T., and Hume, D.A. (2004). Interferon-gamma: an overview of signals, mechanisms and functions. J Leukoc Biol 75, 163-189.
71. Seif, M., Einsele, H., and Loffler, J. (2019). CAR T Cells Beyond Cancer: Hope for Immunomodulatory Therapy of Infectious Diseases. Front Immunol 10, 2711.
72. Shetty, A.K. (2020). Mesenchymal Stem Cell Infusion Shows Promise for Combating Coronavirus (COVID-19)- Induced Pneumonia. Aging Dis 11, 462-464.
73. Shi, R., Shan, C., Duan, X., Chen, Z., Liu, P., Song, J., Song, T., Bi, X., Han, C., Wu, L., et al. (2020). A human neutralizing antibody targets the receptor-binding site of SARS-CoV-2. Nature 584, 120-124.
74. Smyth, M.J., Sutton, V.R., Kershaw, M.H., and Trapani, J.A. (1996). Xenospecific cytotoxic T lymphocytes use perforin- and Fas-mediated lytic pathways. Transplantation 62, 1529-1532.
75. Song, J.W., Zhang, C., Fan, X., Meng, F.P., Xu, Z., Xia, P., Cao, W.J., Yang, T., Dai, X.P., Wang, S.Y., et al. (2020). Immunological and inflammatory profiles in mild and severe cases of COVID-19. Nat Commun 11, 3410.
76. Spielman, J., Lee, R.K., and Podack, E.R. (1998). Perforin/Fas-ligand double deficiency is associated with macrophage expansion and severe pancreatitis. J Immunol 161, 7063-7070.
77. Sui, Y., Bekele, Y., and Berzofsky, J.A. (2021). Potential SARS-CoV-2 Immune Correlates of Protection in Infection and Vaccine Immunization. Pathogens 10.
78. Tay, M.Z., Poh, C.M., Renia, L., MacAry, P.A., and Ng, L.F.P. (2020). The trinity of COVID-19: immunity, inflammation and intervention. Nat Rev Immunol 20, 363-374.
79. ter Meulen, J., van den Brink, E.N., Poon, L.L., Marissen, W.E., Leung, C.S., Cox, F., Cheung, C.Y., Bakker, A.Q., Bogaards, J.A., van Deventer, E., et al. (2006). Human monoclonal antibody combination against SARS coronavirus: synergy and coverage of escape mutants. PLoS Med 3, e237.
80. Tharmarajah, E., Buazon, A., Patel, V., Hannah, J.R., Adas, M., Allen, V.B., Bechman, K., Clarke, B.D., Nagra, D., Norton, S., et al. (2021). IL-6 inhibition in the treatment of COVID-19: A meta-analysis and meta-regression. J Infect 82, 178-185.
81. Thomas, P.G., Keating, R., Hulse-Post, D.J., and Doherty, P.C. (2006). Cell-mediated protection in influenza infection. Emerg Infect Dis 12, 48-54.
82. Tian, X., Li, C., Huang, A., Xia, S., Lu, S., Shi, Z., Lu, L., Jiang, S., Yang, Z., Wu, Y., and Ying, T. (2020). Potent binding of 2019 novel coronavirus spike protein by a SARS coronavirus-specific human monoclonal antibody. Emerg Microbes Infect 9, 382-385.
83. Topham, D.J., Tripp, R.A., and Doherty, P.C. (1997). CD8+ T cells clear influenza virus by perforin or Fas-dependent processes. J Immunol 159, 5197-5200.
84. Urra, J.M., Cabrera, C.M., Porras, L., and Rodenas, I. (2020). Selective CD8 cell reduction by SARS-CoV-2 is associated with a worse prognosis and systemic inflammation in COVID-19 patients. Clin Immunol 217, 108486.
85. Varchetta, S., Mele, D., Oliviero, B., Mantovani, S., Ludovisi, S., Cerino, A., Bruno, R., Castelli, A., Mosconi, M., Vecchia, M., et al. (2020). Unique immunological profile in patients with COVID-19. Cell Mol Immunol.
86. Vivier, E., Raulet, D.H., Moretta, A., Caligiuri, M.A., Zitvogel, L., Lanier, L.L., Yokoyama, W.M., and Ugolini, S. (2011). Innate or adaptive immunity? The example of natural killer cells. Science 331, 44-49.
87. Weiskopf, D., Schmitz, K.S., Raadsen, M.P., Grifoni, A., Okba, N.M.A., Endeman, H., van den Akker, J.P.C., Molenkamp, R., Koopmans, M.P.G., van Gorp, E.C.M., et al. (2020). Phenotype and kinetics of SARS-CoV-2-specific T cells in COVID-19 patients with acute respiratory distress syndrome. Sci Immunol 5.
88. Welm, B.E., Dijkgraaf, G.J., Bledau, A.S., Welm, A.L., and Werb, Z. (2008). Lentiviral transduction of mammary stem cells for analysis of gene function during development and cancer. Cell Stem Cell 2, 90-102.
89. Williams, M.A, and Bevan, M.J. (2007). Effector and memory CTL differentiation. Annual review of immunology 25, 171-192.
90. Wrobel, A.G., Benton, D.J., Xu, P., Roustan, C., Martin, S.R., Rosenthal, P.B., Skehel, J.J., and Gamblin, S.J. (2020). SARS-CoV-2 and bat RaTG13 spike glycoprotein structures inform on virus evolution and furin-cleavage effects. Nature structural & molecular biology 27, 763-767.
91. Wu, C., Liu, Y., Yang, Y., Zhang, P., Zhong, W., Wang, Y., Wang, Q., Xu, Y., Li, M., Li, X., et al. (2020). Analysis of therapeutic targets for SARS-CoV-2 and discovery of potential drugs by computational methods. Acta Pharm Sin B.
92. Xu, X., Ong, Y.K., and Wang, Y. (2020). Role of adjunctive treatment strategies in COVID-19 and a review of international and national clinical guidelines. Mil Med Res 7, 22.
93. Yao, Y., Jeyanathan, M., Haddadi, S., Barra, N.G., Vaseghi-Shanjani, M., Damjanovic, D., Lai, R., Afkhami, S., Chen, Y., Dvorkin-Gheva, A., et al. (2018). Induction of Autonomous Memory Alveolar Macrophages Requires T Cell Help and Is Critical to Trained Immunity. Cell 175, 1634-1650 e1617.
94. Yuan, M., Wu, N.C., Zhu, X., Lee, C.-C.D., So, R.T.Y., Lv, H., Mok, C.K.P., and Wilson, I.A. (2020). A highly conserved cryptic epitope in the receptor binding domains of SARS-CoV-2 and SARS-CoV. Science 368, 630-633.
95. Ziegler, S.F., Ramsdell, F., and Alderson, M.R. (1994). The activation antigen CD69. Stem Cells 12, 456-465.

### Sequence Listing

SEQ ID NO: 1: (IgG4 Hinge) 
ESKYGPPCPSCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSS
IEKTISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSL
GK
SEQ ID NO: 2: (SARS-CoV-2 Spike Protein) 
NITNLCPFGEVFNATKFPSVYAWERKKISNCVADYSVLYNSTFFSTFKCYGVSATKL
NDLCFSNVYADSFVVKGDDVRQIAPGQTGVIADYNYKLPDDFMGCVLAWNTRNID
ATSTGNYNYKYRYLRHGKLRPFERDISNVPFSPDGKPCTPPALNCYWPLNDYGFYTT
TGIGYQPYRVVVLSFELLNAPATVCGPKLSTDLI
SEQ ID NO: 3: (CR3022 HCDR1) FITYWIGW
SEQ ID NO: 4: (CR3022 HCDR2) MGIIYPGDSETRYSPSFQGQ
SEQ ID NO: 5: (CR3022 HCDR3) CAGGSGISTPMDVW
SEQ ID NO: 6: (CR3022 LCDR1) CKSSQSVLYSSINKNYLAW
SEQ ID NO: 7: (CR3022 LCDR2) YWASTRESG
SEQ ID NO: 8: (CR3022 LCDR3) CQQYYSTPYTF
SEQ ID NO: 9: (V_{H} CR3022) 
EVQLVQSGTEVKKPGESLKISCKGSGYGFITYWIGWVRQMPGKGLEWMGIIYPGDSE
TRYSPSFQGQVTISADKSINTAYLQWSSLKASDTAIYYCAGGSGISTPMDVWGQGTT
VTVSS
SEQ ID NO: 10: (V_{L} CR3022) 
DIVMTQSPDSLAVSLGERATINCKSSQSVLYSSINKNYLAWYQQKPGQPPKLLIYWA
STRESGVPDRFSGSGSGTDFTLTISSLQAEDVAVYYCQQYYSTPYTFGQGTKVEIK
SEQ ID NO: 11: (Peptide linker) GGGGSGGGGSGGGGS
SEQ ID NO: 12: (CD28 transmembrane domain)
   FWVLVVVGGVLACYSLLVTVAFIIFWV
SEQ ID NO: 13: (CD28 co-stimulatory domain)
   RSKRSRLLHSDYMNMTPRRPGPTRKHYQPYAPPRDFAAYRS
SEQ ID NO: 14: (CD3ζ intracellular signaling domain) 
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQAL
PPR
SEQ ID NO: 15: (Flag tag) DYKDDDDK
SEQ ID NO: 16: (His tag) HHHHHH
SEQ ID NO: 17: (Myc tag) EQKLISEEDL
SEQ ID NO: 18: (HA tag) YPYDVPDYA
SEQ ID NO: 19: (GM-CSF signal peptide) MWLQSLLLLGTVACSIS
SEQ ID NO: 20: (CR3022-IgGmut-28Z full construct) 
MALPVTALLLPLALLLHAARPDIVMTQSPDSLAVSLGERATINCKSSQSVLYSSINKN
YLAWYQQKPGQPPKLLIYWASTRESGVPDRFSGSGSGTDFTLTISSLQAEDVAVYYC
QQYYSTPYTFGQGTKVEIKGGGGSGGGGSGGGGSEVQLVQSGTEVKKPGESLKISCK
GSGYGFITYWIGWVRQMPGKGLEWMGIIYPGDSETRYSPSFQGQVTISADKSINTAY
LQWSSLKASDTAIYYCAGGSGISTPMDVWGQGTTVTVSSDYKDDDDKESKYGPPCP
PCPAPPVAGPSVFLFPPKPKDTLMISRTPEVTCVVVDVSQEDPEVQFNWYVDGVEVH
NAKTKPREEQFQSTYRVVSVLTVLHQDWLNGKEYKCKVSNKGLPSSIEKTISKAKG
QPREPQVYTLPPSQEEMTKNQVSLTCLVKGFYPSDIAVEWESNGQPENNYKTTPPVL
DSDGSFFLYSRLTVDKSRWQEGNVFSCSVMHEALHNHYTQKSLSLSLGKFWVLVVV
GGVLACYSLLVTVAFIIFWVRSKRSRGGHSDYMNMTPRRPGPTRKHYQPYAPPRDF
AAYRSRVKFSRSADAPAYQQGQNQLYNELNLGRREEYDVLDKRRGRDPEMGGKPR
 
RKNPQEGLYNELQKDKMAEAYSEIGMKGERRRGKGHDGLYQGLSTATKDTYDALH
MQALPPR
SEQ ID NO: 21: (CR3022-IgGmut-28Z DNA) 
ATGGCTCTGCCTGTGACAGCTCTGCTGCTGCCTCTGGCTCTGCTTCTGCATGCCGC
CAGACCTGACATCGTGATGACACAGAGCCCTGACAGCCTGGCCGTGTCTCTGGG
AGAAAGAGCCACCATCAACTGCAAGAGCAGCCAGAGCGTGCTGTACTCCAGCAT
CAACAAGAACTACCTGGCCTGGTATCAGCAGAAGCCCGGCCAGCCTCCTAAGCT
GCTGATCTACTGGGCCAGCACCAGAGAAAGCGGCGTGCCCGATAGATTTCTGG
CAGCGGCTCTGGCACCGACTTCACCCTGACAATTAGCTCCCTGCAGGCCGAGGAT
GTGGCCGTGTACTACTGCCAGCAGTACTACAGCACCCCTTACACCTTTGGCCAGG
GCACCAAGGTGGAAATCAAAGGCGGCGGAGGATCTGGCGGAGGTGGAAGTGGC
GGAGGCGGATCTGAAGTTCAGCTGGTGCAGAGCGGCACCGAAGTGAAGAAGCCT
GGCGAGAGCCTGAAGATCTCCTGCAAAGGCTCCGGCTACGGCTTCATCACCTACT
GGATCGGCTGGGTCCGACAGATGCCTGGCAAAGGCCTTGAGTGGATGGGCATCA
TCTACCCCGGCGACAGCGAGACAAGATACAGCCCTAGCTTCCAGGGCCAAGTGA
CCATCAGCGCCGACAAGAGCATCAATACCGCCTACCTGCAGTGGTCCAGCCTGA
AGGCCTCTGACACCGCCATCTACTATTGTGCCGGCGGAAGCGGCATCAGCACCCC
AATGGATGTTTGGGGCCAGGGAACCACCGTGACCGTTTCTTCTGACTACAAGGAC
GACGACGACAAGGAATCTAAGTACGGCCCTCCTTGTCCTCCATGTCCTGCTCCAC
CTGTGGCCGGACCCTCCGTGTTCCTGTTTCCTCCAAAGCCTAAGGACACCCTGAT
GATCAGCAGAACCCCTGAAGTGACCTGCGTGGTGGTGGACGTTTCCCAAGAGGA
CCCTGAGGTGCAGTTCAATTGGTACGTGGACGGCGTGGAAGTGCACAACGCCAA
GACCAAGCCTAGAGAGGAACAGTTCCAGAGCACCTACAGAGTGGTGTCCGTGCT
GACCGTGCTGCACCAGGATTGGCTGAACGGCAAAGAGTACAAGTGCAAGGTGTC
CAACAAGGGCCTGCCTAGCAGCATCGAGAAAACCATCAGCAAGGCCAAGGGCC
AGCCAAGAGAACCCCAGGTGTACACACTGCCTCCAAGCCAAGAGGAAATGACCA
AGAACCAGGTGTCCCTGACCTGCCTGGTCAAGGGCTTCTACCCTTCCGATATCGC
CGTGGAATGGGAGAGCAATGGCCAGCCTGAGAACAACTACAAGACCACACCTCC
TGTGCTGGACAGCGACGGCTCATTCTTCCTGTACAGCAGACTGACCGTGGACAAG
AGCAGATGGCAAGAGGGCAACGTGTTCAGCTGCAGCGTGATGCACGAGGCCCTG
CACAACCACTACACCCAGAAGTCTCTGAGCCTGAGCCTGGGCAAGTTCTGGGTG
CTCGTTGTTGTTGGCGGCGTGCTGGCCTGTTACTCTCTGCTGGTTACCGTGGCCTT
 
CATCATCTTTTGGGTCCGAAGCAAGCGGAGCAGAGGCGGCCACAGCGACTACAT
GAACATGACCCCTAGACGGCCCGGACCAACCAGAAAGCACTACCAGCCTTACGC
TCCTCCTAGAGACTTCGCCGCCTACCGGTCCAGAGTGAAGTTCAGCAGATCCGCC
GATGCTCCCGCCTATCAGCAGGGACAGAACCAGCTGTACAACGAGCTGAACCTG
GGGAGAAGAGAAGAGTACGACGTGCTGGACAAGCGGAGAGGCAGAGATCCTGA
GATGGGCGGCAAGCCCAGACGGAAGAATCCTCAAGAGGGCCTGTATAATGAGCT
GCAGAAAGACAAGATGGCCGAGGCCTACAGCGAGATCGGAATGAAGGGCGAGC
GCAGAAGAGGCAAGGGACACGATGGACTGTACCAGGGCCTGAGCACCGCCACC
AAGGATACCTATGATGCCCTGCACATGCAGGCCCTGCCTCCAAGATAA
SEQ ID NO: 22: (V_{H} CR3104): 
EVQLVESGGGLVQPGGSLRLSCAASGFTFSDHYMDWVRQAPGKGLEWVGRTRNKA
NSYTTEYAASVKGRFTISRDDSKNSLYLQMNSLKTEDTAVYYCARGISPFYFDYWG
QGTLVTVSS
SEQ ID NO: 23: (IgG4mut Hinge) 
ESKYGPPCPPCPAPPVAGPSVFLFPPKPKDTLMISRTPEVTCVVVDVSQEDPEVQFNW
YVDGVEVHNAKTKPREEQFQSTYRVVSVLTVLHQDWLNGKEYKCKVSNKGLPSSIE
KTISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLG
K
SEQ ID NO: 24: (modified CD-28 co-stimulatory domain)
   RSKRSRGGHSDYMNMTPRRPGPTRKHYQPYAPPRDFAAYRS
SEQ ID NO: 25: (CD8α signal peptide) MALPVTALLLPLALLLHAARP
SEQ ID NO: 26: (CD4 signal peptide) MNRGVPFRHLLLVLQLALLPAATQG
SEQ ID NO: 27: (CD28 signal peptide) MLRLLLALNLFPSIQVTG
SEQ ID NO: 28: (Receptor binding domain of the S protein) 
RVVPSGDVVRFPNITNLCPFGEVFNATKFPSVYAWERKKISNCVADYSVLYNSTFFST
FKCYGVSATKLNDLCFSNVYADSFVVKGDDVRQIAPGQTGVIADYNYKLPDDFMGC
 
VLAWNTRNIDATSTGNYNYKYRYLRHGKLRPFERDISNVPFSPDGKPCTPPALNCY
WPLNDYGFYTTTGIGYQPYRVVVLSFELLNAPATVCGPKLSTDLIKNQCVNF
SEQ ID NO: 29: (V_{H} S309) 
QVQLVESGGGVVQPGRSLRLSCAASGFTFSSYIMHWVRQAPGKGLEWVAVISYDGS
NEAYADSVKGRFTISRDNSKNTLYLQMSSLRAEDTGVYYCARETGDYSSSWYDSW
GRGTLVTVSS
SEQ ID NO: 30: (V_{L} S309) 
QLVLTQSPSASASLGASVKLTCTLSSGHSNYAIAWHQQQPEKGPRYLMKVNSDGSH
TKGDGIPDRFSGSSSGAERYLTISSLQSEDEADYYCQTWGTGIQVFGGGTKLTVL
SEQ ID NO: 31: (V_{H} C135) 
QVQLVESGGGVVQPGRSLRLSCAASGFTFSSYAMHWVRQAPGKGLEWVAVIPFDG
RNKYYADSVTGRFTISRDNSKNTLYLQMNSLRAEDTAVYYCASSSGYLFHSDYWGQ
GTLVTVSS
SEQ ID NO: 32: (V_{L} C135) 
DIQMTQSPSTLSASVGDRVTITCRASQSISNWLAWFQQKPGKAPKLLIYEASSLESGV
PSRFSGSGSGTEFTLTISSLQPDDFATYYCQQYNSYPWTFGQGTKVEIK
SEQ ID NO: 33: (V_{H} 4A8) 
EVQLVESGAEVKKPGASVKVSCKVSGYTLTELSMHWVRQAPGKGLEWMGGFDPED
GETMYAQKFQGRVTMTEDTSTDTAYMELSSLRSEDTAVYYCATSTAVAGTPDLFDY
YYGMDVWGQGTTVTVSS
SEQ ID NO: 34: (V_{L} 4A8) 
EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIK
SEQ ID NO: 35: (PDGFRA Hinge) 
QLSLPSILPNENEKVVQLNSSFSLRCFGESEVSWQYPMSEEESSDVEIRNEENNSGLFV
TVLEVSSASAAHTGLYTCYYNHTQTEENELEGRHIYIYVPDPDVAFVPLGMTDYLVI
VEDDDSAIIPCRTTDPETPVTLHNSEGVVPASYDSRQGFNGTFTVGPYICEATVKGKK
FQTIPFNVYALKATSELDLEMEALKTVYKSGETIVVTCAVFNNEVVDLQWTYPGEV
KGKGITMLEEIKVPSIKLVYTLTVPEATVKDSGDYECAARQATREVKEMKKVTISVH
EKGFIEIKPTFSQLEAVNLHEVKHFVVEVRAYPPPRISWLKNNLTLIENLTEITTDVEKI
QEIRYRSKLKLIRAKEEDSGHYTIVAQNEDAVKSYTFELLTQVPSSILDLVDDHHGST
GGQTVRCTAEGTPLPDIEWMICKDIKKCNNETSWTILANNVSNIITEIHSRDRSTVEGR
VTFAKVEETIAVRCLAKNLLGAENRELKLVAPTLRSELTVA
SEQ ID NO: 36: (PDGFRB Hinge) 
LVVTPPGPELVLNVSSTFVLTCSGSAPVVWERMSQEPPQEMAKAQDGTFSSVLTLTN
LTGLDTGEYFCTHNDSRGLETDERKRLYIFVPDPTVGFLPNDAEELFIFLTEITEITIPC
RVTDPQLVVTLHEKKGDVALPVPYDHQRGFSGIFEDRSYICKTTIGDREVDSDAYYV
YRLQVSSINVSVNAVQTVVRQGENITLMCIVIGNEVVNFEWTYPRKESGRLVEPVTD
FLLDMPYHIRSILHIPSAELEDSGTYTCNVTESVNDHQDEKAINITVVESGYVRLLGEV
GTLQFAELHRSRTLQVVFEAYPPPTVLWFKDNRTLGDSSAGEIALSTRNVSETRYVSE
LTLVRVKVAEAGHYTMRAFHEDAEVQLSFQLQINVPVRVLELSESHPDSGEQTVRC
RGRGMPQPNIIWSACRDLKRCPRELPPTLLGNSSEEESQLETNVTYWEEEQEFEVVST
LRLQHVDRPLSVRCTLRNAVGQDTQEVIVVPHSLPFKV
SEQ ID NO: 37: (LAIR1 Hinge) 
QEEDLPRPSISAEPGTVIPLGSHVTFVCRGPVGVQTFRLERESRSTYNDTEDVSQASPS
ESEARFRIDSVSEGNAGPYRCIYYKPPKWSEQSDYLELLVKETSGGPDSPDTEPGSSA
GPTQRPSDNSHNEHAPASQGLKAEHLY
SEQ ID NO: 38: (V_{L} CR3014) 
DIQMTQSPSSLSASVGDRVTITCRASQSISSYLNWYQQKPGKAPKLLIYAASSLQSGV
PSRFSGSGSGTDFTLTISSLQPEDFATYYCQQSYSTPPTFGQGTKVEIK
SEQ ID NO: 39: (CD28 Hinge)
   IEVMYPPPYLDNEKSNGTIIHVKGKHLCPSPLFPGPSKP
SEQ ID NO: 40: (CD8a Hinge)
   KPTTTPAPRPPTPAPTIASQPLSLRPEACRPAAGGAVHTRGLDFACD
SEQ ID NO: 41: (IgG4 CH3 Hinge) 
ESKYGPPCPPCPGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSL
SLSLGK
SEQ ID NO: 42: (tLAIR1 Hinge)
   LLVKETSGGPDSPDTEPGSSAGPTQRPSDNSHNEHAPASQGLKAEHLY
SEQ ID NO: 43: (CR3104 antibody) 
DIQMTQSPSSLSASVGDRVTITCRASQSISSYLNWYQQKPGKAPKLLIYAASSLQSGV
PSRFSGSGSGTDFTLTISSLQPEDFATYYCQQSYSTPPTFGQGTKVEIKGGGGSGGGGS
GGGGSEVQLVESGGGLVQPGGSLRLSCAASGFTFSDHYMDWVRQAPGKGLEWVGR
TRNKANSYTTEYAASVKGRFTISRDDSKNSLYLQMNSLKTEDTAVYYCARGISPFYF
DYWGQGTLVTVSS
SEQ ID NO: 44: (S309 HCDR1) GFTFSSY
SEQ ID NO: 45: (S309 HCDR2) SYDGSN
SEQ ID NO: 46: (S309 HCDR3) ETGDYSSSWYDS
SEQ ID NO: 47: (S309 LCDR1) TLSSGHSNYAIA
SEQ ID NO: 48: (S309 LCDR2) VNSDGSHTKGD
SEQ ID NO: 49: (S309 LCDR2) QTWGTGIQV
SEQ ID NO: 50: (CR3022-IgGmut-28Z full RNA) 
AUGGCUCUGCCUGUGACAGCUCUGCUGCUGCCUCUGGCUCUGCUUCUGCAUGC
CGCCAGACCUGACAUCGUGAUGACACAGAGCCCUGACAGCCUGGCCGUGUCUC
UGGGAGAAAGAGCCACCAUCAACUGCAAGAGCAGCCAGAGCGUGCUGUACUCC
AGCAUCAACAAGAACUACCUGGCCUGGUAUCAGCAGAAGCCCGGCCAGCCUCC
UAAGCUGCUGAUCUACUGGGCCAGCACCAGAGAAAGCGGCGUGCCCGAUAGAU
UUUCUGGCAGCGGCUCUGGCACCGACUUCACCCUGACAAUUAGCUCCCUGCAG
 
GCCGAGGAUGUGGCCGUGUACUACUGCCAGCAGUACUACAGCACCCCUUACAC
CUUUGGCCAGGGCACCAAGGUGGAAAUCAAAGGCGGCGGAGGAUCUGGCGGA
GGUGGAAGUGGCGGAGGCGGAUCUGAAGUUCAGCUGGUGCAGAGCGGCACCG
AAGUGAAGAAGCCUGGCGAGAGCCUGAAGAUCUCCUGCAAAGGCUCCGGCUAC
GGCUUCAUCACCUACUGGAUCGGCUGGGUCCGACAGAUGCCUGGCAAAGGCCU
UGAGUGGAUGGGCAUCAUCUACCCCGGCGACAGCGAGACAAGAUACAGCCCUA
GCUUCCAGGGCCAAGUGACCAUCAGCGCCGACAAGAGCAUCAAUACCGCCUAC
CUGCAGUGGUCCAGCCUGAAGGCCUCUGACACCGCCAUCUACUAUUGUGCCGG
CGGAAGCGGCAUCAGCACCCCAAUGGAUGUUUGGGGCCAGGGAACCACCGUGA
CCGUUUCUUCUGACUACAAGGACGACGACGACAAGGAAUCUAAGUACGGCCCU
CCUUGUCCUCCAUGUCCUGCUCCACCUGUGGCCGGACCCUCCGUGUUCCUGUU
UCCUCCAAAGCCUAAGGACACCCUGAUGAUCAGCAGAACCCCUGAAGUGACCU
GCGUGGUGGUGGACGUUUCCCAAGAGGACCCUGAGGUGCAGUUCAAUUGGUA
CGUGGACGGCGUGGAAGUGCACAACGCCAAGACCAAGCCUAGAGAGGAACAGU
UCCAGAGCACCUACAGAGUGGUGUCCGUGCUGACCGUGCUGCACCAGGAUUGG
CUGAACGGCAAAGAGUACAAGUGCAAGGUGUCCAACAAGGGCCUGCCUAGCAG
CAUCGAGAAAACCAUCAGCAAGGCCAAGGGCCAGCCAAGAGAACCCCAGGUGU
ACACACUGCCUCCAAGCCAAGAGGAAAUGACCAAGAACCAGGUGUCCCUGACC
UGCCUGGUCAAGGGCUUCUACCCUUCCGAUAUCGCCGUGGAAUGGGAGAGCAA
UGGCCAGCCUGAGAACAACUACAAGACCACACCUCCUGUGCUGGACAGCGACG
GCUCAUUCUUCCUGUACAGCAGACUGACCGUGGACAAGAGCAGAUGGCAAGAG
GGCAACGUGUUCAGCUGCAGCGUGAUGCACGAGGCCCUGCACAACCACUACAC
CCAGAAGUCUCUGAGCCUGAGCCUGGGCAAGUUCUGGGUGCUCGUUGUUGUU
GGCGGCGUGCUGGCCUGUUACUCUCUGCUGGUUACCGUGGCCUUCAUCAUCUU
UUGGGUCCGAAGCAAGCGGAGCAGAGGCGGCCACAGCGACUACAUGAACAUGA
CCCCUAGACGGCCCGGACCAACCAGAAAGCACUACCAGCCUUACGCUCCUCCU
AGAGACUUCGCCGCCUACCGGUCCAGAGUGAAGUUCAGCAGAUCCGCCGAUGC
UCCCGCCUAUCAGCAGGGACAGAACCAGCUGUACAACGAGCUGAACCUGGGGA
GAAGAGAAGAGUACGACGUGCUGGACAAGCGGAGAGGCAGAGAUCCUGAGAU
GGGCGGCAAGCCCAGACGGAAGAAUCCUCAAGAGGGCCUGUAUAAUGAGCUGC
AGAAAGACAAGAUGGCCGAGGCCUACAGCGAGAUCGGAAUGAAGGGCGAGCG
CAGAAGAGGCAAGGGACACGAUGGACUGUACCAGGGCCUGAGCACCGCCACCA
AGGAUACCUAUGAUGCCCUGCACAUGCAGGCCCUGCCUCCAAGAUAA
SEQ ID NO: 51: (Flag-28Z DNA) 
ATGGCTCTGCCTGTGACAGCTCTGCTGCTGCCTCTGGCTCTGCTTCTGCATGCCGC
CAGACCTGACTACAAGGACGACGACGACAAGATCGAAGTGATGTACCCTCCTCC
TTACCTGGACAACGAGAAGTCCAACGGCACCATCATCCACGTGAAGGGCAAGCA
CCTGTGTCCTTCTCCACTGTTCCCCGGACCTAGCAAGCCTTTCTGGGTGCTCGTTG
TTGTTGGCGGCGTGCTGGCCTGTTACTCTCTGCTGGTTACCGTGGCCTTCATCATC
TTTTGGGTCCGAAGCAAGCGGAGCAGAGGCGGCCACAGCGACTACATGAACATG
ACCCCTAGACGGCCCGGACCAACCAGAAAGCACTACCAGCCTTACGCTCCTCCT
AGAGACTTCGCCGCCTACCGGTCCAGAGTGAAGTTCAGCAGATCCGCCGATGCT
CCCGCCTATCAGCAGGGACAGAACCAGCTGTACAACGAGCTGAACCTGGGGAGA
AGAGAAGAGTACGACGTGCTGGACAAGCGGAGAGGCAGAGATCCTGAGATGGG
CGGCAAGCCCAGACGGAAGAATCCTCAAGAGGGCCTGTATAATGAGCTGCAGAA
AGACAAGATGGCCGAGGCCTACAGCGAGATCGGAATGAAGGGCGAGCGCAGAA
GAGGCAAGGGACACGATGGACTGTACCAGGGCCTGAGCACCGCCACCAAGGAT
ACCTATGATGCCCTGCACATGCAGGCCCTGCCTCCAAGATAA
SEQ ID NO: 52: (Flag-28Z RNA) 
AUGGCUCUGCCUGUGACAGCUCUGCUGCUGCCUCUGGCUCUGCUUCUGCAUGC
CGCCAGACCUGACUACAAGGACGACGACGACAAGAUCGAAGUGAUGUACCCUC
CUCCUUACCUGGACAACGAGAAGUCCAACGGCACCAUCAUCCACGUGAAGGGC
AAGCACCUGUGUCCUUCUCCACUGUUCCCCGGACCUAGCAAGCCUUUCUGGGU
GCUCGUUGUUGUUGGCGGCGUGCUGGCCUGUUACUCUCUGCUGGUUACCGUGG
CCUUCAUCAUCUUUUGGGUCCGAAGCAAGCGGAGCAGAGGCGGCCACAGCGAC
UACAUGAACAUGACCCCUAGACGGCCCGGACCAACCAGAAAGCACUACCAGCC
UUACGCUCCUCCUAGAGACUUCGCCGCCUACCGGUCCAGAGUGAAGUUCAGCA
GAUCCGCCGAUGCUCCCGCCUAUCAGCAGGGACAGAACCAGCUGUACAACGAG
CUGAACCUGGGGAGAAGAGAAGAGUACGACGUGCUGGACAAGCGGAGAGGCA
GAGAUCCUGAGAUGGGCGGCAAGCCCAGACGGAAGAAUCCUCAAGAGGGCCUG
UAUAAUGAGCUGCAGAAAGACAAGAUGGCCGAGGCCUACAGCGAGAUCGGAA
UGAAGGGCGAGCGCAGAAGAGGCAAGGGACACGAUGGACUGUACCAGGGCCU
GAGCACCGCCACCAAGGAUACCUAUGAUGCCCUGCACAUGCAGGCCCUGCCUC
CAAGAUAA
SEQ ID NO: 53: (CR3014-28Z DNA) 
ATGGCTCTGCCTGTGACAGCTCTGCTGCTGCCTCTGGCTCTGCTTCTGCATGCCGC
CAGACCTGACATCCAGATGACACAGAGCCCTAGCAGCCTGTCTGCCAGCGTGGG
AGACAGAGTGACCATCACCTGTAGAGCCAGCCAGAGCATCAGCTCCTACCTGAA
CTGGTATCAGCAGAAGCCCGGCAAGGCCCCTAAGCTGCTGATCTATGCTGCCAG
CTCTCTGCAGTCTGGCGTGCCCAGCAGATTTTCTGGCAGCGGCTCTGGCACCGAC
TTCACCCTGACCATATCTAGCCTGCAGCCTGAGGACTTCGCCACCTACTACTGCC
AGCAGTCCTACAGCACCCCTCCTACATTTGGCCAGGGCACCAAGGTGGAAATCA
AAGGCGGCGGAGGATCTGGCGGAGGTGGAAGTGGCGGAGGCGGATCTGAAGTG
CAGCTGGTTGAATCAGGTGGCGGCCTGGTTCAACCTGGCGGATCTCTGAGACTGA
GCTGTGCCGCCAGCGGCTTCACCTTCAGCGACCACTATATGGACTGGGTCCGACA
GGCCCCTGGCAAAGGACTTGAGTGGGTCGGACGGACCAGAAACAAGGCCAACA
GCTACACCACAGAGTACGCCGCCTCTGTGAAGGGCAGATTCACCATCAGCCGGG
ACGACAGCAAGAACAGCCTGTACCTGCAGATGAACTCCCTGAAAACCGAGGACA
CCGCCGTGTACTACTGCGCCAGAGGAATCAGCCCCTTCTACTTCGACTATTGGGG
CCAGGGAACCCTCGTGACCGTTTCTTCTGACTACAAGGACGACGACGACAAGAT
CGAAGTGATGTACCCTCCTCCTTACCTGGACAACGAGAAGTCCAACGGCACCATC
ATCCACGTGAAGGGCAAGCACCTGTGTCCTTCTCCACTGTTCCCCGGACCTAGCA
AGCCTTTCTGGGTGCTCGTTGTTGTTGGCGGCGTGCTGGCCTGTTACTCTCTGCTG
GTTACCGTGGCCTTCATCATCTTTTGGGTCCGAAGCAAGCGGAGCAGAGGCGGCC
ACAGCGACTACATGAACATGACCCCTAGACGGCCCGGACCAACCAGAAAGCACT
ACCAGCCTTACGCTCCTCCTAGAGACTTCGCCGCCTACCGGTCCAGAGTGAAGTT
CAGCAGATCCGCCGATGCTCCCGCCTATCAGCAGGGACAGAACCAGCTGTACAA
CGAGCTGAACCTGGGGAGAAGAGAAGAGTACGACGTGCTGGACAAGCGGAGAG
GCAGAGATCCTGAGATGGGCGGCAAGCCCAGACGGAAGAATCCTCAAGAGGGC
CTGTATAATGAGCTGCAGAAAGACAAGATGGCCGAGGCCTACAGCGAGATCGGA
ATGAAGGGCGAGCGCAGAAGAGGCAAGGGACACGATGGACTGTACCAGGGCCT
GAGCACCGCCACCAAGGATACCTATGATGCCCTGCACATGCAGGCCCTGCCTCC
AAGATAA
SEQ ID NO: 54: (CR3014-28Z RNA) 
AUGGCUCUGCCUGUGACAGCUCUGCUGCUGCCUCUGGCUCUGCUUCUGCAUGC
CGCCAGACCUGACAUCCAGAUGACACAGAGCCCUAGCAGCCUGUCUGCCAGCG
 
UGGGAGACAGAGUGACCAUCACCUGUAGAGCCAGCCAGAGCAUCAGCUCCUAC
CUGAACUGGUAUCAGCAGAAGCCCGGCAAGGCCCCUAAGCUGCUGAUCUAUGC
UGCCAGCUCUCUGCAGUCUGGCGUGCCCAGCAGAUUUCUGGCAGCGGCUCUG
GCACCGACUUCACCCUGACCAUAUCUAGCCUGCAGCCUGAGGACUUCGCCACC
UACUACUGCCAGCAGUCCUACAGCACCCCUCCUACAUUUGGCCAGGGCACCAA
GGUGGAAAUCAAAGGCGGCGGAGGAUCUGGCGGAGGUGGAAGUGGCGGAGGC
GGAUCUGAAGUGCAGCUGGUUGAAUCAGGUGGCGGCCUGGUUCAACCUGGCG
GAUCUCUGAGACUGAGCUGUGCCGCCAGCGGCUUCACCUUCAGCGACCACUAU
AUGGACUGGGUCCGACAGGCCCCUGGCAAAGGACUUGAGUGGGUCGGACGGAC
CAGAAACAAGGCCAACAGCUACACCACAGAGUACGCCGCCUCUGUGAAGGGCA
GAUUCACCAUCAGCCGGGACGACAGCAAGAACAGCCUGUACCUGCAGAUGAAC
UCCCUGAAAACCGAGGACACCGCCGUGUACUACUGCGCCAGAGGAAUCAGCCC
CUUCUACUUCGACUAUUGGGGCCAGGGAACCCUCGUGACCGUUUCUUCUGACU
ACAAGGACGACGACGACAAGAUCGAAGUGAUGUACCCUCCUCCUUACCUGGAC
AACGAGAAGUCCAACGGCACCAUCAUCCACGUGAAGGGCAAGCACCUGUGUCC
UUCUCCACUGUUCCCCGGACCUAGCAAGCCUUUCUGGGUGCUCGUUGUUGUUG
GCGGCGUGCUGGCCUGUUACUCUCUGCUGGUUACCGUGGCCUUCAUCAUCUUU
UGGGUCCGAAGCAAGCGGAGCAGAGGCGGCCACAGCGACUACAUGAACAUGAC
CCCUAGACGGCCCGGACCAACCAGAAAGCACUACCAGCCUUACGCUCCUCCUA
GAGACUUCGCCGCCUACCGGUCCAGAGUGAAGUUCAGCAGAUCCGCCGAUGCU
CCCGCCUAUCAGCAGGGACAGAACCAGCUGUACAACGAGCUGAACCUGGGGAG
AAGAGAAGAGUACGACGUGCUGGACAAGCGGAGAGGCAGAGAUCCUGAGAUG
GGCGGCAAGCCCAGACGGAAGAAUCCUCAAGAGGGCCUGUAUAAUGAGCUGCA
GAAAGACAAGAUGGCCGAGGCCUACAGCGAGAUCGGAAUGAAGGGCGAGCGC
AGAAGAGGCAAGGGACACGAUGGACUGUACCAGGGCCUGAGCACCGCCACCAA
GGAUACCUAUGAUGCCCUGCACAUGCAGGCCCUGCCUCCAAGAUAA
SEQ ID NO: 55: (CR3022-28Z DNA) 
ATGGCTCTGCCTGTGACAGCTCTGCTGCTGCCTCTGGCTCTGCTTCTGCATGCCGC
CAGACCTGACATCGTGATGACACAGAGCCCTGACAGCCTGGCCGTGTCTCTGGG
AGAAAGAGCCACCATCAACTGCAAGAGCAGCCAGAGCGTGCTGTACTCCAGCAT
CAACAAGAACTACCTGGCCTGGTATCAGCAGAAGCCCGGCCAGCCTCCTAAGCT
GCTGATCTACTGGGCCAGCACCAGAGAAAGCGGCGTGCCCGATAGATTTTCTGG
 
CAGCGGCTCTGGCACCGACTTCACCCTGACAATTAGCTCCCTGCAGGCCGAGGAT
GTGGCCGTGTACTACTGCCAGCAGTACTACAGCACCCCTTACACCTTTGGCCAGG
GCACCAAGGTGGAAATCAAAGGCGGCGGAGGATCTGGCGGAGGTGGAAGTGGC
GGAGGCGGATCTGAAGTTCAGCTGGTGCAGAGCGGCACCGAAGTGAAGAAGCCT
GGCGAGAGCCTGAAGATCTCCTGCAAAGGCTCCGGCTACGGCTTCATCACCTACT
GGATCGGCTGGGTCCGACAGATGCCTGGCAAAGGCCTTGAGTGGATGGGCATCA
TCTACCCCGGCGACAGCGAGACAAGATACAGCCCTAGCTTCCAGGGCCAAGTGA
CCATCAGCGCCGACAAGAGCATCAATACCGCCTACCTGCAGTGGTCCAGCCTGA
AGGCCTCTGACACCGCCATCTACTATTGTGCCGGCGGAAGCGGCATCAGCACCCC
AATGGATGTTTGGGGCCAGGGAACCACCGTGACCGTTTCTTCTGACTACAAGGAC
GACGACGACAAGATCGAAGTGATGTACCCTCCTCCTTACCTGGACAACGAGAAG
TCCAACGGCACCATCATCCACGTGAAGGGCAAGCACCTGTGTCCTTCTCCACTGT
TCCCCGGACCTAGCAAGCCTTTCTGGGTGCTCGTTGTTGTTGGCGGCGTGCTGGC
CTGTTACTCTCTGCTGGTTACCGTGGCCTTCATCATCTTTTGGGTCCGAAGCAAGC
GGAGCAGAGGCGGCCACAGCGACTACATGAACATGACCCCTAGACGGCCCGGAC
CAACCAGAAAGCACTACCAGCCTTACGCTCCTCCTAGAGACTTCGCCGCCTACCG
GTCCAGAGTGAAGTTCAGCAGATCCGCCGATGCTCCCGCCTATCAGCAGGGACA
GAACCAGCTGTACAACGAGCTGAACCTGGGGAGAAGAGAAGAGTACGACGTGC
TGGACAAGCGGAGAGGCAGAGATCCTGAGATGGGCGGCAAGCCCAGACGGAAG
AATCCTCAAGAGGGCCTGTATAATGAGCTGCAGAAAGACAAGATGGCCGAGGCC
TACAGCGAGATCGGAATGAAGGGCGAGCGCAGAAGAGGCAAGGGACACGATGG
ACTGTACCAGGGCCTGAGCACCGCCACCAAGGATACCTATGATGCCCTGCACAT
GCAGGCCCTGCCTCCAAGATAA
SEQ ID NO: 56: (CR3022-28Z RNA) 
AUGGCUCUGCCUGUGACAGCUCUGCUGCUGCCUCUGGCUCUGCUUCUGCAUGC
CGCCAGACCUGACAUCGUGAUGACACAGAGCCCUGACAGCCUGGCCGUGUCUC
UGGGAGAAAGAGCCACCAUCAACUGCAAGAGCAGCCAGAGCGUGCUGUACUCC
AGCAUCAACAAGAACUACCUGGCCUGGUAUCAGCAGAAGCCCGGCCAGCCUCC
UAAGCUGCUGAUCUACUGGGCCAGCACCAGAGAAAGCGGCGUGCCCGAUAGAU
UUUCUGGCAGCGGCUCUGGCACCGACUUCACCCUGACAAUUAGCUCCCUGCAG
GCCGAGGAUGUGGCCGUGUACUACUGCCAGCAGUACUACAGCACCCCUUACAC
CUUUGGCCAGGGCACCAAGGUGGAAAUCAAAGGCGGCGGAGGAUCUGGCGGA
 
GGUGGAAGUGGCGGAGGCGGAUCUGAAGUUCAGCUGGUGCAGAGCGGCACCG
AAGUGAAGAAGCCUGGCGAGAGCCUGAAGAUCUCCUGCAAAGGCUCCGGCUAC
GGCUUCAUCACCUACUGGAUCGGCUGGGUCCGACAGAUGCCUGGCAAAGGCCU
UGAGUGGAUGGGCAUCAUCUACCCCGGCGACAGCGAGACAAGAUACAGCCCUA
GCUUCCAGGGCCAAGUGACCAUCAGCGCCGACAAGAGCAUCAAUACCGCCUAC
CUGCAGUGGUCCAGCCUGAAGGCCUCUGACACCGCCAUCUACUAUUGUGCCGG
CGGAAGCGGCAUCAGCACCCCAAUGGAUGUUUGGGGCCAGGGAACCACCGUGA
CCGUUUCUUCUGACUACAAGGACGACGACAAGAUCGAAGUGAUGUACCCU
CCUCCUUACCUGGACAACGAGAAGUCCAACGGCACCAUCAUCCACGUGAAGGG
CAAGCACCUGUGUCCUUCUCCACUGUUCCCCGGACCUAGCAAGCCUUUCUGGG
UGCUCGUUGUUGUUGGCGGCGUGCUGGCCUGUUACUCUCUGCUGGUUACCGUG
GCCUUCAUCAUCUUUUGGGUCCGAAGCAAGCGGAGCAGAGGCGGCCACAGCGA
CUACAUGAACAUGACCCCUAGACGGCCCGGACCAACCAGAAAGCACUACCAGC
CUUACGCUCCUCCUAGAGACUUCGCCGCCUACCGGUCCAGAGUGAAGUUCAGC
AGAUCCGCCGAUGCUCCCGCCUAUCAGCAGGGACAGAACCAGCUGUACAACGA
GCUGAACCUGGGGAGAAGAGAAGAGUACGACGUGCUGGACAAGCGGAGAGGC
AGAGAUCCUGAGAUGGGCGGCAAGCCCAGACGGAAGAAUCCUCAAGAGGGCCU
GUAUAAUGAGCUGCAGAAAGACAAGAUGGCCGAGGCCUACAGCGAGAUCGGA
AUGAAGGGCGAGCGCAGAAGAGGCAAGGGACACGAUGGACUGUACCAGGGCC
UGAGCACCGCCACCAAGGAUACCUAUGAUGCCCUGCACAUGCAGGCCCUGCCU
CCAAGAUAA
SEQ ID NO: 57: (CR3022-81-28Z DNA) 
ATGGCTCTGCCTGTGACAGCTCTGCTGCTGCCTCTGGCTCTGCTTCTGCATGCCGC
CAGACCTGACATCGTGATGACACAGAGCCCTGACAGCCTGGCCGTGTCTCTGGG
AGAAAGAGCCACCATCAACTGCAAGAGCAGCCAGAGCGTGCTGTACTCCAGCAT
CAACAAGAACTACCTGGCCTGGTATCAGCAGAAGCCCGGCCAGCCTCCTAAGCT
GCTGATCTACTGGGCCAGCACCAGAGAAAGCGGCGTGCCCGATAGATTTTCTGG
CAGCGGCTCTGGCACCGACTTCACCCTGACAATTAGCTCCCTGCAGGCCGAGGAT
GTGGCCGTGTACTACTGCCAGCAGTACTACAGCACCCCTTACACCTTTGGCCAGG
GCACCAAGGTGGAAATCAAAGGCGGCGGAGGATCTGGCGGAGGTGGAAGTGGC
GGAGGCGGATCTGAAGTTCAGCTGGTGCAGAGCGGCACCGAAGTGAAGAAGCCT
GGCGAGAGCCTGAAGATCTCCTGCAAAGGCTCCGGCTACGGCTTCATCACCTACT
 
GGATCGGCTGGGTCCGACAGATGCCTGGCAAAGGCCTTGAGTGGATGGGCATCA
TCTACCCCGGCGACAGCGAGACAAGATACAGCCCTAGCTTCCAGGGCCAAGTGA
CCATCAGCGCCGACAAGAGCATCAATACCGCCTACCTGCAGTGGTCCAGCCTGA
AGGCCTCTGACACCGCCATCTACTATTGTGCCGGCGGAAGCGGCATCAGCACCCC
AATGGATGTTTGGGGCCAGGGAACCACCGTGACCGTTTCTTCTGACTACAAGGAC
GACGACGACAAGAAGCCCACCACCACACCAGCTCCTAGACCTCCAACTCCTGCT
CCTACAATCGCCAGCCAGCCTCTGTCTCTGAGGCCAGAAGCTTGTAGACCTGCTG
CTGGCGGAGCCGTGCATACAAGAGGACTGGATTTCGCCTGCGACTTCTGGGTGCT
CGTTGTTGTTGGCGGCGTGCTGGCCTGTTACTCTCTGCTGGTTACCGTGGCCTTCA
TCATCTTTTGGGTCCGAAGCAAGCGGAGCAGAGGCGGCCACAGCGACTACATGA
ACATGACCCCTAGACGGCCCGGACCAACCAGAAAGCACTACCAGCCTTACGCTC
CTCCTAGAGACTTCGCCGCCTACCGGTCCAGAGTGAAGTTCAGCAGATCCGCCGA
TGCTCCCGCCTATCAGCAGGGACAGAACCAGCTGTACAACGAGCTGAACCTGGG
GAGAAGAGAAGAGTACGACGTGCTGGACAAGCGGAGAGGCAGAGATCCTGAGA
TGGGCGGCAAGCCCAGACGGAAGAATCCTCAAGAGGGCCTGTATAATGAGCTGC
AGAAAGACAAGATGGCCGAGGCCTACAGCGAGATCGGAATGAAGGGCGAGCGC
AGAAGAGGCAAGGGACACGATGGACTGTACCAGGGCCTGAGCACCGCCACCAA
GGATACCTATGATGCCCTGCACATGCAGGCCCTGCCTCCAAGATAA
SEQ ID NO: 58: (CR0322-8a-28Z RNA) 
AUGGCUCUGCCUGUGACAGCUCUGCUGCUGCCUCUGGCUCUGCUUCUGCAUGC
CGCCAGACCUGACAUCGUGAUGACACAGAGCCCUGACAGCCUGGCCGUGUCUC
UGGGAGAAAGAGCCACCAUCAACUGCAAGAGCAGCCAGAGCGUGCUGUACUCC
AGCAUCAACAAGAACUACCUGGCCUGGUAUCAGCAGAAGCCCGGCCAGCCUCC
UAAGCUGCUGAUCUACUGGGCCAGCACCAGAGAAAGCGGCGUGCCCGAUAGAU
UUUCUGGCAGCGGCUCUGGCACCGACUUCACCCUGACAAUUAGCUCCCUGCAG
GCCGAGGAUGUGGCCGUGUACUACUGCCAGCAGUACUACAGCACCCCUUACAC
CUUUGGCCAGGGCACCAAGGUGGAAAUCAAAGGCGGCGGAGGAUCUGGCGGA
GGUGGAAGUGGCGGAGGCGGAUCUGAAGUUCAGCUGGUGCAGAGCGGCACCG
AAGUGAAGAAGCCUGGCGAGAGCCUGAAGAUCUCCUGCAAAGGCUCCGGCUAC
GGCUUCAUCACCUACUGGAUCGGCUGGGUCCGACAGAUGCCUGGCAAAGGCCU
UGAGUGGAUGGGCAUCAUCUACCCCGGCGACAGCGAGACAAGAUACAGCCCUA
GCUUCCAGGGCCAAGUGACCAUCAGCGCCGACAAGAGCAUCAAUACCGCCUAC
 
CUGCAGUGGUCCAGCCUGAAGGCCUCUGACACCGCCAUCUACUAUUGUGCCGG
CGGAAGCGGCAUCAGCACCCCAAUGGAUGUUGGGGCCAGGGAACCACCGUGA
CCGUUUCUUCUGACUACAAGGACGACGACGACAAGAAGCCCACCACCACACCA
GCUCCUAGACCUCCAACUCCUGCUCCUACAAUCGCCAGCCAGCCUCUGUCUCU
GAGGCCAGAAGCUUGUAGACCUGCUGCUGGCGGAGCCGUGCAUACAAGAGGAC
UGGAUUUCGCCUGCGACUUCUGGGUGCUCGUUGUUGUUGGCGGCGUGCUGGCC
UGUUACUCUCUGCUGGUUACCGUGGCCUUCAUCAUCUUUGGGUCCGAAGCAA
GCGGAGCAGAGGCGGCCACAGCGACUACAUGAACAUGACCCCUAGACGGCCCG
GACCAACCAGAAAGCACUACCAGCCUUACGCUCCUCCUAGAGACUUCGCCGCC
UACCGGUCCAGAGUGAAGUUCAGCAGAUCCGCCGAUGCUCCCGCCUAUCAGCA
GGGACAGAACCAGCUGUACAACGAGCUGAACCUGGGGAGAAGAGAAGAGUAC
GACGUGCUGGACAAGCGGAGAGGCAGAGAUCCUGAGAUGGGCGGCAAGCCCA
GACGGAAGAAUCCUCAAGAGGGCCUGUAUAAUGAGCUGCAGAAAGACAAGAU
GGCCGAGGCCUACAGCGAGAUCGGAAUGAAGGGCGAGCGCAGAAGAGGCAAG
GGACACGAUGGACUGUACCAGGGCCUGAGCACCGCCACCAAGGAUACCUAUGA
UGCCCUGCACAUGCAGGCCCUGCCUCCAAGAUAA
SEQ ID NO: 59: (CR3022-CH-28Z DNA) 
ATGGCTCTGCCTGTGACAGCTCTGCTGCTGCCTCTGGCTCTGCTTCTGCATGCCGC
CAGACCTGACATCGTGATGACACAGAGCCCTGACAGCCTGGCCGTGTCTCTGGG
AGAAAGAGCCACCATCAACTGCAAGAGCAGCCAGAGCGTGCTGTACTCCAGCAT
CAACAAGAACTACCTGGCCTGGTATCAGCAGAAGCCCGGCCAGCCTCCTAAGCT
GCTGATCTACTGGGCCAGCACCAGAGAAAGCGGCGTGCCCGATAGATTTTCTGG
CAGCGGCTCTGGCACCGACTTCACCCTGACAATTAGCTCCCTGCAGGCCGAGGAT
GTGGCCGTGTACTACTGCCAGCAGTACTACAGCACCCCTTACACCTTTGGCCAGG
GCACCAAGGTGGAAATCAAAGGCGGCGGAGGATCTGGCGGAGGTGGAAGTGGC
GGAGGCGGATCTGAAGTTCAGCTGGTGCAGAGCGGCACCGAAGTGAAGAAGCCT
GGCGAGAGCCTGAAGATCTCCTGCAAAGGCTCCGGCTACGGCTTCATCACCTACT
GGATCGGCTGGGTCCGACAGATGCCTGGCAAAGGCCTTGAGTGGATGGGCATCA
TCTACCCCGGCGACAGCGAGACAAGATACAGCCCTAGCTTCCAGGGCCAAGTGA
CCATCAGCGCCGACAAGAGCATCAATACCGCCTACCTGCAGTGGTCCAGCCTGA
AGGCCTCTGACACCGCCATCTACTATTGTGCCGGCGGAAGCGGCATCAGCACCCC
AATGGATGTTTGGGGCCAGGGAACCACCGTGACCGTTTCTTCTGACTACAAGGAC
 
GACGACGACAAGGAATCTAAGTACGGCCCTCCTTGTCCTCCATGTCCTGGCCAGC
CAAGAGAACCCCAGGTGTACACACTGCCTCCAAGCCAAGAGGAAATGACCAAGA
ACCAGGTGTCCCTGACCTGCCTGGTCAAGGGCTTCTACCCTTCCGATATCGCCGT
GGAATGGGAGAGCAATGGCCAGCCTGAGAACAACTACAAGACCACACCTCCTGT
GCTGGACAGCGACGGCTCATTCTTCCTGTACAGCAGACTGACCGTGGACAAGAG
CAGATGGCAAGAGGGCAACGTGTTCAGCTGCAGCGTGATGCACGAGGCCCTGCA
CAACCACTACACCCAGAAGTCTCTGAGCCTGAGCCTGGGCAAGTTCTGGGTGCTC
GTTGTTGTTGGCGGCGTGCTGGCCTGTTACTCTCTGCTGGTTACCGTGGCCTTCAT
CATCTTTTGGGTCCGAAGCAAGCGGAGCAGAGGCGGCCACAGCGACTACATGAA
CATGACCCCTAGACGGCCCGGACCAACCAGAAAGCACTACCAGCCTTACGCTCC
TCCTAGAGACTTCGCCGCCTACCGGTCCAGAGTGAAGTTCAGCAGATCCGCCGAT
GCTCCCGCCTATCAGCAGGGACAGAACCAGCTGTACAACGAGCTGAACCTGGGG
AGAAGAGAAGAGTACGACGTGCTGGACAAGCGGAGAGGCAGAGATCCTGAGAT
GGGCGGCAAGCCCAGACGGAAGAATCCTCAAGAGGGCCTGTATAATGAGCTGCA
GAAAGACAAGATGGCCGAGGCCTACAGCGAGATCGGAATGAAGGGCGAGCGCA
GAAGAGGCAAGGGACACGATGGACTGTACCAGGGCCTGAGCACCGCCACCAAG
GATACCTATGATGCCCTGCACATGCAGGCCCTGCCTCCAAGATAA
SEQ ID NO: 60: (CR3022-CH-28Z RNA) 
AUGGCUCUGCCUGUGACAGCUCUGCUGCUGCCUCUGGCUCUGCUUCUGCAUGC
CGCCAGACCUGACAUCGUGAUGACACAGAGCCCUGACAGCCUGGCCGUGUCUC
UGGGAGAAAGAGCCACCAUCAACUGCAAGAGCAGCCAGAGCGUGCUGUACUCC
AGCAUCAACAAGAACUACCUGGCCUGGUAUCAGCAGAAGCCCGGCCAGCCUCC
UAAGCUGCUGAUCUACUGGGCCAGCACCAGAGAAAGCGGCGUGCCCGAUAGAU
UUUCUGGCAGCGGCUCUGGCACCGACUUCACCCUGACAAUUAGCUCCCUGCAG
GCCGAGGAUGUGGCCGUGUACUACUGCCAGCAGUACUACAGCACCCCUUACAC
CUUUGGCCAGGGCACCAAGGUGGAAAUCAAAGGCGGCGGAGGAUCUGGCGGA
GGUGGAAGUGGCGGAGGCGGAUCUGAAGUUCAGCUGGUGCAGAGCGGCACCG
AAGUGAAGAAGCCUGGCGAGAGCCUGAAGAUCUCCUGCAAAGGCUCCGGCUAC
GGCUUCAUCACCUACUGGAUCGGCUGGGUCCGACAGAUGCCUGGCAAAGGCCU
UGAGUGGAUGGGCAUCAUCUACCCCGGCGACAGCGAGACAAGAUACAGCCCUA
GCUUCCAGGGCCAAGUGACCAUCAGCGCCGACAAGAGCAUCAAUACCGCCUAC
CUGCAGUGGUCCAGCCUGAAGGCCUCUGACACCGCCAUCUACUAUUGUGCCGG
 
CGGAAGCGGCAUCAGCACCCCAAUGGAUGUUUGGGGCCAGGGAACCACCGUGA
CCGUUUCUUCUGACUACAAGGACGACGACGACAAGGAAUCUAAGUACGGCCCU
CCUUGUCCUCCAUGUCCUGGCCAGCCAAGAGAACCCCAGGUGUACACACUGCC
UCCAAGCCAAGAGGAAAUGACCAAGAACCAGGUGUCCCUGACCUGCCUGGUCA
AGGGCUUCUACCCUUCCGAUAUCGCCGUGGAAUGGGAGAGCAAUGGCCAGCCU
GAGAACAACUACAAGACCACACCUCCUGUGCUGGACAGCGACGGCUCAUUCUU
CCUGUACAGCAGACUGACCGUGGACAAGAGCAGAUGGCAAGAGGGCAACGUG
UUCAGCUGCAGCGUGAUGCACGAGGCCCUGCACAACCACUACACCCAGAAGUC
UCUGAGCCUGAGCCUGGGCAAGUUCUGGGUGCUCGUUGUUGUUGGCGGCGUG
CUGGCCUGUUACUCUCUGCUGGUUACCGUGGCCUUCAUCAUCUUUUGGGUCCG
AAGCAAGCGGAGCAGAGGCGGCCACAGCGACUACAUGAACAUGACCCCUAGAC
GGCCCGGACCAACCAGAAAGCACUACCAGCCUUACGCUCCUCCUAGAGACUUC
GCCGCCUACCGGUCCAGAGUGAAGUUCAGCAGAUCCGCCGAUGCUCCCGCCUA
UCAGCAGGGACAGAACCAGCUGUACAACGAGCUGAACCUGGGGAGAAGAGAA
GAGUACGACGUGCUGGACAAGCGGAGAGGCAGAGAUCCUGAGAUGGGCGGCA
AGCCCAGACGGAAGAAUCCUCAAGAGGGCCUGUAUAAUGAGCUGCAGAAAGA
CAAGAUGGCCGAGGCCUACAGCGAGAUCGGAAUGAAGGGCGAGCGCAGAAGA
GGCAAGGGACACGAUGGACUGUACCAGGGCCUGAGCACCGCCACCAAGGAUAC
CUAUGAUGCCCUGCACAUGCAGGCCCUGCCUCCAAGAUAA
SEQ ID NO: 61: (CR3022-IgG4-28Z DNA) 
ATGGCTCTGCCTGTGACAGCTCTGCTGCTGCCTCTGGCTCTGCTTCTGCATGCCGC
CAGACCTGACATCGTGATGACACAGAGCCCTGACAGCCTGGCCGTGTCTCTGGG
AGAAAGAGCCACCATCAACTGCAAGAGCAGCCAGAGCGTGCTGTACTCCAGCAT
CAACAAGAACTACCTGGCCTGGTATCAGCAGAAGCCCGGCCAGCCTCCTAAGCT
GCTGATCTACTGGGCCAGCACCAGAGAAAGCGGCGTGCCCGATAGATTTTCTGG
CAGCGGCTCTGGCACCGACTTCACCCTGACAATTAGCTCCCTGCAGGCCGAGGAT
GTGGCCGTGTACTACTGCCAGCAGTACTACAGCACCCCTTACACCTTTGGCCAGG
GCACCAAGGTGGAAATCAAAGGCGGCGGAGGATCTGGCGGAGGTGGAAGTGGC
GGAGGCGGATCTGAAGTTCAGCTGGTGCAGAGCGGCACCGAAGTGAAGAAGCCT
GGCGAGAGCCTGAAGATCTCCTGCAAAGGCTCCGGCTACGGCTTCATCACCTACT
GGATCGGCTGGGTCCGACAGATGCCTGGCAAAGGCCTTGAGTGGATGGGCATCA
TCTACCCCGGCGACAGCGAGACAAGATACAGCCCTAGCTTCCAGGGCCAAGTGA
 
CCATCAGCGCCGACAAGAGCATCAATACCGCCTACCTGCAGTGGTCCAGCCTGA
AGGCCTCTGACACCGCCATCTACTATTGTGCCGGCGGAAGCGGCATCAGCACCCC
AATGGATGTTTGGGGCCAGGGAACCACCGTGACCGTTTCTTCTGACTACAAGGAC
GACGACGACAAGGAATCTAAGTACGGCCCTCCTTGTCCTCCATGTCCTGCTCCAG
AGTTTCTCGGCGGACCCTCCGTGTTCCTGTTTCCTCCAAAGCCTAAGGACACCCT
GATGATCAGCAGAACCCCTGAAGTGACCTGCGTGGTGGTGGACGTTTCCCAAGA
GGACCCTGAGGTGCAGTTCAATTGGTACGTGGACGGCGTGGAAGTGCACAACGC
CAAGACCAAGCCTAGAGAGGAACAGTTCAACAGCACCTACAGAGTGGTGTCCGT
GCTGACCGTGCTGCACCAGGATTGGCTGAACGGCAAAGAGTACAAGTGCAAGGT
GTCCAACAAGGGCCTGCCTAGCAGCATCGAGAAAACCATCAGCAAGGCCAAGGG
CCAGCCAAGAGAACCCCAGGTGTACACACTGCCTCCAAGCCAAGAGGAAATGAC
CAAGAACCAGGTGTCCCTGACCTGCCTGGTCAAGGGCTTCTACCCTTCCGATATC
GCCGTGGAATGGGAGAGCAATGGCCAGCCTGAGAACAACTACAAGACCACACCT
CCTGTGCTGGACAGCGACGGCTCATTCTTCCTGTACAGCAGACTGACCGTGGACA
AGAGCAGATGGCAAGAGGGCAACGTGTTCAGCTGCAGCGTGATGCACGAGGCCC
TGCACAACCACTACACCCAGAAGTCTCTGAGCCTGAGCCTGGGCAAGTTCTGGGT
GCTCGTTGTTGTTGGCGGCGTGCTGGCCTGTTACTCTCTGCTGGTTACCGTGGCCT
TCATCATCTTTTGGGTCCGAAGCAAGCGGAGCAGAGGCGGCCACAGCGACTACA
TGAACATGACCCCTAGACGGCCCGGACCAACCAGAAAGCACTACCAGCCTTACG
CTCCTCCTAGAGACTTCGCCGCCTACCGGTCCAGAGTGAAGTTCAGCAGATCCGC
CGATGCTCCCGCCTATCAGCAGGGACAGAACCAGCTGTACAACGAGCTGAACCT
GGGGAGAAGAGAAGAGTACGACGTGCTGGACAAGCGGAGAGGCAGAGATCCTG
AGATGGGCGGCAAGCCCAGACGGAAGAATCCTCAAGAGGGCCTGTATAATGAGC
TGCAGAAAGACAAGATGGCCGAGGCCTACAGCGAGATCGGAATGAAGGGCGAG
CGCAGAAGAGGCAAGGGACACGATGGACTGTACCAGGGCCTGAGCACCGCCAC
CAAGGATACCTATGATGCCCTGCACATGCAGGCCCTGCCTCCAAGATAA
SEQ ID NO: 62: (CR3022-IgG4-28Z RNA) 
AUGGCUCUGCCUGUGACAGCUCUGCUGCUGCCUCUGGCUCUGCUUCUGCAUGC
CGCCAGACCUGACAUCGUGAUGACACAGAGCCCUGACAGCCUGGCCGUGUCUC
UGGGAGAAAGAGCCACCAUCAACUGCAAGAGCAGCCAGAGCGUGCUGUACUCC
AGCAUCAACAAGAACUACCUGGCCUGGUAUCAGCAGAAGCCCGGCCAGCCUCC
UAAGCUGCUGAUCUACUGGGCCAGCACCAGAGAAAGCGGCGUGCCCGAUAGAU
 
UUUCUGGCAGCGGCUCUGGCACCGACUUCACCCUGACAAUUAGCUCCCUGCAG
GCCGAGGAUGUGGCCGUGUACUACUGCCAGCAGUACUACAGCACCCCUUACAC
CUUUGGCCAGGGCACCAAGGUGGAAAUCAAAGGCGGCGGAGGAUCUGGCGGA
GGUGGAAGUGGCGGAGGCGGAUCUGAAGUUCAGCUGGUGCAGAGCGGCACCG
AAGUGAAGAAGCCUGGCGAGAGCCUGAAGAUCUCCUGCAAAGGCUCCGGCUAC
GGCUUCAUCACCUACUGGAUCGGCUGGGUCCGACAGAUGCCUGGCAAAGGCCU
UGAGUGGAUGGGCAUCAUCUACCCCGGCGACAGCGAGACAAGAUACAGCCCUA
GCUUCCAGGGCCAAGUGACCAUCAGCGCCGACAAGAGCAUCAAUACCGCCUAC
CUGCAGUGGUCCAGCCUGAAGGCCUCUGACACCGCCAUCUACUAUUGUGCCGG
CGGAAGCGGCAUCAGCACCCCAAUGGAUGUUGGGCCAGGGAACCACCGUGA
CCGUUUCUUCUGACUACAAGGACGACGACGACAAGGAAUCUAAGUACGGCCCU
CCUUGUCCUCCAUGUCCUGCUCCAGAGUUUCUCGGCGGACCCUCCGUGUUCCU
GUUUCCUCCAAAGCCUAAGGACACCCUGAUGAUCAGCAGAACCCCUGAAGUGA
CCUGCGUGGUGGUGGACGUUUCCCAAGAGGACCCUGAGGUGCAGUUCAAUUG
GUACGUGGACGGCGUGGAAGUGCACAACGCCAAGACCAAGCCUAGAGAGGAAC
AGUUCAACAGCACCUACAGAGUGGUGUCCGUGCUGACCGUGCUGCACCAGGAU
UGGCUGAACGGCAAAGAGUACAAGUGCAAGGUGUCCAACAAGGGCCUGCCUA
GCAGCAUCGAGAAAACCAUCAGCAAGGCCAAGGGCCAGCCAAGAGAACCCCAG
GUGUACACACUGCCUCCAAGCCAAGAGGAAAUGACCAAGAACCAGGUGUCCCU
GACCUGCCUGGUCAAGGGCUUCUACCCUUCCGAUAUCGCCGUGGAAUGGGAGA
GCAAUGGCCAGCCUGAGAACAACUACAAGACCACACCUCCUGUGCUGGACAGC
GACGGCUCAUUCUUCCUGUACAGCAGACUGACCGUGGACAAGAGCAGAUGGCA
AGAGGGCAACGUGUUCAGCUGCAGCGUGAUGCACGAGGCCCUGCACAACCACU
ACACCCAGAAGUCUCUGAGCCUGAGCCUGGGCAAGUUCUGGGUGCUCGUUGUU
GUUGGCGGCGUGCUGGCCUGUUACUCUCUGCUGGUUACCGUGGCCUUCAUCAU
CUUUUGGGUCCGAAGCAAGCGGAGCAGAGGCGGCCACAGCGACUACAUGAACA
UGACCCCUAGACGGCCCGGACCAACCAGAAAGCACUACCAGCCUUACGCUCCU
CCUAGAGACUUCGCCGCCUACCGGUCCAGAGUGAAGUUCAGCAGAUCCGCCGA
UGCUCCCGCCUAUCAGCAGGGACAGAACCAGCUGUACAACGAGCUGAACCUGG
GGAGAAGAGAAGAGUACGACGUGCUGGACAAGCGGAGAGGCAGAGAUCCUGA
GAUGGGCGGCAAGCCCAGACGGAAGAAUCCUCAAGAGGGCCUGUAUAAUGAG
CUGCAGAAAGACAAGAUGGCCGAGGCCUACAGCGAGAUCGGAAUGAAGGGCG
AGCGCAGAAGAGGCAAGGGACACGAUGGACUGUACCAGGGCCUGAGCACCGCC
ACCAAGGAUACCUAUGAUGCCCUGCACAUGCAGGCCCUGCCUCCAAGAUAA
SEQ ID NO: 63: (C135-IgG4mut-28Z DNA) 
ATGGCTCTGCCTGTTACAGCTCTGCTGCTGCCTCTGGCTCTGCTTCTGCATGCTGC
TAGACCTCAGGTGCAGCTGGTGGAATCTGGTGGCGGAGTTGTGCAGCCTGGCAG
AAGCCTGAGACTGTCTTGTGCCGCCAGCGGCTTCACCTTTAGCAGCTATGCCATG
CACTGGGTCCGACAGGCCCCTGGCAAAGGACTTGAATGGGTCGCCGTGATTCCCT
TCGACGGCCGGAACAAGTACTACGCCGATAGCGTGACCGGCAGATTCACCATCA
GCCGGGACAACAGCAAGAACACCCTGTACCTGCAGATGAACAGCCTGAGAGCCG
AGGACACCGCCGTGTACTACTGTGCCTCTAGCAGCGGCTACCTGTTCCACAGCGA
TTATTGGGGCCAGGGCACCCTGGTCACAGTTTCTAGCGGAGGCGGAGGAAGTGG
CGGCGGAGGATCTGGCGGTGGTGGTTCTGATATCCAGATGACACAGAGCCCCAG
CACACTGTCTGCCAGCGTGGGAGACAGAGTGACCATCACCTGTAGAGCCAGCCA
GAGCATCAGCAACTGGCTGGCCTGGTTCCAGCAGAAGCCAGGCAAAGCCCCTAA
GCTGCTGATCTACGAGGCCAGCTCTCTGGAAAGCGGCGTGCCAAGCAGATTTTCT
GGCAGCGGCTCTGGCACCGAGTTCACCCTGACCATCAGTAGCCTGCAGCCTGAC
GACTTCGCCACCTACTACTGCCAGCAGTACAACAGCTACCCCTGGACCTTTGGAC
AGGGCACCAAGGTGGAAATCAAGGACTACAAGGACGACGACGACAAGGAATCT
AAGTACGGCCCTCCTTGTCCTCCATGTCCTGCTCCACCTGTGGCCGGACCCTCCGT
GTTCCTGTTTCCTCCAAAGCCTAAGGACACCCTGATGATCAGCAGAACCCCTGAA
GTGACCTGCGTGGTGGTGGACGTTTCCCAAGAGGACCCTGAGGTGCAGTTCAATT
GGTACGTGGACGGCGTGGAAGTGCACAACGCCAAGACCAAGCCTAGAGAGGAA
CAGTTCCAGAGCACCTACAGAGTGGTGTCCGTGCTGACCGTGCTGCACCAGGATT
GGCTGAACGGCAAAGAGTACAAGTGCAAGGTGTCCAACAAGGGCCTGCCTAGCA
GCATCGAGAAAACCATCAGCAAGGCCAAGGGCCAGCCAAGAGAACCCCAGGTG
TACACACTGCCTCCAAGCCAAGAGGAAATGACCAAGAACCAGGTGTCCCTGACC
TGCCTGGTCAAGGGCTTCTACCCTTCCGATATCGCCGTGGAATGGGAGAGCAATG
GCCAGCCTGAGAACAACTACAAGACCACACCTCCTGTGCTGGACAGCGACGGCT
CATTCTTCCTGTACAGCAGACTGACCGTGGACAAGAGCAGATGGCAAGAGGGCA
ACGTGTTCAGCTGCAGCGTGATGCACGAGGCCCTGCACAACCACTACACCCAGA
AGTCTCTGAGCCTGAGCCTGGGCAAGTTCTGGGTGCTCGTTGTTGTTGGCGGCGT
GCTGGCCTGTTACTCTCTGCTGGTTACCGTGGCCTTCATCATCTTTTGGGTCCGAA
GCAAGCGGAGCAGAGGCGGCCACAGCGACTACATGAACATGACCCCTAGACGG
CCCGGACCAACCAGAAAGCACTACCAGCCTTACGCTCCTCCTAGAGACTTCGCCG
CCTACCGGTCCAGAGTGAAGTTCAGCAGATCCGCCGATGCTCCCGCCTATCAGCA
 
GGGACAGAACCAGCTGTACAACGAGCTGAACCTGGGGAGAAGAGAAGAGTACG
ACGTGCTGGACAAGCGGAGAGGCAGAGATCCTGAGATGGGCGGCAAGCCCAGA
CGGAAGAATCCTCAAGAGGGCCTGTATAATGAGCTGCAGAAAGACAAGATGGCC
GAGGCCTACAGCGAGATCGGAATGAAGGGCGAGCGCAGAAGAGGCAAGGACA
CGATGGACTGTACCAGGGCCTGAGCACCGCCACCAAGGATACCTATGATGCCCT
GCACATGCAGGCCCTGCCTCCAAGATAA
SEQ ID NO: 64: (C135-IgG4mut-28Z RNA) 
AUGGCUCUGCCUGUUACAGCUCUGCUGCUGCCUCUGGCUCUGCUUCUGCAUGC
UGCUAGACCUCAGGUGCAGCUGGUGGAAUCUGGUGGCGGAGUUGUGCAGCCU
GGCAGAAGCCUGAGACUGUCUUGUGCCGCCAGCGGCUUCACCUUUAGCAGCUA
UGCCAUGCACUGGGUCCGACAGGCCCCUGGCAAAGGACUUGAAUGGGUCGCCG
UGAUUCCCUUCGACGGCCGGAACAAGUACUACGCCGAUAGCGUGACCGGCAGA
UUCACCAUCAGCCGGGACAACAGCAAGAACACCCUGUACCUGCAGAUGAACAG
CCUGAGAGCCGAGGACACCGCCGUGUACUACUGUGCCUCUAGCAGCGGCUACC
UGUUCCACAGCGAUUAUUGGGGCCAGGGCACCCUGGUCACAGUUUCUAGCGGA
GGCGGAGGAAGUGGCGGCGGAGGAUCUGGCGGUGGUGGUUCUGAUAUCCAGA
UGACACAGAGCCCCAGCACACUGUCUGCCAGCGUGGGAGACAGAGUGACCAUC
ACCUGUAGAGCCAGCCAGAGCAUCAGCAACUGGCUGGCCUGGUUCCAGCAGAA
GCCAGGCAAAGCCCCUAAGCUGCUGAUCUACGAGGCCAGCUCUCUGGAAAGCG
GCGUGCCAAGCAGAUUUUCUGGCAGCGGCUCUGGCACCGAGUUCACCCUGACC
AUCAGUAGCCUGCAGCCUGACGACUUCGCCACCUACUACUGCCAGCAGUACAA
CAGCUACCCCUGGACCUUUGGACAGGGCACCAAGGUGGAAAUCAAGGACUACA
AGGACGACGACGACAAGGAAUCUAAGUACGGCCCUCCUUGUCCUCCAUGUCCU
GCUCCACCUGUGGCCGGACCCUCCGUGUUCCUGUUCCUCCAAAGCCUAAGGA
CACCCUGAUGAUCAGCAGAACCCCUGAAGUGACCUGCGUGGUGGUGGACGUUU
CCCAAGAGGACCCUGAGGUGCAGUUCAAUUGGUACGUGGACGGCGUGGAAGU
GCACAACGCCAAGACCAAGCCUAGAGAGGAACAGUUCCAGAGCACCUACAGAG
UGGUGUCCGUGCUGACCGUGCUGCACCAGGAUUGGCUGAACGGCAAAGAGUAC
AAGUGCAAGGUGUCCAACAAGGGCCUGCCUAGCAGCAUCGAGAAAACCAUCAG
CAAGGCCAAGGGCCAGCCAAGAGAACCCCAGGUGUACACACUGCCUCCAAGCC
AAGAGGAAAUGACCAAGAACCAGGUGUCCCUGACCUGCCUGGUCAAGGGCUUC
UACCCUUCCGAUAUCGCCGUGGAAUGGGAGAGCAAUGGCCAGCCUGAGAACAA
 
CUACAAGACCACACCUCCUGUGCUGGACAGCGACGGCUCAUUCUUCCUGUACA
GCAGACUGACCGUGGACAAGAGCAGAUGGCAAGAGGGCAACGUGUUCAGCUG
CAGCGUGAUGCACGAGGCCCUGCACAACCACUACACCCAGAAGUCUCUGAGCC
UGAGCCUGGGCAAGUUCUGGGUGCUCGUUGUUGUUGGCGGCGUGCUGGCCUG
UUACUCUCUGCUGGUUACCGUGGCCUUCAUCAUCUUUUGGGUCCGAAGCAAGC
GGAGCAGAGGCGGCCACAGCGACUACAUGAACAUGACCCCUAGACGGCCCGGA
CCAACCAGAAAGCACUACCAGCCUUACGCUCCUCCUAGAGACUUCGCCGCCUA
CCGGUCCAGAGUGAAGUUCAGCAGAUCCGCCGAUGCUCCCGCCUAUCAGCAGG
GACAGAACCAGCUGUACAACGAGCUGAACCUGGGGAGAAGAGAAGAGUACGA
CGUGCUGGACAAGCGGAGAGGCAGAGAUCCUGAGAUGGGCGGCAAGCCCAGAC
GGAAGAAUCCUCAAGAGGGCCUGUAUAAUGAGCUGCAGAAAGACAAGAUGGC
CGAGGCCUACAGCGAGAUCGGAAUGAAGGGCGAGCGCAGAAGAGGCAAGGGA
CACGAUGGACUGUACCAGGGCCUGAGCACCGCCACCAAGGAUACCUAUGAUGC
CCUGCACAUGCAGGCCCUGCCUCCAAGAUAA
SEQ ID NO: 65: (S309-IgGmut-28Z DNA) 
ATGGCTCTGCCTGTTACAGCTCTGCTGCTGCCTCTGGCTCTGCTTCTGCATGCTGC
TAGACCTCAGGTGCAGCTGGTTCAGTCTGGCGCCGAAGTGAAGAAACCTGGCGC
CTCTGTGAAGGTGTCCTGCAAGGCCAGCGGCTACCCTTTACCAGCTACGGCATC
AGCTGGGTCCGACAGGCTCCTGGACAAGGCTTGGAATGGATGGGCTGGATCAGC
ACCTACAACGGCAACACCAACTACGCCCAGAAATTCCAGGGCAGAGTGACCATG
ACCACCGACACCAGCACCACCACCGGCTACATGGAACTGCGGAGACTGAGAAGC
GACGACACCGCCGTGTACTACTGCGCCAGAGATTACACCAGAGGCGCTTGGTTT
GGCGAGTCTCTGATCGGCGGCTTCGACAATTGGGGCCAGGGAACACTGGTCACC
GTTTCTAGCGGAGGCGGAGGATCTGGTGGCGGAGGAAGTGGCGGAGGCGGTTCT
GAAATTGTGCTGACACAGAGCCCCGGCACACTGTCACTTTCTCCAGGCGAAAGA
GCCACACTGAGCTGTAGAGCCAGCCAGACCGTGTCTAGCACAAGCCTGGCTTGG
TATCAGCAGAAGCCTGGACAGGCCCCTCGGCTGTTGATCTACGGTGCTTCTAGCC
GCGCCACAGGCATCCCCGATAGATTTCTGGCAGCGGCTCCGGCACCGACTTCAC
CCTGACAATCAGCAGACTGGAACCCGAGGACTTCGCCGTGTATTATTGCCAGCA
GCACGATACCAGCCTGACCTTTGGCGGCGGAACAAAGGTGGAAATCAAGGACTA
CAAGGACGACGACGACAAGGAATCTAAGTACGGCCCTCCTTGTCCTCCATGTCCT
GCTCCACCTGTGGCCGGACCCTCCGTGTTCCTGTTTCCTCCAAAGCCTAAGGACA
 
CCCTGATGATCAGCAGAACCCCTGAAGTGACCTGCGTGGTGGTGGACGTTTCCCA
AGAGGACCCTGAGGTGCAGTTCAATTGGTACGTGGACGGCGTGGAAGTGCACAA
CGCCAAGACCAAGCCTAGAGAGGAACAGTTCCAGAGCACCTACAGAGTGGTGTC
CGTGCTGACCGTGCTGCACCAGGATTGGCTGAACGGCAAAGAGTACAAGTGCAA
GGTGTCCAACAAGGGCCTGCCTAGCAGCATCGAGAAAACCATCAGCAAGGCCAA
GGGCCAGCCAAGAGAACCCCAGGTGTACACACTGCCTCCAAGCCAAGAGGAAAT
GACCAAGAACCAGGTGTCCCTGACCTGCCTGGTCAAGGGCTTCTACCCTTCCGAT
ATCGCCGTGGAATGGGAGAGCAATGGCCAGCCTGAGAACAACTACAAGACCACA
CCTCCTGTGCTGGACAGCGACGGCTCATTCTTCCTGTACAGCAGACTGACCGTGG
ACAAGAGCAGATGGCAAGAGGGCAACGTGTTCAGCTGCAGCGTGATGCACGAG
GCCCTGCACAACCACTACACCCAGAAGTCTCTGAGCCTGAGCCTGGGCAAGTTCT
GGGTGCTCGTTGTTGTTGGCGGCGTGCTGGCCTGTTACTCTCTGCTGGTTACCGTG
GCCTTCATCATCTTTTGGGTCCGAAGCAAGCGGAGCAGAGGCGGCCACAGCGAC
TACATGAACATGACCCCTAGACGGCCCGGACCAACCAGAAAGCACTACCAGCCT
TACGCTCCTCCTAGAGACTTCGCCGCCTACCGGTCCAGAGTGAAGTTCAGCAGAT
CCGCCGATGCTCCCGCCTATCAGCAGGGACAGAACCAGCTGTACAACGAGCTGA
ACCTGGGGAGAAGAGAAGAGTACGACGTGCTGGACAAGCGGAGAGGCAGAGAT
CCTGAGATGGGCGGCAAGCCCAGACGGAAGAATCCTCAAGAGGGCCTGTATAAT
GAGCTGCAGAAAGACAAGATGGCCGAGGCCTACAGCGAGATCGGAATGAAGGG
CGAGCGCAGAAGAGGCAAGGGACACGATGGACTGTACCAGGGCCTGAGCACCG
CCACCAAGGATACCTATGATGCCCTGCACATGCAGGCCCTG
SEQ ID NO: 66: (S309-IgGmut-28Z RNA) 
AUGGCUCUGCCUGUUACAGCUCUGCUGCUGCCUCUGGCUCUGCUUCUGCAUGC
UGCUAGACCUCAGGUGCAGCUGGUUCAGUCUGGCGCCGAAGUGAAGAAACCUG
GCGCCUCUGUGAAGGUGUCCUGCAAGGCCAGCGGCUACCCUUUACCAGCUAC
GGCAUCAGCUGGGUCCGACAGGCUCCUGGACAAGGCUUGGAAUGGAUGGGCU
GGAUCAGCACCUACAACGGCAACACCAACUACGCCCAGAAAUUCCAGGGCAGA
GUGACCAUGACCACCGACACCAGCACCACCACCGGCUACAUGGAACUGCGGAG
ACUGAGAAGCGACGACACCGCCGUGUACUACUGCGCCAGAGAUUACACCAGAG
GCGCUUGGUUUGGCGAGUCUCUGAUCGGCGGCUUCGACAAUUGGGGCCAGGG
AACACUGGUCACCGUUUCUAGCGGAGGCGGAGGAUCUGGUGGCGGAGGAAGU
GGCGGAGGCGGUUCUGAAAUUGUGCUGACACAGAGCCCCGGCACACUGUCACU
 
UUCUCCAGGCGAAAGAGCCACACUGAGCUGUAGAGCCAGCCAGACCGUGUCUA
GCACAAGCCUGGCUUGGUAUCAGCAGAAGCCUGGACAGGCCCCUCGGCUGUUG
AUCUACGGUGCUUCUAGCCGCGCCACAGGCAUCCCCGAUAGAUUUCUGGCAG
CGGCUCCGGCACCGACUUCACCCUGACAAUCAGCAGACUGGAACCCGAGGACU
UCGCCGUGUAUUAUUGCCAGCAGCACGAUACCAGCCUGACCUUUGGCGGCGGA
ACAAAGGUGGAAAUCAAGGACUACAAGGACGACGACGACAAGGAAUCUAAGU
ACGGCCCUCCUUGUCCUCCAUGUCCUGCUCCACCUGUGGCCGGACCCUCCGUG
UUCCUGUUUCCUCCAAAGCCUAAGGACACCCUGAUGAUCAGCAGAACCCCUGA
AGUGACCUGCGUGGUGGUGGACGUUUCCCAAGAGGACCCUGAGGUGCAGUUC
AAUUGGUACGUGGACGGCGUGGAAGUGCACAACGCCAAGACCAAGCCUAGAG
AGGAACAGUUCCAGAGCACCUACAGAGUGGUGUCCGUGCUGACCGUGCUGCAC
CAGGAUUGGCUGAACGGCAAAGAGUACAAGUGCAAGGUGUCCAACAAGGGCC
UGCCUAGCAGCAUCGAGAAAACCAUCAGCAAGGCCAAGGGCCAGCCAAGAGAA
CCCCAGGUGUACACACUGCCUCCAAGCCAAGAGGAAAUGACCAAGAACCAGGU
GUCCCUGACCUGCCUGGUCAAGGGCUUCUACCCUUCCGAUAUCGCCGUGGAAU
GGGAGAGCAAUGGCCAGCCUGAGAACAACUACAAGACCACACCUCCUGUGCUG
GACAGCGACGGCUCAUUCUUCCUGUACAGCAGACUGACCGUGGACAAGAGCAG
AUGGCAAGAGGGCAACGUGUUCAGCUGCAGCGUGAUGCACGAGGCCCUGCACA
ACCACUACACCCAGAAGUCUCUGAGCCUGAGCCUGGGCAAGUUCUGGGUGCUC
GUUGUUGUUGGCGGCGUGCUGGCCUGUUACUCUCUGCUGGUUACCGUGGCCUU
CAUCAUCUUUUGGGUCCGAAGCAAGCGGAGCAGAGGCGGCCACAGCGACUACA
UGAACAUGACCCCUAGACGGCCCGGACCAACCAGAAAGCACUACCAGCCUUAC
GCUCCUCCUAGAGACUUCGCCGCCUACCGGUCCAGAGUGAAGUUCAGCAGAUC
CGCCGAUGCUCCCGCCUAUCAGCAGGGACAGAACCAGCUGUACAACGAGCUGA
ACCUGGGGAGAAGAGAAGAGUACGACGUGCUGGACAAGCGGAGAGGCAGAGA
UCCUGAGAUGGGCGGCAAGCCCAGACGGAAGAAUCCUCAAGAGGGCCUGUAUA
AUGAGCUGCAGAAAGACAAGAUGGCCGAGGCCUACAGCGAGAUCGGAAUGAA
GGGCGAGCGCAGAAGAGGCAAGGGACACGAUGGACUGUACCAGGGCCUGAGC
ACCGCCACCAAGGAUACCUAUGAUGCCCUGCACAUGCAGGCCCUG
SEQ ID NO: 67: (4A8-IgG4mut-28Z DNA) 
ATGGCTCTGCCTGTTACAGCTCTGCTGCTGCCTCTGGCTCTGCTTCTGCATGCCGC
TAGACCTGAGGTGCAGCTGGTTGAATCTGGCGCCGAAGTGAAGAAACCAGGCGC
 
CTCTGTGAAGGTGTCCTGCAAGGTGTCCGGCTACACCCTGACAGAGCTGAGCATG
CACTGGGTCCGACAGGCCCCTGGAAAAGGCCTTGAATGGATGGGCGGCTTCGAC
CCCGAAGATGGCGAGACTATGTACGCCCAGAAATTCCAGGGCAGAGTGACCATG
ACCGAGGACACCAGCACCGACACCGCCTACATGGAACTGAGCAGCCTGAGAAGC
GAGGATACCGCCGTGTACTACTGCGCCACATCTACAGCCGTGGCCGGCACACCT
GACCTGTTCGATTACTACTACGGCATGGACGTGTGGGGCCAGGGCACAACAGTG
ACAGTTTCTTCTGGCGGCGGAGGATCTGGCGGAGGTGGAAGCGGAGGCGGTGGA
TCTGAGATCGTGATGACACAGAGCCCTCTGTCTAGCCCCGTGACACTGGGACAGC
CTGCCAGCATCAGCTGTAGAAGCAGCCAGAGCCTGGTGCACAGCGACGGCAATA
CCTACCTGTCTTGGCTGCAGCAGAGGCCTGGACAGCCTCCTAGACTGCTGATCTA
CAAGATCAGCAACCGGTTCAGCGGCGTGCCCGATAGATTTTCTGGTAGCGGAGC
CGGCACCGACTTCACCCTGAAGATCAGTAGAGTGGAAGCCGAGGACGTGGGCGT
GTACTATTGCACACAGGCCACACAGTTCCCTTACACCTTCGGACAGGGCACCAAG
GTGGACATCAAGGACTACAAGGACGACGACGACAAGGAATCTAAGTACGGCCCT
CCTTGTCCTCCATGTCCTGCTCCACCTGTGGCCGGACCCTCCGTGTTCCTGTTTCC
TCCAAAGCCTAAGGACACCCTGATGATCAGCAGAACCCCTGAAGTGACCTGCGT
GGTGGTGGACGTTTCCCAAGAGGACCCTGAGGTGCAGTTCAATTGGTACGTGGA
CGGCGTGGAAGTGCACAACGCCAAGACCAAGCCTAGAGAGGAACAGTTCCAGA
GCACCTACAGAGTGGTGTCCGTGCTGACCGTGCTGCACCAGGATTGGCTGAACG
GCAAAGAGTACAAGTGCAAGGTGTCCAACAAGGGCCTGCCTAGCAGCATCGAGA
AAACCATCAGCAAGGCCAAGGGCCAGCCAAGAGAACCCCAGGTGTACACACTGC
CTCCAAGCCAAGAGGAAATGACCAAGAACCAGGTGTCCCTGACCTGCCTGGTCA
AGGGCTTCTACCCTTCCGATATCGCCGTGGAATGGGAGAGCAATGGCCAGCCTG
AGAACAACTACAAGACCACACCTCCTGTGCTGGACAGCGACGGCTCATTCTTCCT
GTACAGCAGACTGACCGTGGACAAGAGCAGATGGCAAGAGGGCAACGTGTTCA
GCTGCAGCGTGATGCACGAGGCCCTGCACAACCACTACACCCAGAAGTCTCTGA
GCCTGAGCCTGGGCAAGTTCTGGGTGCTCGTTGTTGTTGGCGGCGTGCTGGCCTG
TTACTCTCTGCTGGTTACCGTGGCCTTCATCATCTTTTGGGTCCGAAGCAAGCGG
AGCAGAGGCGGCCACAGCGACTACATGAACATGACCCCTAGACGGCCCGGACCA
ACCAGAAAGCACTACCAGCCTTACGCTCCTCCTAGAGACTTCGCCGCCTACCGGT
CCAGAGTGAAGTTCAGCAGATCCGCCGATGCTCCCGCCTATCAGCAGGGACAGA
ACCAGCTGTACAACGAGCTGAACCTGGGGAGAAGAGAAGAGTACGACGTGCTG
GACAAGCGGAGAGGCAGAGATCCTGAGATGGGCGGCAAGCCCAGACGGAAGAA
TCCTCAAGAGGGCCTGTATAATGAGCTGCAGAAAGACAAGATGGCCGAGGCCTA
 
CAGCGAGATCGGAATGAAGGGCGAGCGCAGAAGAGGCAAGGACACGATGGAC
TGTACCAGGGCCTGAGCACCGCCACCAAGGATACCTATGATGCCCTGCACATGC
AGGCCCTGCCTCCAAGATAA
SEQ ID NO: 68: (4A8-IgG4mut-28Z RNA) 
AUGGCUCUGCCUGUUACAGCUCUGCUGCUGCCUCUGGCUCUGCUUCUGCAUGC
CGCUAGACCUGAGGUGCAGCUGGUUGAAUCUGGCGCCGAAGUGAAGAAACCA
GGCGCCUCUGUGAAGGUGUCCUGCAAGGUGUCCGGCUACACCCUGACAGAGCU
GAGCAUGCACUGGGUCCGACAGGCCCCUGGAAAAGGCCUUGAAUGGAUGGGCG
GCUUCGACCCCGAAGAUGGCGAGACUAUGUACGCCCAGAAAUUCCAGGGCAGA
GUGACCAUGACCGAGGACACCAGCACCGACACCGCCUACAUGGAACUGAGCAG
CCUGAGAAGCGAGGAUACCGCCGUGUACUACUGCGCCACAUCUACAGCCGUGG
CCGGCACACCUGACCUGUUCGAUUACUACUACGGCAUGGACGUGUGGGGCCAG
GGCACAACAGUGACAGUUUCUUCUGGCGGCGGAGGAUCUGGCGGAGGUGGAA
GCGGAGGCGGUGGAUCUGAGAUCGUGAUGACACAGAGCCCUCUGUCUAGCCCC
GUGACACUGGGACAGCCUGCCAGCAUCAGCUGUAGAAGCAGCCAGAGCCUGGU
GCACAGCGACGGCAAUACCUACCUGUCUUGGCUGCAGCAGAGGCCUGGACAGC
CUCCUAGACUGCUGAUCUACAAGAUCAGCAACCGGUUCAGCGGCGUGCCCGAU
AGAUUUUCUGGUAGCGGAGCCGGCACCGACUUCACCCUGAAGAUCAGUAGAGU
GGAAGCCGAGGACGUGGGCGUGUACUAUUGCACACAGGCCACACAGUUCCCUU
ACACCUUCGGACAGGGCACCAAGGUGGACAUCAAGGACUACAAGGACGACGAC
GACAAGGAAUCUAAGUACGGCCCUCCUUGUCCUCCAUGUCCUGCUCCACCUGU
GGCCGGACCCUCCGUGUUCCUGUUCCUCCAAAGCCUAAGGACACCCUGAUGA
UCAGCAGAACCCCUGAAGUGACCUGCGUGGUGGUGGACGUUUCCCAAGAGGAC
CCUGAGGUGCAGUUCAAUUGGUACGUGGACGGCGUGGAAGUGCACAACGCCA
AGACCAAGCCUAGAGAGGAACAGUUCCAGAGCACCUACAGAGUGGUGUCCGUG
CUGACCGUGCUGCACCAGGAUUGGCUGAACGGCAAAGAGUACAAGUGCAAGG
UGUCCAACAAGGGCCUGCCUAGCAGCAUCGAGAAAACCAUCAGCAAGGCCAAG
GGCCAGCCAAGAGAACCCCAGGUGUACACACUGCCUCCAAGCCAAGAGGAAAU
GACCAAGAACCAGGUGUCCCUGACCUGCCUGGUCAAGGGCUUCUACCCUUCCG
AUAUCGCCGUGGAAUGGGAGAGCAAUGGCCAGCCUGAGAACAACUACAAGACC
ACACCUCCUGUGCUGGACAGCGACGGCUCAUUCUUCCUGUACAGCAGACUGAC
CGUGGACAAGAGCAGAUGGCAAGAGGGCAACGUGUUCAGCUGCAGCGUGAUG
 
CACGAGGCCCUGCACAACCACUACACCCAGAAGUCUCUGAGCCUGAGCCUGGG
CAAGUUCUGGGUGCUCGUUGUUGUUGGCGGCGUGCUGGCCUGUUACUCUCUGC
UGGUUACCGUGGCCUUCAUCAUCUUUGGGUCCGAAGCAAGCGGAGCAGAGGC
GGCCACAGCGACUACAUGAACAUGACCCCUAGACGGCCCGGACCAACCAGAAA
GCACUACCAGCCUUACGCUCCUCCUAGAGACUUCGCCGCCUACCGGUCCAGAG
UGAAGUUCAGCAGAUCCGCCGAUGCUCCCGCCUAUCAGCAGGGACAGAACCAG
CUGUACAACGAGCUGAACCUGGGGAGAAGAGAAGAGUACGACGUGCUGGACA
AGCGGAGAGGCAGAGAUCCUGAGAUGGGCGGCAAGCCCAGACGGAAGAAUCC
UCAAGAGGGCCUGUAUAAUGAGCUGCAGAAAGACAAGAUGGCCGAGGCCUAC
AGCGAGAUCGGAAUGAAGGGCGAGCGCAGAAGAGGCAAGGGACACGAUGGAC
UGUACCAGGGCCUGAGCACCGCCACCAAGGAUACCUAUGAUGCCCUGCACAUG
CAGGCCCUGCCUCCAAGAUAA
SEQ ID NO: 69: (4A8-PDGFRA-28Z DNA) 
ATGGCTCTGCCTGTTACAGCTCTGCTGCTGCCTCTGGCTCTGCTTCTGCATGCCGC
TAGACCTGAGGTGCAGCTGGTTGAATCTGGCGCCGAAGTGAAGAAACCAGGCGC
CTCTGTGAAGGTGTCCTGCAAGGTGTCCGGCTACACCCTGACAGAGCTGAGCATG
CACTGGGTCCGACAGGCCCCTGGAAAAGGCCTTGAATGGATGGGCGGCTTCGAC
CCCGAAGATGGCGAGACTATGTACGCCCAGAAATTCCAGGGCAGAGTGACCATG
ACCGAGGACACCAGCACCGACACCGCCTACATGGAACTGAGCAGCCTGAGAAGC
GAGGATACCGCCGTGTACTACTGCGCCACATCTACAGCCGTGGCCGGCACACCT
GACCTGTTCGATTACTACTACGGCATGGACGTGTGGGGCCAGGGCACAACAGTG
ACAGTTTCTTCTGGCGGCGGAGGATCTGGCGGAGGTGGAAGCGGAGGCGGTGGA
TCTGAGATCGTGATGACACAGAGCCCTCTGTCTAGCCCCGTGACACTGGGACAGC
CTGCCAGCATCAGCTGTAGAAGCAGCCAGAGCCTGGTGCACAGCGACGGCAATA
CCTACCTGTCTTGGCTGCAGCAGAGGCCTGGACAGCCTCCTAGACTGCTGATCTA
CAAGATCAGCAACCGGTTCAGCGGCGTGCCCGATAGATTTTCTGGTAGCGGAGC
CGGCACCGACTTCACCCTGAAGATCAGTAGAGTGGAAGCCGAGGACGTGGGCGT
GTACTATTGCACACAGGCCACACAGTTCCCTTACACCTTCGGACAGGGCACCAAG
GTGGACATCAAGGACTACAAGGACGACGACGACAAGCAGCTTTCATTACCCTCT
ATCCTTCCAAATGAAAATGAAAAGGTTGTGCAGCTGAATTCATCCTTTTCTCTGA
GATGCTTTGGGGAGAGTGAAGTGAGCTGGCAGTACCCCATGTCTGAAGAAGAGA
GCTCCGATGTGGAAATCAGAAATGAAGAAAACAACAGCGGCCTTTTTGTGACGG
 
TCTTGGAAGTGAGCAGTGCCTCGGCGGCCCACACAGGGTTGTACACTTGCTATTA
CAACCACACTCAGACAGAAGAGAATGAGCTTGAAGGCAGGCACATTTACATCTA
TGTGCCAGACCCAGATGTAGCCTTTGTACCTCTAGGAATGACGGATTATTTAGTC
ATCGTGGAGGATGATGATTCTGCCATTATACCTTGTCGCACAACTGATCCCGAGA
CTCCTGTAACCTTACACAACAGTGAGGGGGTGGTACCTGCCTCCTACGACAGCAG
ACAGGGCTTTAATGGGACCTTCACTGTAGGGCCCTATATCTGTGAGGCCACCGTC
AAAGGAAAGAAGTTCCAGACCATCCCATTTAATGTTTATGCTTTAAAAGCAACAT
CAGAGCTGGATCTAGAAATGGAAGCTCTTAAAACCGTGTATAAGTCAGGGGAAA
CGATTGTGGTCACCTGTGCTGTTTTTAACAATGAGGTGGTTGACCTTCAATGGAC
TTACCCTGGAGAAGTGAAAGGCAAAGGCATCACAATGCTGGAAGAAATCAAAGT
CCCATCCATCAAATTGGTGTACACTTTGACGGTCCCCGAGGCCACGGTGAAAGAC
AGTGGAGATTACGAATGTGCTGCCCGCCAGGCTACCAGGGAGGTCAAAGAAATG
AAGAAAGTCACTATTTCTGTCCATGAGAAAGGTTTCATTGAAATCAAACCCACCT
TCAGCCAGTTGGAAGCTGTCAACCTGCATGAAGTCAAACATTTTGTTGTAGAGGT
GCGGGCCTACCCACCTCCCAGGATATCCTGGCTGAAAAACAATCTGACTCTGATT
GAAAATCTCACTGAGATCACCACTGATGTGGAAAAGATTCAGGAAATAAGGTAT
CGAAGCAAATTAAAGCTGATCCGTGCTAAGGAAGAAGACAGTGGCCATTATACT
ATTGTAGCTCAAAATGAAGATGCTGTGAAGAGCTATACTTTTGAACTGTTAACTC
AAGTTCCTTCATCCATTCTGGACTTGGTCGATGATCACCATGGCTCAACTGGGGG
ACAGACGGTGAGGTGCACAGCTGAAGGCACGCCGCTTCCTGATATTGAGTGGAT
GATATGCAAAGATATTAAGAAATGTAATAATGAAACTTCCTGGACTATTTTGGCC
AACAATGTCTCAAACATCATCACGGAGATCCACTCCCGAGACAGGAGTACCGTG
GAGGGCCGTGTGACTTTCGCCAAAGTGGAGGAGACCATCGCCGTGCGATGCCTG
GCTAAGAATCTCCTTGGAGCTGAGAACCGAGAGCTGAAGCTGGTGGCTCCCACC
CTGCGTTCTGAACTCACGGTGGCTTTCTGGGTGCTCGTTGTTGTTGGCGGCGTGCT
GGCCTGTTACTCTCTGCTGGTTACCGTGGCCTTCATCATCTTTTGGGTCCGAAGCA
AGCGGAGCAGAGGCGGCCACAGCGACTACATGAACATGACCCCTAGACGGCCCG
GACCAACCAGAAAGCACTACCAGCCTTACGCTCCTCCTAGAGACTTCGCCGCCTA
CCGGTCCAGAGTGAAGTTCAGCAGATCCGCCGATGCTCCCGCCTATCAGCAGGG
ACAGAACCAGCTGTACAACGAGCTGAACCTGGGGAGAAGAGAAGAGTACGACG
TGCTGGACAAGCGGAGAGGCAGAGATCCTGAGATGGGCGGCAAGCCCAGACGG
AAGAATCCTCAAGAGGGCCTGTATAATGAGCTGCAGAAAGACAAGATGGCCGAG
GCCTACAGCGAGATCGGAATGAAGGGCGAGCGCAGAAGAGGCAAGGGACACGA
 
TGGACTGTACCAGGGCCTGAGCACCGCCACCAAGGATACCTATGATGCCCTGCA
CATGCAGGCCCTGCCTCCAAGATAA
SEQ ID NO: 70: (4A8-PDGFRA-28Z RNA) 
AUGGCUCUGCCUGUUACAGCUCUGCUGCUGCCUCUGGCUCUGCUUCUGCAUGC
CGCUAGACCUGAGGUGCAGCUGGUUGAAUCUGGCGCCGAAGUGAAGAAACCA
GGCGCCUCUGUGAAGGUGUCCUGCAAGGUGUCCGGCUACACCCUGACAGAGCU
GAGCAUGCACUGGGUCCGACAGGCCCCUGGAAAAGGCCUUGAAUGGAUGGGCG
GCUUCGACCCCGAAGAUGGCGAGACUAUGUACGCCCAGAAAUUCCAGGGCAGA
GUGACCAUGACCGAGGACACCAGCACCGACACCGCCUACAUGGAACUGAGCAG
CCUGAGAAGCGAGGAUACCGCCGUGUACUACUGCGCCACAUCUACAGCCGUGG
CCGGCACACCUGACCUGUUCGAUUACUACUACGGCAUGGACGUGUGGGGCCAG
GGCACAACAGUGACAGUUUCUUCUGGCGGCGGAGGAUCUGGCGGAGGUGGAA
GCGGAGGCGGUGGAUCUGAGAUCGUGAUGACACAGAGCCCUCUGUCUAGCCCC
GUGACACUGGGACAGCCUGCCAGCAUCAGCUGUAGAAGCAGCCAGAGCCUGGU
GCACAGCGACGGCAAUACCUACCUGUCUUGGCUGCAGCAGAGGCCUGGACAGC
CUCCUAGACUGCUGAUCUACAAGAUCAGCAACCGGUUCAGCGGCGUGCCCGAU
AGAUUUUCUGGUAGCGGAGCCGGCACCGACUUCACCCUGAAGAUCAGUAGAGU
GGAAGCCGAGGACGUGGGCGUGUACUAUUGCACACAGGCCACACAGUUCCCUU
ACACCUUCGGACAGGGCACCAAGGUGGACAUCAAGGACUACAAGGACGACGAC
GACAAGCAGCUUUCAUUACCCUCUAUCCUUCCAAAUGAAAAUGAAAAGGUUG
UGCAGCUGAAUUCAUCCUUUUCUCUGAGAUGCUUUGGGGAGAGUGAAGUGAG
CUGGCAGUACCCCAUGUCUGAAGAAGAGAGCUCCGAUGUGGAAAUCAGAAAU
GAAGAAAACAACAGCGGCCUUUUUGUGACGGUCUUGGAAGUGAGCAGUGCCU
CGGCGGCCCACACAGGGUUGUACACUUGCUAUUACAACCACACUCAGACAGAA
GAGAAUGAGCUUGAAGGCAGGCACAUUUACAUCUAUGUGCCAGACCCAGAUG
UAGCCUUUGUACCUCUAGGAAUGACGGAUUAUUUAGUCAUCGUGGAGGAUGA
UGAUUCUGCCAUUAUACCUUGUCGCACAACUGAUCCCGAGACUCCUGUAACCU
UACACAACAGUGAGGGGGUGGUACCUGCCUCCUACGACAGCAGACAGGGCUUU
AAUGGGACCUUCACUGUAGGGCCCUAUAUCUGUGAGGCCACCGUCAAAGGAAA
GAAGUUCCAGACCAUCCCAUUUAAUGUUUAUGCUUUAAAAGCAACAUCAGAG
CUGGAUCUAGAAAUGGAAGCUCUUAAAACCGUGUAUAAGUCAGGGGAAACGA
UUGUGGUCACCUGUGCUGUUUUUAACAAUGAGGUGGUUGACCUUCAAUGGAC
 
UUACCCUGGAGAAGUGAAAGGCAAAGGCAUCACAAUGCUGGAAGAAAUCAAA
GUCCCAUCCAUCAAAUUGGUGUACACUUUGACGGUCCCCGAGGCCACGGUGAA
AGACAGUGGAGAUUACGAAUGUGCUGCCCGCCAGGCUACCAGGGAGGUCAAA
GAAAUGAAGAAAGUCACUAUUUCUGUCCAUGAGAAAGGUUUCAUUGAAAUCA
AACCCACCUUCAGCCAGUUGGAAGCUGUCAACCUGCAUGAAGUCAAACAUUUU
GUUGUAGAGGUGCGGGCCUACCCACCUCCCAGGAUAUCCUGGCUGAAAAACAA
UCUGACUCUGAUUGAAAAUCUCACUGAGAUCACCACUGAUGUGGAAAAGAUU
CAGGAAAUAAGGUAUCGAAGCAAAUUAAAGCUGAUCCGUGCUAAGGAAGAAG
ACAGUGGCCAUUAUACUAUUGUAGCUCAAAAUGAAGAUGCUGUGAAGAGCUA
UACUUUUGAACUGUUAACUCAAGUUCCUUCAUCCAUUCUGGACUUGGUCGAU
GAUCACCAUGGCUCAACUGGGGGACAGACGGUGAGGUGCACAGCUGAAGGCAC
GCCGCUUCCUGAUAUUGAGUGGAUGAUAUGCAAAGAUAUUAAGAAAUGUAAU
AAUGAAACUUCCUGGACUAUUUUGGCCAACAAUGUCUCAAACAUCAUCACGGA
GAUCCACUCCCGAGACAGGAGUACCGUGGAGGGCCGUGUGACUUUCGCCAAAG
UGGAGGAGACCAUCGCCGUGCGAUGCCUGGCUAAGAAUCUCCUUGGAGCUGAG
AACCGAGAGCUGAAGCUGGUGGCUCCCACCCUGCGUUCUGAACUCACGGUGGC
UUUCUGGGUGCUCGUUGUUGUUGGCGGCGUGCUGGCCUGUUACUCUCUGCUG
GUUACCGUGGCCUUCAUCAUCUUUUGGGUCCGAAGCAAGCGGAGCAGAGGCGG
CCACAGCGACUACAUGAACAUGACCCCUAGACGGCCCGGACCAACCAGAAAGC
ACUACCAGCCUUACGCUCCUCCUAGAGACUUCGCCGCCUACCGGUCCAGAGUG
AAGUUCAGCAGAUCCGCCGAUGCUCCCGCCUAUCAGCAGGGACAGAACCAGCU
GUACAACGAGCUGAACCUGGGGAGAAGAGAAGAGUACGACGUGCUGGACAAG
CGGAGAGGCAGAGAUCCUGAGAUGGGCGGCAAGCCCAGACGGAAGAAUCCUCA
AGAGGGCCUGUAUAAUGAGCUGCAGAAAGACAAGAUGGCCGAGGCCUACAGC
GAGAUCGGAAUGAAGGGCGAGCGCAGAAGAGGCAAGGGACACGAUGGACUGU
ACCAGGGCCUGAGCACCGCCACCAAGGAUACCUAUGAUGCCCUGCACAUGCAG
GCCCUGCCUCCAAGAUAA
SEQ ID NO: 71: (4A8-PDGFRB-28Z DNA) 
ATGGCTCTGCCTGTTACAGCTCTGCTGCTGCCTCTGGCTCTGCTTCTGCATGCCGC
TAGACCTGAGGTGCAGCTGGTTGAATCTGGCGCCGAAGTGAAGAAACCAGGCGC
CTCTGTGAAGGTGTCCTGCAAGGTGTCCGGCTACACCCTGACAGAGCTGAGCATG
CACTGGGTCCGACAGGCCCCTGGAAAAGGCCTTGAATGGATGGGCGGCTTCGAC
 
CCCGAAGATGGCGAGACTATGTACGCCCAGAAATTCCAGGGCAGAGTGACCATG
ACCGAGGACACCAGCACCGACACCGCCTACATGGAACTGAGCAGCCTGAGAAGC
GAGGATACCGCCGTGTACTACTGCGCCACATCTACAGCCGTGGCCGGCACACCT
GACCTGTTCGATTACTACTACGGCATGGACGTGTGGGGCCAGGGCACAACAGTG
ACAGTTTCTTCTGGCGGCGGAGGATCTGGCGGAGGTGGAAGCGGAGGCGGTGGA
TCTGAGATCGTGATGACACAGAGCCCTCTGTCTAGCCCCGTGACACTGGGACAGC
CTGCCAGCATCAGCTGTAGAAGCAGCCAGAGCCTGGTGCACAGCGACGGCAATA
CCTACCTGTCTTGGCTGCAGCAGAGGCCTGGACAGCCTCCTAGACTGCTGATCTA
CAAGATCAGCAACCGGTTCAGCGGCGTGCCCGATAGATTTTCTGGTAGCGGAGC
CGGCACCGACTTCACCCTGAAGATCAGTAGAGTGGAAGCCGAGGACGTGGGCGT
GTACTATTGCACACAGGCCACACAGTTCCCTTACACCTTCGGACAGGGCACCAAG
GTGGACATCAAGGACTACAAGGACGACGACGACAAGCTGGTCGTCACACCCCCG
GGGCCAGAGCTTGTCCTCAATGTCTCCAGCACCTTCGTTCTGACCTGCTCGGGTT
CAGCTCCGGTGGTGTGGAACGGATGTCCCAGGAGCCCCCACAGGAAATGGCCA
AGGCCCAGGATGGCACCTTCTCCAGCGTGCTCACACTGACCAACCTCACTGGGCT
AGACACGGGAGAATACTTTTGCACCCACAATGACTCCCGTGGACTGGAGACCGA
TGAGCGGAAACGGCTCTACATCTTTGTGCCAGATCCCACCGTGGGCTTCCTCCCT
AATGATGCCGAGGAACTATTCATCTTTCTCACGGAAATAACTGAGATCACCATTC
CATGCCGAGTAACAGACCCACAGCTGGTGGTGACACTGCACGAGAAGAAAGGG
GACGTTGCACTGCCTGTCCCCTATGATCACCAACGTGGCTTTTTTGGTATCTTTGA
GGACAGAAGCTACATCTGCAAAACCACCATTGGGGACAGGGAGGTGGATTCTGA
TGCCTACTATGTCTACAGACTCCAGGTGTCATCCATCAACGTCTCTGTGAACGCA
GTGCAGACTGTGGTCCGCCAGGGTGAGAACATCACCCTCATGTGCATTGTGATCG
GGAATGAGGTGGTCAACTTCGAGTGGACATACCCCCGCAAAGAAAGTGGGCGGC
TGGTGGAGCCGGTGACTGACTTCCTCTTGGATATGCCTTACCACATCCGCTCCAT
CCTGCACATCCCCAGTGCCGAGTTAGAAGACTCGGGGACCTACACCTGCAATGT
GACGGAGAGTGTGAATGACCATCAGGATGAAAAGGCCATCAACATCACCGTGGT
TGAGAGCGGCTACGTGCGGCTCCTGGGAGAGGTGGGCACACTACAATTTGCTGA
GCTGCATCGGAGCCGGACACTGCAGGTAGTGTTCGAGGCCTACCCACCGCCCAC
TGTCCTGTGGTTCAAAGACAACCGCACCCTGGGCGACTCCAGCGCTGGCGAAAT
CGCCCTGTCCACGCGCAACGTGTCGGAGACCCGGTATGTGTCAGAGCTGACACT
GGTTCGCGTGAAGGTGGCAGAGGCTGGCCACTACACCATGCGGGCCTTCCATGA
GGATGCTGAGGTCCAGCTCTCCTTCCAGCTACAGATCAATGTCCCTGTCCGAGTG
CTGGAGCTAAGTGAGAGCCACCCTGACAGTGGGGAACAGACAGTCCGCTGTCGT
 
GGCCGGGGCATGCCCCAGCCGAACATCATCTGGTCTGCCTGCAGAGACCTCAAA
AGGTGTCCACGTGAGCTGCCGCCCACGCTGCTGGGGAACAGTTCCGAAGAGGAG
AGCCAGCTGGAGACTAACGTGACGTACTGGGAGGAGGAGCAGGAGTTTGAGGTG
GTGAGCACACTGCGTCTGCAGCACGTGGATCGGCCACTGTCGGTGCGCTGCACG
CTGCGCAACGCTGTGGGCCAGGACACGCAGGAGGTCATCGTGGTGCCACACTCC
TTGCCCTTTAAGGTGTTCTGGGTGCTCGTTGTTGGCGGCGTGCTGGCCTGTTA
CTCTCTGCTGGTTACCGTGGCCTTCATCATCTTTTGGGTCCGAAGCAAGCGGAGC
AGAGGCGGCCACAGCGACTACATGAACATGACCCCTAGACGGCCCGGACCAACC
AGAAAGCACTACCAGCCTTACGCTCCTCCTAGAGACTTCGCCGCCTACCGGTCCA
GAGTGAAGTTCAGCAGATCCGCCGATGCTCCCGCCTATCAGCAGGGACAGAACC
AGCTGTACAACGAGCTGAACCTGGGGAGAAGAGAAGAGTACGACGTGCTGGAC
AAGCGGAGAGGCAGAGATCCTGAGATGGGCGGCAAGCCCAGACGGAAGAATCC
TCAAGAGGGCCTGTATAATGAGCTGCAGAAAGACAAGATGGCCGAGGCCTACAG
CGAGATCGGAATGAAGGGCGAGCGCAGAAGAGGCAAGGGACACGATGGACTGT
ACCAGGGCCTGAGCACCGCCACCAAGGATACCTATGATGCCCTGCACATGCAGG
CCCTGCCTCCAAGATAA
SEQ ID NO: 72: (4A8-PDGFRB-28Z RNA) 
AUGGCUCUGCCUGUUACAGCUCUGCUGCUGCCUCUGGCUCUGCUUCUGCAUGC
CGCUAGACCUGAGGUGCAGCUGGUUGAAUCUGGCGCCGAAGUGAAGAAACCA
GGCGCCUCUGUGAAGGUGUCCUGCAAGGUGUCCGGCUACACCCUGACAGAGCU
GAGCAUGCACUGGGUCCGACAGGCCCCUGGAAAAGGCCUUGAAUGGAUGGGCG
GCUUCGACCCCGAAGAUGGCGAGACUAUGUACGCCCAGAAAUUCCAGGGCAGA
GUGACCAUGACCGAGGACACCAGCACCGACACCGCCUACAUGGAACUGAGCAG
CCUGAGAAGCGAGGAUACCGCCGUGUACUACUGCGCCACAUCUACAGCCGUGG
CCGGCACACCUGACCUGUUCGAUUACUACUACGGCAUGGACGUGUGGGGCCAG
GGCACAACAGUGACAGUUUCUUCUGGCGGCGGAGGAUCUGGCGGAGGUGGAA
GCGGAGGCGGUGGAUCUGAGAUCGUGAUGACACAGAGCCCUCUGUCUAGCCCC
GUGACACUGGGACAGCCUGCCAGCAUCAGCUGUAGAAGCAGCCAGAGCCUGGU
GCACAGCGACGGCAAUACCUACCUGUCUUGGCUGCAGCAGAGGCCUGGACAGC
CUCCUAGACUGCUGAUCUACAAGAUCAGCAACCGGUUCAGCGGCGUGCCCGAU
AGAUUUUCUGGUAGCGGAGCCGGCACCGACUUCACCCUGAAGAUCAGUAGAGU
GGAAGCCGAGGACGUGGGCGUGUACUAUUGCACACAGGCCACACAGUUCCCUU
 
ACACCUUCGGACAGGGCACCAAGGUGGACAUCAAGGACUACAAGGACGACGAC
GACAAGCUGGUCGUCACACCCCGGGCCAGAGCUUGUCCUCAAUGUCUCCAG
CACCUUCGUUCUGACCUGCUCGGUUCAGCUCCGGUGGUGUGGAACGGAUGU
CCCAGGAGCCCCACAGGAAAUGGCCAAGGCCAGGAUGGCACCUUCUCCAGC
GUGCUCACACUGACCAACCUCACUGGGCUAGACACGGAGAAUACUUUGCAC
CCACAAUGACUCCCGUGGACUGGAGACCGAUGAGCGGAAACGGCUCUACAUCU
UUGUGCCAGAUCCCACCGUGGGCUUCCUCCCUAAUGAUGCCGAGGAACUAUUC
AUCUUUCUCACGGAAAUAACUGAGAUCACCAUUCCAUGCCGAGUAACAGACCC
ACAGCUGGUGGUGACACUGCACGAGAAGAAAGGGGACGUUGCACUGCCUGUCC
CCUAUGAUCACCAACGUGGCUUUUUGGUAUCUUUGAGGACAGAAGCUACAU
CUGCAAAACCACCAUUGGGGACAGGGAGGUGGAUUCUGAUGCCUACUAUGUC
UACAGACUCCAGGUGUCAUCCAUCAACGUCUCUGUGAACGCAGUGCAGACUGU
GGUCCGCCAGGGUGAGAACAUCACCCUCAUGUGCAUUGUGAUCGGGAAUGAG
GUGGUCAACUUCGAGUGGACAUACCCCCGCAAAGAAAGUGGGCGGCUGGUGG
AGCCGGUGACUGACUUCCUCUUGGAUAUGCCUUACCACAUCCGCUCCAUCCUG
CACAUCCCCAGUGCCGAGUUAGAAGACUCGGGGACCUACACCUGCAAUGUGAC
GGAGAGUGUGAAUGACCAUCAGGAUGAAAAGGCCAUCAACAUCACCGUGGUU
GAGAGCGGCUACGUGCGGCUCCUGGGAGAGGUGGGCACACUACAAUUUGCUG
AGCUGCAUCGGAGCCGGACACUGCAGGUAGUGUUCGAGGCCUACCCACCGCCC
ACUGUCCUGUGGUUCAAAGACAACCGCACCCUGGGCGACUCCAGCGCUGGCGA
AAUCGCCCUGUCCACGCGCAACGUGUCGGAGACCCGGUAUGUGUCAGAGCUGA
CACUGGUUCGCGUGAAGGUGGCAGAGGCUGGCCACUACACCAUGCGGGCCUUC
CAUGAGGAUGCUGAGGUCCAGCUCUCCUUCCAGCUACAGAUCAAUGUCCCUGU
CCGAGUGCUGGAGCUAAGUGAGAGCCACCCUGACAGUGGGGAACAGACAGUCC
GCUGUCGUGGCCGGGGCAUGCCCCAGCCGAACAUCAUCUGGUCUGCCUGCAGA
GACCUCAAAAGGUGUCCACGUGAGCUGCCGCCCACGCUGCUGGGGAACAGUUC
CGAAGAGGAGAGCCAGCUGGAGACUAACGUGACGUACUGGGAGGAGGAGCAG
GAGUUUGAGGUGGUGAGCACACUGCGUCUGCAGCACGUGGAUCGGCCACUGUC
GGUGCGCUGCACGCUGCGCAACGCUGUGGGCCAGGACACGCAGGAGGUCAUCG
UGGUGCCACACUCCUUGCCCUUUAAGGUGUUCUGGGUGCUCGUUGUUGUUGGC
GGCGUGCUGGCCUGUUACUCUCUGCUGGUUACCGUGGCCUUCAUCAUCUUUUG
GGUCCGAAGCAAGCGGAGCAGAGGCGGCCACAGCGACUACAUGAACAUGACCC
CUAGACGGCCCGGACCAACCAGAAAGCACUACCAGCCUUACGCUCCUCCUAGA
GACUUCGCCGCCUACCGGUCCAGAGUGAAGUUCAGCAGAUCCGCCGAUGCUCC
 
CGCCUAUCAGCAGGGACAGAACCAGCUGUACAACGAGCUGAACCUGGGGAGAA
GAGAAGAGUACGACGUGCUGGACAAGCGGAGAGGCAGAGAUCCUGAGAUGGG
CGGCAAGCCCAGACGGAAGAAUCCUCAAGAGGGCCUGUAUAAUGAGCUGCAGA
AAGACAAGAUGGCCGAGGCCUACAGCGAGAUCGGAAUGAAGGGCGAGCGCAG
AAGAGGCAAGGGACACGAUGGACUGUACCAGGGCCUGAGCACCGCCACCAAGG
AUACCUAUGAUGCCCUGCACAUGCAGGCCCUGCCUCCAAGAUAA
SEQ ID NO: 73: (4A8-LAIR1-28Z DNA) 
ATGGCTCTGCCTGTTACAGCTCTGCTGCTGCCTCTGGCTCTGCTTCTGCATGCCGC
TAGACCTGAGGTGCAGCTGGTTGAATCTGGCGCCGAAGTGAAGAAACCAGGCGC
CTCTGTGAAGGTGTCCTGCAAGGTGTCCGGCTACACCCTGACAGAGCTGAGCATG
CACTGGGTCCGACAGGCCCCTGGAAAAGGCCTTGAATGGATGGGCGGCTTCGAC
CCCGAAGATGGCGAGACTATGTACGCCCAGAAATTCCAGGGCAGAGTGACCATG
ACCGAGGACACCAGCACCGACACCGCCTACATGGAACTGAGCAGCCTGAGAAGC
GAGGATACCGCCGTGTACTACTGCGCCACATCTACAGCCGTGGCCGGCACACCT
GACCTGTTCGATTACTACTACGGCATGGACGTGTGGGGCCAGGGCACAACAGTG
ACAGTTTCTTCTGGCGGCGGAGGATCTGGCGGAGGTGGAAGCGGAGGCGGTGGA
TCTGAGATCGTGATGACACAGAGCCCTCTGTCTAGCCCCGTGACACTGGGACAGC
CTGCCAGCATCAGCTGTAGAAGCAGCCAGAGCCTGGTGCACAGCGACGGCAATA
CCTACCTGTCTTGGCTGCAGCAGAGGCCTGGACAGCCTCCTAGACTGCTGATCTA
CAAGATCAGCAACCGGTTCAGCGGCGTGCCCGATAGATTTTCTGGTAGCGGAGC
CGGCACCGACTTCACCCTGAAGATCAGTAGAGTGGAAGCCGAGGACGTGGGCGT
GTACTATTGCACACAGGCCACACAGTTCCCTTACACCTTCGGACAGGGCACCAAG
GTGGACATCAAGGACTACAAGGACGACGACGACAAGCAAGAGGACCTGCC
TAGACCTAGCATCTCTGCCGAGCCTGGCACAGTGATCCCTCTGGGAAGCCACGTG
ACCTTCGTGTGTAGAGGACCAGTGGGCGTGCAGACCTTCCGGCTGGAAAGAGAG
AGCAGAAGCACCTACAACGACACCGAGGACGTGTCCCAGGCCTCTCCATCTGAG
AGCGAGGCCAGATTCAGAATCGACAGCGTGTCCGAGGGCAACGCTGGCCCTTAC
CGGTGCATCTACTACAAGCCTCCAAAGTGGAGCGAGCAGAGCGACTACCTGGAA
CTGCTGGTCAAAGAGACATCTGGCGGCCCTGACAGCCCTGATACAGAGCCTGGA
TCTAGCGCCGGACCTACACAGAGGCCCAGCGACAATAGCCACAATGAACACGCC
CCTGCCAGCCAGGGACTGAAAGCCGAACATCTGTACTTCTGGGTGCTCGTTGTTG
TTGGCGGCGTGCTGGCCTGTTACTCTCTGCTGGTTACCGTGGCCTTCATCATCTTT
 
TGGGTCCGAAGCAAGCGGAGCAGAGGCGGCCACAGCGACTACATGAACATGAC
CCCTAGACGGCCCGGACCAACCAGAAAGCACTACCAGCCTTACGCTCCTCCTAG
AGACTTCGCCGCCTACCGGTCCAGAGTGAAGTTCAGCAGATCCGCCGATGCTCCC
GCCTATCAGCAGGGACAGAACCAGCTGTACAACGAGCTGAACCTGGGGAGAAG
AGAAGAGTACGACGTGCTGGACAAGCGGAGAGGCAGAGATCCTGAGATGGGCG
GCAAGCCCAGACGGAAGAATCCTCAAGAGGGCCTGTATAATGAGCTGCAGAAAG
ACAAGATGGCCGAGGCCTACAGCGAGATCGGAATGAAGGGCGAGCGCAGAAGA
GGCAAGGGACACGATGGACTGTACCAGGGCCTGAGCACCGCCACCAAGGATACC
TATGATGCCCTGCACATGCAGGCCCTGCCTCCAAGATAA
SEQ ID NO: 74: (4A8-LAIR1-28Z RNA) 
AUGGCUCUGCCUGUUACAGCUCUGCUGCUGCCUCUGGCUCUGCUUCUGCAUGC
CGCUAGACCUGAGGUGCAGCUGGUUGAAUCUGGCGCCGAAGUGAAGAAACCA
GGCGCCUCUGUGAAGGUGUCCUGCAAGGUGUCCGGCUACACCCUGACAGAGCU
GAGCAUGCACUGGGUCCGACAGGCCCCUGGAAAAGGCCUUGAAUGGAUGGGCG
GCUUCGACCCCGAAGAUGGCGAGACUAUGUACGCCCAGAAAUUCCAGGGCAGA
GUGACCAUGACCGAGGACACCAGCACCGACACCGCCUACAUGGAACUGAGCAG
CCUGAGAAGCGAGGAUACCGCCGUGUACUACUGCGCCACAUCUACAGCCGUGG
CCGGCACACCUGACCUGUUCGAUUACUACUACGGCAUGGACGUGUGGGGCCAG
GGCACAACAGUGACAGUUUCUUCUGGCGGCGGAGGAUCUGGCGGAGGUGGAA
GCGGAGGCGGUGGAUCUGAGAUCGUGAUGACACAGAGCCCUCUGUCUAGCCCC
GUGACACUGGGACAGCCUGCCAGCAUCAGCUGUAGAAGCAGCCAGAGCCUGGU
GCACAGCGACGGCAAUACCUACCUGUCUUGGCUGCAGCAGAGGCCUGGACAGC
CUCCUAGACUGCUGAUCUACAAGAUCAGCAACCGGUUCAGCGGCGUGCCCGAU
AGAUUUUCUGGUAGCGGAGCCGGCACCGACUUCACCCUGAAGAUCAGUAGAGU
GGAAGCCGAGGACGUGGGCGUGUACUAUUGCACACAGGCCACACAGUUCCCUU
ACACCUUCGGACAGGGCACCAAGGUGGACAUCAAGGACUACAAGGACGACGAC
GACAAGCAAGAAGAGGACCUGCCUAGACCUAGCAUCUCUGCCGAGCCUGGCAC
AGUGAUCCCUCUGGGAAGCCACGUGACCUUCGUGUGUAGAGGACCAGUGGGCG
UGCAGACCUUCCGGCUGGAAAGAGAGAGCAGAAGCACCUACAACGACACCGAG
GACGUGUCCCAGGCCUCUCCAUCUGAGAGCGAGGCCAGAUUCAGAAUCGACAG
CGUGUCCGAGGGCAACGCUGGCCCUUACCGGUGCAUCUACUACAAGCCUCCAA
AGUGGAGCGAGCAGAGCGACUACCUGGAACUGCUGGUCAAAGAGACAUCUGG
 
CGGCCCUGACAGCCCUGAUACAGAGCCUGGAUCUAGCGCCGGACCUACACAGA
GGCCCAGCGACAAUAGCCACAAUGAACACGCCCCUGCCAGCCAGGGACUGAAA
GCCGAACAUCUGUACUUCUGGGUGCUCGUUGUUGGCGGCGUGCUGGCCUG
UUACUCUCUGCUGGUUACCGUGGCCUUCAUCAUCUUUUGGGUCCGAAGCAAGC
GGAGCAGAGGCGGCCACAGCGACUACAUGAACAUGACCCCUAGACGGCCCGGA
CCAACCAGAAAGCACUACCAGCCUUACGCUCCUCCUAGAGACUUCGCCGCCUA
CCGGUCCAGAGUGAAGUUCAGCAGAUCCGCCGAUGCUCCCGCCUAUCAGCAGG
GACAGAACCAGCUGUACAACGAGCUGAACCUGGGGAGAAGAGAAGAGUACGA
CGUGCUGGACAAGCGGAGAGGCAGAGAUCCUGAGAUGGGCGGCAAGCCCAGAC
GGAAGAAUCCUCAAGAGGGCCUGUAUAAUGAGCUGCAGAAAGACAAGAUGGC
CGAGGCCUACAGCGAGAUCGGAAUGAAGGGCGAGCGCAGAAGAGGCAAGGGA
CACGAUGGACUGUACCAGGGCCUGAGCACCGCCACCAAGGAUACCUAUGAUGC
CCUGCACAUGCAGGCCCUGCCUCCAAGAUAA
SEQ ID NO: 75: (4A8-tLAIR1-28Z DNA) 
ATGGCTCTGCCTGTTACAGCTCTGCTGCTGCCTCTGGCTCTGCTTCTGCATGCCGC
TAGACCTGAGGTGCAGCTGGTTGAATCTGGCGCCGAAGTGAAGAAACCAGGCGC
CTCTGTGAAGGTGTCCTGCAAGGTGTCCGGCTACACCCTGACAGAGCTGAGCATG
CACTGGGTCCGACAGGCCCCTGGAAAAGGCCTTGAATGGATGGGCGGCTTCGAC
CCCGAAGATGGCGAGACTATGTACGCCCAGAAATTCCAGGGCAGAGTGACCATG
ACCGAGGACACCAGCACCGACACCGCCTACATGGAACTGAGCAGCCTGAGAAGC
GAGGATACCGCCGTGTACTACTGCGCCACATCTACAGCCGTGGCCGGCACACCT
GACCTGTTCGATTACTACTACGGCATGGACGTGTGGGGCCAGGGCACAACAGTG
ACAGTTTCTTCTGGCGGCGGAGGATCTGGCGGAGGTGGAAGCGGAGGCGGTGGA
TCTGAGATCGTGATGACACAGAGCCCTCTGTCTAGCCCCGTGACACTGGGACAGC
CTGCCAGCATCAGCTGTAGAAGCAGCCAGAGCCTGGTGCACAGCGACGGCAATA
CCTACCTGTCTTGGCTGCAGCAGAGGCCTGGACAGCCTCCTAGACTGCTGATCTA
CAAGATCAGCAACCGGTTCAGCGGCGTGCCCGATAGATTTCTGGTAGCGGAGC
CGGCACCGACTTCACCCTGAAGATCAGTAGAGTGGAAGCCGAGGACGTGGGCGT
GTACTATTGCACACAGGCCACACAGTTCCCTTACACCTTCGGACAGGGCACCAAG
GTGGACATCAAGGACTACAAGGACGACGACGACAAGCTGCTGGTCAAAGAGAC
ATCTGGCGGCCCTGACAGCCCTGATACAGAGCCTGGATCTAGCGCCGGACCTAC
ACAGAGGCCCAGCGACAATAGCCACAATGAACACGCCCCTGCCAGCCAGGGACT
 
GAAAGCCGAACATCTGTACTTCTGGGTGCTCGTTGTTGGCGGCGTGCTGGCC
TGTTACTCTCTGCTGGTTACCGTGGCCTTCATCATCTTTTGGGTCCGAAGCAAGCG
GAGCAGAGGCGGCCACAGCGACTACATGAACATGACCCCTAGACGGCCCGGACC
AACCAGAAAGCACTACCAGCCTTACGCTCCTCCTAGAGACTTCGCCGCCTACCGG
TCCAGAGTGAAGTTCAGCAGATCCGCCGATGCTCCCGCCTATCAGCAGGGACAG
AACCAGCTGTACAACGAGCTGAACCTGGGGAGAAGAGAAGAGTACGACGTGCT
GGACAAGCGGAGAGGCAGAGATCCTGAGATGGGCGGCAAGCCCAGACGGAAGA
ATCCTCAAGAGGGCCTGTATAATGAGCTGCAGAAAGACAAGATGGCCGAGGCCT
ACAGCGAGATCGGAATGAAGGGCGAGCGCAGAAGAGGCAAGGGACACGATGGA
CTGTACCAGGGCCTGAGCACCGCCACCAAGGATACCTATGATGCCCTGCACATG
CAGGCCCTGCCTCCAAGATAA
SEQ ID NO: 76: (4A8-tLAIR1-28Z RNA) 
AUGGCUCUGCCUGUUACAGCUCUGCUGCUGCCUCUGGCUCUGCUUCUGCAUGC
CGCUAGACCUGAGGUGCAGCUGGUUGAAUCUGGCGCCGAAGUGAAGAAACCA
GGCGCCUCUGUGAAGGUGUCCUGCAAGGUGUCCGGCUACACCCUGACAGAGCU
GAGCAUGCACUGGGUCCGACAGGCCCCUGGAAAAGGCCUUGAAUGGAUGGGCG
GCUUCGACCCCGAAGAUGGCGAGACUAUGUACGCCCAGAAAUUCCAGGGCAGA
GUGACCAUGACCGAGGACACCAGCACCGACACCGCCUACAUGGAACUGAGCAG
CCUGAGAAGCGAGGAUACCGCCGUGUACUACUGCGCCACAUCUACAGCCGUGG
CCGGCACACCUGACCUGUUCGAUUACUACUACGGCAUGGACGUGUGGGGCCAG
GGCACAACAGUGACAGUUUCUUCUGGCGGCGGAGGAUCUGGCGGAGGUGGAA
GCGGAGGCGGUGGAUCUGAGAUCGUGAUGACACAGAGCCCUCUGUCUAGCCCC
GUGACACUGGGACAGCCUGCCAGCAUCAGCUGUAGAAGCAGCCAGAGCCUGGU
GCACAGCGACGGCAAUACCUACCUGUCUUGGCUGCAGCAGAGGCCUGGACAGC
CUCCUAGACUGCUGAUCUACAAGAUCAGCAACCGGUUCAGCGGCGUGCCCGAU
AGAUUUUCUGGUAGCGGAGCCGGCACCGACUUCACCCUGAAGAUCAGUAGAGU
GGAAGCCGAGGACGUGGGCGUGUACUAUUGCACACAGGCCACACAGUUCCCUU
ACACCUUCGGACAGGGCACCAAGGUGGACAUCAAGGACUACAAGGACGACGAC
GACAAGCUGCUGGUCAAAGAGACAUCUGGCGGCCCUGACAGCCCUGAUACAGA
GCCUGGAUCUAGCGCCGGACCUACACAGAGGCCCAGCGACAAUAGCCACAAUG
AACACGCCCCUGCCAGCCAGGGACUGAAAGCCGAACAUCUGUACUUCUGGGUG
CUCGUUGUUGUUGGCGGCGUGCUGGCCUGUUACUCUCUGCUGGUUACCGUGGC
 
CUUCAUCAUCUUUGGGUCCGAAGCAAGCGGAGCAGAGGCGGCCACAGCGACU
ACAUGAACAUGACCCCUAGACGGCCCGGACCAACCAGAAAGCACUACCAGCCU
UACGCUCCUCCUAGAGACUUCGCCGCCUACCGGUCCAGAGUGAAGUUCAGCAG
AUCCGCCGAUGCUCCCGCCUAUCAGCAGGGACAGAACCAGCUGUACAACGAGC
UGAACCUGGGGAGAAGAGAAGAGUACGACGUGCUGGACAAGCGGAGAGGCAG
AGAUCCUGAGAUGGGCGGCAAGCCCAGACGGAAGAAUCCUCAAGAGGGCCUGU
AUAAUGAGCUGCAGAAAGACAAGAUGGCCGAGGCCUACAGCGAGAUCGGAAU
GAAGGGCGAGCGCAGAAGAGGCAAGGGACACGAUGGACUGUACCAGGGCCUG
AGCACCGCCACCAAGGAUACCUAUGAUGCCCUGCACAUGCAGGCCCUGCCUCC
AAGAUAA
SEQ ID NO: 77: (CR3014-IgG4mut-28Z DNA) 
ATGGCTCTGCCTGTGACAGCTCTGCTGCTGCCTCTGGCTCTGCTTCTGCATGCCGC
CAGACCTGACATCCAGATGACACAGAGCCCTAGCAGCCTGTCTGCCAGCGTGGG
AGACAGAGTGACCATCACCTGTAGAGCCAGCCAGAGCATCAGCTCCTACCTGAA
CTGGTATCAGCAGAAGCCCGGCAAGGCCCCTAAGCTGCTGATCTATGCTGCCAG
CTCTCTGCAGTCTGGCGTGCCCAGCAGATTTTCTGGCAGCGGCTCTGGCACCGAC
TTCACCCTGACCATATCTAGCCTGCAGCCTGAGGACTTCGCCACCTACTACTGCC
AGCAGTCCTACAGCACCCCTCCTACATTTGGCCAGGGCACCAAGGTGGAAATCA
AAGGCGGCGGAGGATCTGGCGGAGGTGGAAGTGGCGGAGGCGGATCTGAAGTG
CAGCTGGTTGAATCAGGTGGCGGCCTGGTTCAACCTGGCGGATCTCTGAGACTGA
GCTGTGCCGCCAGCGGCTTCACCTTCAGCGACCACTATATGGACTGGGTCCGACA
GGCCCCTGGCAAAGGACTTGAGTGGGTCGGACGGACCAGAAACAAGGCCAACA
GCTACACCACAGAGTACGCCGCCTCTGTGAAGGGCAGATTCACCATCAGCCGGG
ACGACAGCAAGAACAGCCTGTACCTGCAGATGAACTCCCTGAAAACCGAGGACA
CCGCCGTGTACTACTGCGCCAGAGGAATCAGCCCCTTCTACTTCGACTATTGGGG
CCAGGGAACCCTCGTGACCGTTTCTTCTGACTACAAGGACGACGACGACAAGGA
ATCTAAGTACGGCCCTCCTTGTCCTCCATGTCCTGCTCCACCTGTGGCCGGACCCT
CCGTGTTCCTGTTTCCTCCAAAGCCTAAGGACACCCTGATGATCAGCAGAACCCC
TGAAGTGACCTGCGTGGTGGTGGACGTTTCCCAAGAGGACCCTGAGGTGCAGTT
CAATTGGTACGTGGACGGCGTGGAAGTGCACAACGCCAAGACCAAGCCTAGAGA
GGAACAGTTCCAGAGCACCTACAGAGTGGTGTCCGTGCTGACCGTGCTGCACCA
GGATTGGCTGAACGGCAAAGAGTACAAGTGCAAGGTGTCCAACAAGGGCCTGCC
 
TAGCAGCATCGAGAAAACCATCAGCAAGGCCAAGGGCCAGCCAAGAGAACCCC
AGGTGTACACACTGCCTCCAAGCCAAGAGGAAATGACCAAGAACCAGGTGTCCC
TGACCTGCCTGGTCAAGGGCTTCTACCCTTCCGATATCGCCGTGGAATGGGAGAG
CAATGGCCAGCCTGAGAACAACTACAAGACCACACCTCCTGTGCTGGACAGCGA
CGGCTCATTCTTCCTGTACAGCAGACTGACCGTGGACAAGAGCAGATGGCAAGA
GGGCAACGTGTTCAGCTGCAGCGTGATGCACGAGGCCCTGCACAACCACTACAC
CCAGAAGTCTCTGAGCCTGAGCCTGGGCAAGTTCTGGGTGCTCGTTGTTGTTGGC
GGCGTGCTGGCCTGTTACTCTCTGCTGGTTACCGTGGCCTTCATCATCTTTTGGGT
CCGAAGCAAGCGGAGCAGAGGCGGCCACAGCGACTACATGAACATGACCCCTA
GACGGCCCGGACCAACCAGAAAGCACTACCAGCCTTACGCTCCTCCTAGAGACT
TCGCCGCCTACCGGTCCAGAGTGAAGTTCAGCAGATCCGCCGATGCTCCCGCCTA
TCAGCAGGGACAGAACCAGCTGTACAACGAGCTGAACCTGGGGAGAAGAGAAG
AGTACGACGTGCTGGACAAGCGGAGAGGCAGAGATCCTGAGATGGGCGGCAAG
CCCAGACGGAAGAATCCTCAAGAGGGCCTGTATAATGAGCTGCAGAAAGACAAG
ATGGCCGAGGCCTACAGCGAGATCGGAATGAAGGGCGAGCGCAGAAGAGGCAA
GGGACACGATGGACTGTACCAGGGCCTGAGCACCGCCACCAAGGATACCTATGA
TGCCCTGCACATGCAGGCCCTGCCTCCAAGATAA
SEQ ID NO: 78: (CR3014-IgG4mut-28Z RNA) 
AUGGCUCUGCCUGUGACAGCUCUGCUGCUGCCUCUGGCUCUGCUUCUGCAUGC
CGCCAGACCUGACAUCCAGAUGACACAGAGCCCUAGCAGCCUGUCUGCCAGCG
UGGGAGACAGAGUGACCAUCACCUGUAGAGCCAGCCAGAGCAUCAGCUCCUAC
CUGAACUGGUAUCAGCAGAAGCCCGGCAAGGCCCCUAAGCUGCUGAUCUAUGC
UGCCAGCUCUCUGCAGUCUGGCGUGCCCAGCAGAUUUCUGGCAGCGGCUCUG
GCACCGACUUCACCCUGACCAUAUCUAGCCUGCAGCCUGAGGACUUCGCCACC
UACUACUGCCAGCAGUCCUACAGCACCCCUCCUACAUUUGGCCAGGGCACCAA
GGUGGAAAUCAAAGGCGGCGGAGGAUCUGGCGGAGGUGGAAGUGGCGGAGGC
GGAUCUGAAGUGCAGCUGGUUGAAUCAGGUGGCGGCCUGGUUCAACCUGGCG
GAUCUCUGAGACUGAGCUGUGCCGCCAGCGGCUUCACCUUCAGCGACCACUAU
AUGGACUGGGUCCGACAGGCCCCUGGCAAAGGACUUGAGUGGGUCGGACGGAC
CAGAAACAAGGCCAACAGCUACACCACAGAGUACGCCGCCUCUGUGAAGGGCA
GAUUCACCAUCAGCCGGGACGACAGCAAGAACAGCCUGUACCUGCAGAUGAAC
UCCCUGAAAACCGAGGACACCGCCGUGUACUACUGCGCCAGAGGAAUCAGCCC
 
CUUCUACUUCGACUAUUGGGGCCAGGGAACCCUCGUGACCGUUUCUUCUGACU
ACAAGGACGACGACGACAAGGAAUCUAAGUACGGCCCUCCUUGUCCUCCAUGU
CCUGCUCCACCUGUGGCCGGACCCUCCGUGUUCCUGUUCCUCCAAAGCCUAA
GGACACCCUGAUGAUCAGCAGAACCCCUGAAGUGACCUGCGUGGUGGUGGACG
UUUCCCAAGAGGACCCUGAGGUGCAGUUCAAUUGGUACGUGGACGGCGUGGA
AGUGCACAACGCCAAGACCAAGCCUAGAGAGGAACAGUUCCAGAGCACCUACA
GAGUGGUGUCCGUGCUGACCGUGCUGCACCAGGAUUGGCUGAACGGCAAAGA
GUACAAGUGCAAGGUGUCCAACAAGGGCCUGCCUAGCAGCAUCGAGAAAACCA
UCAGCAAGGCCAAGGGCCAGCCAAGAGAACCCCAGGUGUACACACUGCCUCCA
AGCCAAGAGGAAAUGACCAAGAACCAGGUGUCCCUGACCUGCCUGGUCAAGGG
CUUCUACCCUUCCGAUAUCGCCGUGGAAUGGGAGAGCAAUGGCCAGCCUGAGA
ACAACUACAAGACCACACCUCCUGUGCUGGACAGCGACGGCUCAUUCUUCCUG
UACAGCAGACUGACCGUGGACAAGAGCAGAUGGCAAGAGGGCAACGUGUUCA
GCUGCAGCGUGAUGCACGAGGCCCUGCACAACCACUACACCCAGAAGUCUCUG
AGCCUGAGCCUGGGCAAGUUCUGGGUGCUCGUUGUUGUUGGCGGCGUGCUGG
CCUGUUACUCUCUGCUGGUUACCGUGGCCUUCAUCAUCUUUUGGGUCCGAAGC
AAGCGGAGCAGAGGCGGCCACAGCGACUACAUGAACAUGACCCCUAGACGGCC
CGGACCAACCAGAAAGCACUACCAGCCUUACGCUCCUCCUAGAGACUUCGCCG
CCUACCGGUCCAGAGUGAAGUUCAGCAGAUCCGCCGAUGCUCCCGCCUAUCAG
CAGGGACAGAACCAGCUGUACAACGAGCUGAACCUGGGGAGAAGAGAAGAGU
ACGACGUGCUGGACAAGCGGAGAGGCAGAGAUCCUGAGAUGGGCGGCAAGCCC
AGACGGAAGAAUCCUCAAGAGGGCCUGUAUAAUGAGCUGCAGAAAGACAAGA
UGGCCGAGGCCUACAGCGAGAUCGGAAUGAAGGGCGAGCGCAGAAGAGGCAA
GGGACACGAUGGACUGUACCAGGGCCUGAGCACCGCCACCAAGGAUACCUAUG
AUGCCCUGCACAUGCAGGCCCUGCCUCCAAGAUAA
SEQ ID NO: 79: (Omicron BA.2 S protein) 
MFVFLVLLPLVSSQCVNLTTRTQLPPAYTNSFTRGVYYPDKVFRSSVLHSTQDLFLPF
FSNVTWFHVISGTNGTKRFDNPVLPFNDGVYFASIEKSNIIRGWIFGTTLDSKTQSLLI
VNNATNVVIKVCEFQFCNDPFLDHKNNKSWMESEFRVYSSANNCTFEYVSQPFLMD
LEGKQGNFKNLREFVFKNIDGYFKIYSKHTPIIVREPEDLPQGFSALEPLVDLPIGINIT
RFQTLLALHRSYLTPGDSSSGWTAGAAAYYVGYLQPRTFLLKYNENGTITDAVDCA
LDPLSETKCTLKSFTVEKGIYQTSNFRVQPTESIVRFPNITNLCPFDEVFNATRFASVY
 
AWNRKRISNCVADYSVLYNLAPFFTFKCYGVSPTKLNDLCFTNVYADSFVIRGDEVR
QIAPGQTGNIADYNYKLPDDFTGCVIAWNSNKLDSKVSGNYNYLYRLFRKSNLKPFE
RDISTEIYQAGNKPCNGVAGFNCYFPLRSYSFRPTYGVGHQPYRVVVLSFELLHAPA
TVCGPKKSTNLVKNKCVNFNFNGLKGTGVLTESNKKFLPFQQFGRDIADTTDAVRD
PQTLEILDITPCSFGGVSVITPGTNTSNQVAVLYQGVNCTEVPVAIHADQLTPTWRVY
STGSNVFQTRAGCLIGAEYVNNSYECDIPIGAGICASYQTQTKSHRRARSVASQSIIAY
TMSLGAENSVAYSNNSIAIPTNFTISVTTEILPVSMTKTSVDCTMYICGDSTECSNLLL
QYGSFCTQLKRALTGIAVEQDKNTQEVFAQVKQIYKTPPIKYFGGFNFSQILPDPSKP
SKRSFIEDLLFNKVTLADAGFIKQYGDCLGDIAARDLICAQKFKGLTVLPPLLTDEMI
AQYTSALLAGTITSGWTFGAGAALQIPFAMQMAYRFNGIGVTQNVLYENQKLIANQ
FNSAIGKIQDSLSSTASALGKLQDVVNHNAQALNTLVKQLSSKFGAISSVLNDIFSRL
DKVEAEVQIDRLITGRLQSLQTYVTQQLIRAAEIRASANLAATKMSECVLGQSKRVD
FCGKGYHLMSFPQSAPHGVVFLHVTYVPAQEKNFTTAPAICHDGKAHFPREGVFVS
NGTHWFVTQRNFYEPQIITTDNTFVSGNCDVVIGIVNNTVYDPLQPELDSFKEELDKY
FKNHTSPDVDLGDISGINASVVNIQKEIDRLNEVAKNLNESLIDLQELGKYEQYIKWP
WYIWLGFIAGLIAIVMVTIMLCCMTSCCSCLKGCCSCGSCCKFDEDDSEPVLKGVKL
HYT
SEQ ID NO: 80: (Flag-28Z peptide) 
MALPVTALLLPLALLLHAARPDYKDDDDKIEVMYPPPYLDNEKSNGTIIHVKGKHLC
PSPLFPGPSKPFWVLVVVGGVLACYSLLVTVAFIIFWVRSKRSRGGHSDYMNMTPRR
PGPTRKHYQPYAPPRDFAAYRSRVKFSRSADAPAYQQGQNQLYNELNLGRREEYDV
LDKRRGRDPEMGGKPRRKNPQEGLYNELQKDKMAEAYSEIGMKGERRRGKGHDG
LYQGLSTATKDTYDALHMQALPPR
SEQ ID NO: 81: (CR3014-28Z peptide) 
MALPVTALLLPLALLLHAARPDIQMTQSPSSLSASVGDRVTITCRASQSISSYLNWYQ
QKPGKAPKLLIYAASSLQSGVPSRFSGSGSGTDFTLTISSLQPEDFATYYCQQSYSTPP
TFGQGTKVEIKGGGGSGGGGSGGGGSEVQLVESGGGLVQPGGSLRLSCAASGFTFSD
HYMDWVRQAPGKGLEWVGRTRNKANSYTTEYAASVKGRFTISRDDSKNSLYLQM
NSLKTEDTAVYYCARGISPFYFDYWGQGTLVTVSSDYKDDDDKIEVMYPPPYLDNE
KSNGTIIHVKGKHLCPSPLFPGPSKPFWVLVVVGGVLACYSLLVTVAFIIFWVRSKRS
RGGHSDYMNMTPRRPGPTRKHYQPYAPPRDFAAYRSRVKFSRSADAPAYQQGQNQ
 
LYNELNLGRREEYDVLDKRRGRDPEMGGKPRRKNPQEGLYNELQKDKMAEAYSEI
GMKGERRRGKGHDGLYQGLSTATKDTYDALHMQALPPR
SEQ ID NO: 82: (CR3022-28Z peptide) 
MALPVTALLLPLALLLHAARPDIVMTQSPDSLAVSLGERATINCKSSQSVLYSSINKN
YLAWYQQKPGQPPKLLIYWASTRESGVPDRFSGSGSGTDFTLTISSLQAEDVAVYYC
QQYYSTPYTFGQGTKVEIKGGGGSGGGGSGGGGSEVQLVQSGTEVKKPGESLKISCK
GSGYGFITYWIGWVRQMPGKGLEWMGIIYPGDSETRYSPSFQGQVTISADKSINTAY
LQWSSLKASDTAIYYCAGGSGISTPMDVWGQGTTVTVSSDYKDDDDKIEVMYPPPY
LDNEKSNGTIIHVKGKHLCPSPLFPGPSKPFWVLVVVGGVLACYSLLVTVAFIIFWVR
SKRSRGGHSDYMNMTPRRPGPTRKHYQPYAPPRDFAAYRSRVKFSRSADAPAYQQG
QNQLYNELNLGRREEYDVLDKRRGRDPEMGGKPRRKNPQEGLYNELQKDKMAEA
YSEIGMKGERRRGKGHDGLYQGLSTATKDTYDALHMQALPPR
SEQ ID NO: 83: (CR3022-81-28Z peptide) 
MALPVTALLLPLALLLHAARPDIVMTQSPDSLAVSLGERATINCKSSQSVLYSSINKN
YLAWYQQKPGQPPKLLIYWASTRESGVPDRFSGSGSGTDFTLTISSLQAEDVAVYYC
QQYYSTPYTFGQGTKVEIKGGGGSGGGGSGGGGSEVQLVQSGTEVKKPGESLKISCK
GSGYGFITYWIGWVRQMPGKGLEWMGIIYPGDSETRYSPSFQGQVTISADKSINTAY
LQWSSLKASDTAIYYCAGGSGISTPMDVWGQGTTVTVSSDYKDDDDKKPTTTPAPR
PPTPAPTIASQPLSLRPEACRPAAGGAVHTRGLDFACDFWVLVVVGGVLACYSLLVT
VAFIIFWVRSKRSRGGHSDYMNMTPRRPGPTRKHYQPYAPPRDFAAYRSRVKFSRSA
DAPAYQQGQNQLYNELNLGRREEYDVLDKRRGRDPEMGGKPRRKNPQEGLYNELQ
KDKMAEAYSEIGMKGERRRGKGHDGLYQGLSTATKDTYDALHMQALPPR
SEQ ID NO. 84: (CR3022-CH3-28Z peptide) 
MALPVTALLLPLALLLHAARPDIVMTQSPDSLAVSLGERATINCKSSQSVLYSSINKN
YLAWYQQKPGQPPKLLIYWASTRESGVPDRFSGSGSGTDFTLTISSLQAEDVAVYYC
QQYYSTPYTFGQGTKVEIKGGGGSGGGGSGGGGSEVQLVQSGTEVKKPGESLKISCK
GSGYGFITYWIGWVRQMPGKGLEWMGIIYPGDSETRYSPSFQGQVTISADKSINTAY
LQWSSLKASDTAIYYCAGGSGISTPMDVWGQGTTVTVSSDYKDDDDKESKYGPPCP
PCPGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGKFWV
 
LVVVGGVLACYSLLVTVAFIIFWVRSKRSRGGHSDYMNMTPRRPGPTRKHYQPYAP
PRDFAAYRSRVKFSRSADAPAYQQGQNQLYNELNLGRREEYDVLDKRRGRDPEMG
GKPRRKNPQEGLYNELQKDKMAEAYSEIGMKGERRRGKGHDGLYQGLSTATKDTY
DALHMQALPPR
SEQ ID NO. 85: (CR022-IgG4-28Z peptide) 
MALPVTALLLPLALLLHAARPDIVMTQSPDSLAVSLGERATINCKSSQSVLYSSINKN
YLAWYQQKPGQPPKLLIYWASTRESGVPDRFSGSGSGTDFTLTISSLQAEDVAVYYC
QQYYSTPYTFGQGTKVEIKGGGGSGGGGSGGGGSEVQLVQSGTEVKKPGESLKISCK
GSGYGFITYWIGWVRQMPGKGLEWMGIIYPGDSETRYSPSFQGQVTISADKSINTAY
LQWSSLKASDTAIYYCAGGSGISTPMDVWGQGTTVTVSSDYKDDDDKESKYGPPCP
PCPAPEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISKAK
GQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGKFWVLVV
VGGVLACYSLLVTVAFIIFWVRSKRSRGGHSDYMNMTPRRPGPTRKHYQPYAPPRD
FAAYRSRVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKDTYDAL
HMQALPPR
SEQ ID NO. 86: (C135-IgG4mut-28Z peptide) 
MALPVTALLLPLALLLHAARPQVQLVESGGGVVQPGRSLRLSCAASGFTFSSYAMH
WVRQAPGKGLEWVAVIPFDGRNKYYADSVTGRFTISRDNSKNTLYLQMNSLRAEDT
AVYYCASSSGYLFHSDYWGQGTLVTVSSGGGGSGGGGSGGGGSDIQMTQSPSTLSA
SVGDRVTITCRASQSISNWLAWFQQKPGKAPKLLIYEASSLESGVPSRFSGSGSGTEF
TLTISSLQPDDFATYYCQQYNSYPWTFGQGTKVEIKDYKDDDDKESKYGPPCPPCPA
PPVAGPSVFLFPPKPKDTLMISRTPEVTCVVVDVSQEDPEVQFNWYVDGVEVHNAK
TKPREEQFQSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSDG
SFFLYSRLTVDKSRWQEGNVFSCSVMHEALHNHYTQKSLSLSLGKFWVLVVVGGVL
ACYSLLVTVAFIIFWVRSKRSRGGHSDYMNMTPRRPGPTRKHYQPYAPPRDFAAYRS
RVKFSRSADAPAYQQGQNQLYNELNLGRREEYDVLDKRRGRDPEMGGKPRRKNPQ
 
EGLYNELQKDKMAEAYSEIGMKGERRRGKGHDGLYQGLSTATKDTYDALHMQAL
PPR
SEQ ID NO. 87: (S309-IgG4mut-28Z peptide) 
MALPVTALLLPLALLLHAARPQVQLVQSGAEVKKPGASVKVSCKASGYPFTSYGIS
WVRQAPGQGLEWMGWISTYNGNTNYAQKFQGRVTMTTDTSTTTGYMELRRLRSD
DTAVYYCARDYTRGAWFGESLIGGFDNWGQGTLVTVSSGGGGSGGGGSGGGGSEI
VLTQSPGTLSLSPGERATLSCRASQTVSSTSLAWYQQKPGQAPRLLIYGASSRATGIP
DRFSGSGSGTDFTLTISRLEPEDFAVYYCQQHDTSLTFGGGTKVEIKDYKDDDDKES
KYGPPCPPCPAPPVAGPSVFLFPPKPKDTLMISRTPEVTCVVVDVSQEDPEVQFNWY
VDGVEVHNAKTKPREEQFQSTYRVVSVLTVLHQDWLNGKEYKCKVSNKGLPSSIEK
TISKAKGQPREPQVYTLPPSQEEMTKNQVSLTCLVKGFYPSDIAVEWESNGQPENNY
KTTPPVLDSDGSFFLYSRLTVDKSRWQEGNVFSCSVMHEALHNHYTQKSLSLSLGKF
WVLVVVGGVLACYSLLVTVAFIIFWVRSKRSRGGHSDYMNMTPRRPGPTRKHYQP
YAPPRDFAAYRSRVKFSRSADAPAYQQGQNQLYNELNLGRREEYDVLDKRRGRDPE
MGGKPRRKNPQEGLYNELQKDKMAEAYSEIGMKGERRRGKGHDGLYQGLSTATK
DTYDALHMQALPPR
SEQ ID NO: 88: (4A8-IgG4mut-28Z peptide) 
MALPVTALLLPLALLLHAARPEVQLVESGAEVKKPGASVKVSCKVSGYTLTELSMH
WVRQAPGKGLEWMGGFDPEDGETMYAQKFQGRVTMTEDTSTDTAYMELSSLRSE
DTAVYYCATSTAVAGTPDLFDYYYGMDVWGQGTTVTVSSGGGGSGGGGSGGGGS
EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIKDYKD
DDDKESKYGPPCPPCPAPPVAGPSVFLFPPKPKDTLMISRTPEVTCVVVDVSQEDPEV
QFNWYVDGVEVHNAKTKPREEQFQSTYRVVSVLTVLHQDWLNGKEYKCKVSNKG
LPSSIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSL
SLSLGKFWVLVVVGGVLACYSLLVTVAFIIFWVRSKRSRGGHSDYMNMTPRRPGPT
RKHYQPYAPPRDFAAYRSRVKFSRSADAPAYQQGQNQLYNELNLGRREEYDVLDK
RRGRDPEMGGKPRRKNPQEGLYNELQKDKMAEAYSEIGMKGERRRGKGHDGLYQ
GLSTATKDTYDALHMQALPPR
SEQ ID NO: 89: (4A8-PDGFRA-28Z peptide) 
MALPVTALLLPLALLLHAARPEVQLVESGAEVKKPGASVKVSCKVSGYTLTELSMH
WVRQAPGKGLEWMGGFDPEDGETMYAQKFQGRVTMTEDTSTDTAYMELSSLRSE
DTAVYYCATSTAVAGTPDLFDYYYGMDVWGQGTTVTVSSGGGGSGGGGSGGGGS
EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIKDYKD
DDDKQLSLPSILPNENEKVVQLNSSFSLRCFGESEVSWQYPMSEEESSDVEIRNEENN
SGLFVTVLEVSSASAAHTGLYTCYYNHTQTEENELEGRHIYIYVPDPDVAFVPLGMT
DYLVIVEDDDSAIIPCRTTDPETPVTLHNSEGVVPASYDSRQGFNGTFTVGPYICEATV
KGKKFQTIPFNVYALKATSELDLEMEALKTVYKSGETIVVTCAVFNNEVVDLQWTY
PGEVKGKGITMLEEIKVPSIKLVYTLTVPEATVKDSGDYECAARQATREVKEMKKVT
ISVHEKGFIEIKPTFSQLEAVNLHEVKHFVVEVRAYPPPRISWLKNNLTLIENLTEITTD
VEKIQEIRYRSKLKLIRAKEEDSGHYTIVAQNEDAVKSYTFELLTQVPSSILDLVDDH
HGSTGGQTVRCTAEGTPLPDIEWMICKDIKKCNNETSWTILANNVSNIITEIHSRDRST
VEGRVTFAKVEETIAVRCLAKNLLGAENRELKLVAPTLRSELTVAFWVLVVVGGVL
ACYSLLVTVAFIIFWVRSKRSRGGHSDYMNMTPRRPGPTRKHYQPYAPPRDFAAYRS
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQAL
PPR
SEQ ID NO: 90: (4A8-PDGFRB-28Z peptide) 
MALPVTALLLPLALLLHAARPEVQLVESGAEVKKPGASVKVSCKVSGYTLTELSMH
WVRQAPGKGLEWMGGFDPEDGETMYAQKFQGRVTMTEDTSTDTAYMELSSLRSE
DTAVYYCATSTAVAGTPDLFDYYYGMDVWGQGTTVTVSSGGGGSGGGGSGGGGS
EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIKDYKD
DDDKLVVTPPGPELVLNVSSTFVLTCSGSAPVVWERMSQEPPQEMAKAQDGTFSSV
LTLTNLTGLDTGEYFCTHNDSRGLETDERKRLYIFVPDPTVGFLPNDAEELFIFLTEIT
EITIPCRVTDPQLVVTLHEKKGDVALPVPYDHQRGFFGIFEDRSYICKTTIGDREVDSD
AYYVYRLQVSSINVSVNAVQTVVRQGENITLMCIVIGNEVVNFEWTYPRKESGRLVE
PVTDFLLDMPYHIRSILHIPSAELEDSGTYTCNVTESVNDHQDEKAINITVVESGYVRL
LGEVGTLQFAELHRSRTLQVVFEAYPPPTVLWFKDNRTLGDSSAGEIALSTRNVSET
RYVSELTLVRVKVAEAGHYTMRAFHEDAEVQLSFQLQINVPVRVLELSESHPDSGEQ
TVRCRGRGMPQPNIIWSACRDLKRCPRELPPTLLGNSSEEESQLETNVTYWEEEQEFE
 
VVSTLRLQHVDRPLSVRCTLRNAVGQDTQEVIVVPHSLPFKVFWVLVVVGGVLACY
SLLVTVAFIIFWVRSKRSRGGHSDYMNMTPRRPGPTRKHYQPYAPPRDFAAYRSRVK
FSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR
SEQ ID NO: 91: (4A8-LAIR1-28Z peptide) 
MALPVTALLLPLALLLHAARPEVQLVESGAEVKKPGASVKVSCKVSGYTLTELSMH
WVRQAPGKGLEWMGGFDPEDGETMYAQKFQGRVTMTEDTSTDTAYMELSSLRSE
DTAVYYCATSTAVAGTPDLFDYYYGMDVWGQGTTVTVSSGGGGSGGGGSGGGGS
EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIKDYKD
DDDKQEEDLPRPSISAEPGTVIPLGSHVTFVCRGPVGVQTFRLERESRSTYNDTEDVS
QASPSESEARFRIDSVSEGNAGPYRCIYYKPPKWSEQSDYLELLVKETSGGPDSPDTE
PGSSAGPTQRPSDNSHNEHAPASQGLKAEHLYFWVLVVVGGVLACYSLLVTVAFIIF
WVRSKRSRGGHSDYMNMTPRRPGPTRKHYQPYAPPRDFAAYRSRVKFSRSADAPA
YQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR
SEQ ID NO: 92: (tLAIR1-28Z peptide) 
MALPVTALLLPLALLLHAARPEVQLVESGAEVKKPGASVKVSCKVSGYTLTELSMH
WVRQAPGKGLEWMGGFDPEDGETMYAQKFQGRVTMTEDTSTDTAYMELSSLRSE
DTAVYYCATSTAVAGTPDLFDYYYGMDVWGQGTTVTVSSGGGGSGGGGSGGGGS
EIVMTQSPLSSPVTLGQPASISCRSSQSLVHSDGNTYLSWLQQRPGQPPRLLIYKISNR
FSGVPDRFSGSGAGTDFTLKISRVEAEDVGVYYCTQATQFPYTFGQGTKVDIKDYKD
DDDKLLVKETSGGPDSPDTEPGSSAGPTQRPSDNSHNEHAPASQGLKAEHLYFWVL
VVVGGVLACYSLLVTVAFIIFWVRSKRSRGGHSDYMNMTPRRPGPTRKHYQPYAPP
RDFAAYRSRVKFSRSADAPAYQQGQNQLYNELNLGRREEYDVLDKRRGRDPEMGG
KPRRKNPQEGLYNELQKDKMAEAYSEIGMKGERRRGKGHDGLYQGLSTATKDTYD
ALHMQALPPR
SEQ ID NO: 93: (CR3014-IgG4mut-28Z peptide) 
MALPVTALLLPLALLLHAARPDIQMTQSPSSLSASVGDRVTITCRASQSISSYLNWYQ
QKPGKAPKLLIYAASSLQSGVPSRFSGSGSGTDFTLTISSLQPEDFATYYCQQSYSTPP
 
TFGQGTKVEIKGGGGSGGGGSGGGGSEVQLVESGGGLVQPGGSLRLSCAASGFTFSD
HYMDWVRQAPGKGLEWVGRTRNKANSYTTEYAASVKGRFTISRDDSKNSLYLQM
NSLKTEDTAVYYCARGISPFYFDYWGQGTLVTVSSDYKDDDDKESKYGPPCPPCPAP
PVAGPSVFLFPPKPKDTLMISRTPEVTCVVVDVSQEDPEVQFNWYVDGVEVHNAKT
KPREEQFQSTYRVVSVLTVLHQDWLNGKEYKCKVSNKGLPSSIEKTISKAKGQPREP
QVYTLPPSQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGKFWVLVVVGGVL
ACYSLLVTVAFIIFWVRSKRSRGGHSDYMNMTPRRPGPTRKHYQPYAPPRDFAAYRS
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQAL
PPR
SEQ ID NO: 94: (C135 CDRH3) ASSSGYLFHSDY
SEQ ID NO: 95: (C135 CDRL3) QQYNSYPWT
SEQ ID NO: 96: (4A8 CDRH1) GYTLTELS
SEQ ID NO: 97: (4A8 CDRH2) FDPEDGET
SEQ ID NO: 98: (4A8 CDRH3) ATSTAVAGTPDLFDYYYGMDV
SEQ ID NO: 99: (4A8 CDRL1) QSLVHSDGNTY
SEQ ID NO: 100: (4A8 CDRL2) KIS
SEQ ID NO: 101: (4A8 CDRL3) TQATQFPYT
SEQ ID NO: 102: (CR3014 CDRH1) FSDHYMDW
SEQ ID NO: 103: (CR3014 CDRH2) VGRTRNKANSYTTEYAASVKGR
SEQ ID NO: 104: (CR3014 CDRH3) CARGISPFYFDYW
SEQ ID NO: 105: (CR3014 CDRL1) CRASQSISSYLNW
SEQ ID NO: 106: (CR3014 CDRL2) YAASSLQSG
SEQ ID NO: 107: (CR3014 CDRL3) CQQSYSTPPTF
SEQ ID NO: 108: (COVID BA.4/5 S Protein) 
MFVFLVLLPLVSSQCVNLTTRTQLPPAYTNSFTRGVYYPDKVFRSSVLHSTQDLFLPF
FSNVTWFHVISGTNGTKRFDNPVLPFNDGVYFASIEKSNIIRGWIFGTTLDSKTQSLLI
VNNATNVVIKVCEFQFCNDPFLDHKNNKSWMESEFRVYSSANNCTFEYVSQPFLMD
LEGKQGNFKNLREFVFKNIDGYFKIYSKHTPIIVREPEDLPQGFSALEPLVDLPIGINIT
RFQTLLALHRSYLTPGDSSSGWTAGAAAYYVGYLQPRTFLLKYNENGTITDAVDCA
LDPLSETKCTLKSFTVEKGIYQTSNFRVQPTESIVRFPNITNLCPFDEVFNATRFASVY
AWNRKRISNCVADYSVLYNLAPFFTFKCYGVSPTKLNDLCFTNVYADSFVIRGDEVR
QIAPGQTGNIADYNYKLPDDFTGCVIAWNSNKLDSKVSGNYNYRYRLFRKSNLKPFE
RDISTEIYQAGNKPCNGVAGVNCYFPLQSYSFRPTYGVGHQPYRVVVLSFELLHAPA
TVCGPKKSTNLVKNKCVNFNFNGLKGTGVLTESNKKFLPFQQFGRDIADTTDAVRD
PQTLEILDITPCSFGGVSVITPGTNTSNQVAVLYQGVNCTEVPVAIHADQLTPTWRVY
STGSNVFQTRAGCLIGAEYVNNSYECDIPIGAGICASYQTQTKSHRRARSVASQSIIAY
TMSLGAENSVAYSNNSIAIPTNFTISVTTEILPVSMTKTSVDCTMYICGDSTECSNLLL
QYGSFCTQLKRALTGIAVEQDKNTQEVFAQVKQIYKTPPIKYFGGFNFSQILPDPSKP
SKRSFIEDLLFNKVTLADAGFIKQYGDCLGDIAARDLICAQKFKGLTVLPPLLTDEMI
AQYTSALLAGTITSGWTFGAGAALQIPFAMQMAYRFNGIGVTQNVLYENQKLIANQ
FNSAIGKIQDSLSSTASALGKLQDVVNHNAQALNTLVKQLSSKFGAISSVLNDIFSRL
DKVEAEVQIDRLITGRLQSLQTYVTQQLIRAAEIRASANLAATKMSECVLGQSKRVD
FCGKGYHLMSFPQSAPHGVVFLHVTYVPAQEKNFTTAPAICHDGKAHFPREGVFVS
NGTHWFVTQRNFYEPQIITTDNTFVSGNCDVVIGIVNNTVYDPLQPELDSFKEELDKY
FKNHTSPDVDLGDISGINASVVNIQKEIDRLNEVAKNLNESLIDLQELGKYEQYIKWP
WYIWLGFIAGLIAIVMVTIMLCCMTSCCSCLKGCCSCGSCCKFDEDDSEPVLKGVKL
HYT
SEQ ID NO: 109: (4-1BB costimulatory signaling region)
   KRGRKKLLYIFKQPFMRPVQTTQEEDGCSCRFPEEEEGGCEL
SEQ ID NO: 110: (2B4 costimulatory signaling region) 
WRRKRKEKQSETSPKEFLTIYEDVKDLKTRRNHEQEQTFPGGGSTIYSMIQSQSSAPT
SQEPAYTLYSLIQPSRKSGSRKRNHSPSFNSTIYEVIGKSQPKAQNPARLSRKELENFD
VYS
SEQ ID NO: 111: (OX40 costimulatory signaling region) 
AGGGACCAG AGGCTGCCCC CCGATGCCCA CAAGCCCCCT GGGGGAGGCA
GTTTCCGGAC CCCCATCCAA GAGGAGCAGG CCGACGCCCA CTCCACCCTG
GCCAAGATC
SEQ ID NO: 112: (CD19 isoform 1)
SEQ ID NO: 113: (CD19 isoform 2) 
MPPPRLLFFLLFLTPMEVRPEEPLVVKVEEGDNAVLQCLKGTSDGPTQQLTWSRESP
LKPFLKLSLGLPGLGIHMRPLAIWLFIFNVSQQMGGFYLCQPGPPSEKAWQPGWTVN
VEGSGELFRWNVSDLGGLGCGLKNRSSEGPSSPSGKLMSPKLYVWAKDRPEIWEGE
PPCLPPRDSLNQSLSQDLTMAPGSTLWLSCGVPPDSVSRGPLSWTHVHPKGPKSLLSL
ELKDDRPARDMWVMETGLLLPRATAQDAGKYYCHRGNLTMSFHLEITARPVLWH
WLLRTGGWKVSAVTLAYLIFCLCSLVGILHLQRALVLRRKRKRMTDPTRRFFKVTPP
PGSGPQNQYGNVLSLPTPTSGLGRAQRWAAGLGGTAPSYGNPSSDVQADGALGSRS
PPGVGPEEEEGEGYEEPDSEEDSEFYENDSNLGQDQLSQDGSGYENPEDEPLGPEDE
DSFSNAESYENEDEELTQPVARTMDFLSPHGSAWDPSREATSLAGSQSYEDMRGILY
AAPQLRSIRGQPGPNHEEDADSYENMDNPDGPDPAWGGGGRMGTWSTR
SEQ ID NO: 114: (ICOS costimulatory signaling region) ACAAAAAAGA AGTATTCATC CAGTGTGCAC GACCCTAACG GTGAATACAT GTTCATGAGA GCAGTGAACA CAGCCAAAAA ATCCAGACTC ACAGATGTGA CCCTA
SEQ ID NO: 115: (DAP10 costimulatory signaling region) RPRRSPAQDGKVYINMPGRG
SEQ ID NO: 116: (DAP 12 costimulatory signaling region)
   ESPYQELQGQRSDVYSDLNTQ

## Claims

1. A polypeptide comprising:
(a) an antibody or a fragment thereof that specifically binds a tumor antigen,
(b) a hinge region comprising 120 or more amino acid residues,
(c) a transmembrane domain,
(d) one or more co-stimulatory domains, and
(e) an intracellular signaling domain,
wherein the hinge region comprises:

QEEDLPRPSISAEPGTVIPLGSHVTFVCRGPVGVQTFRLERESRSTYNDTEDVSQA
SPSESEARFRIDSVSEGNAGPYRCIYYKPPKWSEQSDYLELLVKETSGGPDSPDTEPGSSA
GPTQRPSDNSHNEHAPASQGLKAEHLY (SEQ ID NO: 37).

2. The polypeptide of claim 1, wherein the transmembrane domain comprises a CD28 transmembrane domain comprising FWVLVVVGGVLACYSLLVTVAFIIFWV (SEQ ID NO: 12) or an equivalent thereof.

3. The polypeptide of claim 1 or 2, wherein the co-stimulatory domain comprises RSKRSRGGHSDYMNMTPRRPGPTRKHYQPY APPRDF AA YRS (SEQ ID NO: 24) or an equivalent thereof, and/or wherein the intracellular signaling domain comprises a CD3ζ intracellular signaling domain comprising RVKFSRSADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQALPPR (SEQ ID NO: 14) or an equivalent thereof.

4. The polypeptide of any one of claims 1-3, wherein the polypeptide is recombinant or isolated.

5. A polynucleotide encoding the polypeptide of any one of claims 1-4, or a polynucleotide complementary thereto.

6. A vector comprising the polynucleotide of claim 5.

7. A cell comprising one or more of: the polypeptide of any one of claims 1-4, the polynucleotide of claim 5, or the vector of claim 6.

8. The cell of claim 7, wherein the cell is a eukaryotic cell, and the eukaryotic cell is an immune cell, optionally wherein the immune cell is selected from a T cell, a B cell, a NK cell, a NKT cell, a dendritic cell, a myeloid cell, a monocyte, or a macrophage.

9. An immune cell comprising one or more of: the polypeptide of any one of claims 1-4, the polynucleotide of claim 5, or the vector of claim 6, optionally wherein the immune cell is selected from a T cell, a B cell, a NK cell, a NKT cell, a dendritic cell, a myeloid cell, a monocyte, or a macrophage.

10. A T cell comprising:
(i) granzyme B, or a polynucleotide encoding the granzyme B, or both;
(ii) perforin, or a polynucleotide encoding the perforin, or both; and
(iii) one or more of: the polypeptide of any one of claims 1-4, the polynucleotide of claim 5, or the vector of claim 6, optionally further comprising:
(iv) CD69, or a polynucleotide encoding the CD69, or both; or
(v) IFN-y, or a polynucleotide encoding the IFN-y, or both; or
both (iv) and (v).

11. A histocompatible T cell comprising one or more of: the polypeptide of any one of claims 1-4, the polynucleotide of claim 5, or the vector of claim 6, optionally wherein the histocompatible T cell lacks a functional endogenous T cell receptor or a human leukocyte antigen (HLA) molecule or both.

12. A cell population comprising the cell of any one of claims 7-11.

13. A composition comprising a carrier and one or more of: the polypeptide of any one of claims 1-4, the polynucleotide of claim 5, the vector of claim 6, the cell of any one of claims 7-11, or the cell population of claim 12, optionally wherein the carrier is a pharmaceutically acceptable carrier.

14. A method for producing the cell of any one of claims 7-11, comprising introducing the polynucleotide of claim 5, the vector of claim 6, or both to a cell, optionally further comprising isolating the cell introduced with the polynucleotide or the vector or both.

15. The cell of any one of claims 7-11, the cell population of claim 12, or the composition of claim 13 for use in a method of treating a subject having or suspected of having cancer.

## Patentansprüche

1. Polypeptid, das Folgendes umfasst:
(a) einen Antikörper oder ein Fragment davon, der/das spezifisch ein Tumorantigen bindet,
(b) eine Gelenkregion, die 120 oder mehr Aminosäurereste umfasst,
(c) eine Transmembrandomäne,
(d) eine oder mehrere co-stimulatorische Domänen und
(e) eine intrazelluläre Signaldomäne,
wobei die Gelenkregion Folgendes umfasst:

QEEDLPRPSISAEPGTVIPLGSHVTFVCRGPVGVQTFRLERESRSTYNDTEDVSQA
SPSESEARFRIDSVSEGNAGPYRCIYYKPPKWSEQSDYLELLVKETSGGPDSPDTEPGSSA
GPTQRPSDNSHNEHAPASQGLKAEHLY (SEQ ID NO: 37).

2. Polypeptid nach Anspruch 1, wobei die Transmembrandomäne eine CD28-Transmembrandomäne umfasst, die FWVLVVVGGVLACYSLLVTVAFIIFWV (SEQ ID NO: 12) oder ein Äquivalent davon umfasst.

3. Polypeptid nach Anspruch 1 oder 2, wobei die co-stimulatorische Domäne RSKRSRGGHSDYMNMTPRRPGPTREHYOPYAPPRDFAAYRS (SEQ ID NO: 24) oder ein Äquivalent davon umfasst und/oder wobei die intrazelluläre Signaldomäne eine intrazelluläre CD3ζ-Signaldomäne umfasst, die
RVKFSRSADAPAYQQGQNQLYNELNLGRREEYDVLDKRRGRDPEMGGKPRRKENPQEG LYNELQKDKMAEAYSEIGMKGERRRGKGHDGLYQGLSTATKDTYDALHMQALPPR (SEQ ID NO: 14) oder ein Äquivalent davon umfasst.

4. Polypeptid nach einem der Ansprüche 1 bis 3, wobei das Polypeptid rekombinant oder isoliert ist.

5. Polynukleotid, das für ein Polypeptid nach einem der Ansprüche 1 bis 4 kodiert, ein oder Polynukleotid, das komplementär dazu ist.

6. Vektor, der ein Polynukleotid nach Anspruch 5 umfasst.

7. Zelle, die eines oder mehrere der Folgenden umfasst: ein Polypeptid nach einem der Ansprüche 1 bis 4, ein Polynukleotid nach Anspruch 5 oder einen Vektor nach Anspruch 6.

8. Zelle nach Anspruch 7, wobei die Zelle eine eukaryotische Zelle ist und die eukaryotische Zelle eine Immunzelle ist, wobei die Immunzelle gegebenenfalls aus einer T-Zelle, einer B-Zelle, einer NK-Zelle, einer NKT-Zelle, einer dendritischen Zelle, einer myeloischen Zelle, einem Monozyten oder einem Makrophagen ausgewählt ist.

9. Immunzelle, die eines oder mehrere der Folgenden umfasst: ein Polypeptid nach einem der Ansprüche 1 bis 4, ein Polynukleotid nach Anspruch 5 oder einen Vektor nach Anspruch 6, wobei die Immunzelle gegebenenfalls aus einer T-Zelle, einer B-Zelle, einer NK-Zelle, einer NKT-Zelle, einer dendritischen Zelle, einer myeloischen Zelle, einem Monozyten oder einem Makrophagen ausgewählt ist.

10. T-Zelle,die Folgendes umfasst:
(i) Granzym B oder ein Polynukleotid, das für das Granzym B kodiert, oder beide,
(ii) Perforin oder ein Polynukleotid, das für das Perforin kodiert, oder beide und
(iii) eines oder mehrere der Folgenden: ein Polypeptid nach einem der Ansprüche 1 bis 4, ein Polynukleotid nach Anspruch 5 oder einen Vektor nach Anspruch 6;
wobei die Zelle gegebenenfalls weiters Folgendes umfasst:
(iv) CD69 oder ein Polynukleotid, das für das CD69 kodiert, oder beides oder
(v) IFN-γ oder ein Polynukleotid, das für das IFN-γ kodiert, oder beides oder sowohl (iv) als auch (v).

11. Histokompatible T-Zelle, die eines oder mehrere der Folgenden umfasst: ein Polypeptid nach einem der Ansprüche 1 bis 4, ein Polynukleotid nach Anspruch 5 oder einen Vektor nach Anspruch 6, wobei der histokompatiblen T-Zelle gegebenenfalls ein funktioneller endogener T-Zell-Rezeptor oder ein menschliches Leukozytenantigen(HLA)-Molekül oder beides fehlt.

12. Zellpopulation, die eine Zelle nach einem der Ansprüche 7 bis 11 umfasst.

13. Zusammensetzung, die einen Träger und eines oder mehrere der Folgenden umfasst: ein Polypeptid nach einem der Ansprüche 1 bis 4, ein Polynukleotid nach Anspruch 5, einen Vektor nach Anspruch 6, eine Zelle nach einem der Ansprüche 7 bis 11 oder eine Zellpopulation nach Anspruch 12, wobei der Träger gegebenenfalls ein pharmazeutisch annehmbarer Träger ist.

14. Verfahren zur Herstellung einer Zelle nach einem der Ansprüche 7 bis 11, welches das Einführen eines Polynukleotids nach Anspruch 5, eines Vektors nach Anspruch 6 oder beides in eine Zelle umfasst, wobei das Verfahren gegebenenfalls weiters das Isolieren der Zelle, in die das Polynukleotid oder der Vektor oder beide eingeführt wurden, umfasst.

15. Zelle nach einem der Ansprüche 7 bis 11, Zellpopulation nach Anspruch 12 oder Zusammensetzung nach Anspruch 13 zur Verwendung in einem Verfahren zur Behandlung eines Individuums, das an Krebs leidet oder bei dem vermutet wird, dass es an Krebs leidet.

## Revendications

1. Polypeptide, comprenant :
(a) un anticorps ou un fragment de celui-ci qui se lie spécifiquement à un antigène tumoral,
(b) une région charnière comprenant 120 résidus d'acides aminés ou plus,
(c) un domaine transmembranaire,
(d) un ou plusieurs domaines de co-stimulation, et
(e) un domaine de signalisation intracellulaire, dans lequel la région charnière comprend :

QEEDLPRPSISAEPGTVIPLGSHVTFVCRGPVGVQTFRLERESRSTYNDTEDVS
QASPSESEARFRIDSVSEGNAGPYRCIYKPPKWSEQSDYLELLVKETSGGPDSPDTEPG
SSAGPTQRPSDNSHNEHAPASQGLKAEHLY (SEQ ID NO : 37).

2. Polypeptide selon la revendication 1, dans lequel le domaine transmembranaire comprend un domaine transmembranaire CD28 comprenant FWVLVVVVGGVLACYSLLVTVAFIIFWV (SEQ ID NO : 12) ou un équivalent de celui-ci.

3. Polypeptide selon la revendication 1 ou 2, dans lequel le domaine de co-stimulation comprend RSKRSRGGHSDYMNMTPRRPGPTRKHYQPYAPPRDFAAYRS (SEQ ID NO : 24) ou un équivalent de celle-ci, et/ou dans lequel le domaine de signalisation intracellulaire comprend un domaine de signalisation intracellulaire CD3ζ comprenant RVKFSRSADAPAYQQGQNQLYNELNLGRREEYDVLDKRRGRDPEMGGKPRRKNPQEGLY NELQKDKMAEAYSEIGMKGERRRGKGHDGLYQGLSTATKDTYDALHMQALPPR (SEQ ID NO : 14) ou un équivalent de celle-ci.

4. Polypeptide selon l'une quelconque des revendications 1 à 3, dans lequel le polypeptide est recombinant ou isolé.

5. Polynucléotide codant pour le polypeptide selon l'une quelconque des revendications 1 à 4, ou un polynucléotide complémentaire de celui-ci.

6. Vecteur comprenant l'acide nucléique selon la revendication 5.

7. Cellule comprenant un ou plusieurs parmi : le polypeptide selon l'une quelconque des revendications 1 à 4, le polynucléotide selon la revendication 5, ou le vecteur selon la revendication 6.

8. Cellule selon la revendication 7, dans laquelle la cellule est une cellule eucaryote, et la cellule eucaryote est une cellule immunitaire, facultativement dans laquelle la cellule immunitaire est choisie parmi une cellule T, une cellule B, une cellule NK, une cellule NKT, une cellule dendritique, une cellule myéloïde, un monocyte ou un macrophage.

9. Cellule immunitaire comprenant un ou plusieurs parmi : le polypeptide selon l'une quelconque des revendications 1 à 4, le polynucléotide selon la revendication 5 ou le vecteur selon la revendication 6, facultativement dans laquelle la cellule immunitaire est choisie parmi une cellule T, une cellule B, une cellule NK, une cellule NKT, une cellule dendritique, une cellule myéloïde, un monocyte ou un macrophage.

10. Cellule T, comprenant :
(i) le granzyme B, ou un polynucléotide codant pour le granzyme B, ou les deux ;
(ii) la perforine, ou un polynucléotide codant pour la perforine, ou les deux ; et
(iii) un ou plusieurs parmi : le polypeptide selon l'une quelconque des revendications 1 à 4, le polynucléotide selon la revendication 5 ou le vecteur selon la revendication 6, comprenant en outre facultativement :
(iv) CD69, ou un polynucléotide codant pour le CD69, ou les deux ; ou
(v) IFN-*y*, ou un polynucléotide codant pour l'IFN-*y*, ou les deux ; ou à la fois (iv) et (v).

11. Cellule T histocompatible comprenant un ou plusieurs parmi : le polypeptide selon l'une quelconque des revendications 1 à 4, le polynucléotide selon la revendication 5 ou le vecteur selon la revendication 6, facultativement dans laquelle la cellule T histocompatible est dépourvue d'un récepteur de cellule T endogène fonctionnel ou d'une molécule d'antigène de leucocyte humain (HLA) ou des deux.

12. Population cellulaire comprenant la cellule selon l'une quelconque des revendications 7 à 11.

13. Composition comprenant un support et un ou plusieurs parmi : le polypeptide selon l'une quelconque des revendications 1 à 4, le polynucléotide selon la revendication 5, le vecteur selon la revendication 6, la cellule selon l'une quelconque des revendications 7 à 11, ou la population cellulaire selon la revendication 12, facultativement dans laquelle le support est un support pharmaceutiquement acceptable.

14. Procédé de production de la cellule selon l'une quelconque des revendications 7 à 11, comprenant l'introduction du polynucléotide de la revendication 5, du vecteur de la revendication 6, ou des deux dans une cellule, comprenant en outre facultativement l'isolation de la cellule dans laquelle a été introduit le polynucléotide ou le vecteur, ou les deux.

15. Cellule selon l'une quelconque des revendications 7 à 11, population cellulaire selon la revendication 12 ou composition selon la revendication 13 pour utilisation dans un procédé de traitement d'un sujet souffrant ou suspecté de souffrir d'un cancer.
